# EUROPÄISCHE PATENTSCHRIFT

(11) **EP 2 167 499 B1**
(45) Veröffentlichungstag und Bekanntmachung des Hinweises auf die Patenterteilung: **14.12.2011**
(21) Anmeldenummer: 08773422.4
(22) Anmeldetag: 13.06.2008
(51) Int. Cl.: C07D 413/14, A61K 31/4439, A61P 7/02

(54) **SUBSTITUIERTE OXAZOLIDINONE UND IHRE VERWENDUNG**
SUBSTITUTED OXAZOLIDINONES AND USE THEREOF
OXAZOLIDINONES SUBSTITUÉES ET LEUR UTILISATION

(30) Priorität: 20.06.2007 DE 102007028406
(43) Veröffentlichungstag der Anmeldung: 31.03.2010
(73) Patentinhaber: Bayer Pharma Aktiengesellschaft, 13353 Berlin (DE)
(72) Erfinder: ALLERHEILIGEN, Swen, 45259 Essen (DE); BAUSER, Marcus, 10439 Berlin (DE); SCHIROK, Hartmut, 40764 Langenfeld (DE); HÄRTER, Michael, D-51375 Leverkusen (DE); SIEGEL, Stephan, 10779 Berlin (DE); RESTER, Ulrich, 42115 Wuppertal (DE); GERDES, Christoph, 51063 Köln (DE); HEITMEIER, Stefan, 42489 Wülfrath (DE); VON DEGENFELD, Georges, 51373 Leverkusen (DE); BUCHMÜLLER, Anja, 45259 Essen (DE); DITTRICH-WENGENROTH, Elke, 42113 Wuppertal (DE); SAATMANN, Uwe, 42117 Wuppertal (DE); STRASSBURGER, Julia, 42115 Wuppertal (DE); GNOTH, Mark, Jean, 40822 Mettmann (DE); LANG, Dieter, 42553 Velbert (DE)
(86) Internationale Anmeldenummer: PCT/EP2008/004746
(87) Internationale Veröffentlichungsnummer: WO 2008/155069

(56) Entgegenhaltungen:
- WO-A-2007/039134
- US-A1- 2003 153 610
- US-A1- 2006 069 260

## Beschreibung

Die Erfindung betrifft neue substituierte Oxazolidinone, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von thromboembolischen Erkrankungen.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden bzw. minimiert werden. Die Blutstillung nach Gefäßverletzung erfolgt im wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und dem extrinsischen System, die in einem abschließenden gemeinsamen Reaktionsweg münden. Hierbei kommen den Faktoren Xa und IIa (Thrombin) Schlüsselrollen zu.

Faktor Xa bündelt die Signale der beiden Gerinnungswege, da er sowohl über Faktor VIIa/Tissue Factor (extrinsischer Weg) wie auch den Tenase Komplex (intrisischer Weg) durch Umsetzung von Faktor X entsteht. Die aktivierte Serinprotease Xa spaltet Prothrombin zu Thrombin.

Thrombin überträgt über einen Strauss von Umsetzungen die Signale aus der Kaskade auf den Gerinnungsstatus des Blutes. Thrombin spaltet direkt Fibrinogen zu Fibrin. Es aktiviert den zur Stabilisierung des Fibringerinnsels notwendigen Faktor XIII zu Faktor XIIIa. Darüber hinaus ist Thrombin ein potenter Auslöser der Thrombozytenaggregation (über PAR-1 Aktivierung), die ebenfalls einen erheblichen Beitrag bei der Hämostase leistet. Mittels Aktivierung von TAFI (Thrombin-aktivierbarer Fibrinolyse Inhibitor) zu TAFIa hemmt Thrombin im Komplex mit Thrombomodulin die Auflösung des Gerinnsels. Aktivierung der Faktoren V und VIII führt zur Potenzierung der Thrombinproduktion und damit wiederum zur Verstärkung der Gerinnungsreaktion, das im Komplex mit Thrombomodulin hergestellte aktivierte Protein C wirkt dieser verstärkten Thrombinproduktion entgegen und verhindert so das Überschießen der Hämostase (Thrombose).

Neben frei im Blut befindlichem Faktor X und Thrombin sind auch gebundene Formen bekannt. Während der Entstehung eines Fibringerinnsels werden Thrombin und Prothrombinase (Faktor Xa im Komplex) an das Fibringerüst gebunden. Diese Enzymmoleküle besitzen weiterhin Aktivität und können durch das körpereigene Anti-Thrombin III nicht inhibiert werden. Gerinnsel besitzen also auf diese Weise ein generelles koagulatives Potenzial.

Im Verlauf vieler Herzkreislauf- und Stoffwechselerkrankungen kommt es infolge systemischer Faktoren, wie z.B. Hyperlipidämie, Diabetes oder Rauchen, infolge von Blutflußveränderungen mit Stase, wie z.B. beim Vorhofflimmern, oder infolge pathologischer Gefäßwandveränderungen, z.B. endothelialer Dysfunktionen oder Atherosklerose, zu einer erhöhten Neigung von Gerinnung- und Thrombozytenaktivierung. Diese unerwünschte und überschießende Hämostase kann durch Bildung fibrin- und plättchenreicher Thromben zu thromboembolischen Erkrankungen und thrombotischen Komplikationen mit lebensbedrohlichen Zuständen führen.

Die Hämostase unterliegt einem komplexen Regulationsmechanismus. Eine unkontrollierte Aktivierung des Gerinnungssystems oder eine defekte Hemmung der Aktivierungsprozesse kann die Bildung von lokalen Thrombosen oder Embolien in Gefäßen (Arterien, Venen, Lymphgefäßen) oder Herzhöhlen bewirken. Dies kann zu schwerwiegenden thrombotischen oder thromboembolischen Erkrankungen führen. Darüber hinaus kann eine systemische Hyperkoagulabilität zu einer Verbrauchskoagulopathie im Rahmen einer disseminierten intravasalen Gerinnung führen. Thromboembolische Komplikationen treten ferner auf bei mikroangiopathischen hämolytischen Anämien, extrakorporalen Blutkreisläufen, wie Hämodialyse, sowie Herzklappenprothesen und Stents.

Thromboembolische Erkrankungen sind die häufigste Ursache von Morbidität und Mortalität in den meisten industrialisierten Ländern [Heart Disease: A Textbook of Cardiovascular Medicine, Eugene Braunwald, 5. Auflage, 1997, W.B. Saunders Company, Philadelphia].

Die aus dem Stand der Technik bekannten Antikoagulantien, d.h. Stoffe zur Hemmung oder Verhinderung der Blutgerinnung, weisen verschiedene, oftmals gravierende Nachteile auf. Eine effiziente Behandlungsmethode bzw. Prophylaxe von thrombotischen/thromboembolischen Erkrankungen erweist sich in der Praxis deshalb als sehr schwierig und unbefriedigend.

In der Therapie und Prophylaxe von thromboembolischen Erkrankungen findet zum einen Heparin Verwendung, das parenteral oder subkutan appliziert wird. Aufgrund günstigerer pharmakokinetischer Eigenschaften wird zwar heutzutage zunehmend niedermolekulares Heparin bevorzugt; allerdings können auch hierdurch die im folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen [Pschyrembel, Klinisches Wörterbuch, 257. Auflage, 1994, Walter de Gruyter Verlag, Seite 610, Stichwort "Heparin"; Römpp Lexikon Chemie, Version 1.5, 1998, Georg Thieme Verlag Stuttgart, Stichwort "Heparin"]. Niedermolekulare Heparine besitzen zwar eine geringere Wahrscheinlichkeit zur Ausbildung einer Heparin-induzierten Thrombocytopenie, sind aber auch nur subkutan applizierbar. Dies gilt auch für Fondaparinux, einem synthetisch hergestellten, selektiven Faktor Xa Inhibitor mit einer langen Halbwertszeit.

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig [J. Hirsh, J. Dalen, D.R. Anderson et al., "Oral anticoagulants: Mechanism of action, clinical effectiveness, and optimal therapeutic range" Chest 2001, 119, 8S-21S; J. Ansell, J. Hirsh, J. Dalen et al., "Managing oral anticoagulant therapy" Chest 2001, 119, 22S-38S; P.S. Wells, A.M. Holbrook, N.R. Crowther et al., "Interactions of warfarin with drugs and food" Ann. Intern. Med. 1994, 121, 676-683]. Darüber hinaus sind weitere Nebenwirkungen wie gastrointestinale Störungen, Haarausfall und Hautnekrosen beschrieben.

Daneben werden in geringerem Umfang Thrombininhibitoren eingesetzt. Hirudin ist ein Protein, das sehr potent Thrombin hemmt. In recombinanter Form wird es intravenös als Reserve-Antikoagulanz appliziert. Mit Bivalirudin steht ein 20 Aminosäure-Stück aus Hirudin zur Verfügung, das eine sehr kurze Halbwertszeit hat und auch nicht oral verfügbar ist. Das gleiche gilt für den direkten nicht-peptidischen niedermolekularen Thrombininhibitor Argatroban [J.H.Sohn, et al. Appl.Microbiol.Biotechnol.2001,57,606-613; T.Galdwell Clin. Ther. 2002, 24, 38-58; G.Escolar, Drugs of Today 2006, 42, 223 ].

Ein weiterer Therapieansatz sieht die alleinige Inhibition von Faktor Xa vor. [J. Hauptmann, J. Stürzebecher, Thrombosis Research 1999, 93, 203; S.A.V. Raghavan, M. Dikshit, "Recent advances in the status and targets of antithrombotic agents" Drugs Fut. 2002, 27, 669-683; H.A. Wieland, V. Laux, D. Kozian, M. Lorenz, "Approaches in anticoagulation: Rationales for target positioning" Curr. Opin. Investig. Drugs 2003, 4, 264-271; U.J. Ries, W. Wienen, "Serine proteases as targets for antithrombotic therapy" Drugs Fut. 2003, 28, 355-370; L.-A. Linkins, J.I. Weitz, "New anticoagulant therapy" Annu. Rev. Med. 2005, 56, 63-77; A. Casimiro-Garcia et al., "Progress in the discovery of Factor Xa inhibitors" Expert Opin. Ther. Patents 2006, 15, 119-145].

Dabei ist gezeigt worden, dass verschiedene, sowohl peptidische wie nicht-peptidische Verbindungen in Tiermodellen als Faktor Xa-Inhibitoren wirksam sind. Eine große Anzahl von direkten Faktor Xa-Inhibitoren ist bislang bekannt [J.M. Walenga, W.P. Jeske, D. Hoppensteadt, J. Fareed, "Factor Xa Inhibitors: Today and beyond" Curr. Opin. Investig. Drugs 2003, 4, 272-281; J. Ruef, H.A. Katus, "New antithrombotic drugs on the horizon" Expert Opin. Investig. Drugs 2003, 12, 781-797; M.L. Quan, J.M. Smallheer, "The race to an orally active Factor Xa inhibitor: Recent advances" Curr. Opin. Drug Discovery & Development 2004, 7, 460-469]. Oxazolidinone als nicht-peptidische, niedermolekulare Faktor Xa-Inhibitoren sind beschrieben in WO 01/47919 (US2003153610) und WO 2007/039134).

In jüngster Zeit sind Ansätze beschrieben worden, in denen niedermolekulare Thrombin- und Faktor Xa Inhibitoren in verschiedenen Mischungsverhältnissen in-vitro und in-vivo getestet wurden. Dabei zeigte sich ein starkes synergistisches Potential. Mit Tanogitran ist eine niedermolekulare Substanz beschrieben worden, die sowohl Thrombin als auch Faktor Xa hemmt, aber eine starke Präferenz auf Thrombinhemmung besitzt. Diese in der Entwicklung befindliche Substanz ist nicht oral bioverfügbar.

Bei antithrombotischen Arzneimitteln ist die therapeutische Breite von zentraler Bedeutung: Der Abstand zwischen der therapeutisch wirksamen Dosis zur Gerinnungshemmung und der Dosis, bei der Blutungen auftreten können, sollte möglichst groß sein, so dass eine maximale therapeutische Wirksamkeit bei minimalem Risikoprofil erreicht wird.

Wie die Experimente mit Mischungen von niedermolekularen Thrombin- und Faktor Xa Inhibitoren zeigen, würden Verbindungen, die sowohl Thrombin als auch Faktor Xa hemmen, durch ihren dualen Charakter einen besonders starken Synergismus besitzen und dadurch besonders effektiv die Entstehung von Thromben bekämpfen können. Damit hemmen die Verbindungen die beiden entscheidenden Enzyme der Koagulationskaskade, ohne die einzelnen Enzyme vollständig blocken zu müssen. Der übriggebliebene Rest an Faktor Xa und Thrombin führt zu einer intakten Blutstillung und somit zu einer besonders vorteilhaften therapeutischen Breite. In einem Arteriovenösem-Shunt Modell im Kaninchen konnte gezeigt werden, dass Co-Administration von nur schwach antithrombotisch wirksamen Dosierungen des selektiven FXa Inhibitors PD0313052 und des selektiven Thrombin Inhibitors Argatroban zu einem starken, überadditiven antithrombotischen Effekt führen. Zudem wurde bei der Kombination der Einzeldosen mit dem maximalen synergistischen Effekt keine Verstärkung von Blutungen beobachtet. Diese Beobachtungen lassen den Schluss zu, dass die simultane Inhibition von Thrombin und FXa die therapeutische Breite in Bezug auf Abstand der antithrombotischen Wirkung zum Blutungsrisiko vergrößert (Journal of Thrombosis and Haemostasis, 4: 834-841).

Dieser Synergismus zeigt sich besonders deutlich bei Betrachtung der Entwicklung der Prothrombinzeit in Abhängigkeit von der Substanzkonzentration im direkten Vergleich zu reinen Faktor Xa- und Thrombin-Inhibitoren. Diese starke Wirkung auf die beiden entscheidenden Enzyme der Gerinnungskaskade wird als besonders vorteilhaft angesehen, wenn ein hohes Risiko für Thrombenbildungen besteht bzw. die Bildung von Thromben zu einer fatalen Erkrankung führen kann. Beides trifft beispielsweise auf die atherothrombotischen Erkrankungen aus dem Bereich des Akuten Koronarsyndroms bzw. die Situation nach einem akuten Myokardinfarkt zu.

Desweiteren würden Verbindungen, die sowohl Thrombin als auch Faktor Xa hemmen, im Gegensatz zu Heparinen, Hirudin und Vitamin K-Antagonisten auch gegen Fibringerinnsel-gebundene Koagulationsfaktoren aktiv. Die Begrenzung des thrombotischen Potentials eines schon vorhandenen Gerinnsels ist ein kritischer Punkt in der Prävention eines arteriellen Verschlusses. Dieses geschieht besonders effektiv durch Hemmung sowohl der vorhandenen Thrombin-Aktivität als auch der Bildung von neuem Thrombin im Gerinnsel. Während ein reiner Thrombin-Inhibitor die lawinenartige Thrombinproduktion durch den Gerinnsel-gebundenen Faktor Xa-enthaltenen Prothrombinase-Komplex nicht verhindern kann und der inhibitorische Effekt damit bei einer stark stimulierten Koagulation von der großen Menge produziertem Thrombin überkompensiert werden kann, können reine Faktor Xa-Inhibitoren die schon vorhandene Thrombin-Aktivität nicht hemmen. Da diese auch durch physiologische Mechanismen nicht mehr hemmbar ist, stellt dies Gerinnselgebundene Thrombin ein besonders großes Risiko dar. Im Gegensatz dazu sind duale Verbindungen, d. h. Verbindungen, die sowohl Thrombin als auch Faktor Xa hemmen, in der Lage, sowohl die Thrombinproduktion als auch die Thrombinaktivität auf Gerinnseln zu hemmen und damit auch potentielles Gerinnselwachstum zu verhindern.

US 2006069260 beschreibt die Synthese von N-Aryl-pyridonen.

Eine Aufgabe der vorliegenden Erfindung ist daher die Bereitstellung von dualen Verbindungen, d. h. Verbindungen, die sowohl Thrombin als auch Faktor Xa hemmen und durch Hemmung der Thrombinproduktion und -aktivität auf Gerinnseln deren potentielles Wachstum verhindern, mit einem breiten therapeutischen Fenster, zur Bekämpfung von Erkrankungen, insbesondere von thromboembolischen Erkrankungen, bei Menschen und Tieren.

Gegenstand der Erfindung sind Verbindungen der Formel 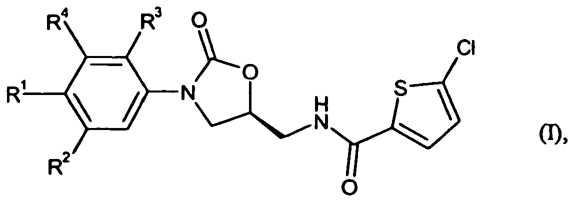 in welcher
- R¹: für eine Gruppe der Formel 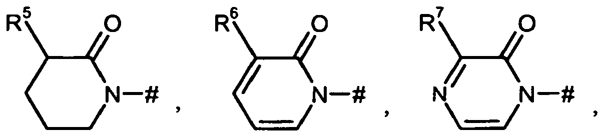 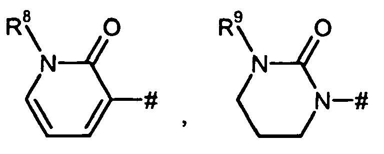 steht,
wobei
# die Anknüpfstelle an den Phenylring ist,
R⁵ für Wasserstoff oder C₁-C₃-Alkyl steht,
worin Alkyl substituiert sein kann mit einem Substituenten Hydroxy,
R⁶ für Wasserstoff, Trifluormethyl, Trifluormethoxy, Difluormethoxy, C₁-C₃-Alkyl oder C₁-C₄-Alkoxy steht,
worin Alkoxy substituiert sein kann mit einem Substituenten, wobei der Substituent ausgewählt wird aus der Gruppe bestehend aus Halogen, Hydroxy, Trifluormethyl, Aminocarbonyl, C₁-C₃-Alkoxy, C₁-C₃-Alkylcarbonyl, C₁-C₄-Alkylaminocarbonyl und Morpholinocarbonyl,
R⁷ für Wasserstoff, C₁-C₃-Alkyl oder C₁-C₄-Alkoxy steht,
worin C₂-C₃-Alkyl und C₂-C₄-Alkoxy substituiert sein können mit einem Substituenten Hydroxy,
R⁸ für Wasserstoff oder C₁-C₃-Alkyl steht,
worin C₂-C₃-Alkyl substituiert sein kann mit einem Substituenten Hydroxy,
R⁹ für Wasserstoff oder C₁-C₃-Alkyl steht,
worin C₂-C₃-Alkyl substituiert sein kann mit einem Substituenten Hydroxy,
- R²: für Chlor, Trifluormethyl, Trifluormethoxy, Difluormethoxy, Methyl, Ethyl, n-Propyl, iso-Propyl, Methoxy, Ethoxy, Methoxymethyl, Ethoxymethyl oder Cyclopropyl steht,
- R³: für Wasserstoff, C₁-C₃-Alkoxy oder C₁-C₃-Alkylamino steht,
- R⁴: für Wasserstoff oder Methyl steht,
und ihre Salze, ihre Solvate und die Solvate ihrer Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäwe, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl per se und "Alk" und "Alkyl" in Alkoxy, Alkylamino, Alkylcarbonyl und Alkylaminocarbonyl steht für einen linearen Alkylrest mit in der Regel 1 bis 3, bevorzugt 1 oder 2 Kohlenstoffatomen, beispielhaft und vorzugsweise für Methyl, Ethyl und n-Propyl.
Alkoxy steht beispielhaft und vorzugsweise für Methoxy, Ethoxy und n-Propoxy.
Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, iso-Propylamino, *N,N*-Dimethylamino, *N,N*-Diethylamino, *N*-Ethyl-*N*-methylamino, *N-*Methyl-*N*-n-propylamino und *N*-iso-Propyl-*N*-n-propylamino. C₁-C₃-Akylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.
Alkylcarbonyl steht beispielhaft und vorzugsweise für Methylcarbonyl, Ethylcarbonyl, n-Propylcarbonyl und iso-Propylcarbonyl.
Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, iso-Propylaminocarbonyl, tert-Butylaminocarbonyl, *N,N*-Dimethylaminocarbonyl, *N,N*-Diethylaminocarbonyl, *N*-Ethyl-*N*-methylaminocarbonyl, *N-*Methyl-*N*-n-propylaminocarbonyl, *N*-iso-Propyl-*N*-n-propylaminocarbonyl und *N*-tert-Butyl-*N* methylaminocarbonyl. C₁-C₄-Alkylaminocarbonyl steht beispielsweise für einen Monoalkylaminocarbonylrest mit 1 bis 4 Kohlenstoffatomen oder für einen Dialkylaminocarbonylrest mit jeweils 1 bis 4 Kohlenstoffatomen pro Alkylsubstituent.

In den Formeln der Gruppe, die für R¹ stehen kann, steht der Endpunkt der Linie, neben der jeweils ein # steht, nicht für ein Kohlenstoffatom beziehungsweise eine CH₂-Gruppe sondern ist Bestandteil der Bindung zu dem Atom, an das R¹ gebunden ist.

Ein Symbol * an einem Kohlenstoffatom bedeutet, dass die Verbindung hinsichtlich der Konfiguration an diesem Kohlenstoffatom in enantiomerenreiner Form vorliegt, worunter im Rahmen der vorliegenden Erfindung ein Enantiomerenüberschuss (enantiomeric excess) von mehr als 90% verstanden wird (> 90% ee).

Bevorzugt sind Verbindungen der Formel (I), in welcher
- R¹: für eine Gruppe der Formel 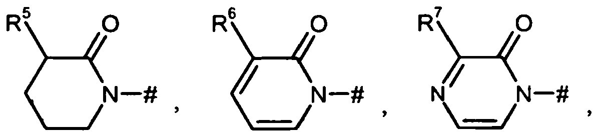 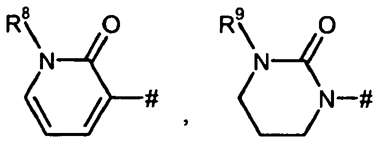 steht,
wobei
# die Anknüpfstelle an den Phenylring ist,
R⁵ für Wasserstoff oder C₁-C₃-Alkyl steht,
worin Alkyl substituiert sein kann mit einem Substituenten Hydroxy,
R⁶ für Wasserstoff, Trifluormethyl, Trifluormethoxy, C₁-C₃-Alkyl oder C₁-C₄-Alkoxy steht,
worin Alkoxy substituiert sein kann mit einem Substituenten, wobei der Substituent ausgewählt wird aus der Gruppe bestehend aus Halogen, Hydroxy, Trifluormethyl, Aminocarbonyl, C₁-C₃-Alkoxy, C₁-C₃-Akylcarbonyl, C₁-C₄-Alkylaminocarbonyl und Morpholinocarbonyl,
R⁷ für C₁-C₄-Alkoxy steht,
R⁸ für C₁-C₃-Alkyl steht,
R⁹ für Wasserstoff oder C₁-C₃-Alkyl steht,
worin C₂-C₃-Alkyl substituiert sein kann mit einem Substituenten Hydroxy,
- R²: für Chlor, Trifluormethyl, Methyl, Ethyl, n-Propyl, iso-Propyl, Methoxy, Ethoxy, Methoxymethyl, Ethoxymethyl oder Cyclopropyl steht,
- R³: für Wasserstoff, C₁-C₃-Alkoxy oder C₁-C₃-Alkylamino steht,
- R⁴: für Wasserstoff oder Methyl steht,
und ihre Salze, ihre Solvate und die Solvate ihrer Salze.

Bevorzugt sind auch Verbindungen der Formel (I), in welcher
- R¹: für eine Gruppe der Formel 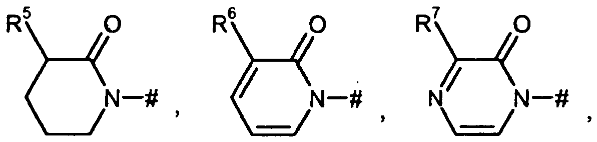 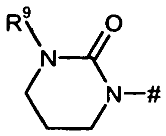 steht,
wobei
# die Anknüpfstelle an den Phenylring ist,
R⁵ für Wasserstoff oder C₁-C₃-Alkyl steht,
worin Alkyl substituiert sein kann mit einem Substituenten Hydroxy,
R⁶ für Wasserstoff, Trifluormethyl, Trifluormethoxy, C₁-C₃-Alkyl oder C₁-C₄-Alkoxy steht,
worin Alkoxy substituiert sein kann mit einem Substituenten, wobei der Substituent ausgewählt wird aus der Gruppe bestehend aus Halogen, Hydroxy, Trifluormethyl, Aminocarbonyl, C₁-C₃-Alkoxy, C₁-C₃-Alkylcarbonyl, C₁-C₄-Alkylaminocarbonyl und Morpholinocarbonyl,
k⁷ für C₁-C₄-Alkoxy steht,
R⁹ für Wasserstoff oder C₁-C₃-Alkyl steht,
worin C₂-C₃-Alkyl substituiert sein kann mit einem Substituenten Hydroxy,
- R²: für Chlor, Trifluormethyl, Methyl, Ethyl, n-Propyl, iso-Propyl, Methoxy, Ethoxy, Methoxymethyl, Ethoxymethyl oder Cyclopropyl steht,
- R³: für Wasserstoff, C₁-C₃-Alkoxy oder C₁-C₃-Alkylamino steht,
- R⁴: für Wasserstoff oder Methyl steht,
und ihre Salze, ihre Solvate und die Solvate ihrer Salze.

Bevorzugt sind auch Verbindungen der Formel (I), in welcher
- R¹: für eine Gruppe der Formel 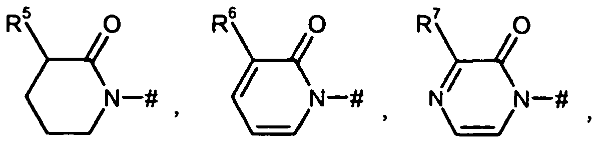 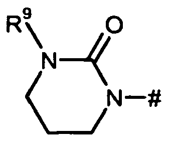 steht,
wobei
# die Anknüpfstelle an den Phenylring ist,
R⁵ für Wasserstoff oder C₁-C₃-Alkyl steht,
worin Alkyl substituiert sein kann mit einem Substituenten Hydroxy,
R⁶ für Wasserstoff, C₁-C₃-Alkyl oder C₁-C₄-Alkoxy steht,
worin Alkoxy substituiert sein kann mit einem Substituenten, wobei der Substituent ausgewählt wird aus der Gruppe bestehend aus Hydroxy, Aminocarbonyl, C₁-C₃-Alkoxy, C₁-C₃-Alkylcarbonyl und C₁-C₄-Alkylaminocarbonyl,
R⁷ für C₁-C₄-Alkoxy steht,
R⁹ für Wasserstoff oder C₁-C₃-Alkyl steht,
worin C₂-C₃-Alkyl substituiert sein kann mit einem Substituenten Hydroxy,
- R²: für Chlor, Methyl, Ethyl, n-Propyl, iso-Propyl, Methoxy, Ethoxy, Methoxymethyl, Ethoxymethyl oder Cyclopropyl steht,
- R³: für Wasserstoff steht,
- R⁴: für Wasserstoff oder Methyl steht,
und ihre Salze, ihre Solvate und die Solvate ihrer Salze.

Bevorzugt sind auch Verbindungen der Formel (I), in welcher
- R¹: für eine Gruppe der Formel 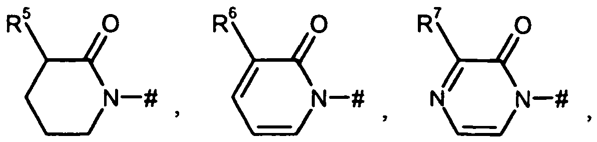 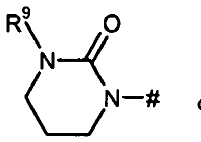 steht,
wobei
# die Anknüpfstelle an den Phenylring ist,
R⁵ für Wasserstoff steht,
R⁶ für Wasserstoff oder C₁-C₄-Alkoxy steht,
worin Alkoxy substituiert sein kann mit einem Substituenten Hydroxy,
R⁷ für Ethoxy steht,
R⁹ für Wasserstoff, Methyl oder 2-Hydroxyeth-1-yl steht,
- R²: für Methyl, iso-Propyl, Methoxy, Ethoxy, Methoxymethyl oder Cyclopropyl steht,
- R³: für Wasserstoff steht,
- R⁴: für Wasserstoff oder Methyl steht,
und ihre Salze, ihre Solvate und die Solvate ihrer Salze.

Bevorzugt sind auch Verbindungen der Formel (I), in welcher
- R¹: für eine Gruppe der Formel 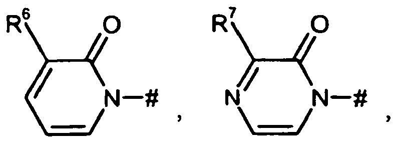 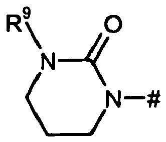 steht,
wobei
# die Anknüpfstelle an den Phenylring ist,
R⁶ für Wasserstoff oder C₁-C₄-Alkoxy steht,
worin Alkoxy substituiert sein kann mit einem Substituenten Hydroxy,
R⁷ für Ethoxy steht,
R⁹ für Wasserstoff, Methyl oder 2-Hydroxyeth-1-yl steht.

Bevorzugt sind auch Verbindungen der Formel (I), in welcher
- R¹: für eine Gruppe der Formel 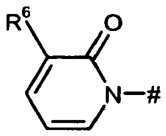 steht,
wobei
# die Anknüpfstelle an den Phenylring ist,
R⁶ für Wasserstoff oder C₁-C₄-Alkoxy steht,
worin Alkoxy substituiert sein kann mit einem Substituenten Hydroxy.

Bevorzugt sind auch Verbindungen der Formel (I), in welcher R² für Chlor, Trifluormethyl, Methyl, Ethyl, n-Propyl, iso-Propyl, Methoxy, Ethoxy, Methoxymethyl, Ethoxymethyl oder Cyclopropyl steht.

Bevorzugt sind auch Verbindungen der Formel (I), in welcher R² für Methyl, iso-Propyl, Methoxy, Ethoxy, Methoxymethyl oder Cyclopropyl steht.

Bevorzugt sind auch Verbindungen der Formel (I), in welcher R² für Chlor, Methyl oder Methoxy steht.

Bevorzugt sind auch Verbindungen der Formel (I), in welcher R² für Methyl steht.

Bevorzugt sind auch Verbindungen der Formel (I), in welcher R³ für Wasserstoff steht.

Bevorzugt sind auch Verbindungen der Formel (I), in welcher R⁴ für Wasserstoff steht.

Bevorzugt sind auch Verbindungen der Formel (I), in welcher R⁶ für Wasserstoff, Trifluormethyl, Trifluormethoxy, C₁-C₃-Alkyl oder C₁-C₄-Alkoxy steht, worin Alkoxy substituiert sein kann mit einem Substituenten, wobei der Substituent ausgewählt wird aus der Gruppe bestehend aus Halogen, Hydroxy, Trifluormethyl, Aminocarbonyl, C₁-C₃-Alkoxy, C₁-C₃-Alkylcarbonyl, C₁-C₄-Alkylaminocarbonyl und Morpholinocarbonyl.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (I), oder ihrer Salze, ihrer Solvate oder der Solvate ihrer Salze, wobei
[A] die Verbindung der Formel 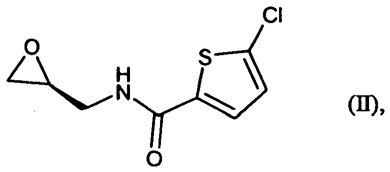 in der ersten Stufe mit Verbindungen der Formel 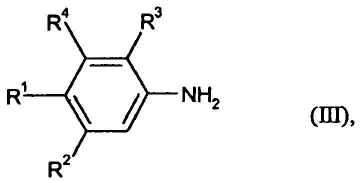 in welcher R¹, R², R³ und R⁴ die oben angegebene Bedeutung haben,
   zu Verbindungen der Formel 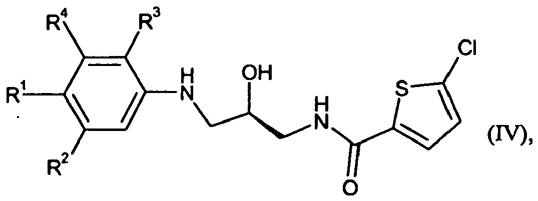 in welcher R¹, R², R³ und R⁴ die oben angegebene Bedeutung haben,
   umgesetzt werden,
   und in der zweiten Stufe in Anwesenheit von Phosgen oder Phosgenäquivalenten wie z.B. Carbonyldiimidazol (CDI) zu den Verbindungen der Formel (I) cyclisiert werden
   oder
[B] die Verbindungen der Formel 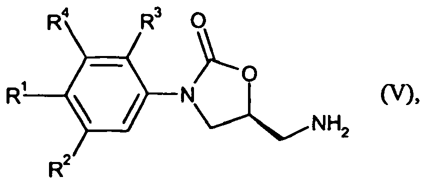 in welcher R¹, R², R³ und R⁴ die oben angegebene Bedeutung haben,
   mit Verbindungen der Formel 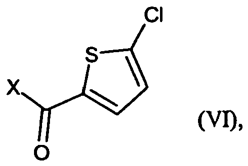 in welcher
   - X: für Halogen, bevorzugt Brom oder Chlor, oder Hydroxy steht,
   umgesetzt werden.

Sind Hydroxygruppen während des Verfahrens geschützt, z. B. durch eine Silylschutzgruppe, so werden diese nach Beendigung des Verfahrens [A] oder [B] nach dem Fachmann bekannten Methoden abgespalten, z. B. durch Umsetzung mit Tetrabutylammoniumfluorid in einem Lösungsmittel, wie z. B. Tetrahydrofuran, oder durch Umsetzung mit Chlorwasserstoff in Methanol.

Die freie Base der Salze kann zum Beispiel durch Chromatographie an einer Reversed Phase Säule mit einem Acetonitril-Wasser-Gradienten unter Zusatz einer Base erhalten werden, insbesondere durch Verwendung einer RP18 Phenomenex Luna C18(2) Säule und Diethylamin als Base, oder durch Lösen der Salze in einem organischen Lösungsmittel und Ausschütteln mit wässrigen Lösungen von basischen Salzen wie Natriumhydrogencarbonat.

Weiterer Gegenstand der Erfindung ist ein Verfahren zur Herstellung der Verbindungen der Formel (I) oder ihrer Solvate, bei dem Salze der Verbindungen oder Solvate der Salze der Verbindungen durch Chromatographie unter Zusatz einer Base in die Verbindungen überführt werden.

Die Umsetzung der ersten Stufe nach Verfahren [A] erfolgt im Allgemeinen in inerten Lösungsmitteln, in Gegenwart einer Lewissäure, bevorzugt in einem Temperaturbereich von Raumtemperatur bis zum Rückfluss des Lösungsmittels bei Normaldruck.

Inerte Lösungsmittel sind beispielsweise polare, aprotische Lösungsmittel wie beispielsweise Acetonitril, Butyronitril, Dichlormethan oder Chloroform, bevorzugt ist Acetonitril.

Lewis-Säuren sind beispielsweise Magnesiumperchlorat, Ytterbium(III)trifluormethansulfonat, Lithiumbromid, Magnesiumtriflat oder Aluminiumtrichlorid, bevorzugt ist Magnesiumperchlorat.

Die Umsetzung der zweiten Stufe nach Verfahren [A] erfolgt im Allgemeinen in inerten Lösungsmitteln, in Gegenwart einer Base, bevorzugt in einem Temperaturbereich von Raumtemperatur bis zum Rückfluss des Lösungsmittels bei Normaldruck.

Inerte Lösungsmittel sind beispielsweise polare, aprotische Lösungsmittel wie beispielsweise Acetonitril oder Butyronitril.

Basen sind beispielsweise starke, tertiäre Aminbasen wie beispielsweise 4-*N,N*-Dimethylarminopyridin.

Bevorzugt ist die Reaktion mit *N,N*'-Carbonyldiimidazol als Kohlensäure-Äquivalent unter Zusatz von 4-*N,N*-Dimethylaminopyridin als Base.

Wenn bei Verfahren [B] X für Halogen steht, erfolgt die Umsetzung im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Tetrahydrofuran, Methylenchlorid, Pyridin, Dioxan oder Dimethylformamid, bevorzugt ist Tetrahydrofuran oder Methylenchlorid.

Basen sind beispielsweise Triethylamin, Diisopropylethylamin oder *N*-Methylmorpholin, bevorzugt ist Diisopropylethylamin.

Wenn bei Verfahren [B] X für Hydroxy steht, erfolgt die Umsetzung im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Als Dehydratisierungsreagenzien eignen sich hierbei beispielsweise Carbodiimide wie z.B. *N,N'-*Diethyl-, *N,N*,'-Dipropyl-, *N,N*'-Diisopropyl-, *N,N*'-Dicyclohexylcarbodiimid, *N*-(3-Dimethylaminoisopropyl)-*N*'-ethylcarbodiimid-hydrochlorid (EDC), *N*-Cyclohexylcarbodiimid-*N*'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert-*Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)-phosphoniumhexafluorophosphat, oder *O*-(Benzotriazol-1-yl)-*N,N,N',N'*-tetra-methyluroniumhexafluorophosphat (HBTU), 2-(2-Oxo-1-(2H)-pyridyl)-1,1,3,3-tetramethyluroniumtetrafluoroborat (TPTU) oder *O*-(7-Azabenzotriazol-1-yl)-*N,N,N',N'*-tetramethyl-uroniumhexafluorophosphat (HATU), oder 1-Hydroxybenzotriazol (HOBt), oder Benzotriazol-1-yloxytris(dimethylamino)-phosphoniumhexafluorophosphat (BOP), oder *N*-Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Vorzugsweise wird die Kondensation mit HATU oder mit EDC in Gegenwart von HOBt durchgeführt.

Die Verbindungen der Formeln (II) und (VI) sind bekannt oder lassen sich nach bekannten Verfahren aus den entsprechenden Ausgangsverbindungen synthetisieren.

Die Verbindungen der Formel (III) sind bekannt oder können hergestellt werden, indem in Verbindungen der Formel 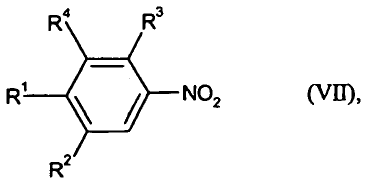 in welcher R¹, R², R³ und R⁴ die oben angegebene Bedeutung haben,
die Nitrogruppe reduziert wird.

Die Umsetzung erfolgt im Allgemeinen mit einem Reduktionsmittel in inerten Lösungsmitteln, bevorzugt in einem Temperaturbereich von Raumtemperatur bis zum Rückfluss der Lösungsmittel bei Normaldruck bis 3 bar.

Reduktionsmittel sind beispielsweise Palladium auf Aktivkohle und Wasserstoff, Zinndichlorid, Titantrichlorid, Hydrazinhydrat und Raney-Nickel, oder Ammoniumformiat und Palladium auf Aktivkohle in einem Gemisch aus Ethanol und Essigsäureethylester, bevorzugt ist Palladium auf Aktivkohle und Wasserstoff oder Zinndichlorid.

Inerte Lösungsmittel sind beispielsweise Ether wie Diethylether, Methyl-*tert*.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder *tert*.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, als Lösungsmittel sind bevorzugt Methanol, Ethanol, iso-Propanol oder im Falle von Zinndichlorid in Dimethylformamid.

Die Verbindungen der Formel (VII) sind bekannt, lassen sich nach bekannten Verfahren aus den entsprechenden Ausgangsverbindungen synthetisieren oder können analog den im Beispielteil beschriebenen Verfahren hergestellt werden.

Die Verbindungen der Formel (V) sind bekannt oder können hergestellt werden, indem in Verbindungen der Formel 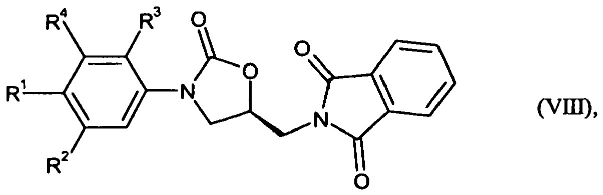 in welcher R¹, R², R³ und R⁴ die oben angegebene Bedeutung haben,
die Phthalimid-Schutzgruppe gespalten wird.

Die Umsetzung erfolgt im Allgemeinen mit einer wässrigen Methylamin-Lösung oder einer Hydrazinhydrat-Lösung in Ethanol, bevorzugt mit einer wässrigen Methylamin-Lösung unter Rückfluss der Lösungsmittel bei Normaldruck.

Die Verbindungen der Formel (VIII) sind bekannt, lassen sich wie unter Verfahren [A] beschrieben herstellen oder lassen sich nach bekannten Verfahren aus den entsprechenden Ausgangsverbindungen synthetisieren.

Die Herstellung der erfindungsgemäßen Verbindungen kann durch das folgende Syntheseschema veranschaulicht werden:

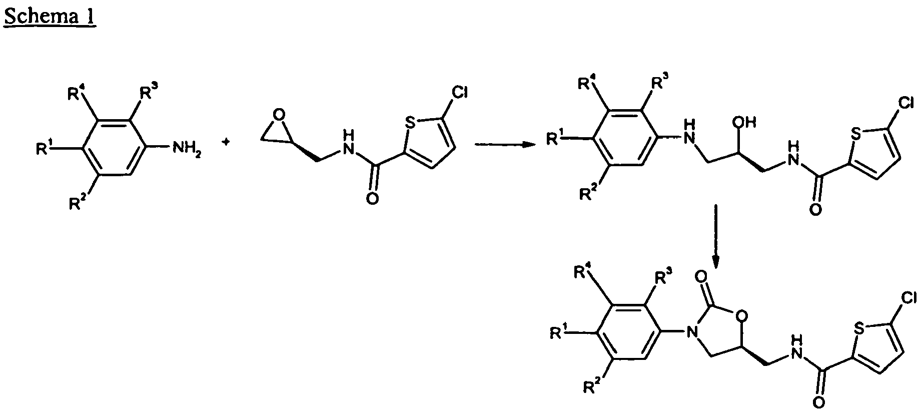

Die erfindungsgemäßen Verbindungen zeigen ein nicht vorhersehbares, wertvolles pharmakologisches Wirkspektrum.

Sie eignen sich daher zur Verwendung als Arzneimittel zur Behandlung und/oder Prophylaxe von Krankheiten bei Menschen und Tieren.

Bei den erfindungsgemäßen Verbindungen handelt es sich um duale Inhibitoren der Blutgerinnungsfaktoren Xa und Thrombin (Faktor IIa), die insbesondere als Antikoagulantien wirken. Die Verbindungen hemmen sowohl Thrombin als auch Faktor Xa, verhindern durch Hemmung der Thrombinproduktion und -aktivität auf Gerinnseln deren potentielles Wachstum und weisen ein breites therapeutisches Fenster auf.

Weiterer Gegenstand der vorliegenden Erfindung ist der Einsatz der erfindungsgemäßen Verbindungen zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag.

Die die erfindungsgemäßen Verbindungen eignen sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit künstlichen Herzklappen.

Thromboembolische Komplikationen treten ferner auf bei mikroangiopathischen hämolytischen Anämien, extrakorporalen Blutkreisläufen, wie Hämodialyse, sowie Herzklappenprothesen.

Außerdem kommen die erfindungsgemäßen Verbindungen auch für die Prophylaxe und/oder Behandlung von atherosklerotischen Gefäßerkrankungen und entzündlichen Erkrankungen wie rheumatische Erkrankungen des Bewegungsapparats in Betracht, darüber hinaus ebenso für die Prophylaxe und/oder Behandlung der Alzheimer'schen Erkrankung. Außerdem können die erfindungsgemäßen Verbindungen zur Inhibition des Tumorwachstums und der Metastasenbildung, bei Mikroangiopathien, altersbedingter Makula-Degeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Außerdem kommen die erfindungsgemäßen Verbindungen auch für die Prophylaxe und/oder Behandlung von pulmonaler Hypertonie in Betracht.

Der Begriff "pulmonale Hypertonie" umfasst bestimmte Formen der pulmonalen Hypertonie, wie sie z.B. von der Weltgesundheitsorganisation (WHO) festgelegt worden sind (Clinical Classification of Pulmonary Hypertension, Venedig 2003). Als Beispiele seien genannt, die pulmonale arterielle Hypertonie, die pulmonale Hypertonie bei Erkrankungen des linken Herzens, die pulmonale Hypertonie bei Lungenerkrankung und/oder Hypoxie und die Pulmonale Hypertonie aufgrund chronischer Thrombembolien (CTEPH).

Die "pulmonale arterielle Hypertonie" beinhaltet die Idiopathische Pulmonale Arterielle Hypertonie (IPAH, früher auch als primäre pulmonale Hypertonie bezeichnet), die Familiär bedingte Pulmonale Arterielle Hypertonie (FPAH) und die Assoziierte Pulmonal-Arterielle Hypertonie (APAH), die assoziiert ist mit Kollagenosen, kongenitalen systemisch-pulmonalen Shuntvitien, portaler Hypertension, HIV-Infektionen, der Einnahme bestimmter Drogen und Medikamente, mit anderen Erkrankungen (Schilddrüsenerkrankungen, Glykogenspeicherkrankheiten, Morbus Gaucher, hereditäre Teleangiektasie, Hämoglobinopathien, myeloproliferative Erkrankungen, Splenektomie), mit Erkrankungen mit einer signifikanten venösen/kapillären Beteiligung wie der pulmonal-venookklusiven Erkrankung und der pulmonal-kapillären Hämangiomatose, sowie die persistierende pulmonale Hypertonie der Neugeborenen.

Die pulmonale Hypertonie bei Erkrankungen des linken Herzens beinhaltet die Erkrankung des linken Vorhofes oder Ventrikels und Mitral- oder Aortenklappenfehler.

Die pulmonale Hypertonie bei Lungenerkrankung und/oder Hypoxie beinhaltet chronisch obstruktive Lungenerkrankungen, interstitielle Lungenerkrankung, Schlafapnoe-Syndrom, alveolärer Hypoventilation, chronische Höhenkrankheit und anlagebedingte Fehlbildungen.

Die Pulmonale Hypertonie aufgrund chronischer Thrombembolien (CTEPH) beinhaltet den thrombembolischen Verschluss proximaler Lungenarterien, den thrombembolischen Verschluss distaler Lungenarterien und nicht-thrombotische Lungenembolien (Tumor, Parasiten, Fremdkörper).

Weiterer Gegenstand der vorliegenden Erfindung ist die Verwendung der erfindungsgemäßen Verbindungen zur Herstellung von Arzneimitteln zur Behandlung und/oder Prophylaxe von pulmonaler Hypertonie bei Sarkoidose, Histiozytose X und Lymphangiomatosis.

Außerdem kommen die erfindungsgemäßen Substanzen auch zur Behandlung von pulmonalen und hepatischen Fibrosen in Betracht.

Außerdem kommen die erfindungsgemäßen Verbindungen auch für die Behandlung und/oder Prophylaxe von Sepsis (oder Septikämie), Systemic Inflammatory Syndrome (SIRS), septische Organdysfunktion, septisches Organversagen und Muliorganversagen, Acute Respiratory Distress Syndrome (ARDS), Acute Lung Injury (ALI), Septischer Schock, DIC ("Disseminated Intravascular coagulation", oder "Verbrauchskoagulopathie") und/oder des septischen Organversagens in Betracht.

"Sepsis" wird definiert als das Vorliegen einer Infektion und eines "systemic inflammatory response syndrome" (nachfolgend mit "SIRS" bezeichnet). SIRS tritt im Rahmen von Infekten, aber auch von anderen Zuständen wie Verletzungen, Verbrennungen, Schock, Operationen, Ischämie, Pankreatitis, Reanimation oder Tumoren auf. Nach der Definition des ACCP/SCCM Consensus Conference Committee von 1992 (Crit Care Med 1992;20:864-874) werden die zur Diagnose "SIRS" erforderlichen Symptome zur Diagnose und Meßparameter beschrieben (u.a. veränderte Körpertemperatur, erhöhte Herzfrequenz, Atemschwierigkeiten und verändertes Blutbild). In der späteren (2001) SCCM/ESICM/ACCP/ATS/SIS International Sepsis Definitions Conference wurden die Kriterien im Wesentlichen beibehalten, in Details jedoch verfeinert (Levy et al., Crit Care Med 2003;31:1250-1256).

Im Verlauf einer Sepsis kann es zur generalisierten Aktivierung des Gerinnungssystems kommen ("Disseminated Intravascular coagulation", oder "Verbrauchskoagulopathie", nachfolgend als "DIC" bezeichnet) mit Mikrothrombosierung in verschiedenen Organen und sekundärer Blutungskomplikationen. Außerdem kann es es zur endothelialen Schädigung mit Erhöhung der Gefäßpermeabilität und Austritt von Flüssigkeit und Proteinen in den Extravasalraum kommen. Im weiteren Verlauf kann ein Versagen eines Organs (z.B. Nierenversagen, Leberversagen, Atemversagen, zentralnervöse Defizite und Herz-/Kreislaufversagen) oder zum Multiorganversagen kommen. "Septischer Schock" bezeichnet das Auftreten einer behandlungspflichtigen Blutdruckernedrigung, die eine weitere Organschädigung begünstigt und mit einer Verschlechterung der Prognose einhergeht.

Krankheitserreger können Bakterien (gram-negativ und gram-positiv), Pilze, Viren und/oder Eukaryonten sein. Eintrittspforte bzw. Primärinfektion können z.B. Pneumonie, Harnwegsinfekt, Peritonitis sein. Die Infektion kann, muß aber nicht zwingend, mit einer Bakteriämie einhergehen.

DIC und/oder SIRS können im Rahmen einer Sepsis, aber auch infolge von Operationen, Tumorerkrankungen, Verbrennungen oder anderen Verletzungen auftreten. Bei der DIC kommt es an der Oberfäche von geschädigten Endothelzellen, Fremdkörperoberflächen oder veretztem extravaskulärem Gewebe zur massiven Aktivierung des Gerinnungssystems. Als Folge kommt es zur Gerinnung in kleinen Gefäßen verschiedener Organe mit Hypoxie und anschließender Organdysfunktion. Sekundär kommt es zum Verbrauch von Gerinnungsfaktoren (z.B. Faktor X, Prothrombin und Fibrinogen) und Plättchen, wodurch die Gerinnungsfähigkeit des Blutes herabgesetzt wird und schwere Blutungen auftreten können.

Die Therapie der Sepsis besteht einerseits in der konsequenten Beseitigung der infektiösen Ursache, z.B. durch operative Herdsanierung und Antibiose. Andererseits besteht sie in der temporären intensivmedizinischen Unterstützung der beeinträchtigten Organsysteme. Therapien der verschiedenen Stadien dieser Erkrankung sind z.B. in folgender Publikation beschrieben (Dellinger et al., Crit Care Med 2004;32:858-873). Für die DIC existieren keine erwiesenermaßen effektive Therapien.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Antibiotische Therapie
   Verschiedene Antibiotika oder antifungale Medikamenten-Kombinationen kommen in Frage, entweder als kalkulierte Therapie (vor Vorliegen des mikrobiellen Befundes) oder als spezifische Therapie.
- Flüssigkeitstherapie
   z.B. Kristalloide oder kolloidale Flüssigkeiten.
- Vasopressoren
   z.B. Norepinephrine, Dopamine oder Vasopressin
- Inotrope Therapie
   z.B. Dobutamin
- Kortikosteroide
   z.B. Hydrokortison, oder Fludrokortison
- rekombinantes humanes aktivierte Protein C
   Xigris
- Blutprodukte
   z.B. Erythrozytenkonzentrate, Thrombozytenkonzentrate, Erythropietin oder Fresh Frozen Plasma
- Maschinelle Beatmung bei sepsis-induziertem Acute Lung Injury (ALI) bzw. Acute Respiratory Distress Syndrome (ARDS)
   z.B. Permissive Hyperkapnie, niedrigere Tidalvolumina
- Sedierung, Analgesierung und neuromuskuläre Blockade
   Sedierung: z.B. Diazepam, Lorazepam, Midazolam oder Propofol. Opioide: z.B. Fentanyl, Hydromorphon, Morphin, Meperidin oder Remifentanil. NSAIDs: z.B. Ketorolac, Ibuprofen oder Acetaminophen. Neuromuskuläre Blockade: z.B. Pancuronium
- Glukose-Kontrolle
   z.B. Insulin, Glukose
- Nierenersatzverfahren
   z.B. kontinuierliche veno-venöse-Hämofiltration oder intermittierende Hämodialyse. Dopamin niedrig-dosiert zur renalen Protektion.
- Antikoagulantien
   z.B. zur Thromboseprophylaxe oder bei Nierenersatzverfahren, z.B. unfraktionierte Heparine, low molecular weight Heparine, Heparinoide, Hirudin, Bivalirudin oder Argatroban.
- Bikarbonat-Therapie
- Streßulkusprophylaxe
   z.B. H2-Rezeptorinhibitoren, Antazida

Die erfindungsgemäßen Verbindungen können darüber hinaus auch zur Verhinderung von Koagulation *ex vivo* eingesetzt werden, z.B. zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor Xa und/oder Faktor IIa enthalten.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer antikoagulatorisch wirksamen Menge der erfindungsgemäßen Verbindung.

Weiterer Gegenstand der vorliegenden Erfindung ist ein Verfahren zur Verhinderung der Blutkoagulation *in vitro,* insbesondere bei Blutkonserven oder biologischen Proben, die Faktor Xa und/oder Faktor IIa enthalten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren wie beispielsweise Lovastatin (Mevacor; US 4,231,938), Simvastatin (Zocor; US 4,444,784), Pravastatin (Pravachol; US 4,346,227), Fluvastatin (Lescol; US 5,354,772) und Atorvastatin (Lipitor; US 5,273,995);
- Koronartherapeutika/Vasodilatatoren, insbesondere ACE-(Angiotensin-Converting-Enzyme)-Inhibitoren wie beispielsweise Captopril, Lisinopril, Enalapril, Ramipril, Cilazapril, Benazepril, Fosinopril, Quinapril und Perindopril, oder AII-(Angiotensin II)-Rezeptor-Antagonisten wie beispielsweise Embusartan (US 5,863,930), Losartan, Valsartan, Irbesartan, Candesartan, Eprosartan und Temisarta, oder β-Adrenozeptor-Antagonisten wie beispielsweise Carvedilol, Alprenolol, Bisoprolol, Acebutolol, Atenolol, Betaxolol, Carteolol, Metoprolol, Nadolol, Penbutolol, Pindolol, Propanolol und Timolol, oder alpha-1-Adrenozeptor-Antagonisten wie beispielsweise Prazosin, Bunazosin, Doxazosin und Terazosin, oder Diuretika wie beispielsweise Hydrochlorothiazid, Furosemid, Bumetanid, Piretanid, Torasemid, Amilorid und Dihydralazin, oder Calciumkanal-Blocker wie beispielsweise Verapamil und Diltiazem, oder Dihydropyridin-Derivate wie beispielsweise Nifedipin (Adalat) und Nitrendipin (Bayotensin), oder Nitropräparate wie beispielsweise Isosorbid-5-mononitrat, Isosorbid-dinitrat und Glyceroltrinitrat, oder Substanzen, die eine Erhöhung von cyclischem Guanosinmonophosphat (cGMP) bewirken, wie beispielsweise Stimulatoren der löslichen Guanylatcyclase (WO 98/16223, WO 98/16507, WO 98/23619, WO 00/06567, WO 00/06568, WO 00/06569, WO 00/21954, WO 00/66582, WO 01/17998, WO 01/19776, WO 01/19355, WO 01/19780, WO 01/19778, WO 07/045366, WO 07/045367, WO 07/045369, WO 07/045370, WO 07/045433);
- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren) wie beispielsweise Gewebsplasminogen-Aktivator (t-PA), Streptokinase, Reteplase und Urokinase;
- antikoagulatorisch wirksame Substanzen (Antikoagulantien) wie beispielsweise Heparin (UFH), niedermolekulare Heparine (NMH) wie beispielsweise Tinzaparin, Certoparin, Parnaparin, Nadroparin, Ardeparin, Enoxaparin, Reviparin, Dalteparin, Danaparoid,
   **AVE 5026** (Sanofi-Aventis, Company Presentation 2008, February 12 ),
   **M118** (Momenta Pharmaceuticals Inc, *Press Release* 2008, February 14),
   **ORG42675** (Organon International Inc, Company World Wide Website 2007, April),
      und direkte Thrombin Inhibitoren (DTI) wie beispielsweise
   **Exanta (Ximelagatran)** 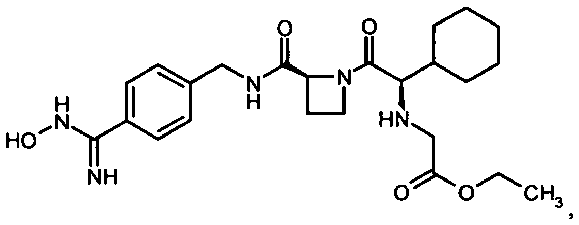
   **Rendix (Dabigatran)** 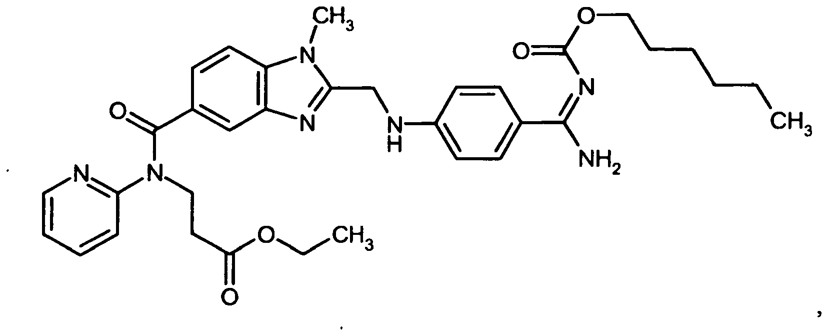
   **AZD-0837** [AstraZeneca Annual Report **2006**, 19. März 2007] 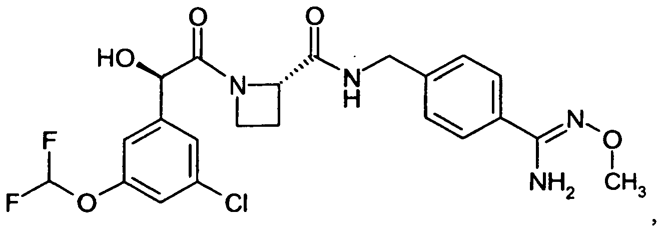
   **SSR-182289A** [J. Lorrain et al. Journal of Pharmacology and Experimental Therapeutics 2003, 304, 567-574; J-M Altenburger et al. Bioorg.Med.Chem. 2004, 12, 1713-1730] 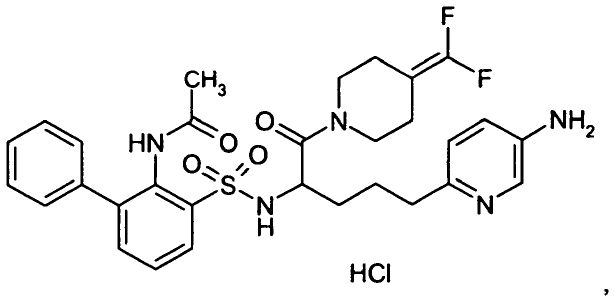
   **TGN-167** [S. Combe et al. Blood 2005, 106, abstract 1863 (ASH 2005)],
   ***N*-[(Benzyloxy)carbonyl]-*L*-phenylalanyl-*N*-[(1*S*)-1-(dihydroxyboryl)-4-methoxybutyl]-*D*-prolinamid** [WO 2005/084685] 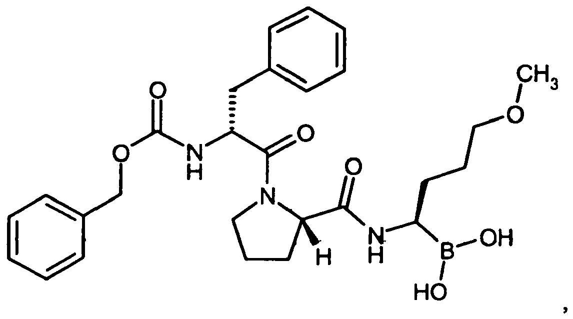
   **Sofigatran** [WHO Drug Information 2007, 21, 77] 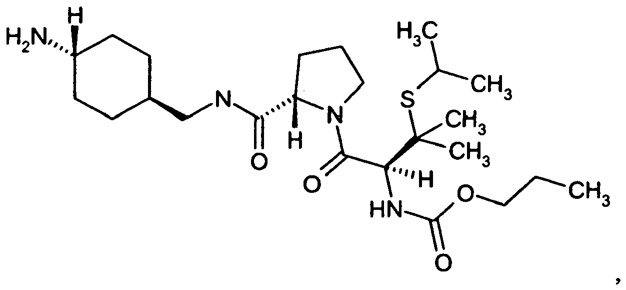
   **MCC-977** [Mitsubishi Pharma website pipeline **2006,** 25. Juli 2006],
   **MPC-0920** [Press Release: "Myriad Genetics Begins Phase 1 Trial of Anti-Thrombin Drug MPC-0920", Myriad Genetics Inc, 02. Mai 2006] und
   **TGN-255** (Flovagatran) 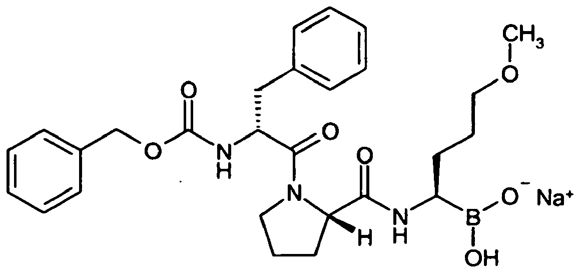 und direkte Faktor Xa-Inhibitoren wie beispielsweise
   **Rivaroxaban (BAY 59-7939):** 5-Chlor-*N*-({(5*S*)-2-oxo-3-[4-(3-oxomorpholin-4-yl)phenyl]-1,3-oxazolidin-5-yl}methyl)thiophen-2-carboxamid [WO 2001/47919] 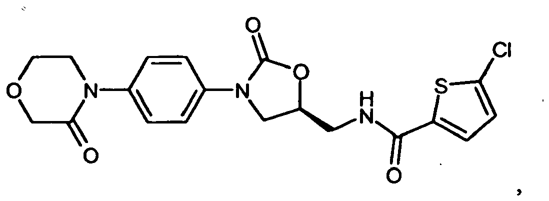
   **AX-1826** [S. Takehana et al. Japanese Journal of Pharmacology 2000, 82 (Suppl. 1), 213P; T. Kayahara et al. Japanese Journal of Pharmacology 2000, 82 (Suppl. 1), 213P],
   **Tanogitran (BIBT-986, prodrug: BIBT-1011):** *N*-[(1*R*)-1-(2-[({4-[Amino(imino)methyl]-phenyl} amino)methyl]-1-methyl-1*H*-benzimidazol-5-yl}-1-methyl-2-oxo-2-pyrrolidin-1-ylethyl]glycin [American Chemical Society - 226th National Meeting, New York City, NY, USA, 2003] 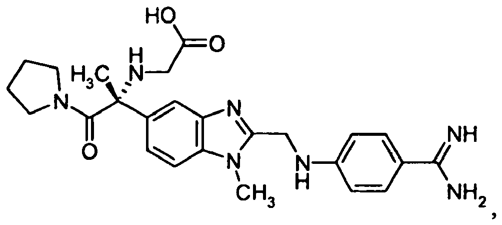 Verbindungen, die mit WO 2004/056784 offengelegt wurden.,
   **YM-150** [Y. Iwatsuki et al. Blood 2006, 108*,* abstract 911 (ASH 2006)],
      *N*-{4-Brom-2-[(5-chlorpyridin-2-yl)carbamoyl]-6-hydroxyphenyl}-1-isopropylpiperidin-4-carboxamid [JP 2005/179272] 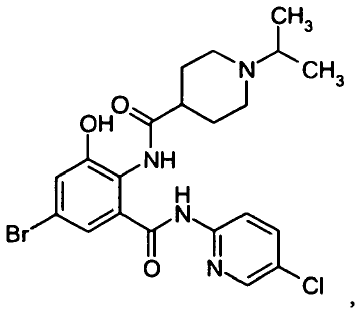 Verbindungen, die mit WO 2000/242270 offengelegt wurden.,
   **AZ12300547:** 6-[4-({(2*S*)-4-[(3-Chlor-1*H*-indol-6-yl)sulfonyl]-2-methyl-6-oxopiperazin-1-yl}methyl)-phenyl]-2-methylpyridazin-3(2*H*)-on [K.L Granberg et al. American Chemical Society - 232th National Meeting, San Francisco, USA, 2006, MEDI 391] 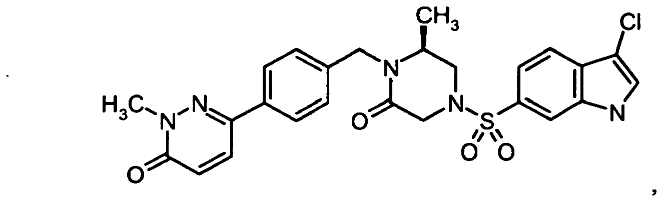 Verbindungen, die mit WO 2007/008142 offengelegt wurden.,
   **Razaxaban (DPC-906):** 1-(3-Amino-1,2-benzisoxazol-5-yl)-*N*-(4-{2-[(dimethylamino)-methyl]-1*H*-imidazol-1-yl}-2-fluorphenyl)-3-(trifluormethyl)-1*H*-pyrazol-5-carboxamid [J.Med.Chem. 2005, 48, 1729-1744] 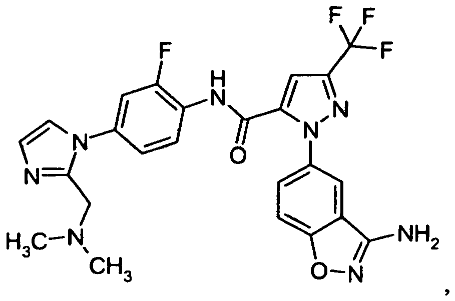
   **Apixaban (BMS-562247):** 1-(4-Methoxyphenyl)-7-oxo-6-[4-(2-oxopiperidin-1-yl)phenyl]-4,5,6,7-tetrahydro-1*H*-pyrazolo[3,4-c]pyridin-3-carboxamid [WO 2003/026652, WO 2003/049681] 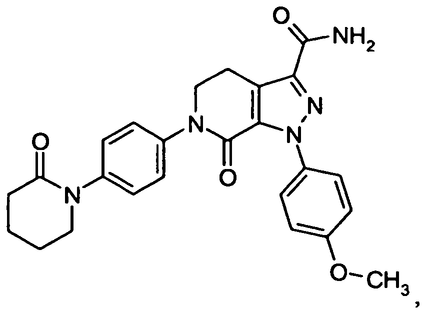
   **BMS-691648:** 3-Chlor-*N*-[(3S,4R)-1-(methylsulfonyl)-4-{[4-(2-oxopyridin-1(2H)-yl)benzoyl]-amino}piperidin-3-yl]-1*H*-indol-6-carboxamid [T. Güngör et al. Drugs Fut. 2006, 31(Suppl A): abstract P118; WO 2004/082687] 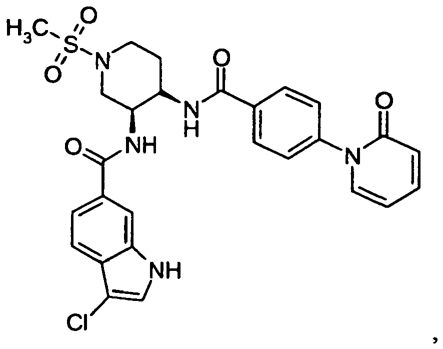
   **DX-9065a:** (2*S*)-3-{7-[Amino(imino)methyl]-2-naphthyl}-2-(4-{[(3*S*)-1-ethanimidoylpyrrolidin-3-yl]oxy}phenyl)propansäure [T. Nagahara et al. J.Med.Chem. 1994, 37, 1200-1207] 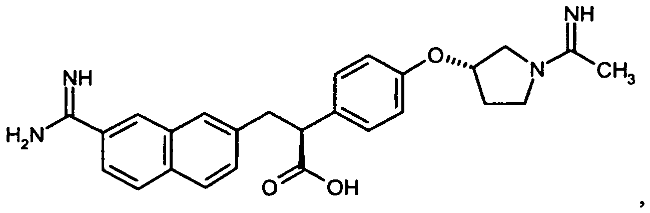
   **DU-176b** [Y. Morishima et al. Blood 2004, 104*,* abstract 1862 (ASH 2004); T. Fukuda et al. Blood 2004, 104, abstract 1852 (ASH 2004); T. Furugohri et al. Blood 2004, 104, abstract 1851 (ASH 2004)],
      *N*-(5-Chlorpyridin-2-yl)-*N*'-[(1*S*,2*R*,4*S*)-4-(dimethylcarbamoyl)-2-{[(5-methyl-4,5,6,7-tetrahydro[1,3]thiazolo[5,4-c]pyridin-2-yl)carbonyl]amino}cyclohexyl]ethandiamid [US 2005/0020645, WO 2005/47296] 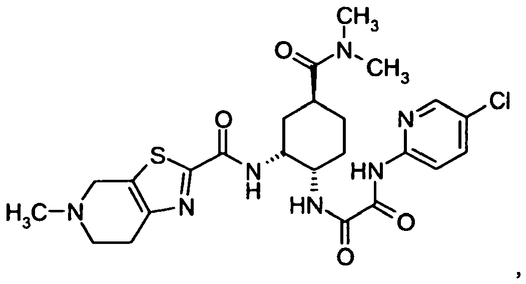 Verbindungen, die mit US 2005/0020645 offengelegt wurden.,
   **LY517717:** *N*-{(1*R*)-2-[4-(1-Methylpiperidin-4-yl)piperazin-1-yl]-2-oxo-1-phenylethyl}-1*H* indol-6-carboxamid [WO 2000/76971, WO 2002/100847] 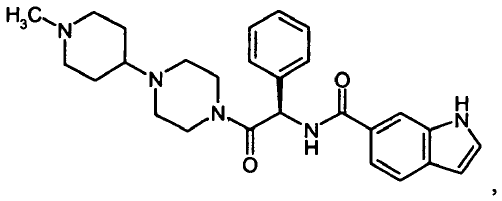
   **813893** [Proteinase Inhibitor Design - Fourth SCI-RSC Symposium, Proteinase 2004: Strategies for New Medicines (Part I), London],
      6-Chlor-*N*-{(3*S*)-1-[(1*S*)-1-methyl-2-morpholin-4-yl-2-oxoethyl]-2-oxopyrrolidin-3-yl}naphtha-len-2-sulfonamid [N.S. Watson et al. Bioorg.Med.Chem.Lett. 2006, 16, 3784; WO 2002/100830; WO 2002/100886] 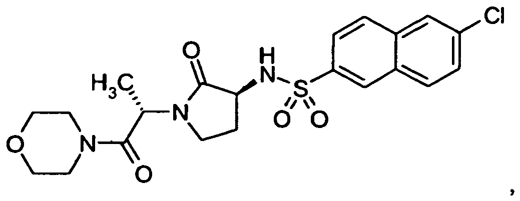
   **KFA-1982 (prodrug of KFA-1829)** [T. Koizumi et al. Journal of Thrombosis and Hemostasis 2003, 1 Suppl 1, P2022],
   **EMD-503982** [Merck KGaA Annual Report 2006, 48-49],
   **EMD-495235:** 5-Chlor-*N*-[(1*R*)-1-(methoxymethyl)-2-{[3-methyl-4-(3-oxomorpholin-4-yl)-phenyl]amino}-2-oxoethyl]thiophen-2-carboxamid [Bioorg.Med.Chem.Lett. 2004, 14, 5817-5822] 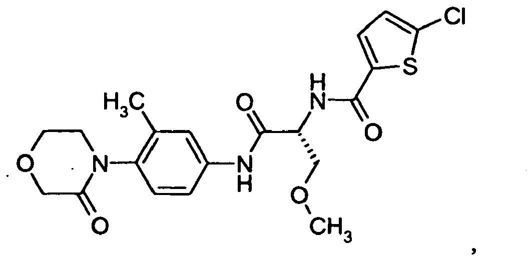
   **M-55113**: 4-[(6-Chlor-2-naphthyl)sulfonyl]-1-[(1-pyridin-4-ylpiperidin-4-yl)methyl]piperazin-2-on [H. Nishida et al. Chem.Pharm.Bull. 2001, 49, 1237-1244] 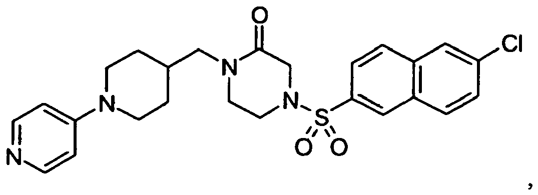
   **M-55551/M-55555**: (2*R*)-4-[(6-Chlor-2-naphthyl)sulfonyl]-6-oxo-1-[(1-pyridin-4-ylpiperidin-4-yl)methyl]piperazin-2-carbonsäure [H. Nishida et al. Chem.Pharm.Bull. 2002, 50, 1187-1194] 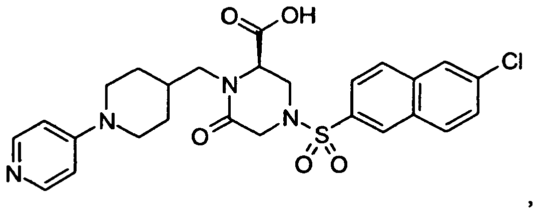
   **M-55190**: (2*R*)-4-[(6-Chlor-2-naphthyl)sulfonyl]-6-oxo-1-[(1-pyridin-4-ylpiperidin-4-yl)-methyl]piperazin-2-carbonsäureethylester [H. Nishida et al. 16th Int Symp Med Chem, Bologna, 18-22 Sept 2000, Abst PA-125] 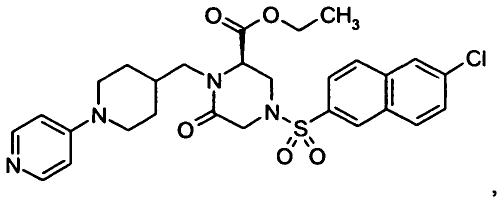
   **M-55532**: 7-[(6-Chlor-2-naphthyl)sulfonyl]-8a-(methoxymethyl)-1'-pyridin-4-yltetrahydro-5*H*-spiro[1,3-oxazolo[3,2-a]pyrazin-2,4'-piperidin]-5-on [H. Nishida et al. 228th ACS National Meeting, Philadelphia, August 22-26, 2004, MEDI-251; H. Nishida et al. Chem.Pharm.Bull. 2004, 52, 406-412; *dito* 459-462] 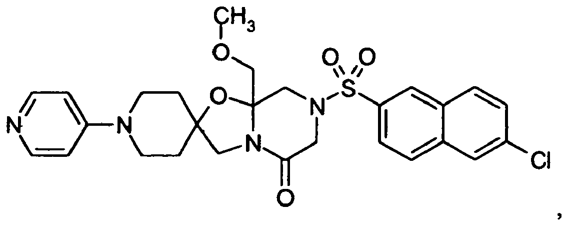 *N*-({7-[(5-Chlor-1*H*-indol-2-yl)sulfonyl]-5-oxo-1'-propionyltetrahydro-8a*H*-spiro[1,3-oxazolo-[3,2-a]pyrazin-2,4'-piperidin]-8a-yl}methyl)-*N*-methylglycin [WO 2006/106804] 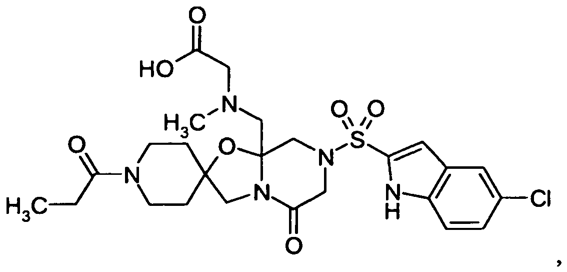
   **PRT54021** [U. Sinha et al. Blood 2006, 108*,* abstract 907 (ASH 2006); K. Abe et al. Blood 2006, 108*,* abstract 901 (ASH 2006)],
      Verbindungen, die mit WO 2006/002099 offengelegt wurden.,
   **Otamixaban (FXV-673, RPR-130673):** (2*R*,3*R*)-2-{3-[Amino(imino)methyl]benzyl}-3-{[4-(1-oxidopyridin-4-yl)benzoyl]amino}butansäuremethylester [V. Chu et al. Thrombosis Research 2001, 103, 309-324; K.R. Guertin et al. Bioorg Med.Chem.Lett. 2002, 12, 1671-1674] 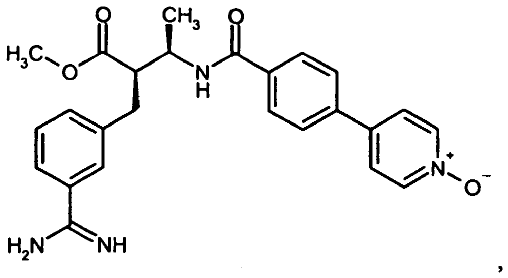
   **AVE3247** [Sanofi Aventis *Company* Presentation, Paris **2007,** February 13],
   **SAR377142 (SSR-7142)** [Sanofi Aventis *Company* Presentation, Paris **2007,** February 13],
   **HMR-2906** [XVIIth Congress of the International Society for Thrombosis and Haemostasis, Washington D.C., USA, 14-21 Aug 1999**;** Generating greater value from our products and pipeline. Aventis SA Company Presentation, 05 Feb **2004],**
   **Idraparinux** [Harry R. Büller et al. Blood, 2006, 108, abstract 571 (ASH 2006)] und
   **Fondaparinux;**
- plättchenaggregationshemmende Substanzen (Plättchenaggregationshemmer, Thrombozytenaggregationshemmer) wie beispielsweise Acetylsalicylsäure (wie beispielsweise Aspirin), Ticlopidin (Ticlid), Clopidogrel (Plavix) und Prasugrel;
- Fibrinogen-Rezeptor-Antagonisten (Glycoprotein-IIb/IIIa-Antagonisten) wie beispielsweise Abciximab, Eptifibatide, Tirofiban, Lamifiban, Lefradafiban und Fradafiban;
- sowie Antiarrhythmika.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 5 mg/kg, vorzugsweise etwa 0.01 bis 1 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen

- ca.: circa
- CDI: Carbonyldiimidazol
- d: Tag(e), Dublett (bei NMR)
- DC: Dünnschicht-Chromatographie
- DCI: direkte chemische Ionisation (bei MS)
- dd: Doppeltes Dublett (bei NMR)
- DMAP: 4-Dimethylaminopyridin
- DMF: *N,N-*Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- h: Stunde(n)
- HPLC: Hochdruck-, Hochleistungsflüssigchromatographie
- LC-MS: Flüssigchromatographie-gekoppelte Massenspektroskopie
- LDA: Lithiumdiisopropylamid
- m: Multiplett (bei NMR)
- min: Minute(n)
- MS: Massenspektroskopie
- NMR: Kernresonanzspektroskopie
- RP: reverse phase (bei HPLC)
- RT: Raumtemperatur
- Rₜ: Retentionszeit (bei HPLC)
- s: Singulett (bei NMR)
- THF: Tetrahydrofuran

### LC-MS- und HPLC-Methoden

**Methode 1 (HPLC):** Instrument: HP 1100 mit DAD-Detektion; Säule: Kromasil 100 RP-18, 60 mm x 2.1 mm, 3.5 µm; Eluent A: 5 ml Perchlorsäure (70%ig) / 1 Wasser, Eluent B: Acetonitril; Gradient: 0 min 2%B, 0.5 min 2%B, 4.5 min 90%B, 6.5 min 90%B, 6.7 min 2%B, 7.5 min 2%B; Fluß: 0.75 ml/min; Säulentemperatur: 30°C; Detektion: UV 210 nm.

**Methode 2 (HPLC):** Instrument: HP 1100 mit DAD-Detektion; Säule: Kromasil 100 RP-18, 60 mm x 2.1 mm, 3.5 µm; Eluent A: 5 ml Perchlorsäure (70%ig) / 1 Wasser, Eluent B: Acetonitril; Gradient: 0 min 2%B, 0.5 min 2%B, 4.5 min 90%B, 9 min 90%B, 9.2 min 2%B, 10 min 2%B; Fluß: 0.75 ml/min; Säulentemperatur: 30°C; Detektion: UV 210 nm.

**Methode 3 (LC-MS):** Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Methode 4 (LC-MS):** Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

**Methode 5 (LC-MS):** Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208- 400 nm.

**Methode 6 (LC-MS):** Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

**Methode 7 (LC-MS):** Instrument: Micromass Platform LCZ mit HPLC Agilent Serie 1100; Säule: Thermo Hypersil GOLD 3µ 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100%A → 0.2 min 100%A → 2.9 min 30%A → 3.1 min 10%A → 5.5 min 10%A; Ofen: 50°C; Fluss: 0.8 ml/min; UV-Detektion: 210 nm.

**Methode 8 (LC-MS):** Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 210 nm.

**Methode 9 (GC-MS):** Instrument: Micromass GCT, GC6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (1.7 min halten).

**Methode 10 (GC-MS):** Instrument: Micromass GCT, GC6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

**Methode 11 (HPLC, Enantiomerentrennung):** Säule: Daicel Chiralpak AD-H, 5 µm, 250 mm x 4.6 mm; Laufmittel: Ethanol/Isohexan 70:30; Fluss: 1 ml/min; Temperatur: 40°C; UV-Detektion: 220 nm.

### Ausgangsverbindungen

### Beispiel 1A

### 5-Chlor-N-[(2S)-oxiran-2-ylmethyl]thiophen-2-carboxamid

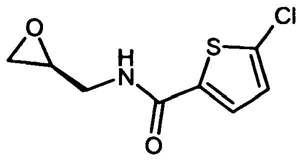

Beispiel 1A wird hergestellt, wie in WO04/1015 (Beispiel 6A) beschrieben.

### Beispiel 2A

### 1-(2-Chlor-4-nitrophenyl)-3-methoxypyridin-2(1H)-on

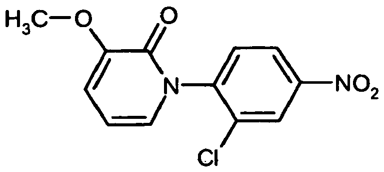

15 g (120 mmol) 3-Methoxy-pyridon werden in 120 ml wasserfreiem Dimethylsulfoxid gelöst und unter Rühren mit 16.1 g (144 mmol) Kalium-tert.-butylat versetzt. Nach 30 min werden 21.0 g (120 mmol) 3-Chlor-4-fluor-nitrobenzol zugegeben und es wird auf 80°C erhitzt. Nach 16 h lässt man erkalten, versetzt mit 1N Salzsäure und extrahiert mehrmals mit Dichlormethan. Die vereinigten Dichlormethan-Phasen werden mit Wasser und gesättigter Natriumchloridlösung gewaschen, über Natriumsulfat getrocknet, filtriert und im Vakuum zur Trockene eingedampft. Der erhaltene Rückstand wird mit tert.-Butylmethylether verrührt und abfiltriert. Der Feststoff wird im Vakuum getrocknet. Es werden 22.8 g (60% d. Th.) der gewünschten Verbindung erhalten.
¹H-NMR (400 MHz, DMSO-*d*₆, *δlppm*): 8.55 (d, 1H), 8.3 (dd, 1H), 7.85 (d, 1H), 7.15 (d, 1H), 6.9 (d, 1H), 6.3 (t, 1H), 3.65 (s, 3H).
HPLC (Methode 4): Rₜ = 1.80 min.
MS (ESIpos, *m*/*z*): 281 (M+H)⁺.

### Beispiel 3A

### 1-(4-Amino-2-chlorphenyl)-3-methoxypyridin-2(1H)-on

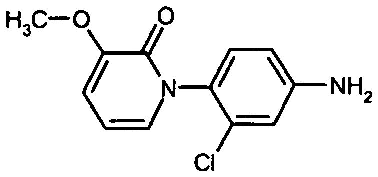

309 mg (0.72 mmol) Beispiel 2A werden in 7.5 ml Ethanol gelöst und mit 994 mg (4.41 mmol) Zinnchlorid-dihydrat versetzt. Es wird 1 h zum Rückfluß erhitzt und dann die Reaktion durch Zugabe von 50 ml Wasser beendet. Mit Natronlauge wird auf pH = 10 gestellt und dreimal mit Essigsäureethylester extrahiert. Die vereinigten organischen Phasen werden über Natriumsulfat getrocknet. Anschließend wird filtriert und das Filtrat vom Lösungsmittel befreit. Das Produkt wird ohne weitere Aufreinigung weiter umgesetzt.
¹H-NMR (400 MHz, DMSO-*d*₆, *δ*/*ppm*): 7.05-6.94 (m, 2H), 6.84 (dd, 1H), 6.72 (d, 1H), 6.56 (dd, 1H), 6.17 (dd, 1H), 5.63 (br.s, 2H), 3.12 (s, 3H).
HPLC (Methode 1): Rₜ = 3.38 min.
MS (DCI, *m*/*z*): 251 (M+H)⁺.

### Beispiel 4A

### 1-(2-Chlor-4-nitrophenyl)-3-hydroxypyridin-2(1H)-on

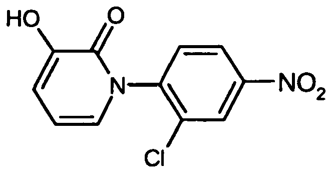

22.8 g (81 mmol) Beispiel 2A werden in 600 ml wasserfreiem Dichlormethan gelöst und auf 0°C gekühlt. Bei dieser Temperatur werden 203 ml (203 mmol) einer 1N Bortribromid-Lösung in Dichlormethan zugetropft. Es wird weitere 2 h bei 0°C gerührt bevor mit Wasser verdünnt wird. Die Kühlung wird entfernt, es wird mit Dichlormethan verdünnt und die organische Phase wird abgetrennt. Die Wasserphase wird mit Dichlormethan extrahiert, die vereinigten organischen Phasen werden mit gesättigter Natriumchloridlösung gewaschen, über Natriumsulfat getrocknet, filtriert und im Vakuum zur Trockene eingedampft. Der Rückstand wird mit Diisopropylether verrührt, abfiltriert und im Vakuum getrocknet. Man erhält 17.8 g (82% d. Th.) der gewünschten Verbindung.
¹H-NMR (400 MHz, DMSO-*d*₆, *δ*/*ppm*): δ = 9.55 (s, 1H), 8.6 (s, 1H), 8.35 (dd, 1H), 7.85 (d, 1H), 7.05 (d, 1H), 6.8 (d, 1H), 6.3 (t, 1H).
LC-MS (Methode 4): Rₜ = 1.48 min
MS (ESIpos): m/z = 267 (M+H)⁺

### Beispiel 5A

### 1-(2-Chlor-4-nitrophenyl)-3-(2-hydroxyethoxy)pyridin-2(1H)-on

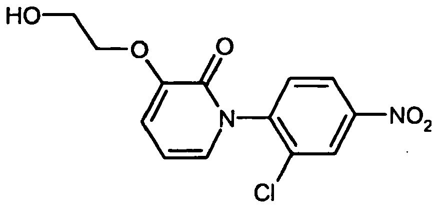

17.8 g (66.7 mmol) Beispiel 4A werden in 500 ml wasserfreiem N,N-Dimethylformamid gelöst und mit 18.5 g (134 mmol) Kaliumcarbonat versetzt. Nach 30 min werden 13.1 g (100 mmol) 2-Bromethanol zugegeben und auf 60°C erhitzt. Nach 5 h wird auf RT abgekült und mit 1N Salzsäure auf pH 2 gebracht. Die Reaktionslösung wird mehrmals mit Dichlormethan extrahiert und die vereinigten organischen Phasen werden mit gesättigter Natriumchloridlösung gewaschen, über Natriumsulfat getrocknet, filtriert und im Vakuum zur Trockene eingedampft. Der Rückstand wird aus Essigsäureethylester umkristallisiert, die Kristalle abfiltriert und im Vakuum getrocknet. Man erhält 13.4 g (65% d. Th.) der gewünschten Verbindung.
¹H-NMR (400 MHz, DMSO-*d*₆, *δlppm*): δ = 8.5 (s, 1H), 8.35 (dd, 1H), 7.85 (d, 1H), 7.15 (d, 1H), 6.95 (d, 1H), 6.3 (t, 1H), 4.95 (t, 1H), 3.9 (m, 2H), 3.7 (m, 2H).
LC-MS (Methode 4): Rₜ = 1.83 min
MS (ESIpos): m/z = 311 (M+H)⁺

### Beispiel 6A

### 3-(2-{[tert-Butyl(diphenyl)silyl]oxy}ethoxy)-1-(2-chlor-4-nitrophenyl)pyridin-2(1H)-on

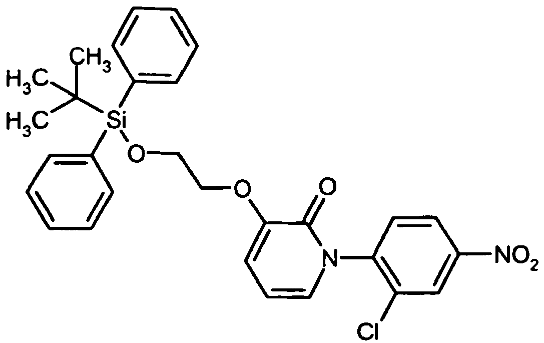

13.4 g (43.1 mmol) Beispiel 5A werden in 50 ml wasserfreiem N,N-Dimethylformamid gelöst und mit 3.5 g (52 mmol) Imidazol versetzt. Bei RT wird langsam 13.0 g (47 mmol) tert-Butyl(chlor)diphenylsilan zugetropft. Nach beendeter Zugabe wir weitere 4 h bei RT nachgerührt, dann mit Wasser verdünnt und die Reaktionslösung wird mehrmals mit Essigsäureethylester extrahiert. Die vereinigten organischen Phasen werden zweimal mit 1N Salzsäure und einmal mit gesättigter Natriumchloridlösung gewaschen, mit Natriumsulfat getrocknet, filtriert und im Vakuum zur Trockene eingedampft. Der Rückstand wird im Vakuum getrocknet. Man erhält 25.4 g (91% d. Th.) der gewünschten Verbindung.
¹H-NMR (400 MHz, DMSO-*d*₆, *δ*/*ppm*): δ = 8.55 (d, 1H), 8.35 (dd, 1H), 7.85 (d, 1H), 7.7 (m, 5H), 7.4 (m, 5H), 7.2 (d, 1H), 6.95 (d, 1H), 6.3 (t, 1H), 4.1 (m, 2H), 3.9 (m, 2H), 1.0 (s, 9H).
LC-MS (Methode 4): Rₜ = 3.12 min
MS (ESIpos): m/z = 549 (M+H)⁺

### Beispiel 7A

### 1-(4-Amino-2-chlorphenyl)-3-(2-{[tert-butyl(diphenyl)silyl]oxy}ethoxy)pyridin-2(1H)-on

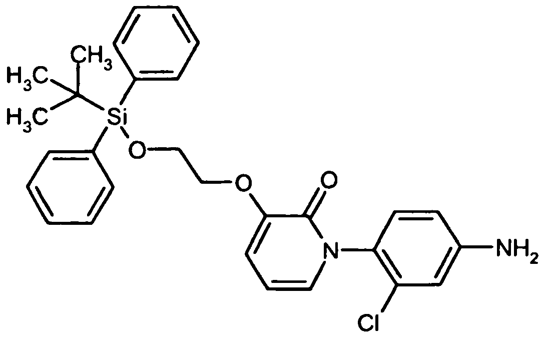

25.4 g (41.6 mmol) Beispiel 6A werden in 300 ml Tetrahydrofuran gelöst und mit 0.5 g Raney Nickel versetzt. Es werden 5.2 g (104 mmol) Hydrazinhydrat zugesetzt und die Reaktionsmischung auf 80°C erhitzt. Es tritt eine heftige Gasentwicklung ein die nach 1 h abklingt. Es wird erneut dieselbe Menge Raney Nickel und Hydrazinhydrat zugegeben und weiter erhitzt. Dies wird zweimal bis zur vollständigen Reduktion wiederholt. Dann lässt man erkalten, filtriert über Kieselgel und wäscht mit Tetrahydrofuran nach. Die Lösung wird im Vakuum zur Trockene eingedampft und der Rückstand wird im Vakuum getrocknet. Man erhält 20.0 g (93% d. Th.) der gewünschten Verbindung.
¹H-NMR (400 MHz, DMSO-*d*₆, *δlppm*): δ = 7.7 (d, 4H), 7.45 (m, 6H), 7.2 (m, 2H), 6.9 (dd, 1H), 6.65 (d, 1H), 6.6 (dd, 1H), 6.15 (t, 1H), 5.65 (m, 2H), 4.1 (m, 2H), 3.95 (m, 2H), 1.0 (s, 9H).
LC-MS (Methode 5): Rₜ = 3.14 min
MS (ESIpos): m/z = 519 (M+H)⁺

### Beispiel 8A

### N-[(2R)-3-({4-[3-(2-{[tert-Butyl(diphenyl)silyl]oxy}ethoxy)-2-oxopyridin-1(2H)-yl]-3-chlorphenyl}amino)-2-hydroxypropyl]-5-chlorthiophen-2-carboxamid

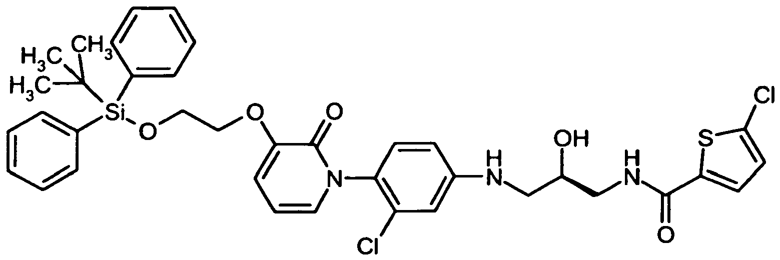

690 mg (1.33 mmol) Beispiel 7A werden in 10 ml wasserfreiem Acetonitril gelöst und bei 0°C mit 318 mg (1.46 mmol) Beispiel 1A versetzt. Es werden 445 mg (1.99 mmol) Magnesiumperchlorat zugesetzt und die Kühlung entfernt. Nach 6 h werden erneut 318 mg (1.46 mmol) Beispiel 1A zugegeben und weitere 16 h bei RT gerührt. Es wird mit Wasser verdünnt und die Reaktionslösung mehrmals mit Essigsäureethylester extrahiert. Die vereinigten organischen Phasen werden über Natriumsulfat getrocknet, filtriert und im Vakuum zur Trockene eingedampft und der Rückstand wird im Vakuum getrocknet. Man erhält 1.4 g (99% d. Th.) der gewünschten Verbindung.
LC-MS (Methode 5): Rₜ = 3.25 min
MS (ESIpos): m/z= 736 (M+H)⁺

### Beispiel 9A

### N-[((5S)-3-{4-[3-(2-{[tert-Butyl(diphenyl)silyl]oxy}ethoxy)-2-oxopyridin-1(2H)-yl]-3-chlorphenyl}-2-oxo-1,3-oxazolidin-5-yl)methyl]-5-chlorthiophen-2-carboxamid

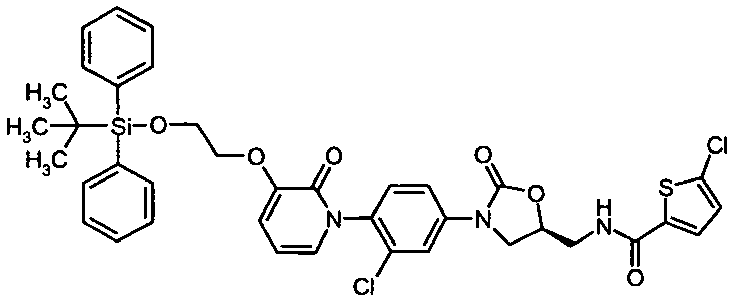

1.4 g (2 mmol) Beispiel 8A werden in 25 ml wasserfreiem Butyronitril gelöst und mit 5 mg (0.04 mmol) 4-Dimethylaminopyridin und 658 mg (4.1 mmol) N,N-Carbonyldiimidazol versetzt. Es wird 5 h zum Rückfluss erhitzt. Man lässt erkalten und verdünnt mit 150 ml Dichlormethan. Die organische Phase wird abgetrennt, mit Wasser und gesättigter Natriumchloridlösung gewaschen, über Natriumsulfat getrocknet, filtriert und im Vakuum zur Trockene eingedampft. Der Rückstand wird in Dimethylsulfoxid gelöst und mit präparativer HPLC mit einem Wasser/Acetonitril Gradienten getrennt. Man erhält 0.74 g (48% d. Th.) der gewünschten Verbindung.
¹H-NMR (400 MHz, DMSO-*d*₆, *δ*/*ppm*): δ = 9.0 (t, 1H), 7.9 (d, 1H), 7.7 (m, 5H), 7.6 (m, 2H), 7.35-7.55 (m, 6H), 7.2 (d, 1H), 7.1 (dd, 1H), 6.9 (d, 1H), 6.2 (t, 1H), 4.9 (m, 1H), 4.3 (t, 1H), 4.1 (m, 2H), 3.95 (m, 2H), 3.9 (m, 1H), 3.6 (m, 2H), 1.0 (s, 9H).
LC-MS (Methode 5): Rₜ = 3.13 min
MS (ESIpos): m/z = 762 (M+H)⁺

### Beispiel 10A

### 3-Methoxy-1-(2-methyl-4-nitrophenyl)pyridin-2(1H)-on

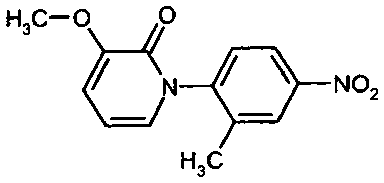

28.5 g (228 mol) 3-Methoxypyridin-2(1H)-on werden in 850 ml Dimethylsulfoxid gelöst und bei RT mit 31 g (273 mmol) Kalium-tert.-butylat versetzt. Die Suspension wird 30 min bei RT gerührt, bevor 35 g (228 mmol) 1-Fluor-2-methyl-4-nitrobenzol zugesetzt werden und die Reaktionslösung für 20 h auf 80°C erhitzt wird. Dann wird vorsichtig mit 1 1 Wasser verdünnt und mit 1N Salzsäure auf pH 1-2 gebracht. Die Lösung wird mehrmals mit Dichlormethan extrahiert. Die vereinigten organischen Extrakte werden mit Wasser und gesättigter Natriumchloridlösung gewaschen, über Natriumsulfat getrocknet, filtriert und im Vakuum eingedampft. Der erhaltene Feststoff wird mit wenig tert-Butylmethylether gewaschen, abfiltriert und im Vakuum getrocknet. Man erhält 42.8 g (72% d. Th.) der gewünschten Verbindung.
¹H-NMR (400 MHz, DMSO-*d₆, δ*/*ppm*): δ = 8.35 (d, 1H), 8.18 (dd, 1H), 7.57 (d, 1H), 7.13 (d, 1H), 6.95 (dd, 1H), 6.32 (t, 1H), 3.75 (s, 3H), 2.25 (s, 3H).
LC-MS (Methode 3): Rₜ = 1.45 min
MS (ESIpos): m/z = 261 (M+H)⁺

### Beispiel 11A

### 3-Hydroxy-1-(2-methyl-4-nitrophenyl)pyridin-2(1H)-on

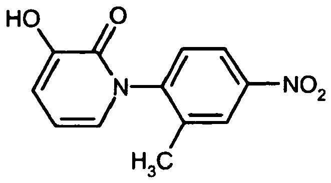

23.3 g (90 mmol) Beispiel 10A werden in 730 ml wasserfreiem Dichlormethan gelöst und auf 0°C gekühlt. Innerhalb von 10 Minuten werden 224 ml (224 mmol) einer 1N Bortribromid Lösung in Dichlormethan zugetropft, bevor weitere 1.5 h bei dieser Temperatur gerührt wird. Der Ansatz wird mit 200 ml Wasser versetzt und die Wasserphase wird dreimal mit Dichlormethan extrahiert. Die vereinigten organischen Phasen werden mit gesättigter Natriumchloridlösung gewaschen, über Natriumsulfat getrocknet und abfiltriert. Das Lösemittel wird im Vakuum entfernt und der erhaltene Feststoff wird mit tert.-Butylmethylether gewaschen und abfiltriert. Man erhält 20.1 g (91% d. Th.) der gewünschten Verbindung.
¹H-NMR (400 MHz, DMSO-*d*₆, *δ*/*ppm*): δ = 9.50 (s, 1H), 8.42 (d, 1H), 8.20 (dd, 1H), 7.6 (d, 1H), 7.05 (d, 1H), 6.85 (dd, 1H), 6.25 (t, 1H), 2.25 (s, 3H).
LC-MS (Methode 3): Rₜ = 1.45 min
MS (ESIpos): m/z = 246 (M+H)⁺

### Beispiel 12A

### 3-[(2-Methoxyethoxy)methoxy]-1-(2-methyl-4-nitrophenyl)pyridin-2(1H)-on

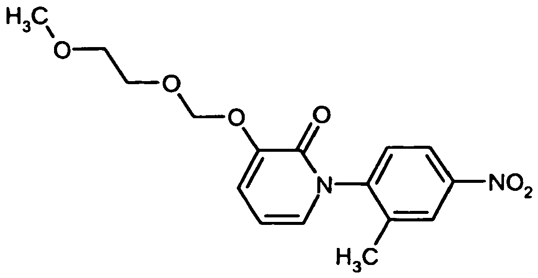

10.0 g (41 mmol) Beispiel 11A werden in wasserfreiem Dichlormethan gelöst und mit 13.6 g (89 mmol) 1,8-Diazabicyclo(5.4.0)undec-7-en versetzt. Zu dieser Lösung wird bei 25°C langsam portionsweise 8.6 g (69 mmol) 1-(Chlormethoxy)-2-methoxyethan zugegeben. Nach einer weiteren Stunde wird die Lösung über Kieselgel filtriert und im Vakuum zur Trockene eingedampft. Man erhält 20.1 g (91% d. Th.) der gewünschten Verbindung.
¹H-NMR (400 MHz, DMSO-*d*₆, *δ*/*ppm*): δ = 8.32 (d, 1H), 8.19 (dd, 1H), 7.6 (d, 1H), 7.22 (dd, 1H), 7.15 (dd, 1H), 6.3 (t, 1H), 5.25 (s, 2H), 3.78-3.72 (m, 2H), 3.51-3.45 (m, 2H), 3.23 (s, 3H), 2.15 (s, 3H).
LC-MS (Methode 3): Rₜ = 1.79 min
MS (ESIpos): m/z = 335 (M+H)⁺

### Beispiel 13A

### 1-(4-Amino-2-methylphenyl)-3-[(2-methoxyethoxy)methoxy]pyridin-2(1H)-on

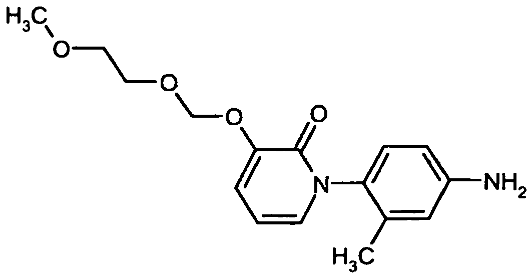

12 g (36 mmol) Beispiel 12A werden in 1.2 1 eines 1:1 1 Gemisches aus Essigsäureethylester und Ethanol gelöst und mit 0.1 Equivalenten Palladium auf Kohle und mit 11.3 g (180 mmol) Ammoniumformiat versetzt. Es wird zwei Stunden auf 80°C erhitzt. Man läßt erkalten, filtriert über Kieselgel und entfernt das Lösemittel im Vakuum. Man erhält 10.6 g (88% d. Th.) der gewünschten Verbindung.
¹H-NMR (400 MHz, DMSO-*d*₆, *δ*/*ppm*): δ = 7.12-7.05 (m, 1H), 6.80 (d, 1H), 6.51-6.42 (m, 2H), 6.17 (t, 1H), 5.21 (s, 2H), 3.76-3.71 (m, 2H), 3.49-3.45 (m, 2H), 3.23 (s, 3H), 1.85 (s, 3H).
LC-MS (Methode 3): Rₜ = 1.10 min
MS (ESIpos): m/z = 305 (M+H)⁺

### Beispiel 14A

### 5-Chlor-N-[((5S)-3-{4-[3-[(2-methoxyethoxy)methoxy]-2-oxopyridin-1(2H)-yl]-3-methylphenyl}-2-oxo-1,3-oxazolidin-5-yl)methyl]thiophen-2-carboxamid

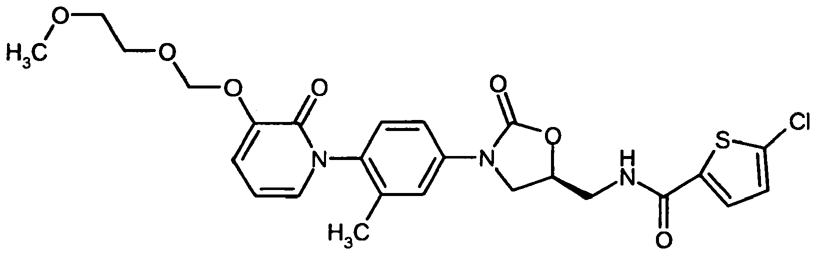

9.2 g (30 mmol) Beispiel 13A werden in 580 ml Acetonitril gelöst und auf 0°C gekühlt. Bei dieser Temperatur werden 7.3 g (34 mmol) Beispiel 1A zugegeben und dann weitere 10 Minuten gerührt. Es wird mit 10.2 g (46 mmol) Magnesiumperchlorat versetzt, die Kühlung entfernt und weiter 17 h nachgerührt. Dann werden 14.8 g (91 mmol) Carbonyldiimidaziol und 0.37 g (3 mmol) N,N-4-Dimethylaminopyridin zugegeben und man erhitzt für 4 h auf 60°C. Nach Erkalten wird weitere 1.6 h bei RT gerührt und dann im Vakuum zur Trockene eingedampft. Der Rückstand wird mit 1N Salzsäure und Essigsäureethylester versetzt und heftig gerührt. Nach 15 min werden die Phasen getrennt, die wässrige Phase dreimal mit Essigsäureethylester extrahiert und die organischen Phasen vereinigt. Nach Waschen mit gesättigter Natriumchloridlösung wird getrocknet und im Vakuum zur Trockene eingedampft. Man erhält 17.2 g (95% d. Th.) der gewünschten Verbindung.
¹H-NMR (400 MHz, DMSO-*d*₆, *δ*/*ppm*): δ = 9.0 (t, 1H), 7.70 (d, 1H), 7.56-7.49 (m, 2H), 7.26 (d, 1H), 7.20 (d, 1H), 6.95 (dd, 1H), 6.80 (dd, 1H), 6.20 (t, 1H), 4.90-4.81 (m, 1H), 4.24 (t, 1H), 3.92-3.85 (m, 1H), 3.70-3.55 (m, 3H), 3.52-3.25 (m, 2H), 2.55 (s, 3H), 2.08-2.02 (m, 3H), 1.9 (s, 3H).
LC-MS (Methode 4): Rₜ = 2.13 min
MS (ESIpos): m/z = 548 (M+H)⁺

### Beispiel 15A

### 5-Chlor-N-({(5S)-3-[4-(3-hydroxy-2-oxopyridin-1(2H)-yl)-3-methylphenyl]-2-oxo-1,3-oxazolidin-5-yl}methyl)thiophen-2-carboxamid

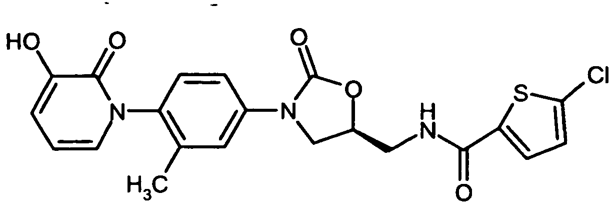

18 g (33 mmol) Beispiel 14A wird in 50 ml Trifluoressigsäure gelöst und 3 h nachgerührt, bevor im Vakuum zur Trockene eingedampft wird. Der Rückstand (22.3 g) wird in Portionen zu je 2 g in je 8.5 ml Dimethylsulfoxid gelöst und mit präparativer HPLC mit einem Wasser/Acetonitril Gradienten gereinigt. Die Produktfraktionen werden vereinigt, vom Acetonitril befreit und die enstandenen Kristalle abfiltriert. Das Filtrat wird mit Essigsäureethylester extrahiert, die Essigsäureethylesterphasen werden vereinigt und mit gesättigter Natriumchloridlösung gewaschen getrocknet und im Vakuum eingedampft. Der Rückstand wird mit den Kristallen vereinigt und im Vakuum getrocknet. Man erhält 9.95 g (65% d. Th.) der gewünschten Verbindung.
¹H-NMR (400 MHz, DMSO-*d*₆, δ*lppm*): δ = 9.2 (br. s, 1H), 9.00 (t, 1H), 7.70 (d, 1H), 7.56-7.48 (m, 2H), 7.26 (d, 1H), 7.20 (d, 1H), 6.95 (dd, 1H), 6.86 (dd, 1H), 6.2 (t, 1H), 4.92-4.80 (m, 1H), 4.22 (t, 1H), 3.92-3.82 (m, 1H), 3.62 (t, 2H), 2.04 (s, 3H).
LC-MS (Methode 4) Rₜ = 2.09 min
MS (ESIpos): m/z = 460 (M+H)⁺

### Beispiel 16A

### 1-(4-Amino-2-methylphenyl)-3-methoxypyridin-2(1H)-on

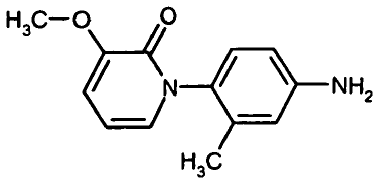

188 mg (0.724 mmol) Beispiel 10A werden in 10 ml Ethanol gelöst und mit 654 g (2.89 mmol) Zinnchlorid-dihydrat versetzt. Es wird 1 h zum Rückfluß erhitzt, dann abgekühlt und mit 50 ml Wasser verdünnt. Mit Natronlauge wird auf pH = 10 gestellt und anschließend dreimal mit Essigsäureethylester extrahiert. Die vereinigten organischen Phasen werden über Natriumsulfat getrocknet. Nach Filtration wird das Filtrat vom Lösungsmittel befreit. Das Reaktionsprodukt wird ohne weitere Aufreinigung umgesetzt.
¹H-NMR (400 MHz, DMSO-*d*₆, δ/*ppm*): 6.96 (dd, 1H), 6.85 (dd, 1H), 6.77 (d, 1H), 6.49-6.41 (m, 2H), 6.16 (dd, 1H), 5.21 (br.s, 2H), 3.72 (s, 3H), 1.84 (s, 3H).
LC-MS (Methode 2): Rₜ = 2.70 min.
MS (ESIpos, *m*/*z*): 231 (M+H)⁺.

### Beispiel 17A

### 1-(2,6-Dimethyl-4-nitrophenyl)-3-methoxypyridin-2(1H)-on

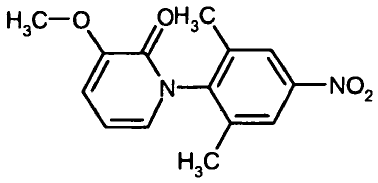

336 mg (2.69 mmol) 3-Methoxypyridinon in 10 ml DMF werden bei 0°C mit 452 mg (4.03 mmol) Kalium-tert.-butylat versetzt und es wird 30 min bei Raumtemperatur gerührt. 500 mg (2.96 mmol) 1-Fluor-2,5-dimethyl-4-nitrobenzol werden zugefügt und es wird bei 80°C gerührt. Nach 22 h wird auf 120°C erhitzt und weitere 20 h gerührt. Dann wird abgekühlt und in 100 ml Wasser und 15 ml gesättigte wässrige Natriumchlorid-Lösung gegeben. Es wird dreimal mit je 300 ml Essigsäureethylester extrahiert und die vereinigten organischen Phasen werden über Natriumsulfat getrocknet. Nach Filtration werden die Lösungsmittel im Vakuum entfernt und der Rückstand durch Chromatographie an Kieselgel (Cyclohexan/Essigsäureethylester 1:1) gereinigt. Man erhält 383 mg (52% d. Th.) der gewünschten Verbindung.
¹H-NMR (300 MHz, DMSO-*d*₆, δ/*ppm*): 8.16 (s, 2H), 7.04 (dd, 1H), 6.97 (dd, 1H), 6.38 (dd, 1H), 3.77 (s, 3H), 2.08 (s, 6H).
HPLC (Methode 1): Rₜ = 2.84 min.
MS (DCI, *m*/*z*): 275 (M+H)⁺.

### Beispiel 18A

### 1-(4-Amino-2,6-dimethylphenyl)-3-methoxypyridin-2(1H)-on

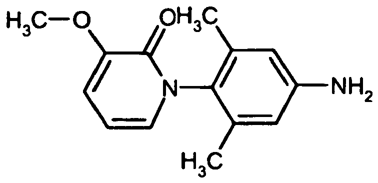

300 mg (1.09 mmol) Beispiel 17A werden in 20 ml Ethanol gelöst und mit 987 g (4.38 mmol) Zinnchlorid-dihydrat versetzt. Es wird 1 h zum Rückfluß erhitzt, dann abgekühlt und mit 150 ml Wasser verdünnt. Mit Natronlauge wird auf pH = 10 gestellt und anschließend dreimal mit Essigsäureethylester extrahiert. Die vereinigten organischen Phasen werden über Natriumsulfat getrocknet. Nach Filtration wird das Filtrat vom Lösungsmittel befreit. Das Reaktionsprodukt (204 mg) wird ohne weitere Aufreinigung umgesetzt.
¹H-NMR (300 MHz, DMSO-*d*₆, δ/*ppm*): 6.93-6.83 (m, 2H), 6.33 (s, 2H), 6.19 (dd, 1H), 5.12 (br.s, 2H), 3.72 (s, 3H), 1.78 (s, 6H).
LC-MS (Methode 1): Rₜ = 2.90 min.
MS (DCI, *m*/*z*): 245 (M+H)⁺.

### Beispiel 19A

### 1-(2,6-Dimethyl-4-nitrophenyl)-3-hydroxypyridin-2(1H)-on

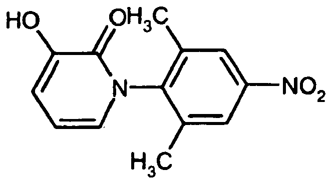

1.95 g (7.11 mmol) Beispiel 17A in 30 ml Dichlormethan werden bei 0°C mit 17.7 ml (17.7 mmol) einer 1-molaren Lösung von Bortribromid in Dichlormethan versetzt. Nach 2.5 h wird abgekühlt und mit 150 ml Dichlormethan verdünnt. Anschließend wird vorsichtig mit 150 ml Wasser versetzt und es wird 10 min gerührt. Nach Phasentrennung wird die wässrige Phase mit Dichlormethan gewaschen. Die vereinigten organischen Phasen werden mit gesättigter wässriger Natriumchlorid-Lösung gewaschen und dann über Natriumsulfat getrocknet. Nach Filtration werden die Lösungsmittel im Vakuum entfernt. Das so erhaltene Reaktionsprodukt (1.85 g) wird ohne weitere Aufreinigung umgesetzt.
¹H-NMR (400 MHz, DMSO-*d*₆, δ/*ppm*): 9.45 (s, 1H), 8.16 (s, 2H), 6.94 (dd, 1H), 6.86 (dd, 1H), 6.31 (dd, 1H), 2.10 (s, 6H).
LC-MS (Methode 3): Rₜ = 1.58 min.
MS (ESIpos, *m*/*z*): 261 (M+H)⁺.

### Beispiel 20A

### 1-(2,6-Dimethyl-4-nitrophenyl)-3-[(2-methoxyethoxy)methoxy]pyridin-2(1H)-on

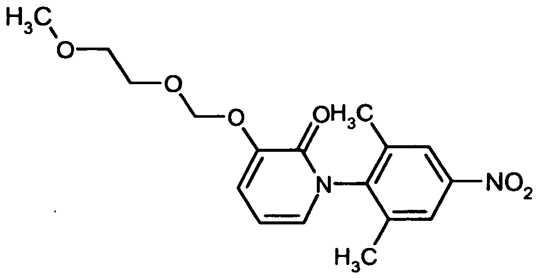

1.37 g (5.28 mmol) Beispiel 19A in 60 ml Dichlormethan und 1.73 ml (11.6 mmol) 1,8-Diazabicyclo(5.4.0)undec-7-en werden bei 0°C mit einer Lösung von 1.12 g (8.97 mmol) Methoxyethoxymethlychlorid in 15 ml Dichlormethan versetzt. Es wird 1 h bei RT gerührt. Die Lösungsmittel werden im Vakuum entfernt und das erhaltene Reaktionsprodukt durch Chromatographie an Kieselgel (Cyclohexan/Essigsäureethylester 2:1) gereinigt. Man erhält 1.02 g (55% d. Th.) der gewünschten Verbindung.
¹H-NMR (400 MHz, DMSO-*d*₆, δ/*ppm*): 8.16 (s, 2H), 7.22-7.13 (m, 2H), 6.37 (dd, 1H), 5.26 (s, 2H), 3.79-3.73 (m, 2H), 3.50-3.45 (m, 2H), 3.31 (s, 3H), 2.10 (s, 6H).
HPLC (Methode 1): Rₜ = 3.85 min.
MS (ESIpos, *m*/*z*): 349 (M+H)⁺.

### Beispiel 21A

### 1-(4-Amino-2,6-dimethylphenyl)-3-[(2-methoxyethoxy)methoxy]pyridin-2(1H)-on

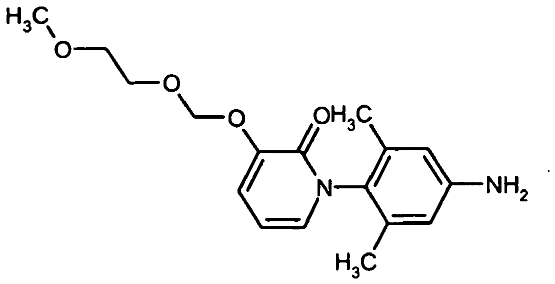

1.00 g (2.81 mmol) Beispiel 20A in 14 ml Ethanol werden mit 905 mg (14.4 mmol) Ammoniumformiat und 50 mg Palladium auf Kohle versetzt. Es wird 2 h bei 90°C gerührt. Nach Abkühlen wird über Kieselgur abgesaugt und zweimal mit Ethanol gespült. Die Lösungsmittel der gesammelten Filtrate werden im Vakuum entfernt. Das erhaltene Reaktionsprodukt (895 mg) wird ohne weitere Aufreinigung umgesetzt.
LC-MS (Methode 6): Rₜ = 1.42 min.
MS (ESIpos, *mlz*): 319 (M+H)⁺.

### Beispiel 22A

### 5-Chlor-N-{[(5S)-3-(4-{3-[(2-methoxyethoxy)methoxy]-2-oxopyridin-1(2H)-yl}-3-methylphenyl)-2-oxo-1,3-oxazolidin-5-yl]methyl}thiophen-2-carboxamid

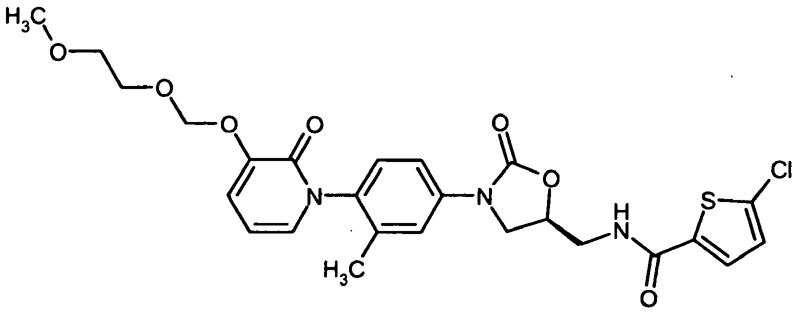

Eine Lösung von 875 mg (2.75 mmol) des Produktes aus Beispiel 21A in 50 ml Acetonitril wird mit 658 mg (3.03 mmol) des Produktes aus Beispiel 1A versetzt. Der Suspension werden 921 mg (4.13 mmol) Magnesiumperchlorat zugefügt, wonach man 6.5 h bei RT rühren lässt. Dann werden 1.15 g (6.88 mmol) 1,1'-Carbonyldiimidazol und 33 mg (0.28 mmol) DMAP hinzugesetzt und es wird bei RT gerührt. Nach 17 h wird mit 140 ml Wasser und 100 ml Essigsäureethylester verdünnt. Die Wasserphase wird zweimal mit Essigsäureethylester extrahiert und die vereinigten organischen Phasen werden über Natriumsulfat getrocknet. Nach Filtration wird vom Lösungsmittel befreit und der Rückstand durch Chromatographie an Kieselgel (Dichlormethan/Ethanol 40:1 -> 20:1) gereinigt. Nach Entfernen der Lösungsmittel erhält man 853 mg (50% d. Th.) des gewünschten Produktes.
HPLC (Methode 1): Rₜ = 4.11 min.
MS (ESIpos, *m*/*z*): 562 (M+H)⁺.

### Beispiel 23A

### 5-Chlor-N-({(5S)-3-[4-(3-hydroxy-2-oxopyridin-1(2H)-yl)-3,5-dimethylphenyl]-2-oxo-1,3-oxazolidin-5-yl}methyl)thiophen-2-carboxamid

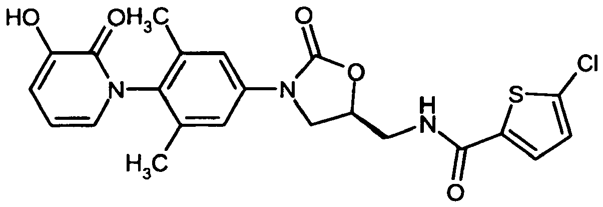

Zu einer Lösung von 750 mg (1.33 mmol) des Produktes aus Beispiel 22A in 3 ml Dichlormethan werden bei 0°C langsam 13.3 ml (173 mmol) Trifluoressigsäure getropft. Nach 5 h bei 0°C wird mit 200 ml Wasser und 80 ml Essigsäureethylester verdünnt. Mit Natriumhydrogencarbonat wird dann pH = 6 eingestellt und die Phasen werden getrennt. Die Wasserphase wird zweimal mit Essigsäureethylester extrahiert und die vereinigten organischen Phasen werden über Natriumsulfat getrocknet. Nach Filtration wird vom Lösungsmittel befreit. Man erhält 652 mg (100% d. Th.) des gewünschten Produktes.
¹H-NMR (400 MHz, DMSO-*d*₆, δ/*ppm*): 9.25 (s, 1H), 8.98 (t, 1H), 7.70 (d, 1H), 7.39 (dd, 2H), 7.20 (d, 1H), 6.84 (dd, 1H), 6.24 (dd, 1H), 4.91-4.80 (m, 1H), 4.21 (dd, 1H), 3.90-3.83 (m, 1H), 3.70-3.57 (m, 2H), 1.97 (s, 6H).
HPLC (Methode 1): Rₜ = 4.09 min.
MS (DCI, *m*/*z*): 474 (M+H)⁺.

### Beispiel 24A

### 1-[2-(Difluormethoxy)-4-nitrophenyl]-3-methoxypyridin-2(1H)-on

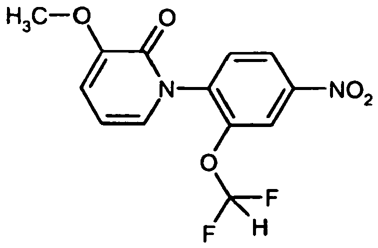

302 mg (2.41 mmol) 3-Methoxy-Pyridon werden in 8.2 ml wasserfreiem Dimethylsulfoxid gelöst und mit 325 mg (2.89 mmol) Kalium-tert.-butylat versetzt. Nach 30 min werden 500 mg (2.41 mmol) 2-(Difluormethoxy)-1-fluor-4-nitrobenzol zugegeben und 20 h auf 80°C erhitzt. Die Reaktionslösung wird mit Dichlormethan verdünnt und mit Wasser und 1N Salzsäure gewaschen. Nach Trocknen der organischen Phase über Natriumsulfat wird filtriert und im Vakuum zur Trockene eingedampft. Der Rückstand wird in Dimethylsulfoxid gelöst und mit präparativer HPLC mit einem Wasser/Acetonitril Gradienten aufgereinigt. Die Produktfraktionen werden vereinigt und im Vakuum zur Trockene eingedampft. Man erhält 207 mg (28% d. Th.) des gewünschten Produktes.
¹H-NMR (400 MHz, DMSO-*d*₆, δ/*ppm*): δ = 8.3 (dd, 1H), 8.2 (d, 1H), 7.8 (d, 1H), 7.4 (t, 1H), 7.2 (d, 1H), 6.9 (d, 1H), 6.3 (t, 1H), 3.75 (s, 3H).
LC-MS (Methode 4): Rₜ = 1.83 min
MS (ESIpos): m/z = 313 (M+H)⁺

### Beispiel 25A

### 1-[4-Amino-2-(difluormethoxy)phenyl]-3-methoxypyridin-2(1H)-on

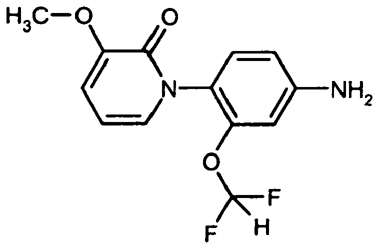

200 mg (0.641 mmol) Beispiel werden in 20 ml einer 1:1 Mischung aus Ethanol und Essigsäureethylester gelöst und mit 202 mg (3.20 mmol) Ammoniumformiat und 50 mg Palladium auf Kohle versetzt. Es wird auf 80°C erhitzt. Nach 1 h lässt man erkalten und filtriert über Kieselgel. Dann wird mit einem 1:1 Gemisch aus Ethanol und Essigsäureethylester nachgewaschen und das Filtrat im Vakuum zur Trockene eingedampft. Man erhält 190 mg (100% d. Th.) des gewünschten Produktes.
¹H-NMR (400 MHz, DMSO-*d*₆, δ/*ppm*): δ = 6.75-7.15 (m, 4H), 6.5 (s, 2H), 6.2 (t, 1H), 5.65 (m, 2H), 3.7 (s, 3H).
LC-MS (Methode 3): Rₜ = 1.30 min
MS (ESIpos): m/z = 283 (M+H)⁺

### Beispiel 26A

### 2-(Brommethyl)-1-fluor-4-nitrobenzol

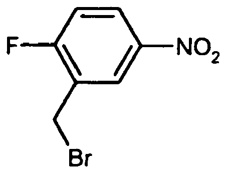

186 g (1.2 mol) 2-Fluor-5-nitro-toluol werden in 1.2 1 Tetrachlormethan gelöst und mit 214 g (1.20 mol) N-Brom-succinimid versetzt. 19.7 g (120 mmol) Azo-diisobutyronitril werden zugegeben und es wird unter Rückfluß erhitzt. Nach 16 h lässt man erkalten, filtriert und dampft im Vakuum zur Trockene ein. Der Rückstand wird in 300 ml Dichlormethan gelöst und mit 300 g Seesand versetzt. Dann wird erneut im Vakuum zur Trockene eingeengt und der Rückstand auf eine 1 kg Kieselgelsäule gegeben. Es wird mit einer 20:1 Mischung aus Cyclohexan und Essigsäureethylester chromatographiert und die Produktfraktionen werden im Vakuum zur Trockene eingedampft. Der Rückstand wird mit Cyclohexan kristallisiert und im Vakuum getrocknet. Man erhält 92 g (32% d. Th.) des gewünschten Produktes.
¹H-NMR (400 MHz, DMSO-*d*₆, δ/*ppm*): δ = 8.57-8.52 (m, 1H), 8.33-8.27 (m, 1H), 7.56 (t, 1H), 4.62 (s, 2H).
GC-MS (Methode 9): Rₜ = 7.79 min
MS (ESIpos): m/z = 154 (M-Br)⁺

### Beispiel 27A

### 1-Fluor-2-(methoxymethyl)-4-nitrobenzol

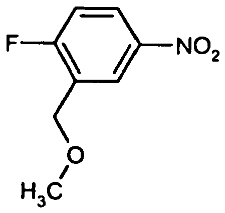

30 g (128 mmol) der Verbindung aus Beispiel 26A werden in 1.3 1 wasserfreiem Toluol gelöst und mit 45 g (192 mmol) Silber(I)oxid und 24.6 g (769 mmol) wasserfreiem Methanol versetzt. Es wird 16 h auf 60°C erhitzt. Dann lässt man erkalten und filtriert über Kieselgel. Das Produkt wird mit einem Gradienten von Cyclohexan und Cyclohexan/Essigsäureethylester 25:1 fraktioniert eluiert. Die Produktfraktionen werden im Vakuum zur Trockene eingedampft und im Vakuum getrocknet. Man erhält 17 g (72% d. Th.) des gewünschten Produktes.
¹H-NMR (400 MHz, DMSO-*d*₆, δ/*ppm*): δ = 8.41-8.36 (m, 1H), 8.22-8.16 (m, 1H), 7.26 (t, 1H), 4.58 (s, 2H), 3.49 (s, 3H).
GC-MS (Methode 9): Rt = 6.52 min
MS (ESIpos): m/z = 154 (M-OCH₃)⁺

### Beispiel 28A

### 3-Methoxy-1-[2-(methoxymethyl)-4-nitrophenyl]pyridin-2(1H)-on

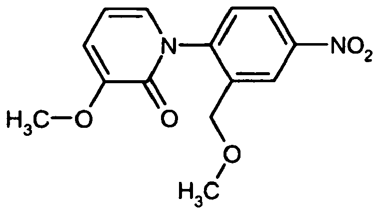

1.69 g (13.5 mmol) 3-Methoxy-Pyridon werden in 50 ml wasserfreiem Dimethylsulfoxid gelöst und mit 1.82 g (16.2 mmol) Kalium-tert.-butylat versetzt. Nach 1 h werden 2.5 g (13.5 mmol) Beispiel 27A gelöst in 10 ml wasserfreiem Dimethylsulfoxid zugegeben und es wird 3 h auf 80°C erhitzt. Man lässt erkalten und lässt weitere 48 h stehen, bevor die Reaktionslösung mit Essigsäureethylester verdünnt und mit Wasser und 1N Salzsäure gewaschen wird. Die organische Phase wird über Magnesiumsulfat getrocknet, filtriert und im Vakuum zur Trockene eingedampft. Der Rückstand wird mit tert.-Butylmethylether versetzt und die sich abscheidenden Kristalle werden abfiltriert und im Vakuum getrocknet. Man erhält 2.05 g (52% d. Th.) des gewünschten Produktes.
¹H-NMR (400 MHz, DMSO-*d*₆, δ/*ppm*): δ = 8.45 (d, 1H), 8.25 (dd, 1H), 7.6 (d, 1H), 7.15 (d, 1H), 6.9 (d, 1H), 6.3 (t, 1H), 4.25 (q, 2H), 3.75 (s, 3H) 3.2 (s, 3H).
LC-MS (Methode 4): Rₜ = 1.82 min
MS (ESIpos): m/z = 291 (M+H)⁺

### Beispiel 29A

### 1-[4-Amino-2-(methoxymethyl)phenyl]-3-methoxypyridin-2(1H)-on

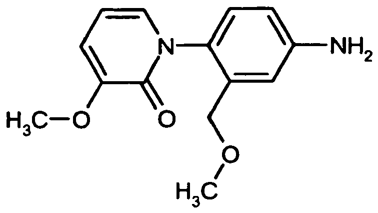

2.00 g (6.89 mmol) Beispiel 28A werden in 140 ml einer 1:1 Mischung aus Ethanol und Essigsäureethylester gelöst und mit 2.17 g (34.4 mmol) Ammoniumformiat und 200 mg Palladium auf Kohle versetzt. Es wird auf 80°C erhitzt. Nach 30 min lässt man erkalten und filtriert über Kieselgel. Es wird mit einem 1:1 Gemisch aus Ethanol und Essigsäureethylester nachgewaschen und das Filtrat im Vakuum zur Trockene eingedampft. Man erhält 1.90 g (99% d. Th.) des gewünschten Produktes.
LC-MS (Methode 4): Rₜ = 1.34 min
MS (ESIpos): m/z = 261 (M+H)⁺

### Beispiel 30A

### 1-[2-(Methoxymethyl)-4-nitrophenyl]-3-methylpyridin-2(1H)-on

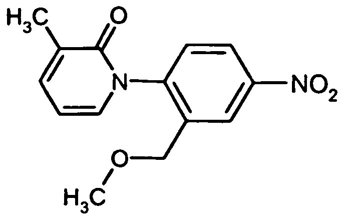

1.47 g (13.5 mmol) 3-Methyl-2-Pyridon werden in 50 ml wasserfreiem Dimethylsulfoxid gelöst und mit 1.82 g (16.2 mmol) Kalium-tert.-butylat versetzt. Nach 1 h werden 2.5 g (13.5 mmol) Beispiel 27A, gelöst in 10 ml wasserfreiem Dimethylsulfoxid zugegeben und es wird 3 h auf 80°C erhitzt. Man lässt erkalten und weitere 48 h stehen, bevor die Reaktionslösung mit Essigsäureethylester verdünnt und mit Wasser und 1N Salzsäure gewaschen wird. Die organische Phase wird über Magnesiumsulfat getrocknet, filtriert und im Vakuum zur Trockene eingedampft. Der Rückstand wird auf Kieselgel aufgezogen und an Kieselgel mit einer Mischung aus Cyclohexan und Essigsäureethylester 1:1 getrennt. Die Produktfraktionen werden vereinigt und im Vakuum zur Trockene eingedampft. Man erhält 2.05 g (55% d. Th.) des gewünschten Produktes.
¹H-NMR (400 MHz, DMSO-*d*₆, δ/*ppm*): δ = 8.45 (d, 1H), 8.25 (dd, 1H), 7.6 (d, 1H), 7.4 (m, 2H), 6.3 (t, 1H), 4.4-4.1 (q, 2H), 3.25 (s, 3H) 2.05 (s, 3H).

### Beispiel 31A

### 1-[4-Amino-2-(methoxymethyl)phenyl]-3-methylpyridin-2(1H)-on

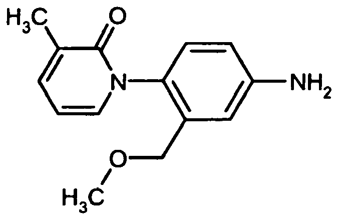

2.00 g (7.29 mmol) Beispiel 30A werden in 150 ml einer 1:1 Mischung aus Ethanol und Essigsäureethylester gelöst und mit 2.60 g (36.5 mmol) Ammoniumformiat und 200 mg Palladium auf Kohle versetzt. Es wird auf 80°C erhitzt. Nach 30 min lässt man erkalten und filtriert über Kieselgel. Es wird mit einem 1:1 Gemisch aus Ethanol und Essigsäureethylester nachgewaschen und das Filtrat im Vakuum zur Trockene eingedampft. Man erhält 1.81 g (99% d. Th.) des gewünschten Produktes.
LC-MS (Methode 4): Rₜ= 1.47 min
MS (ESIpos): m/z = 245 (M+H)⁺

### Beispiel 32A

### 2-(Ethoxymethyl)-1-fluor-4-nitrobenzol

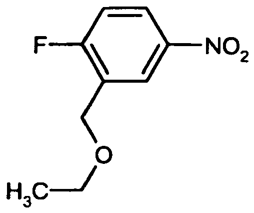

15 g (64.1 mmol) Beispiel 26A werden in 500 ml wasserfreiem Toluol gelöst und mit 22.3 g (96.1 mmol) Silber(I)oxid und 187 ml (3.20 mol) wasserfreiem Ethanol versetzt. Nach 96 h bei 60°C läßt man erkalten und filtriert über Kieselgel. Es wird mit Toluol nachgewaschen und das Filtrat im Vakuum zur Trockene eingedampft. Der Rückstand wird mit präparativer HPLC mit einem Gradienten aus Wasser und Acetonitril aufgereinigt. Die Produktfraktionen werden vereinigt und im Vakuum zur Trockene eingedampft. Man erhält 3.0 g (24% d. Th.) des gewünschten Produktes.
¹H-NMR (400 MHz, DMSO-*d*₆, δ/*ppm*): δ = 8.45 (m, 1H), 8.25 (m, 1H), 7.5 (t, 1H), 4.6 (s, 2H), 3.55 (q, 2H), 1.2 (t, 3H).
GC-MS (Methode 9): Rₜ = 6.91 min
MS (ESIpos): m/z = 154 (M-OC₂H₅)⁺

### Beispiel 33A

### 1-[2-(Ethoxymethyl)-4-nitrophenyl]-3-methylpyridin-2(1H)-on

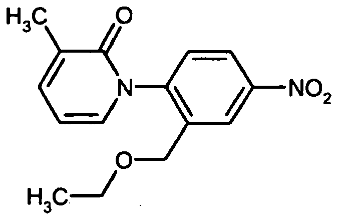

273 mg (2.51 mmol) 3-Methyl-2-pyridon werden in 10 ml wasserfreiem Dimethylsulfoxid gelöst und mit 338 g (3.01 mmol) Kalium-tert.-butylat versetzt. Nach 1 h werden 500 mg (2.51 mmol) Beispiel 32A zugegeben und es wird 16 h auf 80°C erhitzt. Man lässt erkalten und versetzt mit gesättigter Natriumchloridlösung. Die Mischung wird mehrmals mit Dichlormethan extrahiert, die organische Phase zweimal mit Wasser und zweimal mit gesättigter Natriumchloridlösung gewaschen, über Natriumsulfat getrocknet, filtriert und im Vakuum zur Trockene eingedampft. Der Rückstand wird in 5 ml Dimethylsulfoxid gelöst und mit präparativer HPLC mit einem Gradienten aus Wasser und Acetonitril aufgereinigt. Die Produktfraktionen werden vereinigt und im Vakuum zur Trockene eingedampft. Man erhält 430 mg (59% d. Th.) des gewünschten Produktes.
¹H-NMR (400 MHz, DMSO-*d₆*, δ/*ppm*): δ= 8.4 (d, 1H), 8.3 (dd, 1H), 7.6 (d, 1H), 7.45 (m, 2H), 6.3 (t, 1H), 4.4-4.1 (q, 2H), 3.4 (m, 2H) 2.05 (s, 3H), 1.1 (t, 3H).
LC-MS (Methode 4) Rₜ = 2.04 min
MS (ESIpos): m/z = 289 (M+H)⁺

### Beispiel 34A

### 1-[4-Amino-2-(ethoxymethyl)phenyl]-3-methylpyridin-2(1H)-on

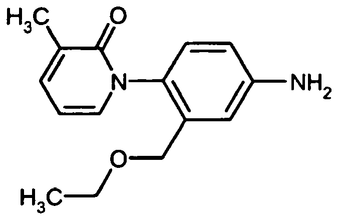

425 mg (1.47 mmol) Beispiel 33A werden in 30 ml einer 1:1 Mischung aus Ethanol und Essigsäureethylester gelöst und mit 465 mg (7.37 mmol) Ammoniumformiat und 50 mg Palladium auf Kohle versetzt. Es wird auf 80°C erhitzt. Nach 30 min lässt man erkalten und filtriert über Kieselgel. Es wird mit Essigsäureethylester nachgewaschen und das Filtrat im Vakuum zur Trockene eingedampft. Man erhält 388 mg (99% d. Th.) des gewünschten Produktes.
LC-MS (Methode 4): Rₜ = 1.28 min
MS (ESIpos): m/z = 259 (M+H)⁺

### Beispiel 35A

### (2-Fluor-5-nitrobenzyl)(triphenyl)phosphonium-bromid

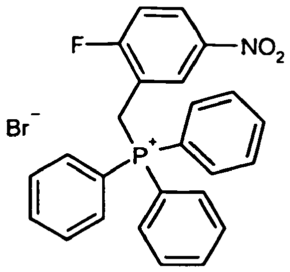

20 g (85.5 mmol) der Verbindung aus Beispiel 26A werden in 250 ml wasserfreiem Toluol gelöst und mit 22.4 g (85.5 mmol) Triphenylphosphin versetzt. Die Lösung wird 16 h unter Rückfluss erhitzt, wobei sich ein Niederschlag abscheidet. Man lässt erkalten und filtriert den Niederschlag ab. Nach Waschen mit Diethylether wird im Vakuum getrocknet. Man erhält 39 g (92% d. Th.) des gewünschten Produktes.
¹H-NMR (400 MHz, DMSO-*d₆*, *δ*/*ppm*): δ = 8.30-8.23 (m, 1H), 7.98-7.88 (m, 4H), 7.81-7.70 (m, 12H), 7.45 (t, 1H), 5.32 (d, 2H).

### Beispiel 36A

### 1-Fluor-4-nitro-2-[prop-1-en-1-yl]benzol

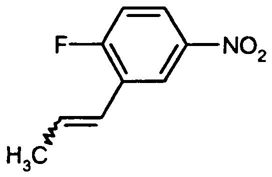

Bei 10°C werden zu einer Lösung von 13.5 g (27.3 mmol) der Verbindung aus Beispiel 35A in 145 ml Dioxan 5.99 g (32.7 mmol) Natriumbis(trimethylsiliy)amid getropft. Es wird 1 h bei dieser Temperatur gerührt. Dann wird eine Lösung von 2.40 g (54.5 mmol) Acetaldehyd in 5 ml Dioxan zugegeben und die Reaktion 1 h bei RT gerührt, anschließend mit 400 ml Wasser versetzt, dreimal mit Dichlormethan extrahiert und die vereinigten organischen Phasen zweimal mit gesättigter wässriger Natriumchlorid-Lösung gewaschen. Nach Trocknen über Natriumsulfat und anschließender Filtration wird das Lösungsmittel im Vakuum entfernt. Man reinigt mittels Chromatographie an Kieselgel (Eluens: Cyclohexan/Essigsäureethylester = 40:1). Es werden 5.2 g (100% d. Th.) des gewünschten Produktes als E/Z-Isomeren-Gemisch erhalten.
¹H-NMR (400 MHz, DMSO-d₆, *δ*/ppm): δ = 8.47-8.05 (m, 2H), 7.58-7.42 (m, 1H), 6.70-6.05 (m, 2H), 1.90-1.78 (m, 3H).
GC-MS (Methode 10): Rₜ = 2.64 und 2.70 min
MS (ESIpos): m/z = 181 (M+H⁺)⁺

### Beispiel 37A

### 1-{4-Nitro-2-[prop-1-en-1-yl]phenyl}pyridin-2(1H)-on

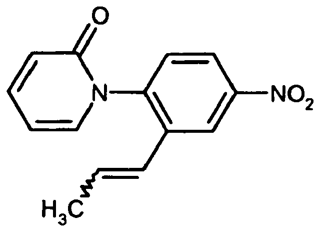

223 mg (2.35 mmol) Pyridin-2(1H)-on werden in 10 ml wasserfreiem Dimethylsulfoxid gelöst und mit 315 mg (2.82 mmol) Kalium-tert.-butylat versetzt. Nach 1 h werden 500 mg (2.76 mmol) Beispiel 36A zugegeben und es wird 15 h auf 80°C erhitzt. Man lässt erkalten und verdünnt mit Wasser und gesättigter Natriumchloridlösung. Die Lösung wird mehrmals mit Dichlormethan extrahiert. Die vereinigten organischen Phasen werden mit Wasser und mit gesättigter Natriumchloridlösung gewaschen, über Natriumsulfat getrocknet, filtriert und im Vakuum zur Trockene eingedampft. Der Rückstand wird mit präparativer HPLC mit einem Gradienten aus Wasser und Acetonitril aufgereinigt. Die Produktfraktionen werden vereinigt und im Vakuum zur Trockene eingedampft. Man erhält 103 mg (17% d. Th.) des gewünschten Produktes.
¹H-NMR (400 MHz, DMSO-*d₆, δ*/ppm): δ = 8.5 (d, 1H), 8.2 (dd, 1H), 7.65-7.50 (m, 3H), 6.65-6.50 (m, 2H), 6.4 (t, 1 H), 6.0 (d, 1H), 1.8 (dd, 3H).
LC-MS (Methode 5): Rₜ = 1.88 min
MS (ESIpos): m/z = 257 (M+H)⁺

### Beispiel 38A

### 1-(4-Amino-2-propylphenyl)pyridin-2(1H)-on

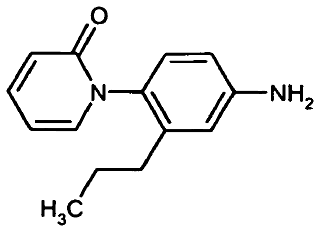

103 mg (0.40 mmol) Beispiel 37A werden in 10 ml einer 1:1 Mischung aus Ethanol und Essigsäureethylester gelöst und im Durchfluss in einem H-Cube (Fa. Thales-Nanotechnologies, Ungarn) bei Normaldruck an Palladium auf Kohle hydriert. Die erhaltene Lösung wird im Vakuum zur Trockene eingedampft. Man erhält 90 mg (83% d. Th.) des gewünschten Produktes.
LC-MS (Methode 7): Rₜ = 2.77 min
MS (ESIpos): m/z = 229 (M+H)⁺

### Beispiel 39A

### 1-(2-Methyl-4-nitrophenyl)-3-(trifluormethyl)pyridin-2(1H)-on

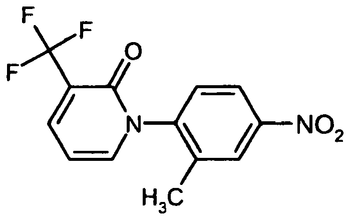

2.5 g (15.3 mmol) 3-(Trifluormethyl)pyridin-2(1H)-on werden in 40 ml Dimethylsulfoxid gelöst und bei Raumtemperatur mit 2.58 g (22.9 mmol) Kalium-tert.-butylat versetzt. Die Suspension wird 30 min bei Raumtemperatur gerührt, bevor 2.6 g (16.9 mmol) 1-Fluor-2-methyl-4-nitrobenzol zugesetzt werden und die Reaktionslösung für 20 h auf 80°C erhitzt wird. Es wird vorsichtig mit Wasser verdünnt. Die Lösung wird mehrmals mit Dichlormethan extrahiert. Die vereinigten organischen Extrakte werden mit Wasser und gesättigter Natriumchloridlösung gewaschen, über Magnesiumsulfat getrocknet, filtriert und im Vakuum eingedampft. Man löst den Rückstand in Acetonitril an reinigt mit präparativer HPLC mit einem Gradienten aus Wasser und Acetonitril auf. Die Produktfraktionen werden vereinigt und im Vakuum zur Trockene eingedampft. Man erhält 1.87 g (41% d. Th.) des gewünschten Produktes.
¹H-NMR (400 MHz, DMSO-*d₆*, *δ*/*ppm*): 8.4 (d, 1H), 8.2 (dd, 1H), 8.15 (d, 1H), 8.0 (dd, 1H), 7.7 (d, 1H), 6.55 (t, 1H), 2.2 (s, 3H).
LC-MS (Methode 6): Rₜ = 2.26 min
MS (ESIpos): m/z = 488 (M+H)⁺

### Beispiel 40A

### 1-(4-Amino-2-methylphenyl)-3-(trifluormethyl)pyridin-2(1H)-on

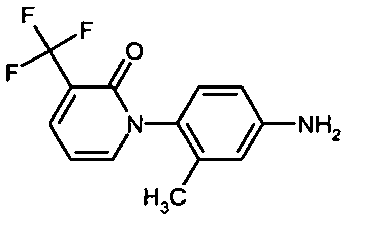

800 mg (2.68 mmol) Beispiel 39A werden in 108 ml einer 1:1 Mischung aus Ethanol und Essigsäureethylester gelöst und mit 1.02 g (16.1 mmol) Ammoniumformiat und 50 mg Palladium auf Kohle versetzt. Es wird auf 80°C erhitzt. Nach 45 min lässt man erkalten und filtriert über Kieselgel. Es wird mit Essigsäureethylester nachgewaschen und das Filtrat im Vakuum zur Trockene eingedampft. Man erhält 780 mg (100% d. Th.) des gewünschten Produktes.
¹H-NMR (400 MHz, DMSO-*d₆*, *δ*/*ppm*): δ = 8.05 (d, 1H), 7.8 (d, 1H), 6.9 (d, 1H), 6.5-6.3 (m, 3H), 5.3 (s, 2H), 1.8 (s, 3H).
LC-MS (Methode 3): Rₜ= 1.42 min
MS (ESIpos): m/z = 269 (M+H)⁺

### Beispiel 41A

### 3-Brom-1-(2-methyl-4-nitrophenyl)pyridin-2(1H)-on

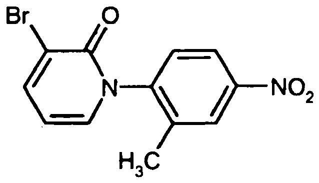

44.5 g (280 mmol) 3-Brompyridin-2(1H)-on werden in 750 ml wasserfreiem Dimethylsulfoxid gelöst und bei Raumtemperatur portionsweise mit 33.4 g (298 mmol) Kalium-tert-butylat versetzt. Die Suspension wird 1 h bei dieser Temperatur gerührt, bevor 38.5 g (280 mmol) 1-Fluor-2-methyl-4-nitrobenzol zugesetzt werden und die Reaktionslösung für 20 h auf 80°C erhitzt wird. Man läßt erkalten und es wird vorsichtig mit Wasser verdünnt. Der ausgefallene kristalline Niederschlag wird abfiltriert, mit etwas Wasser gewaschen und im Vakuum getrocknet. Es werden 62 g (80% d. Th.) des gewünschten Produktes erhalten.
¹H-NMR (400 MHz, DMSO-d₆, δ/*ppm*): δ = 8.34 (d, 1H), 8.21 (dd, 1H), 8.10 (dd, 1H), 7.71-7.63 (m, 2H), 6.36 (t, 1H), 2.17 (s, 3H).
LC-MS (Methode 3): Rₜ = 1.72 min
MS (ESIpos): m/z = 309 (M+H)⁺

### Beispiel 42A

### 1-(2-Methyl-4-nitrophenyl)-3-vinylpyridin-2(1H)-on

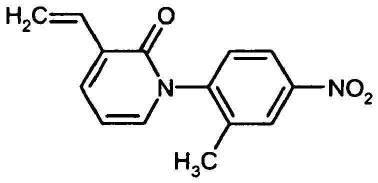

50 g (162 mmol) der Verbindung aus Beispiel 41A werden in 700 ml wasserfreiem Dioxan gelöst und mit 62 g (194 mmol) Tributylvinyl-zinn und 4.7 g (4.0 mmol) Tetrakis(triphenylphosphin)-palladium versetzt und es wird 15 h am Rückfluss erhitzt. Man lässt erkalten und filtriert über Kieselgur. Es wird mit Essigsäureethylester nachgewaschen und die vereinigten Filtrate werden im Vakuum zur Trockene eingeengt. Der Rückstand wird auf Kieslegel aufgezogen und an 800 g Kieselgel mit einem Gradienten aus Cyclohexan und Essigsäureethylester chromatographiert. Es werden 27 g (62% d. Th.) des gewünschten Produktes erhalten.
¹H-NMR (400 MHz, DMSO-d₆, *δ*/*ppm*): δ = 8.35 (d, 1H), 8.2 (dd, 1H), 7.75 (dd, 1H), 7.60 (d, 1H), 7.55 (dd, 1H), 6.75 (dd, 1H), 6.45 (t, 1H), 6.15 (dd, 1H), 5.30 (dd, 1H), 2.17 (s, 3H).
LC-MS (Methode 4): Rₜ = 1.86 min
MS (ESIpos): m/z = 257 (M+H)⁺

### Beispiel 43A

### 3-(2-Hydroxyethyl)-1-(2-methyl-4-nitrophenyl)pyridin-2(1H)-on

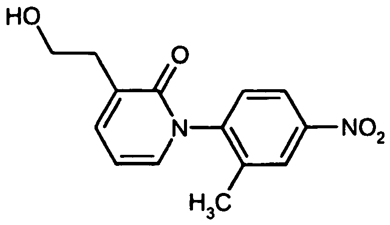

38 g (148 mmol) der Verbindung aus Beispiel 42A werden unter Eiskühlung innerhalb von 45 min mit einer Lösung von 40 g (326 mmol) 9-Borabicyclo[3.3.1]nonan in 650 ml Tetrahydrofuran versetzt. Bei dieser Temperatur wird eine weitere Stunde nachgerührt, bevor mit einer Lösung von 30 g (747 mmol) Natriumhydroxid in 740 ml Wasser innerhalb von 15 min versetzt wird. Es werden 151 ml einer 30%igen Wasserstoffperoxidlösung so zugegeben, dass die Temperatur 30°C nicht übersteigt. Nach beendeter Zugabe wird die Kühlung entfernt und es werden weitere 30 min nachgerührt. Es wird mehrmals mit Essigsäureethylester extrahiert. Die vereinigten organischen Phasen werden mit einer Lösung aus 780 g (1.63 mol) Natriumdisulfit gewaschen, die organische Phase abgetrennt und die wässrige Phase erneut mit Essigsäureethylester extrahiert. Die vereinigten organischen Phasen werden mit gesättigter Natriumchloridlösung gewaschen, über Magnesiumsulfat getrocknet und im Vakuum zur Trockene eingedampft. Der Rückstand wird auf Kieselgel aufgezogen und mit einem Gradienten aus Cyclohexan und Essigsäureethylester chromatographiert. Die produkthaltigen Fraktionen werden vereinigt und im Vakuum zur Trockene eingeengt. Es werden 38 mg (93% d. Th.) des gewünschten Produktes erhalten.
¹H-NMR (400 MHz, DMSO-d₆, *δ*/ppm): δ = 8.33 (d, 1H), 8.18 (d, 1H), 7.57 (d, 1H), 7.48-7.40 (m, 2H), 6.33 (t, 1H), 4.58 (t, 1H), 3.62-3.50 (m, 2H), 2.62 (t, 2H), 2.15 (s, 3H).
LC-MS (Methode 6): Rₜ = 1.57 min
MS (ESIpos): m/z = 275 (M+H)⁺

### Beispiel 44A

### 3-(2-Ethoxyethyl)-1-(2-methyl-4-nitrophenyl)pyridin-2(1H)-on

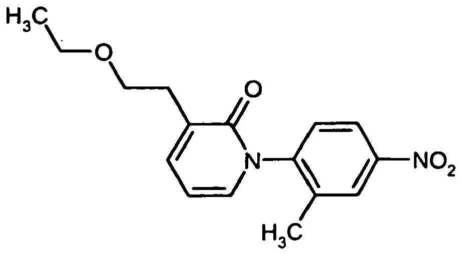

135 mg (0.49 mmol) Beispiel 43A werden in 1 ml wasserfreiem N,N-Dimethylformamid gelöst und mit 24 mg (0.59 mmol) Natriumhydrid versetzt. Man läßt weitere 30 min rühren bevor 153 mg (1 mmol) Jodethan zugegeben werden. Nach 3 h werden erneut 48 mg (1.21 mmol) Natriumhydrid und 307 mg (1.97 mmol) Iodethan zugegeben bevor weitere 30 min nachgerührt wird. Es wird mit Wasser versetzt, filtriert, und das Filtrat wird mit präparativer HPLC mit einem Gradienten aus Wasser und Acetonitril aufgereinigt. Die Produktfraktionen werden vereinigt und im Vakuum zur Trockene eingedampft. Man erhält 92 mg (62% d. Th.) des gewünschten Produktes.
¹H-NMR (400 MHz, DMSO-*d₆* δ/*ppm*): δ = 8.35 (d, 1H), 8.2 (dd, 1H), 7.6 (d, 1H), 7.45 (m, 2H), 6.35 (t, 1H), 3.6 (t, 2H), 3.4 (q, 2H), 2.7 (t, 2H), 2.15 (s, 3H), 1.1 (t, 3H).
LC-MS (Methode 5): Rₜ = 2.00 min
MS (ESIpos): m/z = 303 (M+H)⁺

### Beispiel 45A

### 1-(4-Amino-2-methylphenyl)-3-(2-ethoxyethyl)pyridin-2(1H)-on

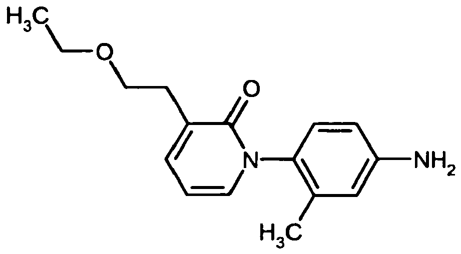

90 mg (0.30 mmol) Beispiel 44A werden in 12 ml einer 1:1 Mischung aus Ethanol und Essigsäureethylester gelöst und mit 113 mg (1.79 mmol) Ammoniumformiat und 50 mg Palladium auf Kohle versetzt. Es wird auf 80°C erhitzt. Man lässt nach 1 h erkalten und filtriert über Kieselgel. Es wird mit Essigsäureethylester nachgewaschen und das Filtrat im Vakuum zur Trockene eingedampft. Man erhält 80 mg (99% d. Th.) des gewünschten Produktes.
¹H-NMR (400 MHz, DMSO-*d₆ δlppm*): δ = 7.4 (d, 1H), 7.3 (dd, 1H), 6.8 (d, 1H), 6.5-6.4 (m, 2H), 6.2 (t, 1H), 5.2 (s,2H), 3.55 (t, 2H), 3.45 (q, 2H), 2.65 (t, 2H), 1.8 (s, 3H); 1.1 (t, 3H).
LC-MS (Methode 3): Rₜ = 1.30 min
MS (ESIpos): m/z=273 (M+H)⁺

### Beispiel 46A

### 3-(2-Methoxyethyl)-1-(2-methyl-4-nitrophenyl)pyridin-2(1H)-on

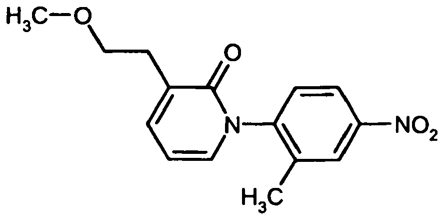

135 mg (0.49 mmol) Beispiel 43A werden in 1 ml wasserfreiem N,N-Dimethylformamid gelöst und mit 24 mg (0.59 mmol) Natriumhydrid versetzt. Man läßt weitere 30 min rühren bevor 139 mg (1 mmol) Jodmethan zugegeben werden. Nach 3 h werden erneut 48 mg (1.21 mmol) Natriumhydrid und 278 mg (1.97 mmol) Iodmethan zugegeben und es wird weitere 30 min nachgerührt. Dann wird mit Wasser versetzt, filtriert, und das Filtrat wird mit präparativer HPLC mit einem Gradienten aus Wasser und Acetonitril aufgereinigt. Die Produktfraktionen werden vereinigt und im Vakuum zur Trockene eingedampft. Man erhält 94 mg (66% d. Th.) des gewünschten Produktes.
¹H-NMR (400 MHz, DMSO-*d₆*, *δ*/*ppm*): δ = 8.3 (d, 1H), 8.15 (dd, 1H), 7.6 (d, 1H), 7.45 (d, 2H), 6.35 (t, 1H), 3.55 (t, 2H), 3.3 (s, 3H), 2.7 (t, 2H), 2.15 (s, 3H).
LC-MS (Methode 5): Rₜ = 1.82 min
MS (ESIpos): m/z = 289 (M+H)⁺

### Beispiel 47A

### 1-(4-Amino-2-methylphenyl)-3-(2-methoxyethyl)pyridin-2(1H)-on

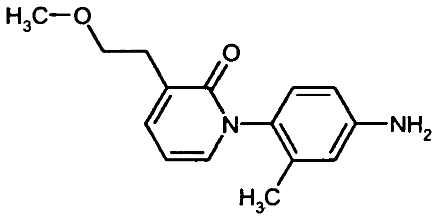

93 mg (0.32 mmol) Beispiel 46A werden in 13 ml einer 1:1 Mischung aus Ethanol und Essigsäureethylester gelöst und mit 122 mg (1.94 mmol) Ammoniumformiat und 50 mg Palladium auf Kohle versetzt. Es wird auf 80°C erhitzt. Nach 1 h lässt man erkalten und filtriert über Kieselgel. Es wird mit Essigsäureethylester nachgewaschen und das Filtrat im Vakuum zur Trockene eingedampft. Man erhält 83 mg (93% d. Th.) des gewünschten Produktes.
¹H-NMR (400 MHz, DMSO-*d₆*, *δ*/*ppm*): δ = 7.35 (d, 1H), 7.3 (dd, 1H), 6.8 (d, 1H), 6.5-6.4 (m, 2H), 6.2 (t, 1H), 5.2 (s, 2H), 3.55 (t, 2H), 3.35 (s, 3H), 2.65 (t, 2H), 1.8 (s, 3H).
LC-MS (Methode 3): Rₜ = 1.12 min
MS (ESIpos): m/z = 259 (M+H)⁺

### Beispiel 48A

### 1-(2-Fluorpyridin-3-yl)propan-1-ol

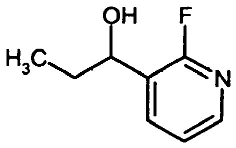

Man kühlt eine frisch bereitete LDA-Lösung (68.0 mmol) in 500 ml THF auf-75°C und setzt 6.00 g (61.8 mmol) 2-Fluorpyridin hinzu. Man läßt 2 h bei dieser Temperatur rühren, wobei sich eine Suspension bildet. Man gibt im Anschluß 4.31 g (74.2 mmol) Propanal hinzu, wobei die Innentemperatur auf -45°C ansteigt. Nach 2 h läßt man auf RT kommen, gießt auf gesättigte Ammoniumchlorid-Lösung und extrahiert dreimal mit *tert*-Butylmethylether. Die vereinten organischen Phasen werden mit Natriumchlorid-Lösung gewaschen, über Natriumsulfat getrocknet und eingeengt. Man reinigt durch Chromatographie an Kieselgel (Eluens Dichlormethan, dann Dichlormethan/Methanol = 25:1). Man erhält 8.00 g (76% d. Th.) der Titelverbindung.
¹H-NMR (400 MHz, DMSO-*d₆*, *δ*/*ppm*): 8.13-8.09 (m, 1H), 7.99 (ddd, 1H), 7.36 (ddd, 1H), 5.42 (d, 1H), 4.68 (dq, 1H), 1.72-1.56 (m, 2H), 0.85 (t, 3H).
HPLC (Methode 4): Rₜ = 1.50 min.
MS (ESIpos, *m*/*z*): 156 (M+H)⁺.

### Beispiel 49A

### 1-(2-Fluorpyridin-3-yl)propylacetat

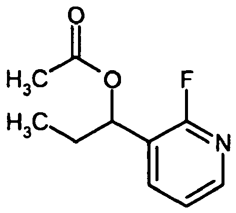

2.75 g (17.7 mmol) des Produktes aus Beispiel 48A werden in 12 ml Eisessig gelöst und mit 4 ml konzentrierter Schwefelsäure versetzt. Man erhitzt 30 min lang auf 130°C und rührt nach dem Abkühlen in Eiswasser ein. Man stellt mit Natronlauge einen pH-Wert von 8 ein und extrahiert dann mit Dichlormethan. Die vereinten organischen Phasen werden über Natriumsulfat getrocknet und vorsichtig eingeengt. Man erhält 3.30 g (94% d. Th.) der Titelverbindung, die ohne Aufreinigung weiter umgesetzt wird.
¹H-NMR (400 MHz, DMSO-*d₆, δ*/*ppm*): 8.21-8.18 (m, 1H), 7.97 (ddd, 1H), 7.39 (ddd, 1H), 5.71 (t, 1H), 2.07 (s, 3H), 1.90-1.77 (m, 2H), 0.85 (t, 3H).
HPLC (Methode 4): Rₜ = 2.16 min.
MS (ESIpos, *m*/*z*): 198 (M+H)⁺.

### Beispiel 50A

### 3-Propyl-2-fluorpyridin

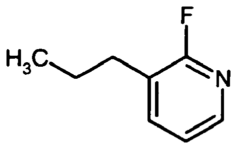

3.50 g (17.7 mmol) des Produktes aus Beispiel 49A werden in 30 ml Ethanol gelöst und mit 2.00 g 10%igem Palladium auf Aktivkohle versetzt. Man hydriert über Nacht in einer Wasserstoffatmosphäre unter normalem Druck. Man saugt ab, wäscht mit Ethanol nach und engt vorsichtig im Vakuum ein. Man erhält 1.9 g ca. 89%iges Produkt (69% d. Th.), das ohne Aufreinigung weiter umgesetzt wird.
¹H-NMR (400 MHz, DMSO-*d₆*, *δ*/*ppm*): 8.08-8.05 (m, 1H), 7.84 (ddd, 1H), 7.28 (ddd, 1H), 2.58 (t, 2H), 1.63-1.54 (m, 2H), 0.90 (t, 3H).
HPLC (Methode 6): Rₜ = 3.54 min.
MS (ESIpos, *m*/*z*): 140 (M+H)⁺.

### Beispiel 51A

### 3-Propylpyridin-2(1H)-on

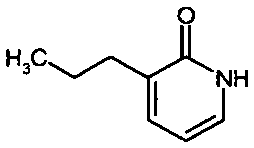

Die Titelverbindung wird analog zu Beispiel 55A aus 1.90 g (13.7 mmol) des Produktes aus Beispiel 50A synthetisiert. Man erhält 1.50 g (80% d. Th.) der gewünschten Verbindung.
¹H-NMR (400 MHz, DMSO-*d*_{*6*,} *δ*/*ppm*): 11.42 (s, breit, 1H), 7.23 (dd, 1H), 7.19 (dd, 1H), 6.09 (t, 1H), 2.33 (t, 2H), 1.55-1.44 (m, 2H), 0.87 (t, 3H).
HPLC (Methode 6): Rₜ = 2.72 min.
MS (ESIpos, *m*/*z*): 138 (M+H)⁺.

### Beispiel 52A

### 1-(2-Methoxy-4-nitrophenyl)-3-propylpyridin-2(1H)-on

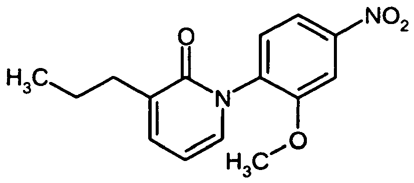

500 mg (3.65 mmol) des Produktes aus Beispiel 51A werden in 7.3 ml DMF gelöst und auf 0°C gekühlt. Man gibt 613 mg (5.47 mmol) Kalium-*tert*-butylat hinzu und lässt 30 min bei RT nachrühren. Dann werden 624 mg (3.65 mmol) 1-Fluor-2-methoxy-4-nitrobenzol hinzugefügt. Man rührt 6 h bei RT, setzt Wasser hinzu und extrahiert viermal mit Essigsäureethylester. Die vereinten organischen Phasen werden über Natriumsulfat getrocknet und eingeengt. Man reinigt durch präparative HPLC. Die erhaltene Produktfraktion wird durch Chromatographie an Kieselgel (Eluens: Dichlormethan, dann Dichlormethan/Methanol 10:1) weiter aufgereinigt. Man erhält 158 mg (15% d. Th.) der gewünschten Verbindung als Feststoff.
¹H-NMR (400 MHz, DMSO-*d₆*, *δ*/*ppm*): 7.97 (d, 1H), 7.93 (dd, 1H), 7.61 (d, 1H), 7.40-7.33 (m, 2H), 6.26 (t, 1H), 3.89 (s, 3H), 2.43-2.36 (m, 2H), 1.60-1.48 (m, 2H), 0.91 (t, 3H).
HPLC (Methode 5): Rₜ = 2.19 min.
MS (ESIpos, *m*/*z*): 289 (M+H)⁺.

### Beispiel 53A

### 1-(4-Amino-2-methoxyphenyl)-3-propylpyridin-2(1H)-on

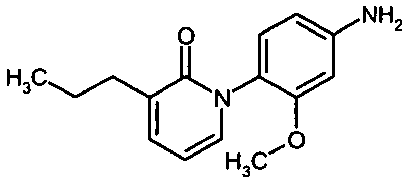

130 mg (0.45 mmol) des Produktes aus Beispiel 52A werden in 4 ml THF vorgelegt. Man gibt 50 mg 10%iges Palladium auf Aktivkohle hinzu und hydriert 1 h bei normalem Druck in einer Wasserstoff-Atmosphäre. Die Reaktionslösung wird dann durch Celite filtriert, das mit Essigsäureethylester nachgespült wird. Man engt im Vakuum ein und erhält 115 mg (99% d. Th.) der kristallinen Titelverbindung.
¹H-NMR (400 MHz, DMSO-*d₆*, *δ*/*ppm*): 7.25-7.19 (m, 2H), 6.80 (d, 1H), 6.32 (d, 1H), 6.17 (dd, 1H), 6.11 (t, 1H), 5.75 (s, breit, 2H), 3.62 (s, 3H), 2.40-2.32 (m, 2H), 1.56-1.46 (m, 2H), 0.90 (t, 3H).
HPLC (Methode 3): Rₜ = 1.56 min.
MS (ESIpos, *m*/*z*): 259 (M+H)⁺.

### Beispiel 54A

### 2-Fluor-3-(2-methoxy-4-nitrophenyl)pyridin

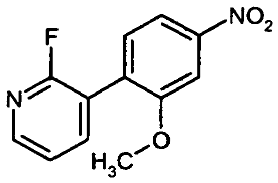

2.00 g (7.17 mmol) 2-Iod-5-nitroanisol, 2.02 g (14.3 mmol) (2-Fluorpyridin-3-yl)boronsäure (A. Bouillon, J.-C. Lancelot, V. Collot, P. R. Bovy, S. Rault, Tetrahedron 2002, 58, 3323-3328.), 1.81 g (21.5 mmol) Natriumhydrogencarbonat und 117 mg (0.14 mmol) [1,1'-Bis(diphenylphosphino)-ferrocen]-palladiumdichlorid werden in einer entgasten Mischung aus 40 ml Dioxan und 10 ml Wasser 1 h auf 100°C erhitzt. Man verdünnt nach dem Abkühlen mit Wasser und extrahiert dreimal mit Essigsäureethylester. Die vereinten organischen Phasen werden über Natriumsulfat getrocknet, über Aktivkohle geklärt und eingeengt. Man reinigt durch Chromatographie an Kieselgel (Eluens: Dichlormethan) und erhält 1.55 g (87% d. Th.) des gewünschten Produktes.
¹H-NMR (400 MHz, DMSO-d₆, *δ*/ppm): 3.32 (d, 1H), 8.02 (ddd, 1H), 7.95 (dd, 1H), 7.92 (d, 1H), 7.64 (d, 1H), 7.49 (ddd, 1H), 4.10 (s, 3H).
HPLC (Methode 6): Rₜ = 3.61 min.
MS (ESIpos, *m*/*z*): 249 (M+H)⁺.

### Beispiel 55A

### 3-(2-Methoxy-4-nitrophenyl)pyridin-2(1H)-on

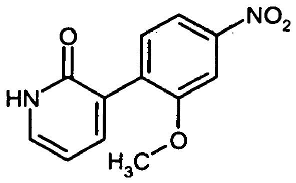

Man erhitzt 1.50 g (6.04 mmol) des Produkts aus Beispiel 54A über Nacht in einer Mischung aus 75 ml Dioxan und 75 ml 4-molarer Salzsäure. Dann stellt man mit Natriumcarbonatlösung alkalisch. Der Niederschlag wird abgesaugt, mit Wasser nachgewaschen und getrocknet. Man erhält 1.43 g (96% d. Th.) der gewünschten Verbindung.
¹H-NMR (400 MHz, DMSO-*d₆,* δ/*ppm*): 11.82 (s, breit, 1H), 7.85 (dd, 1H), 7.82 (d, 1H), 7.57 (d, 1H), 7.52 (dd, 1H), 7.44 (dd, 1H), 6.28 (t, 1H), 3.86 (s, 3H).
HPLC (Methode 3): Rₜ = 1.40 min.
MS (ESIpos, *m*/*z*): 247 (M+H)⁺.

### Beispiel 56A

### 1-Ethyl-3-(2-methoxy-4-nitrophenyl)pyridin-2(1H-on

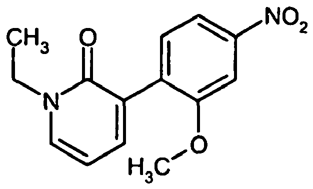

350 mg (1.42 mmol) des Produktes aus Beispiel 55A werden in 3.5 ml DMF suspendiert. Man setzt 393 mg (2.84 mmol) Kaliumcarbonat und 266 mg (1.71 mmol) Iodethan hinzu und läßt 3 h bei RT rühren. Dann wird mit Wasser versetzt und mit Dichlormethan extrahiert. Man trocknet die organische Lösung über Natriumsulfat, engt ein und reingt durch präparative HPLC. Man erhält 224 mg (57% d. Th.) der Titelverbindung.
¹H-NMR (400 MHz, DMSO-*d₆*, *δ*/*ppm*): 7.87-7.81 (m, 2H), 7.78 (d, 1H), 7.56 (d, 1H), 7.48 (dd, 1H), 6.33 (t, 1H), 3.96 (q, 2H), 3.86 (s, 3H), 1.25 (t, 3H).
HPLC (Methode 5): Rₜ = 1.88 min.
MS (ESIpos, *mlz*): 275 (M+H)⁺.

### Beispiel 57A

### 3-(4-Amino-2-methoxyphenyl)-1-ethylpyridin-2(1H)-on BAY 821292, SIRO3378

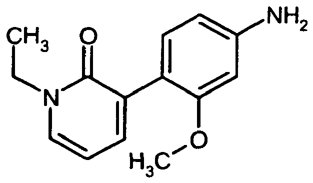

196 mg (0.72 mmol) des Produktes aus Beispiel 56A werden analog Beispiel 53A hydriert. Man erhält 184 mg (98% d. Th.) der Titelverbindung.
¹H-NMR (400 MHz, DMSO-*d₆*, δ/*ppm*): 7.57 (dd, 1H), 7.24 (dd, 1H), 6.91 (d, 1H), 6.25 (d, 1H), 6.19 (t, 1H), 6.14 (dd, 1H), 5.14 (s, 2H), 3.91 (q, 2H), 3.61 (s, 3H), 1.21 (t, 3H).
HPLC (Methode 3): Rₜ = 0.92 min.
MS (ESIpos, *m*/*z*): 244 (M+H)⁺.

### Beispiel 58A

### 3-(2-Methoxy-4-nitrophenyl)-1-methylpyridin-2(1H)-on

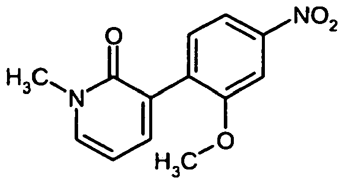

350 mg (1.42 mmol) des Produktes aus Beispiel 55A werden analog Beispiel 56A mit 242 mg (1.71 mmol) Methyliodid alkyliert. Man erhält 347 mg (88% d. Th.) der Titelverbindung.
¹H-NMR (400 MHz, DMSO-d₆, *δ*/*ppm*): 7.85 (d, 1H), 7.82 (d, 1H), 7.78 (dd, 1H), 7.54 (d, 1H), 7.48 (dd, 1H), 6.31 (t, 1H), 3.85 (s, 3H), 3.48 (s, 3H).
HPLC (Methode 4): Rₜ = 1.77 min.
MS (ESIpos, *m*/*z*): 261 (M+H)⁺.

### Beispiel 59A

### 3-(4-Amino-2-methoxyphenyl)-1-methylpyridin-2(1H)-on

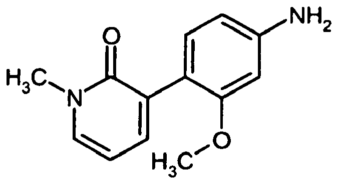

320 mg (1.23 mmol) des Produktes aus Beispiel 58A werden analog Beispiel 53A hydriert. Man erhält 320 mg (85% Reinheit, 96% d. Th.) der Titelverbindung.
¹H-NMR (400 MHz, *DMSO-d₆, δlppm*): 7.57 (dd, 1H), 7.25 (dd, 1H), 6.89 (d, 1H), 6.25 (d, 1H), 6.18 (t, 1H), 6.14 (dd, 1H), 5.14 (s, 2H), 3.61 (s, 3H), 3.43 (s, 3H).
HPLC (Methode 3): Rₜ = 0.62 min.
MS (ESIpos, *m*/*z*): 231 (M+H)⁺.

### Beispiel 60A

### 1-Methyl-3-(2-methyl-4-nitrophenyl)tetrahydropyrimidin-2(1H)-on

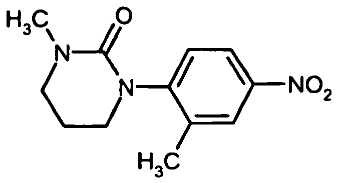

16.1 g (76.1 mmol) Kaliumphosphat und 0.362 g (1.90 mmol) Kupfer(I)iodid werden vorgelegt, die Apparatur ausgeheizt und anschließend mit Argon gespült. 10.0 g (38.1 mmol) 1-Iod-2-methyl-4-nitrobenzol, 6.51 g (57.0 mmol) 1-Methyltetrahydropyrimidin-2(1H)-on und 0.394 g (3.80 mmol) N,N'-Dimethylendiamin in 200 ml Dioxan werden zugegeben und es wird bei 110°C gerührt. Nach 19 h wird nach Abkühlen über Kieselgur abgesaugt und dann dreimal mit 100 ml Essigsäureethylester gewaschen. Die Lösungsmittel wird im Vakuum entfernt und der Rückstand durch Chromatographie an Kieselgel (Cyclohexan/Essigsäureethylester 2:1 -> 1:1) gereinigt. Man erhält 5.94 g (63% d. Th.) der gewünschten Verbindung.
¹H-NMR (400 MHz, DMSO-*d₆*, δ/*ppm*): 8.12 (d, 1H), 8.04 (dd, 1H), 7.43 (d, 1H), 3.78-3.61 (m, 1H), 3.46-3.34 (m, 3H), 2.86 (s, 3H), 2.25 (s, 3H), 2.10-2.01 (m, 2H).
HPLC (Methode 1): Rₜ = 3.73 min.
MS (DCI, *m*/*z*): 250 (M+H)⁺.

### Beispiel 61A

### 1-(4-Amino-2-methylphenyl)-3-methyltetrahydropyrimidin-2(1H)-on

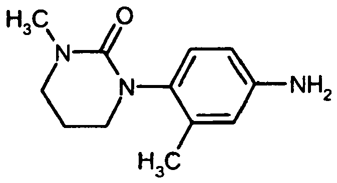

60 mg (0.24 mmol) Beispiel 60A werden in 10 ml Ethanol gelöst und mit 100 mg Palladium auf Kohle versetzt. Es wird 18 h bei RT in einer Wasserstoffatmosphäre unter normalem Druck hydriert. Anschließende wird über Kieselgur filtriert, mit Ethanol nachgewaschen und das Filtrat vom Lösungsmittel befreit. Das Produkt (51 mg) wird ohne weitere Aufreinigung weiter umgesetzt.
¹H-NMR (400 MHz, DMSO-*d₆*, δ/*ppm*): 6.71 (d, 1H), 6.39-6.27 (m, 2H), 4.88 (br.s, 2H), 3.48-3.37 (m, 1H), 3.35-3.19 (m, 3H), 2.80 (s, 3H), 2.03-1.92 (m, 5H).
HPLC (Methode 1): Rₜ = 3.29 min.
MS (ESIpos, *m*/*z*): 220 (M+H)⁺.

### Beispiel 62A

### 1-(2-Methoxy-4-nitrophenyl)-3-methyltetrahydropyrimidin-2(1H)-on

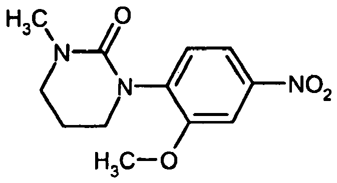

450 mg (3.94 mmol) 1-Methyltetrahydropyrimidin-2(1H)-on in 15 ml DMF werden bei 0°C mit 663 mg (5.91 mmol) Kalium-terl.-butylat versetzt und es wird 30 min bei Raumtemperatur gerührt. 742 mg (4.33 mmol) 1-Fluor-2-methoxy-4-nitrobenzol werden zugefügt und es wird bei RT gerührt. Nach 2 h wird mit 150 ml Wasser und 8 ml gesättigter wässriger Natriumchlorid-Lösung versetzt und dreimal mit je 30 ml Essigsäureethylester extrahiert. Die vereinigten organischen Phasen werden mit 30 ml Wasser gewaschen und anschließend über Natriumsulfat getrocknet. Nach Filtration werden die Lösungsmittel im Vakuum entfernt. Der Rückstand wird durch Chromatographie an Kieselgel (Cyclohexan/Essigsäureethylester 1:5 -> 1:10) gereinigt. Man erhält 457 mg (43% d. Th.) der gewünschten Verbindung.
¹H-NMR (400 MHz, DMSO-*d₆*, *δ*/*ppm*): 7.85 (m, 2H), 7.44-7.39 (m, 1H), 3.90 (s, 3H), 3.48 (t, 2H), 3.38-3.31 (m, 2H), 2.84 (s, 3H), 2.05-1.93 (m, 2H).
HPLC (Methode 1): Rₜ = 3.57 min.
MS (DCI, *m*/*z*): 266 (M+H)⁺.

### Beispiel 63A

### 1-(4-Amino-2-methoxyphenyl)-3-methyltetrahydropyrimidin-2(1H)-on

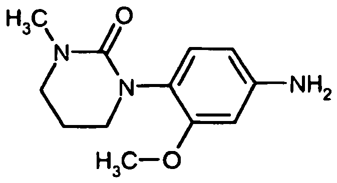

430 mg (1.62 mmol) Beispiel 62A werden in 50 ml THF gelöst und mit 100 mg Palladium auf Kohle versetzt. Es wird 18 h bei RT in einer Wasserstoffatmosphäre unter normalem Druck hydriert. Anschließend wird filtriert, mit THF nachgewaschen und das Filtrat vom Lösungsmittel befreit. Das Reaktionsprodukt (401 mg) wird ohne weitere Aufreinigung weiter umgesetzt.
¹H-NMR (400 MHz, DMSO-*d₆*, δ/*ppm*): 6.69 (d, 1H), 6.22 (d, 1H), 6.06 (dd, 1H), 5.00 (br.s, 2H), 3.65 (s, 3H), 3.37-3.25 (m, 4H), 2.79 (s, 3H), 2.00-1.91 s (m, 2 H).
HPLC (Methode 1): Rₜ = 2.88 min.
MS (ESIpos, *m*/*z*): 236 (M+H)⁺.

### Beispiel 64A

### 1-(2-{[tert-Butyl(diphenyl)silyl]oxy}ethyl)tetrahydropyrimidin-2(1H)-on

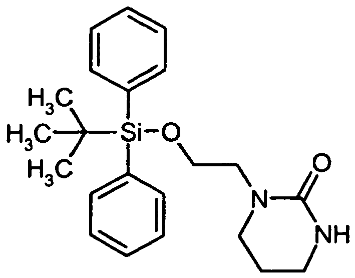

34.3 g (237 mmol) 1-(2-Hydroxyethyl)tetrahydropyrimidin-2(1H)-on (Patent; Chem. Werke Huels; DE 1121617; 1962; Chem.Abstr.; EN; 56; 11601g; 1962) werden in 612 ml DMF gelöst und mit 36.1 g (356 mmol) Triethylamin, 1.45 g (11.9 mmol) DMAP und 85.0 g (309 mmol) tert-Butyl(chlor)diphenylsilan versetzt und 18 h bei RT gerührt. Dann wird in 2000 ml Wasser gegeben und dreimal mit Dichlormethan extrahiert. Die vereinigten organischen Phasen werden zweimal mit Wasser gewaschen und dann über Natriumsulfat getrocknet. Nach Filtration werden die Lösungsmittel im Vakuum entfernt. Der Rückstand wird aus Acetonitril umkristallisiert. Man erhält 71.9 g (78% d. Th.) der gewünschten Verbindung.
¹H-NMR (400 MHz, DMSO-*d₆*, δ/*ppm*): 7.67-7.58 (m, 4H), 7.48-7.39 (m, 6H), 6.17 (br.s, 1H), 3.70 (t, 2H), 3.40-3.26 (m, 4H), 3.17-3.05 (m, 2H), 1.80-1.72 (m, 2H), 0.99 (s, 9H).
HPLC (Methode 1): Rₜ = 4.86min.
MS (ESIpos, *m*/*z*): 383 (M+H)⁺.

### Beispiel 65A

### 1-(2-{[tert-Butyl(diphenyl)silyl]oxy}ethyl)-3-(2-methyl-4-nitrophenyl)tetrahydropyrimidin-2(1H)-on

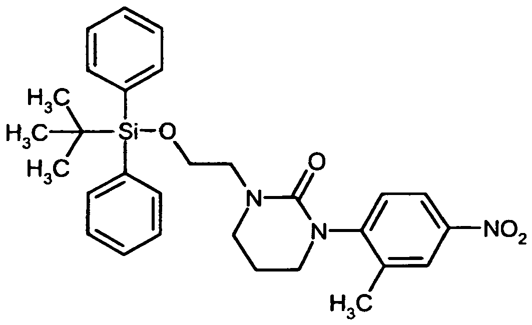

750 mg (1.96 mmol) Beispiel 64A in 9 ml DMF werden bei 0°C mit 330 mg (2.94 mmol) Kalium-tert.-butylat versetzt und es wird 30 min bei Raumtemperatur gerührt. 335 mg (2.16 mmol) 1-Fluor-2-methyl-4-nitrobenzol werden zugefügt und es wird bei 60°C gerührt. Nach 5 h wird abgekühlt und die Reaktionslösung in eine Lösung von 100 ml Wasser mit 10 ml gesättigter wässriger Ammoniumchlorid-Lösung gegeben. Dann wird dreimal mit je 70 ml Essigsäureethylester extrahiert. Die vereinigten organischen Phasen werden mit 100 ml Wasser gewaschen und dann über Natriumsulfat getrocknet. Nach Filtration werden die Lösungsmittel im Vakuum entfernt. Der Rückstand wird durch Chromatographie an Kieselgel (Cyclohexan/Essigsäureethylester 3:1) gereinigt. Man erhält 135 mg (13% d. Th.) der gewünschten Verbindung.
HPLC (Methode 1): Rₜ = 5.17 min.
MS (DCI, *m*/*z*): 518 (M+H)⁺.

### Beispiel 66A

### 1-(4-Amino-2-methylphenyl)-3-(2-{[tert-butyl(diphenyl)silyl]oxy}ethyl)tetrahydropyrimidin-2(1H)-on

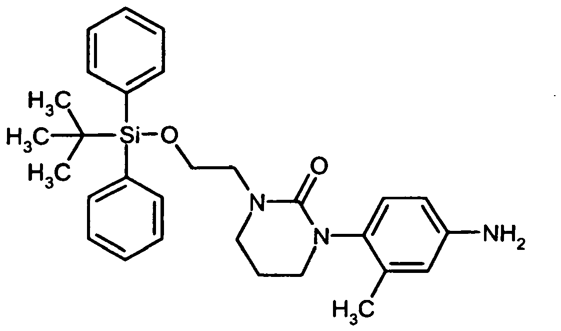

229 mg (0.44 mmol) Beispiel 65A werden in 10 ml THF gelöst und mit 50 mg Palladium auf Kohle versetzt. Es wird 4 h bei RT in einer Wasserstoffatmosphäre unter normalem Druck hydriert. Anschließend wird filtriert, mit THF nachgewaschen und das Filtrat vom Lösungsmittel befreit. Das Reaktionsprodukt (230 mg, Reinheit 67%) wird ohne weitere Aufreinigung weiter umgesetzt.
HPLC (Methode 2): Rₜ = 4.61 min.
MS (DCI, *m*/*z*): 488 (M+H)⁺.

### Beispiel 67A

### N-{[(5S)-3-{4-[3-(2-{[tert-Butyl(diphenyl)silyl]oxy}ethyl)-2-oxotetrahydropyrimidin-1(2H-)7-yl]-3-methylphenyl}-2-oxo-1,3-oxazolidin-5-yl]methyl}-5-chlorthiophen-2-carboxamid

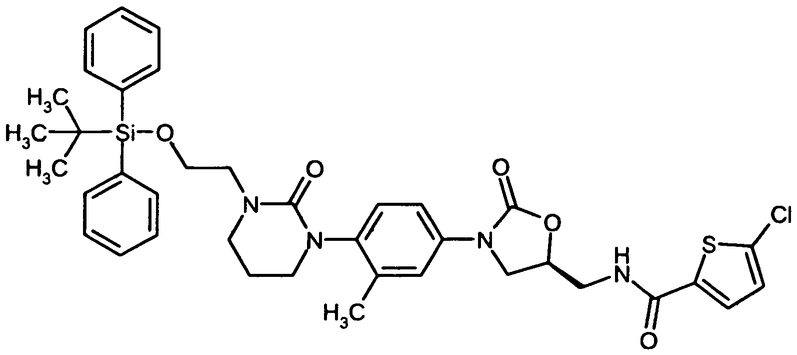

Eine Lösung von 215 mg (0.42 mmol) des Produktes aus Beispiel 66A in 6 ml Acetonitril wird mit 105 mg (0.486 mmol) des Produktes aus Beispiel 1A versetzt. Der Suspension werden 148 mg (0.663 mmol) Magnesiumperchlorat zugefügt, wonach man 18 h bei RT rühren lässt. Dann werden 179 mg (1.11 mmol) 1,1'-Carbonyldiimidazol und 5 mg (0.04 mmol) DMAP hinzugesetzt, und es wird bei 60°C gerührt. Nach 3 h wird abgekühlt und anschließend in 50 ml Wasser gegeben. Nach Zugabe von 50 ml Essigsäureethylester werden die Phasen getrennt und die wässrige Phase dreimal mit Essigsäureethylester extrahiert. Die vereinigten organischen Phasen werden über Natriumsulfat getrocknet. Die Lösungsmittel werden im Vakuum entfernt und der Rückstand durch Chromatographie an Kieselgel (Dichlormethan/Methanol 20:1) gereinigt. Man erhält 241 mg (74% d. Th.) der gewünschten Verbindung.
HPLC (Methode 2): Rₜ = 5.16 min.
MS (ESIpos, *m*/*z*): 731 (M+H)⁺.

### Beispiel 68A

### 1-(2-{[tert-Butyl(diphenyl)silyl]oxy}ethyl)-3-(2-chlor-4-nitrophenyl)tetrahydropyrimidin-2(1H)-on

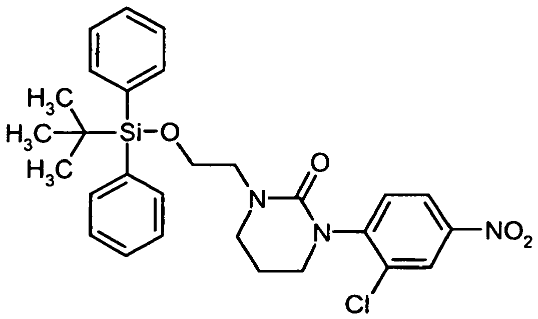

500 mg (1.31 mmol) Beispiel 64A in 6 ml DMF werden bei 0°C mit 220 mg (1.96 mmol) Kalium-tert.-butylat versetzt und es wird 30 min bei Raumtemperatur gerührt. 252 mg (1.44 mmol) 2-Chlor-1-fluor-4-nitrobenzol werden zugefügt und es wird bei RT gerührt. Nach 22 h wird abgekühlt und die Reaktionslösung in eine Lösung von 50 ml Wasser und 8 ml gesättigter wässriger Natriumchlorid-Lösung gegeben. Dann wird dreimal mit je 30 ml Essigsäureethylester extrahiert. Die vereinigten organischen Phasen werden mit 30 ml Wasser gewaschen und dann über Natriumsulfat getrocknet. Nach Filtration werden die Lösungsmittel im Vakuum entfernt. Der Rückstand wird durch Chromatographie an Kieselgel (Cyclohexan/Essigsäureethylester 2:1) gereinigt. Man erhält 200 mg (28% d. Th.) der gewünschten Verbindung.
¹H-NMR (400 MHz, DMSO-d₆, *δ*/*ppm*): 8.34 (d, 1H), 8.21 (dd, 1H), 7.68-7.62 (m, 5H), 7.51-7.42 (m, 6H), 3.76 (t, 2H), 3.58-3.41 (m, 6H), 2.07 (tt, 2H), 1.01 (s, 9H).
HPLC (Methode 2): Rₜ = 5.78 min.
MS (DCI, *m*/*z*): 538 (M+H)⁺.

### Beispiel 69A

### 1-(4-Amino-2-chlorphenyl)-3-(2-{[tert-butyl(diphenyl)silyl]oxy}ethyl)tetrahydropyrimidin-2(1H)-on

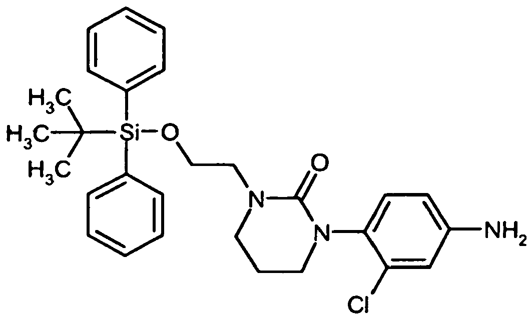

386 mg (0.72 mmol) Beispiel 68A werden in 10 ml THF gelöst und mit 50 mg Palladium auf Kohle versetzt. Es wird 2 h bei RT in einer Wasserstoffatmosphäre unter normalem Druck hydriert. Anschließend wird filtriert, dreimal mit THF nachgewaschen und das Filtrat vom Lösungsmittel befreit. Das Reaktionsprodukt (365 mg, Reinheit 80%) wird ohne weitere Aufreinigung weiter umgesetzt.
¹H-NMR (400 MHz, DMSO-*d₆*, *δ*/*ppm*): 7.66-7.60 (m, 4H), 7.51-7.40 (m, 6H), 6.88 (d, 1H), 6.62 (d, 1H), 6.45 (dd, 1H), 5.33 (br.s., 2H), 3.77-3.68 (m, 2H), 3.50-3.30 (m, 6H), 2.05-1.94 (m, 2H), 1.01 (s, 9H).
HPLC (Methode 2): Rₜ = 4.71 min.
MS (ESIpos, *m*/*z*): 508 (M+H)⁺.

### Beispiel 70A

### N-{[(5S)-3-{4-[3-(2-{[tert-Butyl(diphenyl)silyl]oxy}ethyl)-2-oxotetrahydropyrimidin-1(2H)-yl]-3-chlorphenyl}-2-oxo-1,3-oxazolidin-5-yl]methyl}-5-chlorthiophen-2-carboxamid

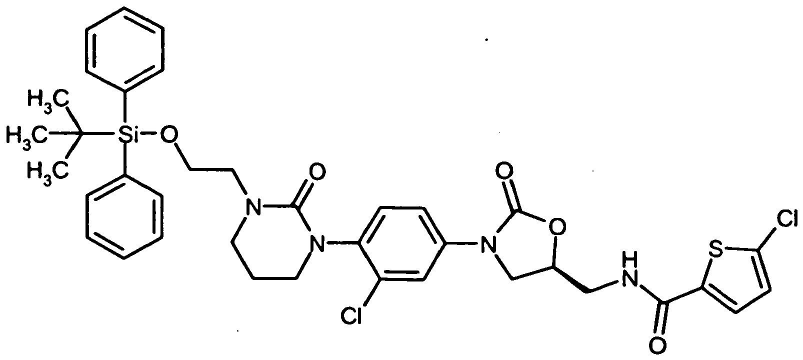

Eine Lösung von 364 mg (0.718 mmol) des Produktes aus Beispiel 69A in 8 ml Acetonitril wird mit 172 mg (0.789 mmol) des Produktes aus Beispiel 1A versetzt. Der Suspension werden 240 mg (1.07 mmol) Magnesiumperchlorat zugefügt, wonach man 15 h bei RT rühren lässt. Dann werden 291 mg (1.79 mmol) 1,1'-Carbonyldiimidazol und 9 mg (0.08 mmol) DMAP hinzugesetzt, und es wird bei 60°C gerührt. Nach 3 h wird abgekühlt und anschließend mit 70 ml Wasser und 50 ml Essigsäureethylester verdünnt. Die Phasen werden getrennt und die wässrige Phase zweimal mit Essigsäureethylester extrahiert. Die vereinigten organischen Phasen werden über Natriumsulfat getrocknet. Die Lösungsmittel werden im Vakuum entfernt und der Rückstand durch Chromatographie an Kieselgel (Dichlormethan/Methanol 20:1) gereinigt. Man erhält 366 mg (59% d. Th.) der gewünschten Verbindung.
¹H-NMR (400 MHz, DMSO-*d₆*, δ/*ppm*): 8.96 (dd, 1H), 7.70 (d, 1H), 7.66-7.58 (m, 5H), 7.48-7.36 (m, 7H), 7.31 (d, 1H), 7.21-7.16 (m, 1H), 4.89-4.78 (m., 1H), 4.19 (dd, 1H), 3.89-3.82 (m, 1H), 3.80-3.68 (m, 2H), 3.60 (t, 2H), 3.52-3.38 (m, 6H), 2.10-1.95 (m, 2H), 1.01 (s, 9H).
HPLC (Methode 2): Rₜ = 5.26 min.
MS (ESIpos, *m*/*z*): 751 (M+H)⁺.

### Beispiel 71A

### 1-(2-{[tert-Butyl(diphenyl)silyl]oxy}ethyl)-3-[4-nitro-2-(trifluormethyl)phenyl]tetrahydropyrimidin-2(1H)-on

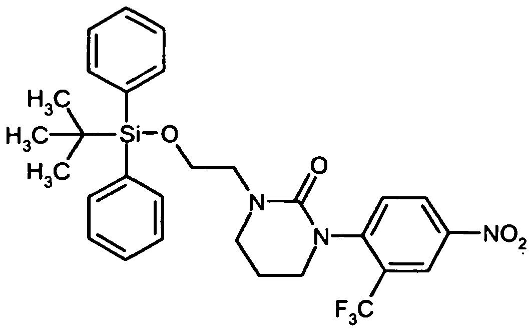

71.8 g (187 mmol) Beispiel 64A in 1300 ml DMF werden bei 0°C mit 31.6 g (281 mmol) Kalium-tert.-butylat versetzt und es wird 30 min bei Raumtemperatur gerührt. 43.2 g (206 mmol) 1-Fluor-4-nitro-2-(trifluormethyl)benzol in 100 ml DMF werden zugefügt und es wird bei RT gerührt. Nach 18 h wird die Reaktionslösung mit 2000 ml Wasser und 50 ml gesättigter wässriger Natriumchlorid-Lösung versetzt. Nach Phasentrennung wird die wässrige Phase dreimal mit je 500 ml Essigsäureethylester extrahiert. Die vereinigten organischen Phasen werden zweimal mit 500 ml Wasser gewaschen und dann über Natriumsulfat getrocknet. Nach Filtration werden die Lösungsmittel im Vakuum entfernt. Der Rückstand wird durch Chromatographie an Kieselgel (Cyclohexan/Essigsäureethylester 5:1 -> 2:1) gereinigt. Man erhält 31.3 g (28% d. Th.) der gewünschten Verbindung.
¹H-NMR (400 MHz, DMSO-*d₆*, *δ*/*ppm*): 8.56 (dd, 1H), 8.45 (d, 1H), 7.74 (d, 1H), 7.68-7.60 (m, 4H), 7.51-7.39 (m, 6H), 3.80-3.62 (m, 3H), 3.58-3.44 (m, 3H), 3.42-3.31 (m, 2H), 2.05 (tt, 2H), 1.01 (s, 9H).
HPLC (Methode 2): Rₜ = 5.50 min.
MS (ESIpos, *m*/*z*): 572 (M+H)⁺.

### Beispiel 72A

### 1-(4-Amino-2-(trifluormethyl)phenyl]-3-(2-{[tert-butyl(diphenyl)silyl]oxy}ethyl)tetrahydropyrimidin-2(1H)-on

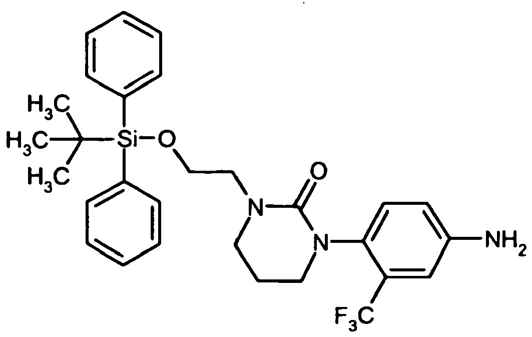

21.2 g (37.1 mmol) Beispiel 71A werden in 700 ml THF gelöst und mit 200 mg Palladium auf Kohle versetzt. Es wird 2 h bei RT in einer Wasserstoffatmosphäre unter normalem Druck hydriert. Anschließend wird filtriert, dreimal mit THF nachgewaschen und das Filtrat vom Lösungsmittel befreit. Das Reaktionsprodukt (20.5 g) wird ohne weitere Aufreinigung weiter umgesetzt.
¹H-NMR (400 MHz, DMSO-*d₆*, δ/*ppm*): 7.68-7.61 (m, 4H), 7.51-7.40 (m, 6H), 6.94 (d, 1H), 6.84 (d, 1H), 6.75 (dd, 1H), 5.53 (br.s., 2H), 3.77-3.65 (m, 2H), 3.50-3.23 (m, 6H), 2.05-1.92 (m, 2H), 1.01 (s, 9H).
HPLC (Methode 2): Rₜ = 5.41 min.
MS (ESIpos, *m*/*z*): 542 (M+H)⁺.

### Beispiel 73A

### N-{[(5S)-3-{4-[3-(2-{[tert-Butyl(diphenyl)silyl]oxy}ethyl)-2-oxotetrahydropyrimidin-1(2H)-yl]-3-(trifluormethyl)phenyl}-2-oxo-1,3-oxazolidin-5-yl]methyl}-5-yl-chlorthiophen-2-carboxamid

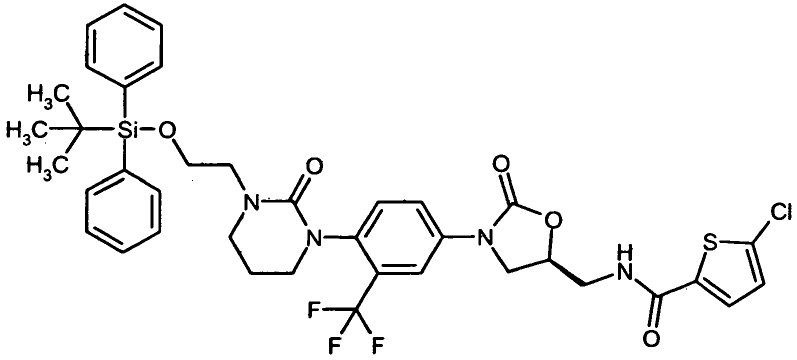

Eine Lösung von 20.5 g (37.8 mmol) des Produktes aus Beispiel 72A in 300 ml Acetonitril wird mit 9.06 g (41.6 mmol) des Produktes aus Beispiel 1A versetzt. Der Suspension werden 12.7 g (56.7 mmol) Magnesiumperchlorat zugefügt, wonach man 15 h bei RT rühren lässt. Dann werden 15.3 g (94.6 mmol) 1,1'-Carbonyldiimidazol und 462 mg (3.78 mmol) DMAP hinzugesetzt, und es wird bei RT gerührt. Nach 15 h wird in 1500 ml Wasser gegeben, die Phasen werden getrennt und die wässrige Phase dreimal mit Essigsäureethylester extrahiert. Die vereinigten organischen Phasen werden über Natriumsulfat getrocknet. Die Lösungsmittel werden im Vakuum entfernt und der Rückstand durch Chromatographie an Kieselgel (Cyclohexan/Essigsäureethylester 1:5) gereinigt. Man erhält 19.7 g (60% d. Th.) der gewünschten Verbindung.
¹H-NMR (400 MHz, DMSO-*d₆*, *δ*/*ppm*): 8.96 (dd, 1H), 7.96 (dd, 1H), 7.69 (d, 1H), 7.66-7.61 (m, 5H), 7.48-7.38 (m, 7H), 7.19 (d, 1H), 4.89-4.80 (m, 1H), 4.28-4.19 (m, 1H), 3.92-3.84 (m, 1H), 3.80-3.68 (m, 2H), 3.65-3.25 (m, 8H), 2.08-1.98 (m, 2H), 1.01 (s, 9H).
HPLC (Methode 2): Rₜ = 6.27 min.
MS (ESIpos, *m*/*z*): 785 (M+H)⁺.

### Beispiel 74A

### 1-[2-(Ethoxymethyl)-4-nitrophenyl]piperidin-2-on

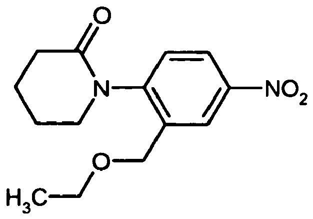

248 mg (2.51 mmol) Piperidin-2-on werden in 10 ml wasserfreiem Dimethylsulfoxid gelöst und mit 338 g (3.01 mmol) Kalium-tert.-butylat versetzt. Nach 1 h werden 500 mg Beispiel 32A zugegeben und es wird 16 h auf 80°C erhitzt. Man lässt erkalten und versetzt mit gesättigter Natriumchloridlösung. Die Mischung wird mehrmals mit Dichlormethan extrahiert, die organische Phase wird zweimal mit Wasser und zweimal mit gesättigter Natriumchloridlösung gewaschen, über Natriumsulfat getrocknet, filtriert und im Vakuum zur Trockene eingedampft. Der Rückstand wird in 5 ml Dimethylsulfoxid gelöst und mit präparativer HPLC mit einem Gradienten aus Wasser und Acetonitril aufgereinigt. Die Produktfraktionen werden vereinigt und im Vakuum zur Trockene eingedampft. Man erhält 162 mg (23% d. Th.) des gewünschten Produktes.
¹H-NMR (400 MHz, DMSO-*d₆*, *δ*/*ppm*): δ = 8.3 (d, 1H), 8.2 (dd, 1H), 7.55 (d, 1H), 4.45 (m, 2H), 3.7 (b, 1H), 3.5 (q, 2H), 3.4 (b, 1H), 2.4 (b, 2H), 1.9 (b, 4H), 1.15 (t, 3H).
LC-MS (Methode 6): Rₜ = 1.99 min
MS (ESIpos): m/z = 279 (M+H)⁺

### Beispiel 75A

### 1-[4-Amino-2-(ethoxymethyl)phenyl]piperidin-2-on

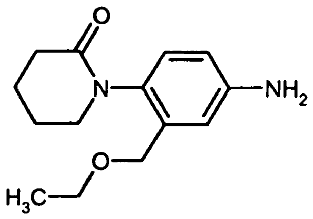

160 mg (0.58 mmol) Beispiel 74A werden in 10 ml einer 1:1 Mischung aus Ethanol und Essigsäureethylester gelöst und mit 181 mg (2.17 mmol) Ammoniumformiat und 20 mg Palladium auf Kohle versetzt. Es wird 30 min auf 80°C erhitzt, man lässt erkalten und filtriert über Kieselgel. Es wird mit Essigsäureethylester und Ethanol nachgewaschen und das Filtrat im Vakuum zur Trockene eingedampft. Man erhält 147 mg (99% d. Th.) des gewünschten Produktes.
LC-MS (Methode 6): Rₜ= 1.04 min
MS (ESIpos): m/z = 249 (M+H)⁺

### Vergleichsbeispiel 76A

### 4-(2-Methyl-4-nitrophenyl)-1,4-oxazepan-5-on

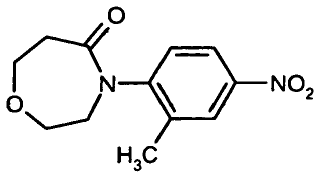

3.32 g (15.6 mmol) Kaliumphosphat werden mit 0.5 g (2.6 mmol) Kupfer(I)iodid in einem Rundkolben vermischt und mehrmals abwechselnd ausgeheizt und mit Argon belüftet. Es werden nacheinander 10 ml wasserfreies Dioxan, 1.5 g (13.0 mmol) 1,4-Oxazepan-5-on, 3.43 g (13.0 mmol) 1-Iod-2-methyl-4-nitrobenzol und 0.46 g (5.21 mmol) N,N-Dimethylethylendiamin zugegeben und 15 h auf 110°C erhitzt. Man läßt erkalten, filtriert über Kieselgel und wäscht das Kieselgel mit Dioxan nach. Die vereinigten Filtrate werden im Vakuum zur Trockene eingedampft. Man löst den Rückstand in Acetonitril an und reinigt mit präparativer HPLC mit einem Gradienten aus Wasser und Acetonitril auf. Die Produktfraktionen werden vereinigt und im Vakuum zur Trockene eingedampft. Man erhält 430 mg (13% d. Th.) des gewünschten Produktes.
¹H-NMR (400 MHz, DMSO-*d₆*, *δ*/*ppm):* δ = 8.2 (s, 1H), 8.1 (d, 1H), 7.5 (d, 1H), 3.9 (m, 5H), 3.8 (m, 1H), 2.9 (m, 1H), 2.8 (m, 1H), 2.3 (s, 3H).
LC-MS (Methode 6): Rₜ = 1.58 min
MS (ESIpos): m/z = 251 (M+H)⁺

### Vergleichsbeispiel 77A

### 4-(4-Amino-2-methylphenyl)-1,4-oxazepan-5-on

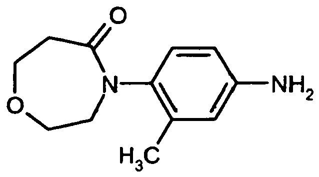

430 mg (1.72 mmol) Beispiel 76A werden in 40 ml einer 1:1 Mischung aus Ethanol und Essigsäureethylester gelöst und mit 650 mg (10.3 mmol) Ammoniumformiat und 50 mg Palladium auf Kohle versetzt. Es wird auf 80°C erhitzt und nach 45 min lässt man erkalten und filtriert über Kieselgel. Es wird mit Essigsäureethylester nachgewaschen und das Filtrat im Vakuum zur Trockene eingedampft. Man erhält 373 mg (98% d. Th.) des gewünschten Produktes.
LC-MS (Methode 7): Rₜ = 1.84 min
MS (ESIpos): m/z = 221 (M+H)⁺

### Beispiel 78A

### 4-(2-Brom-4-nitrophenyl)-1,4-oxazepan-5-on

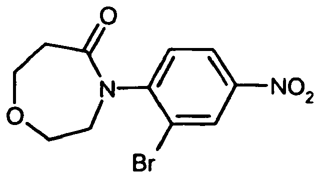

1.57 g (13.6 mmol) 1,4-Oxapan-5-on in 60 ml DMSO werden bei RT mit 2.30 g (20.5 mmol) Kalium-tert.-butylat versetzt und es wird 30 min bei Raumtemperatur gerührt. 3.00 g (13.6 mmol) 2-Brom-1-fluor-4-nitrobenzol werden zugefügt und es wird bei 60°C gerührt. Nach 2 h wird das Heizbad entfernt und anschließend 18 h bei RT gerührt. Dann wird in 600 ml Wasser und 200 ml gesättigter wässriger Natriumchlorid-Lösung gegeben und dreimal mit je 300 ml Essigsäureethylester extrahiert. Die vereinigten organischen Phasen werden über Natriumsulfat getrocknet. Nach Filtration werden die Lösungsmittel im Vakuum entfernt. Der Rückstand wird durch Chromatographie an Kieselgel (Cyclohexan/Essigsäureethylester 1:1; dann Essigsäureethylester) vorgereinigt. Nach erneuter Chromatographie an Kieselgel (Dichlormethan/Methanol 60:1) erhält man 839 mg (20% d. Th.) der gewünschten Verbindung.
¹H-NMR (400 MHz, DMSO-*d₆*, *δ*/*ppm):* 8.50 (d, 1H), 8.28 (dd, 1H), 7.66 (d, 1H), 3.92-3.83 (m, 4H), 3.82-3.75 (m, 2H), 2.90-2.81 (m, 2H).
HPLC (Methode 1): Rₜ = 3.59 min.
MS (DCI, m/z): 316 (M+H)⁺.

### Vergleichsbeispiel 79A

### 4-(2-Cyclopropyl-4-nitrophenyl)-1,4-oxazepan-5-on

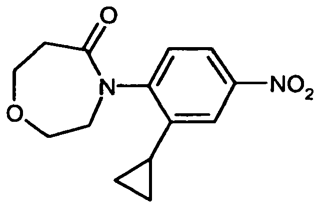

Unter Argon werden 300 mg (0.95 mmol) Beispiel 78A, 327 mg (3.81 mmol) Cyclopropylboronsäure, 33 mg (0.03 mmol) Tetrakis(triphenlyphosphin)palladium (0) und 404 mg (1.90 mmol) Kaliumphosphat in 12 ml Toluol suspendiert. Man rührt 3.5 h bei 120°C und anschließend 10 h bei RT. Dann wird mit 200 ml Wasser und 200 ml Essigsäureethylester versetzt, die wässrige Phase zweimal mit Essigsäureethylester extrahiert und die vereinigten organischen Phasen einmal mit Wasser gewaschen. Nach Trocknen über Natriumsulfat und Filtration werden die Lösungsmittel im Vakuum entfernt. Das Reaktionsprodukt (98% d. Th.) wird ohne weitere Aufreinigung weiter umgesetzt.
HPLC (Methode 1): Rₜ = 3.73 min.
MS (DCI, *m*/*z*): 277 (M+H)⁺.

### Vergleichsbeispiel 80A

### 4-(4-Amino-2-cyclopropylphenyl)-1,4-oxazepan-5-on

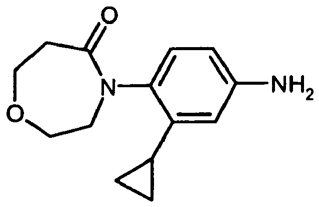

394 mg (1.42 mmol) Beispiel 79A werden in 23 ml Ethanol gelöst und mit 1.29 g (5.70 mmol) Zinnchlorid-dihydrat versetzt. Es wird 20 min zum Rückfluß erhitzt und dann die Reaktion durch Zugabe von 100 ml Wasser beendet. Mit Natronlauge wird auf pH = 10 gestellt und dreimal mit Essigsäureethylester extrahiert. Die vereinigten organischen Phasen werden über Natriumsulfat getrocknet. Anschließend wird filtriert und das Filtrat vom Lösungsmittel befreit. Das Reaktionsprodukt wird ohne weitere Aufreinigung weiter umgesetzt.
LC-MS (Methode 3): Rₜ = 2.13 min.
MS (ESIpos, *m*/*z*): 247 (M+H)⁺.

### Beispiel 81A

### 1-(2-Brom-4-nitrophenyl)tetrahydropyrimidin-2(1H)-on

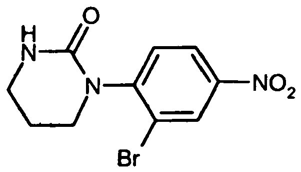

910 mg (9.09 mmol) Tetrahydropyrimidinon in 39 ml DMSO werden bei RT mit 1.53 g (13.6 mmol) Kalium-tert.-butylat versetzt und es wird 30 min bei Raumtemperatur gerührt. 2.00 g (9.09 mmol) 2-Brom-1-fluor-4-nitrobenzol werden zugefügt und es wird bei 60°C gerührt. Nach 18 h wird abgekühlt und in 600 ml Wasser und 160 ml gesättigter wässriger Natriumchlorid-Lösung gegeben. Es wird dreimal mit je 300 ml Essigsäureethylester extrahiert und die vereinigten organischen Phasen werden über Natriumsulfat getrocknet. Nach Filtration werden die Lösungsmittel im Vakuum entfernt und der Rückstand durch Chromatographie an Kieselgel (Dichlormethan/Ethanol 60:1) gereinigt. Man erhält 481 mg (17% d. Th.) der gewünschten Verbindung.
¹H-NMR (400 MHz, DMSO-*d₆*, *δ*/*ppm*): 8.46 (d, 1H), 8.24 (dd, 1H), 7.65 (d, 1H), 6.83 (br. s, 1H), 3.58-3.44 (m, 2H), 3.31-3.23 (m, 2H), 2.03-1.90 (m, 2H).
HPLC (Methode 5): Rₜ = 2.84 min.
MS (ESIpos, *m*/*z*): 300 (M+H)⁺.

### Beispiel 82A

### 1-(2-Ethyl-4-nitrophenyl)tetrahydropyrimidin-2(1H)-on

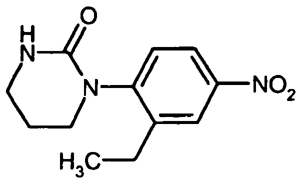

Unter Argon werden 278 mg (0.92 mmol) Beispiel 81A, 410 mg (5.56 mmol) Ethylboronsäure, 64 mg (0.06 mmol) Tetrakis(triphenylphosphin)palladium(0) und 393 mg (1.85 mmol) Kaliumphosphat in 12 ml Toluol suspendiert und bei 120°C gerührt. Nach 20 h wird abermals mit 410 mg (5.56 mmol) Ethylboronsäure, 64 mg (0.06 mmol) Tetrakis(triphenylphosphin)palladium (0) und 393 mg (1.85 mmol) Kaliumphosphat versetzt und anschließend 22 h bei 120°C gerührt. Dann wird mit 100 ml Wasser und 80 ml Essigsäureethylester versetzt und die wässrige Phase zweimal mit Essigsäureethylester extrahiert und die vereinigten organischen Phasen werden über Natriumsulfat getrocknet. Nach Filtration werden die Lösungsmittel im Vakuum entfernt und der Rückstand mit präparativer HPLC mit einem Gradienten aus Wasser und Acetonitril aufgereinigt. Man erhält 22 mg (7% d. Th.) des gewünschten Produktes.
HPLC (Methode 1): Rₜ = 3.70 min.
MS (DCI, *m*/*z*): 250 (M+H)⁺.

### Beispiel 83A

### 1-(4-Amino-2-ethylphenyl)tetrahydropyrimidin-2(1H)-on

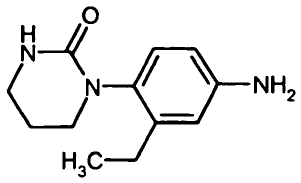

16.8 mg (0.067 mmol) Beispiel 82A werden in 1 ml Ethanol gelöst und mit 60.8 g (0.270 mmol) Zinnchlorid-dihydrat versetzt. Es wird 20 min zum Rückfluß erhitzt, ehe man die Reaktion durch Zugabe von 15 ml Wasser beendet. Mit Natronlauge wird auf pH = 10 gestellt und anschließend dreimal mit Essigsäureethylester extrahiert. Die vereinigten organischen Phasen werden über Natriumsulfat getrocknet. Nach Filtration wird das Filtrat vom Lösungsmittel befreit. Das Reaktionsprodukt wird ohne weitere Aufreinigung umgesetzt.
LC-MS (Methode 7): Rₜ = 2.06 min.
MS (ESIpos, *m*/*z*): 220 (M+H)⁺.

### Beispiel 84A

### 1-(2-Isopropyl-4-nitrophenyl)tetrahydropyrimidin-2(1H)-on

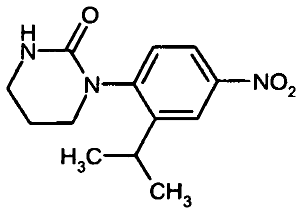

347 mg (3.47 mmol) Tetrahydropyrimidinon in 21 ml DMSO werden bei RT mit 584 mg (5.21 mmol) Kalium-tert.-butylat versetzt und es wird 45 min bei Raumtemperatur gerührt. 700 mg (3.82 mmol) 1-Fluor-2-isopropyl-4-nitrobenzol werden zugefügt und es wird bei 60°C gerührt. Nach 18 h wird abgekühlt und mit 250 ml Wasser verdünnt. Es wird dreimal mit Essigsäureethylester extrahiert und die vereinigten organischen Phasen über Natriumsulfat getrocknet. Nach Filtration werden die Lösungsmittel im Vakuum entfernt und der Rückstand durch Chromatographie an Kieselgel (Cyclohexan/Essigsäureethylester 1:5 -> 1:10) gereinigt. Man erhält 140 mg (14% d. Th.) der gewünschten Verbindung.
¹H-NMR (400 MHz, DMSO-d₆, *δ*/*ppm*): 8.13 (d, 1H), 8.05 (dd, 1H), 7.46 (d, 1H), 6.68 (br. s, 1H), 3.69-3.60 (m, 1H), 3.37-3.21 (m, 2H), 3.14-3.04 (m, 2H), 2.03-1.93 (m, 2H), 1.20 (dd, 6H).
HPLC (Methode 1): Rₜ = 3.87 min.
MS (DCI, *m*/*z*): 264 (M+H)⁺.

### Beispiel 85A

### 1-(4-Amino-2-isopropylphenyl)tetrahydropyrimidin-2(1H)-on

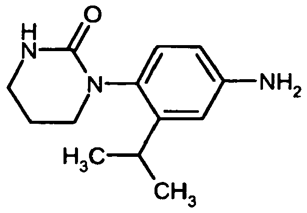

130 mg.(0.493 mmol) Beispiel 84A werden in 8 ml Ethanol gelöst und mit 445 g (1.97 mmol) Zinnchlorid-dihydrat versetzt. Es wird 20 min zum Rückfluß erhitzt, ehe man die Reaktion durch Zugabe von 100 ml Wasser beendet. Mit Natronlauge wird auf pH = 10 gestellt und anschließend dreimal mit Essigsäureethylester extrahiert. Die vereinigten organischen Phasen werden über Natriumsulfat getrocknet. Nach Filtration wird das Filtrat vom Lösungsmittel befreit. Das Reaktionsprodukt (82 mg) wird ohne weitere Aufreinigung umgesetzt.
HPLC (Methode 1): Rₜ = 2.95 min.
MS (ESIpos, *m*/*z*): 234 (M+H)⁺.

### Beispiel 86A

### 1-{4-Nitro-2-[(1E)-prop-1-en-1-yl]phenylpyrimidin-2(1H)-on

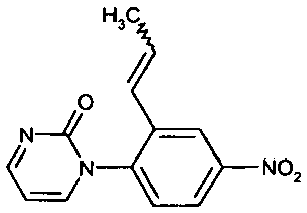

295 mg (2.76 mmol) Pyrimidin-2-on werden in 10 ml wasserfreiem N,N-Dimethylformamid gelöst und mit 371 mg (3.3 mmol) Kalium-tert.-butylat versetzt. Nach 1 h werden 500 mg (2.76 mmol) Beispiel 36A zugegeben und es wird 16 h auf 50°C erhitzt. Man lässt erkalten und filtriert die unlöslichen Bestandteile ab. Die Lösung wird mit präparativer HPLC mit einem Gradienten aus Wasser und Acetonitril aufgereinigt. Die Produktfraktionen werden vereinigt und im Vakuum zur Trockene eingedampft. Man erhält 45 mg (6% d. Th.) der gewünschten Verbindung.
¹H-NMR (400 MHz, DMSO-*d₆*, *δ*/*ppm*): δ = 8.75 (m, 1H), 8.25-8.5 (m, 2H), 8.1 (m, 1H), 7.8 (d, 1H), 6.6 (m, 1H), 6.1 (m, 1H), 5.9 (m, 1H), 1.6-1.8 (dd, 3H).

### Beispiel 87A

### 1-(4-Amino-2-propylphenyl)tetrahydropyrimidin-2(1H)-on

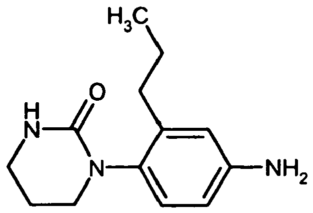

44 mg (0.17 mmol) Beispiel 86A werden in 8 ml einer 1:1 Mischung aus Ethanol und Essigsäureethylester gelöst und mit 65 mg (1.02 mmol) Ammoniumformiat und 50 mg Palladium auf Kohle versetzt. Es wird auf 80°C erhitzt. Nach 45 min lässt man erkalten und filtriert über Kieselgel. Dann wird mit Essigsäureethylester nachgewaschen und das Filtrat im Vakuum zur Trockene eingedampft. Man erhält 42 mg (100% d. Th.) der gewünschten Verbindung.
LC-MS (Methode 7): Rₜ = 2.30 min
MS (ESIpos): m/z = 234 (M+H)⁺

### Beispiel 88A

### 2-Fluor-5-nitrophenol

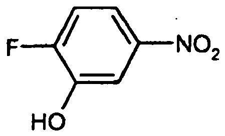

116 ml (116 mmol) Bortribromid (1-molare Lösung in Dichlormethan) werden bei -10°C über einen Zeitraum von 1 h so zu einer Lösung von 5.00 g (29.2 mmol) 1-Fluor-2-methoxy-4-nitrobenzol getropft, dass die Temperatur -5°C nicht übersteigt. Es wird 5 h bei 0°C belassen, dann langsam in 600 ml Eiswasser gegeben und mit 100 ml Essigsäureethylester verdünnt. Nach Phasentrennung wird die wässrige Phase zweimal mit Essigsäureethylester extrahiert. Die vereinigten organischen Phasen werden über Natriumsulfat getrocknet. Nach Filtration werden die Lösungsmittel im Vakuum entfernt. Der Rückstand wird durch Chromatographie an Kieselgel (Cyclohexan/Essigsäureethylester 10: 1) gereinigt. Man erhält 1.45 g (30% d. Th.) der gewünschten Verbindung.
¹H-NMR (400 MHz, DMSO-*d₆*, *δ*/*ppm*): 10.99 (s, 1H), 7.78 (dd, 1H), 7.73 (ddd, 1H), 7.44 (dd, 1H).
HPLC (Methode 1): Rₜ = 3.60 min.
MS (DCI, *m*/*z*): 174 (M+NH₄)⁺.

### Beispiel 89A

### 2-Ethoxy-1-fluor-4-nitrobenzol

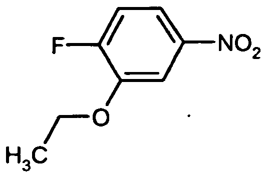

1.00 g (6.37 mmol) Beispiel 88A werden in 15 ml DMF gelöst und mit 1.76 g (12.7 mmol) Kaliumcarbonat versetzt. Anschließend wird 1.19 g (7.64 mmol) Iodethan zugegeben und es wird bei RT gerührt. Nach 1.5 h wird mit je 100 ml Toluol und Wasser versetzt, die Phasen getrennt und die wässrige Phase zweimal mit je 150 ml Toluol extrahiert. Die vereinigten organischen Phasen werden mit gesättigter wässriger Natriumhydrogencarbonat-Lösung gewaschen und dann über Natriumsulfat getrocknet. Es wird im Vakuum vom Lösungsmittel befreit und der erhaltene Feststoff im Hochvakuum getrocknet. Man erhält 1.06 g (89% d. Th.) des gewünschten Produktes.
¹H-NMR (400MHz, DMSO-*d₆*, *δ*/*ppm*): δ = 7.95 (dd, 1H), 7.89 (ddd, 1H), 7.52 (dd, 1H), 4.25 (q, 2H), 1.38 (t, 3H).
GC/MS (Methode 9): Rₜ = 6.42 min.
MS (EI-pos): m/z = 185 (M+H)⁺.

### Beispiel 90A

### 1-(2-Ethoxy-4-nitrophenyl)piperidin-2-on

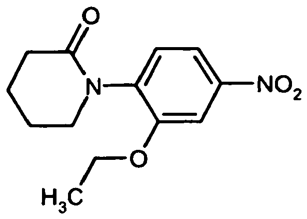

Unter Argon werden 243 mg (2.45 mmol) Piperidin-2-on in 12 ml DMF bei 0°C mit 330 mg (2.95 mmol) Kalium-tert.-butylat versetzt und es wird 30 min bei Raumtemperatur gerührt. 500 mg (2.70 mmol) Beispiel 89A in 30 ml DMF werden zugefügt und es wird bei RT gerührt. Nach 18 h wird in 150 ml gesättigte wässrige Ammoniumchlorid-Lösung gegeben und dann dreimal mit je 100 ml Essigsäureethylester extrahiert. Die vereinigten organischen Phasen werden mit 200 ml Wasser gewaschen und dann über Natriumsulfat getrocknet. Nach Filtration werden die Lösungsmittel im Vakuum entfernt. Der Rückstand wird durch Chromatographie an Kieselgel (Cyclohexan/Essigsäureethylester 1:5) gereinigt. Man erhält 256 mg (39% d. Th.) der gewünschten Verbindung.
¹H-NMR (400 MHz, DMSO-*d₆*, *δ*/*ppm*): 7.89-7.81 (m, 2H), 7.50-7.42 (m, 1H), 4.18 (q, 2H), 3.47 (t, 2H), 2.38 (t, 2H), 1.92-1.78 (m, 4H), 1.33 (t, 3H).
HPLC (Methode 1): Rₜ = 3.93 min.
MS (DCI, *m*/*z*): 265 (M+H)⁺.

### Beispiel 91A

### 1-(4-Amino-2-ethoxyphenyl)piperidin-2-on

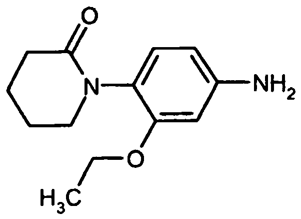

235 mg (0.889 mmol) des Produktes aus Beispiel 90A werden in 40 ml THF gelöst und mit 50 mg 10%-igem Palladium auf Aktivkohle versetzt. Man hydriert über Nacht in einer Wasserstoffatmosphäre unter normalem Druck, saugt dann über Kieselgur ab, wäscht dreimal mit THF nach und engt vorsichtig im Vakuum ein. Man erhält 262 mg Roh-Produkt, das ohne Aufreinigung weiter umgesetzt wird.
¹H-NMR (400 MHz, DMSO-d₆, *δ*/*ppm*): 6.68 (d, 1H), 6.22 (d, 1H), 6.08 (dd, 1H), 5.06 (s. 2H), 3.88 (q, 2H), 3.38-3.28 (m, 2H), 2.33-2.24 (m, 2H), 1.85-1.70 (m, 4H), 1.26 (t, 3H).
LC-MS (Methode 6): Rₜ = 1.37 min.
MS (ESIpos, *m*/*z*): 235 (M+H)⁺.

### Beispiel 92A

### 3-({[tert.-Butyl(diphenyl)silyl]oxy}methyl)piperidin-2-on

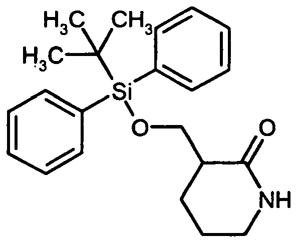

Eine Lösung von 5.00 g (38.7 mmol) 3-Hydroxymethylpiperidin-2-on in 40 ml *N,N-*Dimethylformamid wird nacheinander mit 3.16 g (46.5 mmol) Imidazol und tropfenweise mit 11 ml (42.6 mmol) tert.-Butyl(diphenyl)silylchlorid versetzt. Nach drei Stunden Rühren bei RT wird mit ca. 400 ml Wasser versetzt und dreimal mit Essigsäureethylester extrahiert. Die vereinigten organischen Extrakte werden nacheinander mit gesättigter Ammoniumchlorid-Lösung, Wasser und gesättigter Natriumchlorid-Lösung gewaschen. Nach Trocknen über wasserfreiem Magnesiumsulfat wird filtriert und das Filtrat im Vakuum vom Lösemittel befreit. Der erhaltene Rückstand wird durch Saugfiltration über Kieselgel mit Cyclohexan/Essigsäureethylester 20:1 → 1:1 als Laufmittel gereinigt. Es werden 9.43 g (66% d. Th.) der Titelverbindung erhalten.
¹H-NMR (400 MHz, CDCl₃, *δ*/*ppm*): 7.69-7.65 (m, 4H), 7.42-7.34 (m, 6H), 5.82 (s, breit, 1H), 4.03 (dd, 1H), 3.93 (dd, 1H), 3.32-3.28 (m, 2H), 2.53-2.48 (m, 1H), 2.07-1.99 (m, 1H), 1.96-1.87 (m, 2H), 1.78-1.68 (m, 1H), 1.04 (s, 9H).
HPLC (Methode 3): Rₜ = 2.79 min.
MS (ESIpos, *m*/*z*): 368 (M+H)⁺.

### Beispiel 93A

### 1-(4-Amino-2-chlorphenyl)-3-({[tert.-butyl(diphenyl)silyl]oxy}methyl)piperidin-2-on

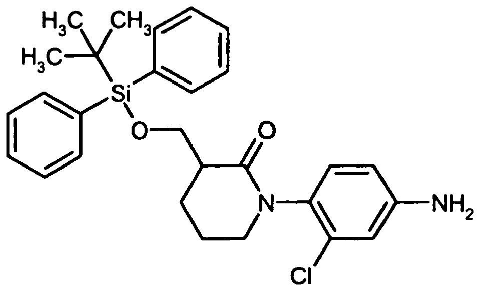

2.75 g (10.8 mmol) 3-Chlor-4-jodanilin werden in 20 ml wasserfreiem Dioxan gelöst und nacheinander mit 4.98 g (13.5 mmol) der Verbindung aus Beispiel 92A, 103 mg (0542 mmol) Kupfer(I)-jodid, 4.61 g (21.7 mmol) Kaliumphosphat und 116 µl (1.085 mmol) *N,N'-*Dimethylethylendiamin versetzt. Die Rückflußapparatur wird durch wiederholtes Anlegen eines leichten Vakuums und Begasen mit Argon inertisiert. Das Reaktionsgemisch wird 15 Stunden zum Rückfluß erhitzt. Nach dieser Zeit läßt man auf RT abkühlen. Es wird mit Wasser versetzt und mit Essigsäureethylester extrahiert. Der organische Extrakt wird nacheinander mit Wasser und gesättigter Natriumchlorid-Lösung gewaschen. Dann wird über wasserfreiem Magnesiumsulfat getrocknet, filtriert und das Filtrat im Vakuum vom Lösemittel befreit. Das so erhaltene Rohprodukt wird zunächst durch Saugfiltration über Kieselgel mit Cyclohexan/Essigsäureethylester 20:1 → 1:4 als Laufmittel gereinigt. Es werden 3.12 g eines immer noch verunreinigten Produktes erhalten, das mittels präparativer HPLC mit einem Acetonitril/WasserGemisch weiter aufgereinigt wird. Es werden 0.71 g (13% d. Th.) der Titelverbindung erhalten.
¹H-NMR (400 MHz, DMSO-d₆, *δ*/*ppm*): 7.67-7.62 (m, 4H), 7.48-7.40 (m, 6H), 6.91 und 6.81 (2 d, zusammen 1H), 6.67 und 6.62 (2 d, zusammen 1H), 6.50 und 6.47 (2 dd, zusammen, 1H), 5.41 (s, breit, 2H), 4.08-4.00 (m, 1H), 3.78-3.73 (m, 1H), 3.50-3.41 (m, 1H), 3.40-3.32 (m, 1H, teilweise überdeckt vom Wassersignal), 2.07-1.96 (m, 3H), 1.92-1.81 (m, 2H), 1.01 und 1.00 (2 s, zusammen 9H).
HPLC (Methode 5): Rₜ = 3.26 min.
MS (ESIpos, *m*/*z*): 493/495 (³⁵Cl/³⁷Cl) (M+H)⁺.

### Beispiel 94A

### N-[(2R)-3-({4-[3-({[tert.-Butyl(diphenyl)silyl]oxy}methyl)-2-oxopiperidin-1-yl)-3-chlorphenyl}-amino)-2-hydroxypropyl]-5-chlorthiophen-2-carbonsäureamid

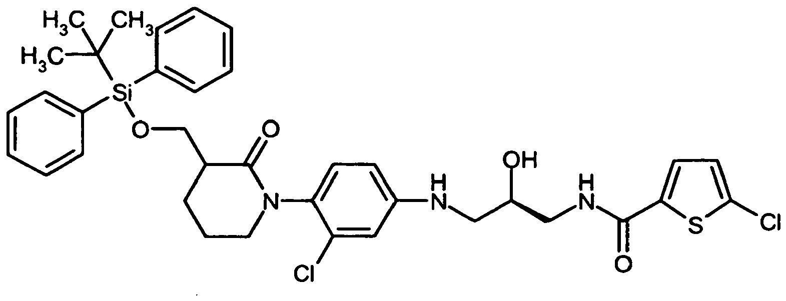

Eine Lösung von 665 mg (1.35 mmol) der Verbindung aus Beispiel 93A und 323 mg (1.48 mmol) der Verbindung aus Beispiel 1A in 3 ml Acetonitril wird mit 452 mg (2.02 mmol) Magnesiumperchlorat versetzt und 15 Stunden bei RT gerührt. Der Ansatz wird ohne Vorbehandlung über eine präparative HPLC mit einem Acetonitril/Wasser-Gemisch aufgereinigt. Es werden 871 mg (91% d. Th.) der Titelverbindung erhalten.
¹H-NMR (400 MHz, DMSO-d₆, *δ*/*ppm*): 8.60 (t, 1H), 7.68-7.62 (m, 5H), 7.48-7.40 (m, 6H), 7.18 (d, 1H), 6.97 und 6.86 (2 d, zusammen 1H), 6.70 und 6.64 (2 d, zusammen 1H), 6.56 und 6.53 (2 dd, zusammen, 1H), 5.98 (t, breit, 1H), 5.11 (d, 1H), 4.09-4.01 (m, 1H), 3.80-3.73 (m, 2H), 3.51-3.42 (m, 1H), 3.38-3.22 (m, 2H, teilweise überdeckt vom Wassersignal), 3.71-3.08 (m, 1H), 3.00-2.93 (m, 1H), 2.58-2.52 (m, 1H), 2.08-1.96 (m, 3H), 1.91-1.82 (m, 2H), 1.01 und 1.00 (2 s, zusammen 9H).
HPLC (Methode 4): Rₜ = 3.40 min.
MS (ESIpos, *m*/*z*): 710/712/714 (Cl₂, ^{3s}Cl/³⁷Cl) (M+H)⁺.

### Beispiel 95A

### N-[((5S)-3-{4-[3-({[tert.-Butyl(diphenyl)silyl]oxy}methyl)-2-oxopiperidin-1-yl]-3-chlorphenyl}-2-oxo-1,3-oxazolidin-5-yl)methyl]-5-chlorthiophen-2-carbonsäureamid

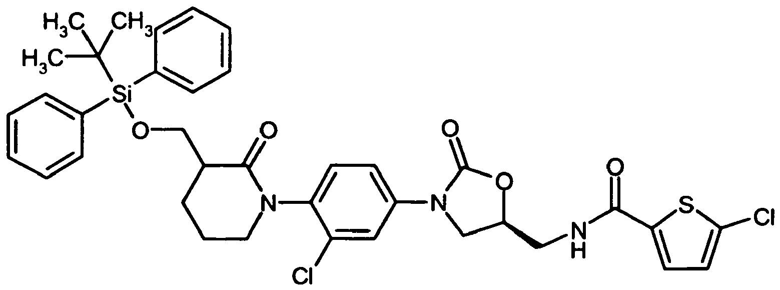

Eine Lösung von 850 mg (1.196 mmol) der Verbindung aus Beispiel 94A und 387 mg (2.39 mmol) Carbonyldiimidazol in 30 ml Butyronitril wird mit 3 mg (0.024 mmol) 4-Dimethylaminopyridin versetzt und zum Rückfluß erhitzt. Nach 15 Stunden wird der grösste. Teil des Lösemittels am Rotationsverdampfer entfernt. Das Produkt wird mittels präparativer HPLC mit einem Acetonitril/Wasser-Gemisch weiter isoliert. Es werden 605 mg (69% d. Th.) der Titelverbindung erhalten.
¹H-NMR (400 MHz, DMSO-d₆, *δ*/*ppm*): 8.97 (t, 1H), 7.80 und 7.72 (2 d, zusammen 1H), 7.68 (d, 1H), 7.66-7.63 (m, 4H), 7.52-7.41 (m, 7H), 7.37 und 7.23 (2 d, zusammen 1H), 7.19 (d, 1H), 4.88-4.82 (m, 1H), 4.22-4.17 (m, 1H), 4.09-4.03 (m, 1H), 3.89-3.83 (m, 1H), 3.81-3.74 (m, 1H), 3.61-3.37 (m, 3H), 2.64-2.57 (m, 1H), 2.11-2.00 (m, 3H), 1.97-1.86 (m, 2H), 1.02 und 1.00 (2 s, zusammen 9H).
HPLC (Methode 4): Rₜ = 3.44 min.
MS (ESIpos, *m*/*z*): 736/738/740 (Cl₂, ³⁵Cl/³⁷Cl) (M+H)⁺.

### Beispiel 96A

### 3-Allylpiperidin-2-on

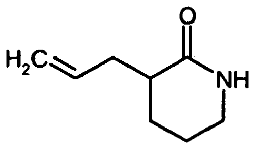

Eine Lösung von 5.00 g (50.4 mmol) Piperidin-2-on in 75 ml wasserfreiem Tetrahydrofuran wird bei einer Temperatur von 0°C tropfenweise mit 69 ml (110 mmol) einer 1.6-molaren Lösung von n-Butyllithium in Hexan versetzt. Nach einer weiteren Stunde bei 0°C wird das Reaktionsgemisch auf -78°C abgekühlt und dann mit einer Lösung von 5.3 ml (60.5 mmol) Allylbromid in 25 ml wasserfreiem Tetrahydrofuran tropfenweise versetzt. Nach beendeter Zugabe läßt man das Reaktionsgemisch innerhalb von 45 Minuten auf RT erwärmen. Dann werden 500 ml Wasser zugesetzt, und es wird dreimal mit je ca. 200 ml Essigsäureethylester extrahiert. Die vereinigten organischen Extrakte werden nacheinander mit Wasser und gesättigter Natriumchloridlösung gewaschen und über wasserfreiem Magnesiumsulfat getrocknet. Nach Filtration wird das Lösemittel am Rotationsverdampfer entfernt. Es werden 4.89 g (70% d. Th.) der Titelverbindung erhalten.
¹H-NMR (400 MHz, CDCl₃, *δ*/*ppm*): 5.99 (s, breit, 1H), 5.83-5.72 (m, 1H), 5.10-5.03 (m, 2H), 3.31-3.27 (m, 2H), 2.70-2.64 (m, 1H), 2.38-2.24 (m, 2H), 1.96-1.88 (m, 2H), 1.77-1.67 (m, 1H), 1.59-1.51 (m, 1H).
HPLC (Methode 1): Rₜ = 3.57 min.
MS (DCI, NH₃, *m*/*z*): 140 (M+H)⁺, 157 (M+NH₄)⁺, 174 (M+N₂H₇)⁺.

### Beispiel 97A

### 3-Allyl-1-(4-amino-2-chlorphenyl)piperidin-2-on

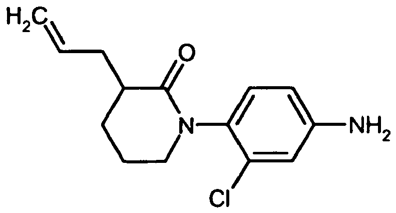

Analog zu dem unter Beispiel 93A beschriebenen Verfahren werden aus 6.00 g (23.7 mmol) 3-Chlor-4-iodanilin und 4.11 g (29.6 mmol) der Verbindung aus Beispiel 96A 1.57 g (25% d. Th.) der Titelverbindung erhalten. Die Aufreinigung erfolgt durch Saugfiltration über Kieselgel mit Cyclohexan/Essigsäureethylester 2:1 → 1:2 als Laufmittel.
¹H-NMR (400 MHz, CDCl₃, *δ*/*ppm*): 6.98 und 6.95 (zwei d, zusammen 1H), 6.75 und 6.73 (zwei d, zusammen 1H), 6.57 und 6.54 (zwei dd, zusammen 1H), 5.90-5.78 (m, 1H), 5.12-5.03 (m, 2H), 3.73 (s, breit, 2H), 3.51-3.38 (m, 2H), 2.78-2.68 (m, 1H), 2.53-2.31 (m, 2H), 2.07-1.85 (m, 4H).
HPLC (Methode 1): Rₜ = 3.88 min.
MS (DCI, NH₃, *m*/*z*): 265/267 (³⁵Cl/³⁷Cl) (M+H)⁺.

### Beispiel 98A

### N-((2R)-3-{[4-(3-Allyl-2-oxopiperidin-1-yl)-3-chlorphenyl]amino}-2-hydroxypropyl)-5-chlorthiophen-2-carbonsäureamid

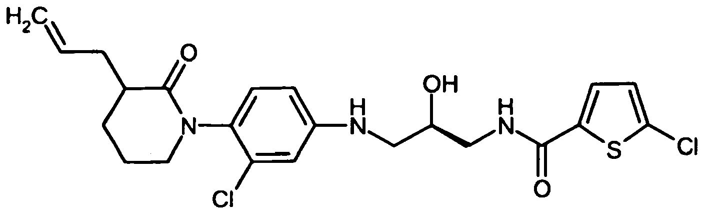

In Analogie zu dem unter Beispiel 94A beschriebenen Verfahren werden aus 1.40 g (5.29 mmol) der Verbindung aus Beispiel 97A 2.09 g (82% d. Th.) der Titelverbindung erhalten. Die Aufreinigung erfolgt durch Saugfiltration über Kieselgel mit Cyclohexan/Essigsäureethylester 5:1 → 1:4 als Laufmittel.
¹H-NMR (400 MHz, CDCl₃, *δ*/*ppm*): 7.32-7.31 (m, 1H), 7.22-7.19 (m, 1H), 6.93-6.84 (m, 2H), 6.56 (s, 1H), 6.43-6.39 (m, 1H), 5.87-5.73 (m, 1H), 5.12-5.07 (m, 2H), 4.58-4.52 (m, 1H), 4.15-4.09 (m, 1H), 3.71-3.64 (m, 1H), 3.60-3.40 (m, 2H), 3.33-3.30 (m, 2H), 3.01-2.96 (m, 2H), 2.73-2.63 (m, 1H), 2.59-2.48 (m, 1H), 2.46-2.32 (m, 1H), 2.07-1.86 (m, 3H), 1.80-1.67 (m, 1H).
HPLC (Methode 2): Rₜ = 4.38 min.
MS (ESIpos, *m*/*z*): 482/484/486 (Cl₂, ³⁵Cl/³⁷Cl) (M+H)⁺.

### Beispiel 99A

### 3-Allyl-1-(4-amino-2-methylphenyl)piperidin-2-on

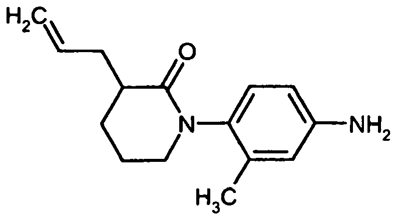

Analog zu dem unter Beispiel 93A beschriebenen Verfahren werden aus 2.68 g (11.5 mmol) 4-Iod-3-methylanilin und 2.0 g (14.3 mmol) der Verbindung aus Beispiel 96A nach präparativer HPLC 1.31 g (47% d. Th.) der Titelverbindung erhalten.
HPLC (Methode 1): Rₜ = 3.76 und 3.98 min.
MS (DCI, NH₃, *m*/*z*): 245 (M+H)⁺, 262 (M+NH₄)⁺.

### Beispiel 100A

### N-((2R)-3-{[4-(3-Allyl-2-oxopiperidin-1-yl)-3-methylphenyl]amino}-2-hydroxypropyl)-5-chlorthiophen-2-carbonsäureamid

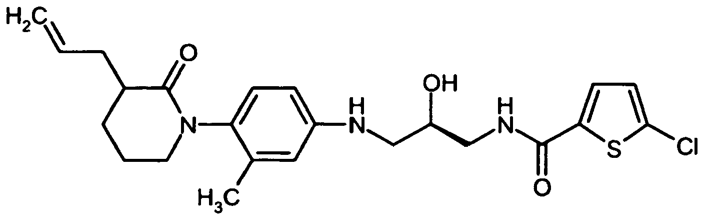

In Analogie zu dem unter Beispiel 94A beschriebenen Verfahren werden aus 1.3 g (5.32 mmol) der Verbindung aus Beispiel 99A nach präparativer HPLC 990 mg (83% d. Th.) der Titelverbindung erhalten.
¹H-NMR (400 MHz, DMSO-d₆, *δ*/*ppm*): 8.60 (t, 1H), 7.69 (d, 1H), 7.18 (d, 1H), 6.79 und 7.75 (2 d, zusammen, 1H), 6.44-6.39 (m, 2H), 5.83-73 (m, 1H), 5.48 und 5.03 (2 s, breit, zusammen 1H), 5.10-5.05 (m, 2H), 3.81-3.76 (m, 1H), 3.48-3.40 (m, 1H), 3.37-3.22 (m, 4H, teilweise überdeckt vom Wassersignal), 3.11-3.05 (m, 1H), 2.98-2.92 (m, 1H), 2.57-2.47 (m, 1H, teilweise überdeckt vom DMSO-Signal), 2.43-2.35 (m, 1H), 2.33-2.18 (m, 1H), 1.93 und 1.92 (2 s, zusammen 3H) 1.92-1.87 (m, 2H), 1.86-1.77 (m, 1H), 1.61-1.53 (m, 1H).
HPLC (Methode 2): Rₜ = 4.16 min.
MS (DCI, NH₃, *m*/*z*): 462/464 (³⁵Cl/³⁷Cl) (M+H)⁺, 479/481 (M+NH₄)⁺.

### Beispiel 101A

### N-({(5S)-3-[4-(3-Allyl-2-oxopiperidin-1-yl)-3-chlorphenyl]-2-oxo-1,3-oxazolidin-5-yl}methyl)-5-chlorthiophen-2-carbonsäureamid

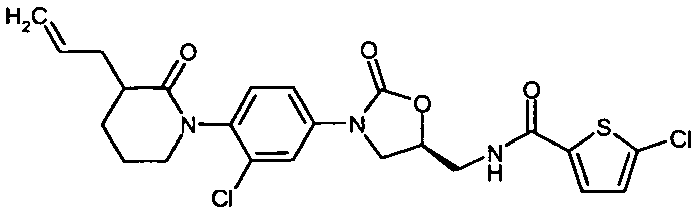

Analog zu dem unter Beispiel 95A beschriebenen Verfahren werden 2.05 g (4.25 mmol) der Verbindung aus Beispiel 98A zu 1.44 g (67% d. Th.) der Titelverbindung als DiastereomerenGemisch umgesetzt. Die Aufreinigung erfolgt durch Saugfiltration über Kieselgel mit Cyclohexan/Essigsäureethylester 5:1 → 1:3 als Laufmittel.
¹H-NMR (400 MHz, CDCl₃, *δ*/*ppm*): 7.72-7.67 (m, 1H), 7.44-7.37 (m, 1H), 7.33-7.31 (m, 1H), 7.23-7.18 (m, 1H), 6.92-6.87 (m, 2H), 5.88-5.77 (m, 1H), 5.12-5.06 (m, 2H), 4.82-4.77 (m, 1H), 4.06-3.98 (m, 1H), 3.82-3.74 (m, 2H), 3.71-3.63 (m, 1H), 3.61-3.42 (m, 2H), 2.77-2.66 (m, 1H), 2.60-2.50 (m, 1H), 2.47-2.32 (m, 1H), 2.09-1.88 (m, 3H), 1.81-1.63 (m, 1H).
HPLC (Methode 2): Rₜ = 4.50 min.
MS (ESIpos, *m*/*z):* 508/510/512 (Cl₂, ³⁵Cl/³⁷Cl) (M+H)⁺.

Das Diastereomerengemisch kann in präparativern Maßstab chromatographisch in die reinen Diastereomere aufgetrennt werden. Dazu werden 1.10 g der Verbindung aus Beispiel 101A in 16 ml des Laufmittels gelöst und in vier Portionen chromatographiert. Es werden 386 mg (35% d. Th.) der Titelverbindung (Diastereomer 2) und 417 mg (38% d. Th.) des Diastereomers 1 erhalten.
Methode: Säule: Daicel Chiralpak AD-H, 5 µm, 250 mm x 20 mm; Laufmittel: Ethanol + 1% Wasser + 0.2% Trifluoressigsäure; Fluß: 15 ml/min; Temperatur: 40°C; UV-Detektion: 220 nm.
Retentionszeit: 10.5 min (Diastereomer 1), 18.8 min (Diastereomer 2)
¹H-NMR (400 MHz, CDCl₃, *δ*/*ppm*): 7.72 und 7.67 (2 d, zusammen 1H), 7.43 und 7.39 (2 dd, zusammen 1H), 7.33 und 7.32 (2 d, zusammen 1H), 7.22 und 7.19 (2 d, zusammen 1H), 6.89 (d, 1H), 6.81 (t, 1H), 5.88-5.77 (m, 1H), 5.12-5.06 (m, 2H), 4.83-4.78 (m, 1H), 4.07-4.00 (m, 1H), 3.80 (dd, 2H), 3.71-3.63 (m, 1H), 3.62-3.41 (m, 2H), 2.77-2.66 (m, 1H), 2.62-2.51 (m, 1H), 2.47-2.32 (m, 1H), 2.09-1.90 (m, 3H), 1.82-1.68 (m, 1H).
HPLC (Methode 4): Rₜ = 2.60 min.
MS (ESIpos, *m*/*z*): 508/510/512 (Cl₂, ³⁵Cl/³⁷Cl) (M+H)⁺.

### Beispiel 102A

### N-({(5S)-3-[4-(3-Allyl-2-oxopiperidin-1-yl)-3-methylphenyl]-2-oxo-1,3-oxazolidin-5-yl}methyl)-5-chlorthiophen-2-carbonsäureamid

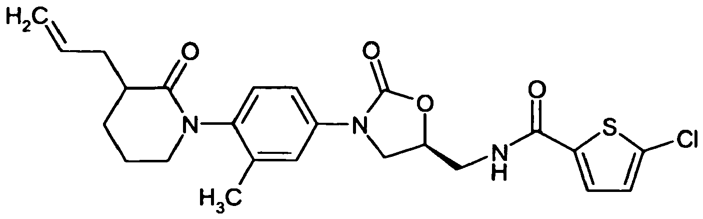

Analog zu dem unter Beispiel 95A beschriebenen Verfahren werden 980 mg (2.12 mmol) der Verbindung aus Beispiel 100A zu 790 mg (76% d. Th.) der Titelverbindung umgesetzt.
¹H-NMR (400 MHz, CDCl₃, *δ*/*ppm*): 7.41-7.37 (m, 2H), 7.33 und 7.32 (2 d, zusammen 1H), 7.12-7.06 (m, 1H), 6.88 (d, 1H), 6.85-6.77 (m, 1H), 5.87-5.77 (m, 1H), 5.12-5.08 (m, 2H), 4.79-4.70 (m, 1H), 4.03-3.98 (m, 1H), 3.82-3.73 (m, 2H), 3.64-3.50 (m, 2H), 3.44-3.22 (m, 1H), 2.77-2.66 (m, 1H), 2.59-2.51 (m, 1H), 2.47-2.31 (m, 1H), 2.19 und 2.18 (2 s, zusammen 3H), 2.09-1.98 (m, 2H), 1.96-1.87 (m, 1H), 1.79-1.68 (m, 1H).
HPLC (Methode 2): Rₜ = 4.56 min.
MS (ESIpos, *m*/*z*): 488/490 (³⁵Cl/³⁷Cl) (M+H)⁺.

### Beispiel 103A

### 1-(5-Chlor-2-methyl-4-nitrophenyl)-3-methylpyridin-2(1H)-on

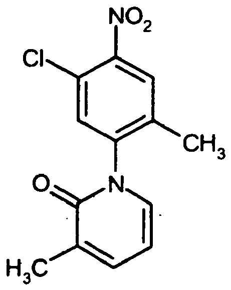

1.50 g (13.8 mmol) 2-Hydroxy-3-methylpyridin in 70 ml DMF werden bei 0°C mit 2.31 g (20.6 mmol) Kalium-tert.-butylat versetzt und es wird 30 min bei Raumtemperatur gerührt. 2.87 g (15.1 mmol) 2-Chlor-4-fluor-5-methylnitrobenzol werden zugefügt und es weiter bei RT gerührt. Nach 17 h wird mit 1000 ml Wasser versetzt und dann dreimal mit Essigsäureethylester extrahiert. Die vereinigten organischen Phasen werden über Natriumsulfat getrocknet und nach Filtration werden die Lösungsmittel im Vakuum entfernt. Der Rückstand wird durch Chromatographie an Kieselgel (Cyclohexan/Essigsäureethylester 2:1) aufgereinigt. Man erhält 2.15 g (56% d. Th.) der gewünschten Verbindung.
¹H-NMR (400 MHz, DMSO-*d₆*, *δ*/*ppm*): 8.18 (s, 1H), 7.85 (s, 1H), 7.48-7.44 (m, 2H), 6.31 (dd, 1H), 2.08 (s, 3H), 2.05 (s, 3H).
HPLC (Methode 1): Rₜ = 4.05 min.
MS (DCI, *m*/*z*): 279 (M+H)⁺.

### Beispiel 104A

### 3-Methyl-1-{2-methyl-5-[methyl(propyl)amino]-4-nitrophenyl}pyridin-2(1H)-on

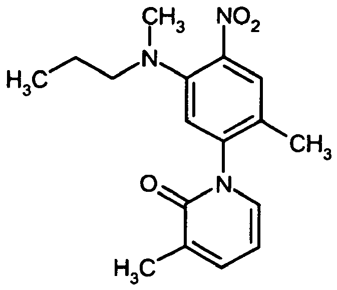

400 mg (1.44 mmol) Beispiel 103A und 315 mg (4.31 mmol) 1-Methylpropylamin in 10 ml DMF werden bei 120°C gerührt. Nach 4 h wird mit 200 ml Wasser versetzt und dann dreimal mit Essigsäureethylester extrahiert. Die vereinigten organischen Phasen werden über Natriumsulfat getrocknet und nach Filtration werden die Lösungsmittel im Vakuum entfernt. Der Rückstand wird durch präparative HPLC mit einem Acetonitril/Wasser-Gemisch aufgereinigt. Man erhält 216 mg (48% d. Th.) der gewünschten Verbindung.
¹H-NMR (400 MHz, DMSO-d₆, *δ*/*ppm*): 7.77 (s, 1H), 7.70-7.45 (m, 2H), 7.14 (s, 1H), 6.26 (dd, 1H), 3.05 (t, 2 H), 2.72 (s, 3H), 2.07 (s, 3H), 2.05 (s, 3H), 1.51 (dt, 2H), 0.81 (t, 3H).
HPLC (Methode 2): Rₜ = 4.36 min.
MS (DCI, *m*/*z*): 316 (M+H)⁺.

### Beispiel 105A

### 1-{4-Amino-2-methyl-5-[methyl(propyl)amino]phenyl}-3-methylpyridin-2(1H)-on

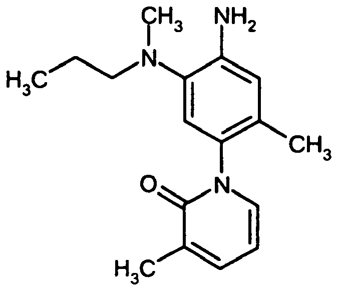

200 mg (0.63 mmol) Beispiel 104A werden in einem Gemisch von 5 ml THF und 5 ml Ethanol gelöst. Dann werden 7.7 mg (0.03 mmol) Platin(IV)oxidhydrat hinzugefügt und es wird 17 h bei RT in einer Wasserstoffatmosphäre unter normalem Druck hydriert. Anschließend wird filtriert und das Filtrat vom Lösungsmittel befreit. Das Reaktionsprodukt wird ohne weitere Aufreinigung weiter umgesetzt.
¹H-NMR (400 MHz, DMSO-*d₆*, *δ*/*ppm*): 7.37-7.33 (m, 1H), 7.29-7.26 (m, 1H), 6.67 (s, 1H), 6.58 (s, 1H), 6.15 (dd, 1H), 4.86 (s, 2H), 2.71 (t, 2H), 2.01 (s, 3H), 1.82 (s, 3H), 1.45 (dt, 2H), 1.36 (s, 3H), 0.85 (t, 3H).
HPLC (Methode 4): Rₜ = 1.75 min.
MS (ESIpos, *m*/*z*): 286 (M+H)⁺.

### Beispiel 106A

### 1-{5-[[2-(Dimethylamino)ethyl](methyl)amino]-2-methyl-4-nitrophenyl}-3-methylpyridin-2(1H)-on

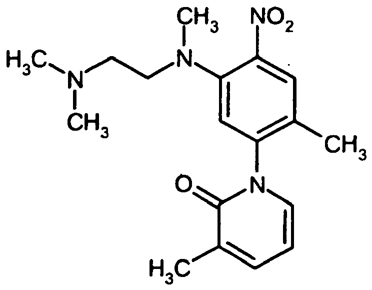

400 mg (1.44 mmol) Beispiel 103A und 880 mg (8.61 mmol) N,N,N'-Trimethylethan-1,2-diamin in 10 ml DMF werden bei 120°C gerührt. Nach 2 h wird mit 200 ml Wasser versetzt und dann dreimal mit Dichlormethan extrahiert. Die vereinigten organischen Phasen werden über Natriumsulfat getrocknet und nach Filtration werden die Lösungsmittel im Vakuum entfernt. Der Rückstand wird durch präparative HPLC mit einem Acetonitril/Wasser-Gemisch aufgereinigt. Man erhält 349 mg (71% d. Th.) der gewünschten Verbindung.
¹H-NMR (400 MHz, DMSO-*d₆*, *δ*/*ppm*): 7.76 (s, 1H), 7.47-7.40 (m, 2H), 7.15 (s, 1H), 6.27 (dd, 1H), 3.18 (t, 2 H), 2.76 (s, 3H), 2.43 (t, 2H), 2.14 (s, 6H), 2.05 (s, 3H), 1.95 (s, 3H).
HPLC (Methode 2): Rₜ = 3.66 min.
MS (DCI, *m*/*z*): 345 (M+H)⁺.

### Beispiel 107A

### 1-{4-Amino-5-[[2-(dimethylamino)ethyl](methyl)amino]-2-methylphenyl}-3-methylpyridin-2(1H)-on

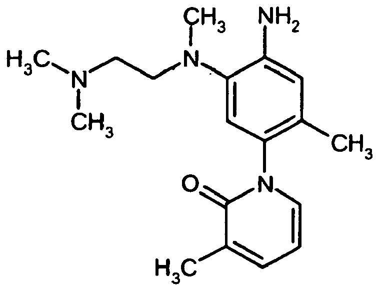

325 mg (0.94 mmol) Beispiel 106A werden in einem Gemisch von 10 ml THF und 10 ml Ethanol gelöst. Dann werden 11 mg (0.05 mmol) Platin(IV)oxidhydrat hinzugefügt und es wird 17 h bei RT in einer Wasserstoffatmosphäre unter normalem Druck hydriert. Anschließend wird filtriert, mit THF nachgewaschen und das Filtrat vom Lösungsmittel befreit. Das Reaktionsprodukt wird ohne weitere Aufreinigung weiter umgesetzt.
¹H-NMR (400 MHz, DMSO-*d₆*, *δ*/*ppm*): 7.29-7.25 (m, 1H), 7.38-7.33 (m, 1H), 6.68 (s, 1H), 6.55 (s, 1H), 6.15 (dd, 1H), 5.75 (s, 2H), 2.79 (t, 2H), 2.55 (s, 3H), 2.39 (t, 2H), 2.21 (s, 6H), 2.02 (s, 3H), 1.83 (s, 3H).
HPLC (Methode 1): Rₜ = 3.26 min.
MS (DCI, *m*/*z*): 315 (M+H)⁺.

### Beispiel 108A

### 3-Methyl-1-(2-methyl-4-nitro-5-propoxyphenyl)pyridin-2(1H)-on

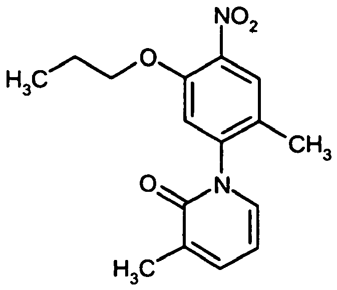

300 mg (1.08 mmol) Beispiel 103A und 151 mg (2.69 mmol) Kaliumhydroxid werden in 7.5 ml 1-Propanol 30 h bei 60°C gerührt. Dann wird mit 200 ml Wasser versetzt und dreimal mit Essigsäureethylester extrahiert. Die vereinigten organischen Phasen werden über Natriumsulfat getrocknet und nach Filtration werden die Lösungsmittel im Vakuum entfernt. Das so erhaltene Produkt wird ohne Aufreinigung weiter umgesetzt.
¹H-NMR (400 MHz, DMSO-*d₆*, *δ*/*ppm*): 7.92 (s, 1H), 7.47-7.41 (m, 2H), 7.33 (s, 1H), 6.29 (dd, 1H), 4.09 (t, 2 H), 2.08 (s, 3H), 1.98 (s, 3H), 1.75-1.66 (m, 2H), 0.95 (t, 3H).
HPLC (Methode 1): Rₜ = 4.33 min.
MS (DCI, *m*/*z*): 320 (M+H)⁺.

### Beispiel 109A

### 1-(4-Amino-2-methyl-5-propoxyphenyl)-3-methylpyridin-2(1H)-on

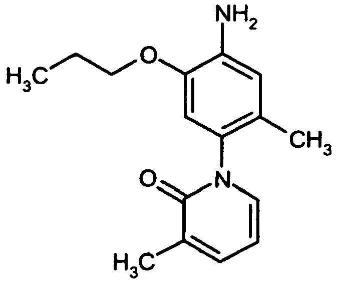

400 mg (1.32 mmol) Beispiel 108A werden in einem Gemisch von 10 ml THF und 10 ml Ethanol gelöst. Dann werden 16 mg (0.07 mmol) Platin(IV)oxidhydrat hinzugefügt und es wird 3 Tage bei RT in einer Wasserstoffatmosphäre unter normalem Druck hydriert. Anschließend wird filtriert, mit THF nachgewaschen und das Filtrat vom Lösungsmittel befreit. Das Reaktionsprodukt wird ohne weitere Aufreinigung weiter umgesetzt.
HPLC (Methode 3): Rₜ = 1.68 min.
MS (ESIpos, *m*/*z*): 273 (M+H)⁺.

### Beispiel 110A

### 1-Benzyl-3,5-dichlorpyrazin-2(1H)-on

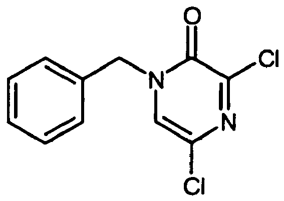

Zu einer Lösung aus 92 g (725 mmol) Oxalylchlorid in 188 ml 1,2-Dichlorbenzol werden 26.5 g (145 mmol) N-Benzylamino-acetonitril gegeben. Die Lösung wird 20 min auf 60°C und 6 h auf 90°C erhitzt. Man lässt erkalten und belässt bei dieser Temperatur für 15 h. Das Lösemittel wird im Vakuum entfernt und der Rückstand mit Kieselgel und mit einem Gradienten von Cyclohexan bis Cyclohexan/Essigsäureethylester 5:1 eluiert. Die Produktfraktionen werden vereinigt und im Vakuum zur Trockene eingedampft. Der Rückstand wird aus Diethylether kristallisiert. Man erhält 22 g (60% d. Th.) der gewünschten Verbindung.
¹H-NMR (400 MHz, CDCl₃, *δ*/*ppm*): δ = 7.4 (m, 3H), 7.35 (m, 2H), 7.15 (s, 1H), 5.1 (s, 2H).
LC-MS (Methode 3): Rₜ = 2.04 min
MS (ESIpos): m/z = 255 (M+H)⁺

### Beispiel 111A

### 1-Benzyl-5-chlor-3-ethoxypyrazin-2(1H)-on

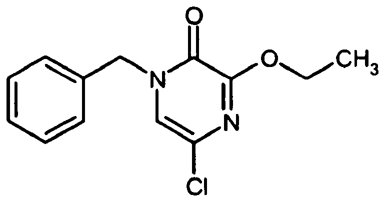

Zu einer Lösung von 1.00 g (3.92 mmol) Beispiel 110A in 8 ml wasserfreiem Ethanol werden 660 mg (7.8 mmol) Kaliumethylat gegeben. Nach 2 h werden 165 mg (196 mmol) Kaliumethylat zugegeben und nach weiteren 30 min abermals 165 mg (196 mmol) Kaliumethylat. Nach weiteren 30 min wird im Vakuum das Lösemittel entfernt und der Rückstand mit Wasser verdünnt. Es wird mehrmals mit Dichlormethan extrahiert, die vereinigten organischen Phasen werden mit gesättigter Natriumchloridlösung gewaschen, über Magnesiumsulfat getrocknet, filtriert und im Vakuum zur Trockene eingedampft. Man erhält 1.0 g (96% d. Th.) der gewünschten Verbindung.
¹H-NMR (400 MHz, CDCl₃, *δ*/*ppm*): δ = 7.4-7.3 (m, 5H), 6.8 (s, 1H), 5.05 (s, 2H), 4.4 (q, 2H), 1.45 (t, 3H).
LC-MS (Methode 5): Rₜ = 2.27 min
MS (ESIpos): m/z = 265 (M+H)⁺

### Beispiel 112A

### 3-Ethoxypyrazin-2(1H)-on

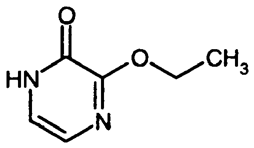

1.4 g (5.4 mmol) Beispiel 111A werden in 200 ml einer Mischung aus Ethanol und Essigsäureethylester 1:1 gelöst. Es werden 200 mg Palladium auf Kohle zugegeben. Es wird 1 h am Rückfluss erhitzt bevor, bei dieser Temperatur, weitere 200 mg Palladium auf Kohle und 2.1 g (32.5 mmol) Ammoniumformiat zugegeben werden. Nach weiteren 30 min werden erneut 200 mg Palladium auf Kohle und 2.1 g (32.5 mmol) Ammoniumformiat zugegeben. Es wird weitere 30 min bei dieser Temperatur gerührt, bevor man erkalten läßt und über Kieselgel filtriert. Das Filtrat wird im Vakuum zur Trockene eingedampft, der Rückstand in Methanol gelöst und mit präparativer HPLC mit einem Gradienten aus Wasser und Acetonitril aufgereinigt. Die Produktfraktionen werden vereinigt und im Vakuum zur Trockene eingedampft. Man erhält 579 mg (79% d. Th.) der gewünschten Verbindung.
¹H-NMR (400 MHz, DMSO-*d₆*, *δ*/*ppm*): δ = 6.95 (d, 1H), 6.75 (d, 1H), 4.25 (q, 2H), 1.3 (t, 3H).
LC-MS (Methode 6): Rₜ, = 2.17 min
MS (ESIpos): m/z = 141 (M+H)⁺

### Beispiel 113A

### 3-Ethoxy-1-(2-methyl-4-nitrophenyl)pyrazin-2(1H)-on

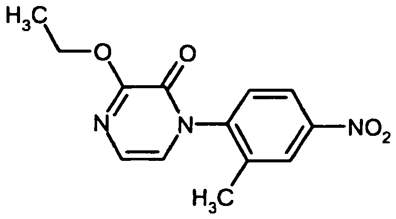

300 mg (2.14 mmol) Beispiel 112A werden in 7 ml Dimethylsulfoxid gelöst und bei Raumtemperatur mit 288 mg (2.6 mmol) Kalium-tert.-butylat versetzt. Die Suspension wird 30 min bei Raumtemperatur gerührt, bevor 332 mg (2.14 mmol) 1-Fluor-2-methyl-4-nitrobenzol zugesetzt werden und die Reaktionslösung für 20 h auf 80°C erhitzt wird. Es wird vorsichtig mit Wasser verdünnt. Die Lösung wird mehrmals mit Dichlormethan extrahiert. Die vereinigten organischen Extrakte werden mit Wasser und gesättigter Natriumchloridlösung gewaschen, über Magnesiumsulfat getrocknet, filtriert und im Vakuum eingedampft. Der Rückstand wird aus Methanol und tert.-Butylmethylether kristallisiert. Man erhält 429 mg (72% d. Th.) der gewünschten Verbindung.
¹H-NMR (400 MHz, CDCl₃, *δ*/*ppm*): δ = 8.25 (d, 1H), 8.2 (dd, 1H), 7.4 (d, 1H), 6.95 (d, 1H), 6.65 (d, 1H), 4.5 (m, 2H), 2.25 (s, 3H), 1.5 (t, 3H).
LC-MS (Methode 5): Rₜ = 1.90 min
MS (ESIpos): m/z = 276 (M+H)⁺

### Beispiel 114A

### 1-(4-Amino-2-methylphenyl)-3-ethoxypyrazin-2(1H)-on

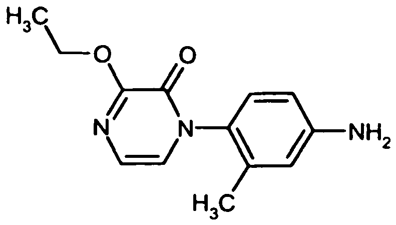

445 mg (1.62 mmol) Beispiel 113A werden in 64 ml einer 1:1 Mischung aus Ethanol und Essigsäureethylester gelöst und mit 611 mg (9.7 mmol) Ammoniumformiat und 50 mg Palladium auf Kohle versetzt. Es wird auf 80°C erhitzt. Nach 1 h lässt man erkalten und filtriert über Kieselgel. Es wird mit Essigsäureethylester nachgewaschen und das Filtrat im Vakuum zur Trockene eingedampft. Man erhält 400 mg (100% d. Th.) der gewünschten Verbindung.
¹H-NMR (400 MHz, DMSO-*d₆ δ*/*ppm*): δ = 7.0 (d, 1H), 6.85 (d, 1H), 6.8 (d, 1H), 6.5-6.4 (m, 2H), 5.3 (s, 2H), 4.3 (q, 2H), 1.8 (s, 3H) 1.3 (t, 3H).
LC-MS (Methode 3): Rₜ= 1.37 min
MS (ESIpos): m/z = 246 (M+H)⁺

### Beispiel 115A

### 1-[2-(Methoxymethyl)-4-nitrophenyl]piperidin-2-on

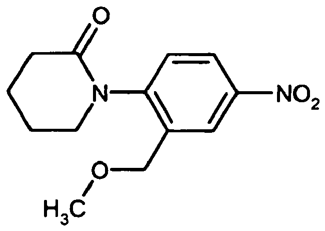

1.34 g (13.5 mmol) Piperidin-2-on werden in 40 ml wasserfreiem Dimethylsulfoxid gelöst und mit 1.82 g (16.2 mmol) Kalium-tert.-butylat versetzt. Nach 1 h werden 2.5 g (13.5 mmol) Beispiel 27A gelöst in 10 ml wasserfreiem Dimethylsulfoxid zugegeben und 3 h auf 80°C erhitzt. Man lässt erkalten und weitere 48 h stehen, bevor die Reaktionslösung mit Essigsäureethylester verdünnt und mit Wasser und 1N Salzsäure gewaschen wird. Die organische Phase wird über Magnesiumsulfat getrocknet, filtriert und im Vakuum zur Trockene eingedampft. Der Rückstand wird auf Kieselgel aufgezogen und an Kieselgel mit einer Mischung aus Cyclohexan und Essigsäureethylester 1:1 getrennt. Die Produktfraktionen werden vereinigt und im Vakuum zur Trockene eingedampft. Man erhält 620 mg (17% d. Th.) der gewünschten Verbindung.
¹H-NMR (400 MHz, DMSO-*d₆*, *δ*/*ppm*): δ = 8.25 (m, 2H), 7.6 (d, 1H), 4.35 (m, 2H), 4.0 (q, 2H), 4.0 (b, 2H), 3.55 (m, 2H), 3.3 (s, 3H) 2.4 (b, 2H).

### Beispiel 116A

### 1-[4-Amino-2-(methoxymethyl)phenyl]piperidin-2-on

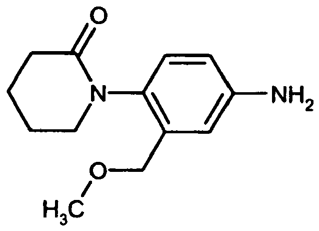

630 mg (2.38 mmol) Beispiel 115A werden in 50 ml einer 1:1 Mischung aus Ethanol und Essigsäureethylester gelöst und mit 752 mg (11.9 mmol) Ammoniumformiat und 65 mg Palladium auf Kohle versetzt. Es wird 30 min auf 80°C erhitzt. Man lässt erkalten und filtriert über Kieselgel. Es wird mit Essigsäureethylester und Ethanol nachgewaschen und das Filtrat im Vakuum zur Trockene eingedampft. Man erhält 560 mg (99% d. Th.) der gewünschten Verbindung.
LC-MS (Methode 6): Rₜ = 1.19 min
MS (ESIpos): m/z = 235 (M+H)⁺

### Ausführungsbeispiele

### Beispiel 1

### 5-Chlor-N-({(5S)-3-[3-chlor-4-(3-methoxy-2-oxopyridin-1(2H)-yl)phenyl]-2-oxo-1,3-oxazolidin-5-yl}methyl)thiophen-2-carboxamid

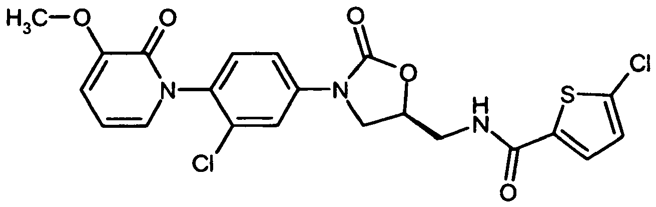

Eine Lösung von 247 mg (0.98 mmol) des Produktes aus Beispiel 3A in 6 ml Acetonitril wird mit 236 mg (1.08 mmol) des Produktes aus Beispiel 1A versetzt. Der Suspension werden 330 mg (1.48 mmol) Magnesiumperchlorat zugefügt, wonach man 17 h bei RT rühren lässt. Dann werden 400 mg (2.46 mmol) 1,1'-Carbonyldiimidazol und 12 mg (0.09 mmol) DMAP hinzugesetzt und es wird auf 60°C erhitzt. Nach 4 h wird mit Wasser, gesättigter wässriger Natriumchlorid-Lösung und Essigsäureethylester verdünnt. Die Wasserphase wird zweimal mit Essigsäureethylester extrahiert und die vereinigten organischen Phasen über Natriumsulfat getrocknet. Nach Filtration wird vom Lösungsmittel befreit und der Rückstand durch Chromatographie an Kieselgel (Dichlormethan/Methanol 20:1) vorgereinigt. Anschließende Reinigung mittels präparativer HPLC mit einem Acetonitril/Wasser-Gemisch liefert 65 mg (13% d. Th.) des gewünschten Produktes.
¹H-NMR (400 MHz, DMSO-*d₆*, *δ*/*ppm*): 8.97 (t, 1H), 7.86 (dd, 1H), 7.60 (d, 1H), 7.63-7.54 (m, 1H), 7.49 (d, 1H), 7.19 (d, 1H), 7.07 (dd, 1H), 6.90 (dd, 1H), 6.25 (dd, 1H), 4.93-4.82 (m, 1H), 4.24 (dd, 1H), 3.99 (dd, 1H), 3.74 (s, 3H), 3.65-3.56 (m, 2H).
HPLC (Methode 2): Rₜ = 4.03 min.
MS (ESIpos, *m*/*z*): 494 (M+H)⁺.

### Beispiel 2

### 5-Chlor-N-({(5S)-3-[4-(3-methoxy-2-oxopyridin-1(2H)-yl)-3-methylphenyl]-2-oxo-1,3-oxazolidin-5-yl}methyl)thiophen-2-carboxamid

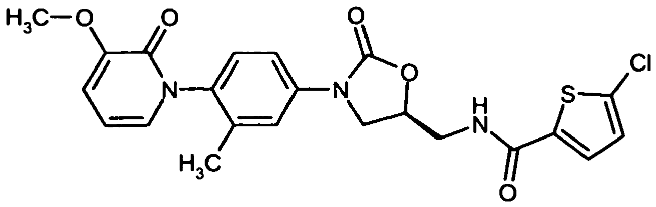

Eine Lösung von 106 mg (0.460 mmol) des Produktes aus Beispiel 16A in 9 ml Acetonitril wird mit 110 mg (0.506 mmol) des Produktes aus Beispiel 1A versetzt. Der Suspension werden 154 mg (0.690 mmol) Magnesiumperchlorat zugefügt, wonach man 18 h bei RT rühren lässt. Dann werden 186 mg (1.15 mmol) 1,1'-Carbonyldiimidazol und 5 mg (0.05 mmol) DMAP hinzugesetzt und es wird auf 60°C erhitzt. Nach 4 h wird abgekühlt und mit Wasser verdünnt. Die Wasserphase wird dreimal mit Essigsäureethylester extrahiert, die vereinigten organischen Phasen mit gesättigter wässriger Natriumchloridlösung gewaschen und über Natriumsulfat getrocknet. Nach Filtration wird vom Lösungsmittel befreit und der Rückstand durch Chromatographie an Kieselgel (Dichlormethan/Ethanol 20:1) gereinigt. Nach Entfernen der Lösungsmittel erhält man 150 mg (68% d. Th.) des gewünschten Produktes.
¹H-NMR (400 MHz, *DMSO-d₆, δ*/*ppm*): 8.97 (t, 1H), 7.69 (d, 1H), 7.57-7.45 (m, 2H), 7.28-7.13 (m, 2H), 7.03 (d, 1H), 6.90 (d, 1H), 6.24 (dd, 1H), 4.93-4.80 (m, 1H), 4.22 (dd, 1H), 3.92-3.85 (m, 1H), 3.74 (s, 3H), 3.62 (dd, 2H), 2.00 (s, 3H).
HPLC (Methode 2): Rₜ = 4.04 min.

### Beispiel 3

### 5-Chlor-N-({(5S)-3-[4-(3-methoxy-2-oxopyridin-1(2H)-yl)-3,5-dimethylphenyl]-2-oxo-1,3-oxazolidin-5-yl} methyl)thiophen-2-carboxamid

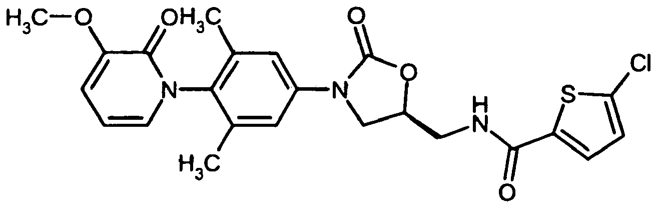

Eine Lösung von 195 mg (0.799 mmol) des Produktes aus Beispiel 18A in 10 ml Acetonitril wird mit 191 mg (0.878 mmol) des Produktes aus Beispiel 1A versetzt. Der Suspension werden 267 mg (1.197 mmol) Magnesiumperchlorat zugefügt, wonach man 5 h bei RT rühren lässt. Dann werden 259 mg (1.59 mmol) 1,1'-Carbonyldiimidazol und 9 mg (0.08 mmol) DMAP hinzugesetzt und es wird auf 60°C erhitzt. Nach 24 h wird abgekühlt und mit 50 ml Wasser verdünnt. Die Wasserphase wird dreimal mit Essigsäureethylester extrahiert und die vereinigten organischen Phasen über Natriumsulfat getrocknet. Nach Filtration wird vom Lösungsmittel befreit und der Rückstand durch Chromatographie an Kieselgel (Dichlormethan/Ethanol 20:1) gereinigt. Nach Entfernen der Lösungsmittel erhält man 258 mg (65% d. Th.) des gewünschten Produktes.
¹H-NMR 300 MHz, DMSO-*d₆*, *δ*/*ppm*): 8.98 (t, 1H), 7.69 (d, 1H), 7.42-7.32 (m, 2H), 7.20 (dd, 1H), 7.00-6.8 (m, 2H), 6.28 (dd, 1H), 4.91-4.80 (m, 1H), 4.20 (dd, 1H), 3.90-3.82 (m, 1H), 3.75 (s, 3H), 3.60 (dd, 2H), 1.95 (s, 6H).
HPLC (Methode 2): Rₜ = 4.05 min.
MS (ESIpos, *m*/*z*): 494 (M+H)⁺.

### Beispiel 4

### 5-Chlor-N-({(5S)-3-[4-(3-ethoxy-2-oxopyridin-1(2H)-yl)-3-methylphenyl]-2-oxo-1,3-oxazolidin-5-yl}methyl)thiophen-2-carboxamid

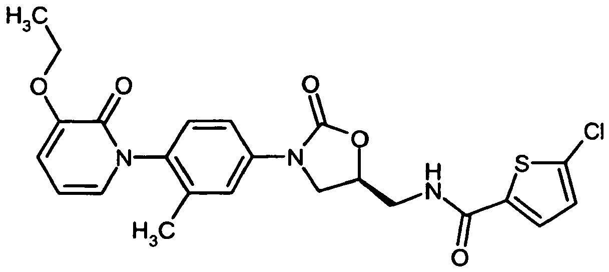

250 mg (0.544 mmol) Beispiel 15A werden in 2.5 ml wasserfreiem N,N-Dimethylformamid gelöst, mit 150 mg (1.1 mmol) Kaliumcarbonat versetzt und 30 min nachgerührt. Es werden 128 mg (0.872 mmol) Iodethan zugegeben und es wird 5 h auf 60°C erhitzt. Dann wird mit 1 ml Dimethylsulfoxid verdünnt, filtriert und mit präparativer HPLC mit einem Wasser/Acetonitril Gradient gereinigt. Die Produktfraktionen werden im Vakuum zur Trockene eingedampft und der Rückstand im Vakuum getrocknet. Nach Entfernen der Lösungsmittel erhält man 104 mg (39% d. Th.) des gewünschten Produktes.
¹H-NMR (400 MHz, DMSO- *d₆, δ*/*ppm*): δ = 8.95 (t, 1H), 7.70 (d, 1H), 7.55-7.46 (m, 2H), 7.25-7.17 (m, 2H), 7.03 (d, 1H), 6.88 (d, 1H), 6.22 (t, 1H), 4.90-4.82 (m, 1H), 4.22 (t, 1H), 4.02-3.92 (m, 2H), 3.91-3.84 (m, 1H), 3.61 (t, 2H), 2.01 (s, 3H), 1.35 (t, 3H).
LC-MS (Methode 4): Rₜ = 2.20 min
MS (ESIpos): m/z = 488 (M+H)⁺
Analog zur Darstellung von Beispiel 4 werden aus dem entsprechenden Alkylhalogenid und Beispiel 15A folgende Derivate hergestellt.

### Beispiel 5

### N-[((5S)-3-{4-[3-(2-Amino-2-oxoethoxy)-2-oxopyridin-1(2H)-yl]-3-methylphenyl}-2-oxo-1,3-oxazolidin-5-yl)methyl]-5-chlorthiophen-2-carboxamid

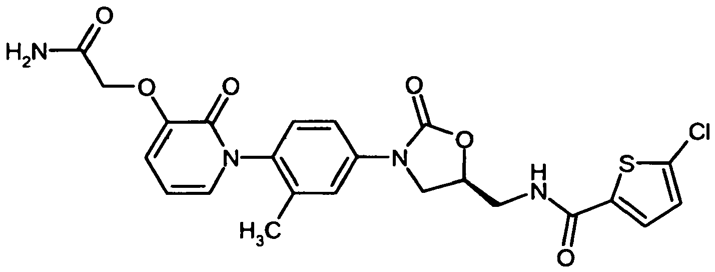

Ausbeute: 41.1 mg (53% d, Th.)
¹H-NMR (400 MHz, DMSO-d₆, *δ*/ppm): δ = 8.99 (t, 1H), 7.70 (d, 1H), 7.56-7.49 (m, 2H), 7.48-7.39 (m, 2H), 7.24 (d, 1H), 7.20 (d, 1H), 7.14 (d, 1H), 6.97 (d, 1H), 6.26 (t, 1H), 4.90-4.82 (m, 1H), 4.45 (s, 2H), 4.22 (t, 1H), 3.91-3.85 (m, 1H), 3.61 (t, 2H), 2.03 (s, 3H).
LC-MS (Methode 4): Rₜ = 1.88 min
MS (ESIpos): m/z = 517 (M+H)⁺

### Beispiel 6

### 5-Chlor-N-[((5S)-3-{4-[3-(2-methoxyethoxy)-2-oxopyridin-1(2H)-yl]-3-methylphenyl}-2-oxo-1,3-oxazolidin-5-yl)methyl]thiophen-2-carboxamid

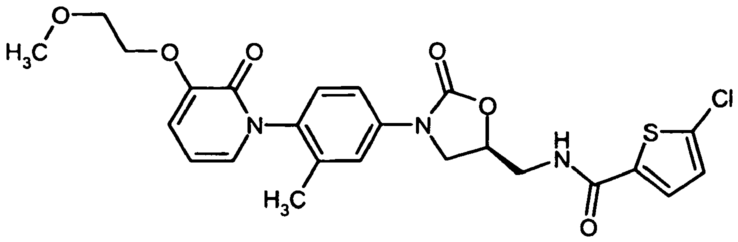

Ausbeute: 39.1 mg (50% d. Th.)
¹H-NMR (400 MHz, DMSO-*d₆*, *δ*/*ppm*): δ = 8.99 (t, 1H), 7.70 (d, 1H), 7.55-7.47 (m, 2H), 7.26-7.18 (m, 2H), 7.05 (d, 1H), 6.93 (d, 1H), 6.22 (t, 1H), 4.90-4.82 (m, 1H), 4.22 (t, 1H), 4.10-3.99 (m, 2H), 3.91-3.81 (m, 1H), 3.66 (t, 2H), 3.61 (t, 2H), 2.54 (s, 3H), 2.02 (s, 3H).
LC-MS (Methode 4): Rₜ = 2.09 min
MS (ESIpos): m/z = 518 (M+H)⁺

### Beispiel 7

### 5-Chlor-N-[((5S)-3-{3-methyl-4-[2-oxo-3-(2-oxopropoxy)pyridin-1(2H)-yl]phenyl}-2-oxo-1,3-oxazolidin-5-yl)methyl]thiophen-2-carboxamid

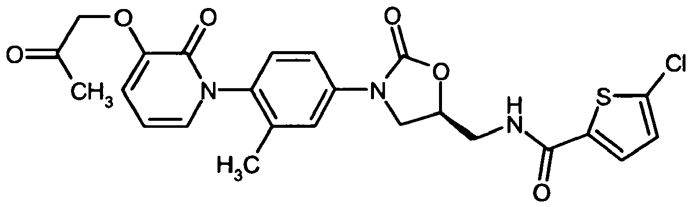

Ausbeute: 24.8 mg (32% d. Th.)
¹H-NMR (400 MHz, DMSO-*d₆*, *δ*/*ppm*): δ = 8.98 (t, 1H), 7.70 (d, 1H), 7.55-7.49 (m, 2H), 7.25 (d, 1H), 7.20(d, 1H), 7.11-7.07 (m, 1H), 6.85 (dd, 1H), 6.20 (t, 1H), 4.90-4.82 (m, 1H), 4.77 (s, 2H), 4.22 (t, 1H), 3.91-3.85 (m, 1H), 3.62 (t, 2H), 2.15 (s, 3H), 2.02 (d, 3H).
LC-MS (Methode 4): Rₜ = 2.04 min
MS (ESIpos): m/z = 516 (M+H)⁺

### Beispiel 8

### 5-Chlor-N-[((5S)-3-{4-[3-(2-fluorethoxy)-2-oxopyridin-1(2H)-yl]-3-methylphenyl}-2-oxo-1,3-oxazolidin-5-yl)methyl]thiophen-2-carboxamid

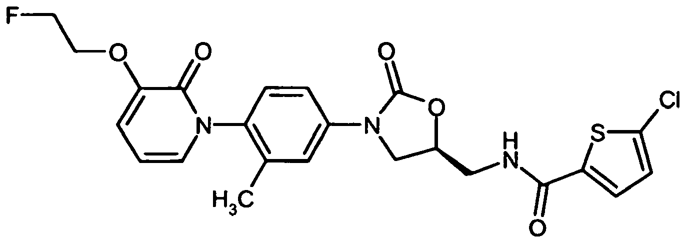

Ausbeute: 52.9 mg (52% d. Th.)
¹H-NMR (400 MHz, DMSO-*d₆*, *δ*/*ppm*): δ = 9.0 (t, 1H), 7.7 (d, 1H), 7.50 (m, 2H), 7.2 (m, 2H), 7.1 (m, 1H), 7.0 (m, 1H), 6.2 (t, 1H), 4.85 (m, 1H), 4.8 (m, 1H), 4.7 (m, 1H), 4.2 (m, 3H), 3.9 (m, 1H), 3.6 (t, 2H), 2.0 (s, 3H).
LC-MS (Methode 4): Rₜ = 2.23 min
MS (ESIpos): m/z= 506 (M+H)⁺

### Beispiel 9

### 5-Chlor-N-[((5S)-3-{4-[3-(2-hydroxy-2-methylpropoxy)-2-oxopyridin-1(2H)-yl]-3-methylphenyl}-2-oxo-1,3-oxazolidin-5-yl)methyl]thiophen-2-carboxamid

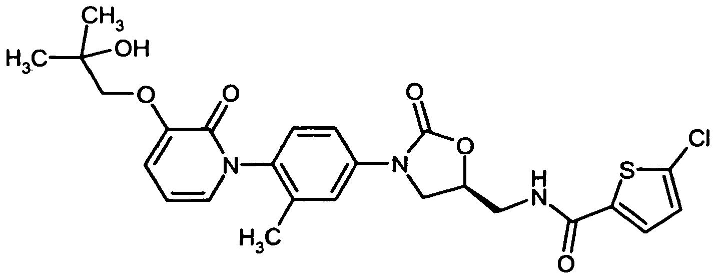

Ausbeute: 17.5 mg (11% d. Th.)
¹H-NMR (400 MHz, DMSO-*d₆, δ*/*ppm*): δ = 8.98 (t, 1H), 7.70 (d, 1H), 7.56-7.47 (m, 2H), 7.26-7.17 (m, 2H), 7.05 (d, 1H), 6.93 (d, 1H), 6.21 (t, 1H), 4.91-4.82 (m, 1H), 4.64 (s, 1H), 4.22 (t, 1H), 3.92-3.84 (m, 1H), 3.77-3.55 (m, 3H), 2.02 (s, 3H), 1.22-1.16 (m, 6H).
LC-MS (Methode 5): Rₜ = 2.05 min
MS (ESIpos): m/z = 532 (M+H)⁺

### Beispiel 10

### 5-Chlor-N-[((5S)-3-{3-methyl-4-[3-(2-morpholin-4-yl-2-oxoethoxy)-2-oxopyridin-1(2H)-yl]phenyl}-2-oxo-1,3-oxazolidin-5-yl)methyl]thiophen-2-carboxamid

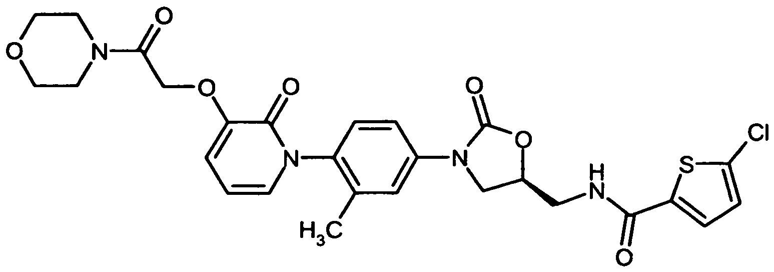

Ausbeute: 50.6 mg (43% d. Th.)
¹H-NMR. (400 MHz, DMSO-*d₆*, *δ*/*ppm*): δ = 8.98 (t, 1H), 7.70 (d, 1H), 7.55-7.49 (m, 2H), 7.26-7.18 (m, 2H), 7.13-7.07 (m, 1H), 6.88 (dd, 1H), 6.22 (t, 1H), 4.90-4.80 (m, 3H), 4.22 (t, 1H), 3.9-3.84 (m, 1H), 3.64-3.38 (m, 10H), 2.02 (s, 3H).
LC-MS (Methode 5): Rₜ = 1.90 min
MS (ESIpos): m/z = 586 (M+H)⁺

### Beispiel 11

### 5-Chlor-N-[((5S)-3-{3-methyl-4-[3-[2-(methylamino)-2-oxoethoxy]-2-oxopyridin-1(2H)-yl]phenyl}-2-oxo-1,3-oxazolidin-5-yl)methyl]thiophen-2-carboxamid

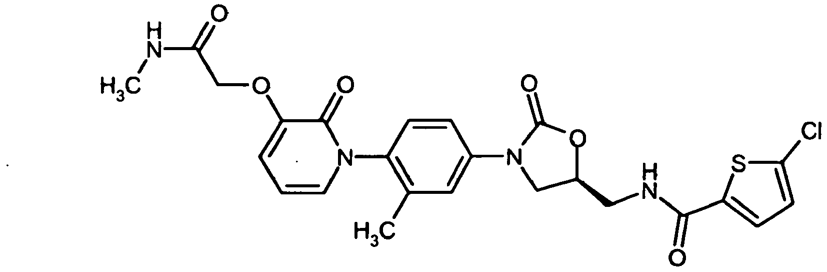

Ausbeute: 43.4 mg (41% d. Th.)
¹H-NMR (400 MHz, DMSO-*d*₆, *δ*/*ppm*): δ = 8.98 (t, 1H), 8.02-7.95 (m, 1H), 7.69 (d, 1H), 7.56-4.49 (b, 2H), 7.25 (d, 1H), 7.20 (d, 1H), 7.14 (d, 1H), 6.93 (d, 1H), 6.25 (t, 1H), 4.90-4.82 (m, 1H), 4.47 (s, 2H), 4.22 (t, 1H), 3.92-3.85 (m, 1H), 3.62 (t, 2H), 2.65 (s, 3H), 2.02 (s, 3H).
LC-MS (Methode 5): Rₜ = 1.87 min
MS (ESIpos): m/z = 531 (M+H)⁺

### Beispiel 12

### N-[((5S)-3-{4-[3-[2-(tert-Butylamino)-2-oxoethoxy]-2-oxopyridin-1(2H)-yl]-3-methylphenyl}-2-oxo-1,3-oxazolidin-5-yl)methyl]-5-chlorthiophen-2-carboxamid

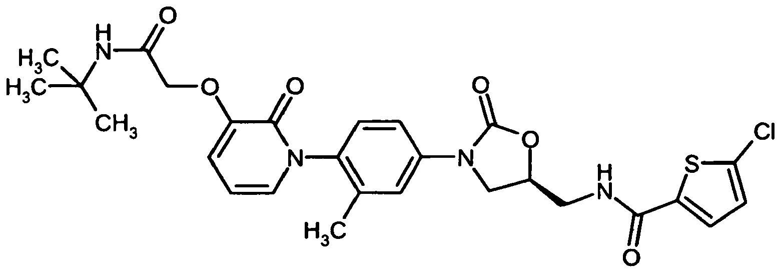

Ausbeute: 40.9 mg (40% d. Th.)
¹H-NMR (400 MHz, DMSO-*d₆*, *δ*/*ppm*): δ = 8.98 (t, 1H), 8.00 (d, 1H), 7.70 (d, 1H), 7.61-7.50 (m, 2H), 7.25 (d, 1H), 7.20 (d, 1H), 7.15 (dd, 1H), 7.02 (dd, 1H), 6.26 (t, 1H), 4.90-4.82 (m, 1H), 4.41 (s, 2H), 4.22 (t, 1H), 3.91-3.85 (m, 1H), 3.61 (t, 2H), 2.02 (s, 3H), 1.27 (s, 9H).
LC-MS (Methode 5): Rₜ = 2.17 min
MS (ESIpos): m/z = 573 (M+H)⁺

### Beispiel 13

### 5-Chlor-N-[((5S)-3-{3-methyl-4-[2-oxo-3-(2,2,2-trifluorethoxy)pyridin-1(2H)-yl]phenyl}-2-oxo-1,3-oxazolidin-5-yl)methyl]thiophen-2-carboxamid

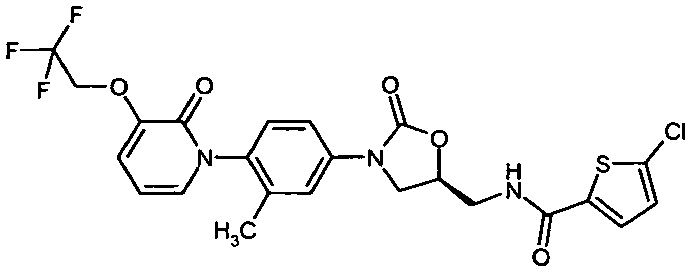

Ausbeute: 11.9 mg (11% d. Th.)
¹H-NMR (400 MHz, CDCl₃, *δ*/*ppm*): δ = 7.51-7.45 (m, 2H), 7.33 (d, 1H), 7.21-7.18 (m, 1H), 7.12 (dd, 1H), 7.00 (dd, 1H), 6.90 (d, 1H), 6.70-6.62 (d, 1H), 6.22 (t, 1H), 4.88-4.78 (m, 1H), 4.66-4.50 (m, 2H), 4.16-4.04 (m, 1H), 3.90-3.82 (m, 2H), 3.76-3.66 (m, 1H), 2.13 (s, 3H).
LC-MS (Methode 3): Rₜ = 2.13 min
MS (ESIpos): m/z = 542 (M+H)⁺

### Beispiel 14

### 5-Chlor-N-[((5S)-3-{4-[3-[2-(dimethylamino)-2-oxoethoxy]-2-oxopyridin-1(2H)-yl]-3-methylphenyl}-2-oxo-1,3-oxazolidin-5-yl)methyl]thiophen-2-carboxamid

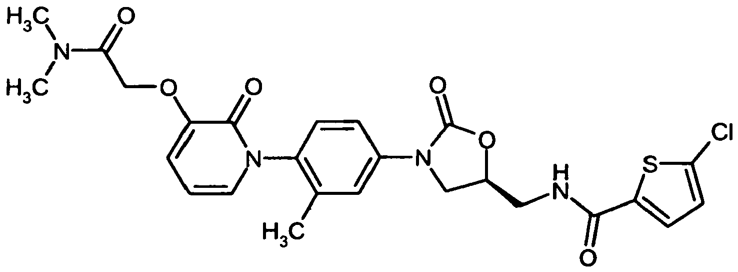

Ausbeute: 51.7 mg (47% d. Th.)
¹H-NMR (400 MHz, DMSO-*d₆*, *δ*/*ppm*): δ = 8.98 (t, 1H), 7.70 (d, 1H), 7.56-7.47 (m, 2H), 7.25 (d, 1H), 7.20 (d, 1H), 7.09 (d, 1H), 6.83 (d, 1H), 6.21 (t, 1H), 4.90-4.82 (m, 1H), 4.78 (s, 2H), 4.22 (t, 1H), 3.92-3.84 (m, 1H), 3.62 (t, 2H), 3.00 (s, 3H), 2.85 (s, 3H), 2.02 (s, 3H).
LC-MS (Methode 3): Rₜ = 1.71 min
MS (ESIpos): m/z = 545 (M+H)⁺

### Beispiel 15

### 5-Chlor-N-[((5S)-3-{4-[3-(2-hydroxyethoxy)-2-oxopyridin-1(2H)-yl]-3-methylphenyl}-2-oxo-1,3-oxazolidin-5-yl)methyl]thiophen-2-carboxamid

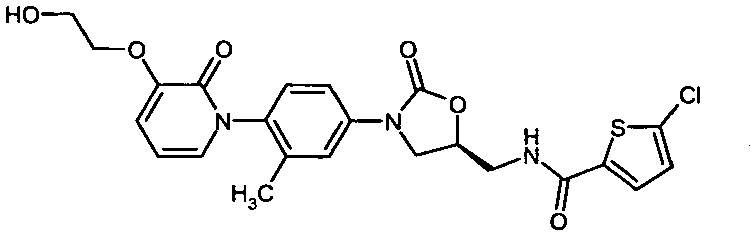

2.0 g (4.35 mmol) Beispiel 15A werden in 9 ml wasserfreiem N,N-Dimethylformamid gelöst, mit 3.6 g (26.1 mmol) Kaliumcarbonat versetzt und 30 min nachgerührt. Es wird mit weiteren 3 ml wasserfreiem N,N-Dimethylformamid verdünnt, mit 3.1 g (13.05 mmol) (2-Bromethoxy)(tert.-butyl)dimethylsilan versetzt und 7 h auf 60°C erhitzt. Man lässt erkalten, filtriert und reinigt mit präparativer HPLC mit einem Wasser/Acetonitril Gradient. Die Produktfraktionen werden im Vakuum zur Trockene eingedampft und der Rückstand im Vakuum getrocknet. Man erhält 1.17 g (53% d. Th.) der gewünschten Verbindung.
¹H-NMR (400 MHz, DMSO-*d₆, δ*/*ppm*): δ = 8.98 (t, 1H), 7.70 (d, 1H), 7.56-7.47 (m, 2H), 7.25-7.17 (m, 2H), 7.05 (dd, 1H), 6.93 (dd, 1H), 6.22 (t, 1H), 4.91 (t, 1H), 4.87-4.82 (m, 1H), 4.22 (t, 1H), 4.00-3.84 (m, 3H), 3.75-3.68 (m, 2H), 3.61 (t, 2H), 2.02 (s, 3H).
LC-MS (Methode 5): Rₜ = 1.93 min
MS (ESIpos): m/z = 504 (M+H)⁺

### Beispiel 16

### 5-Chlor-N-{[(5S)-3-{4-[3-(2-hydroxyethoxy)-2-oxopyridin-1(2H)-yl]-3,5-dimethylphenyl}-2-oxo-1,3-oxazolidin-5-yl]methyl} thiophen-2-carboxamid

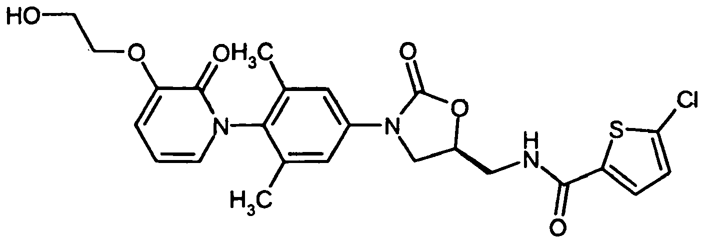

Eine Lösung von 108 mg (0.228 mmol) des Produktes aus Beispiel 23A in 2 ml DMF wird mit 189 mg (1.37 mmol) Kaliumcarbonat versetzt. Nach 1 h bei RT werden 0.15 ml (0.684 mmol) (2-Bromethoxy)-tert.-butyldimethylsilan zugetropft und es wird bei 60°C gerührt. Nach 2 h wird abgekühlt und in 50 ml Wasser gegeben. Nach Phasentrennung wird dreimal mit 80 ml Essigsäureethylester extrahiert. Die vereinigten organischen Phasen werden mit gesättigter wässriger Natriumchlorid-Lösung gewaschen und dann über Natriumsulfat getrocknet. Nach Filtration wird vom Lösungsmittel befreit, der Rückstand in 6 ml Dichlormethan aufgenommen und bei RT mit 2 ml Trifluoressigsäure versetzt. Nach 2 h bei RT wird mit 50 ml Dichlormethan verdünnt, zweimal mit Wasser, dann mit gesättigter wässriger Natriumhydrogencarbonat-Lösung und dann wieder mit Wasser gewaschen. Es wird über Natriumsulfat getrocknet und anschließend vom Lösungsmittel befreit. Da noch Edukt enthalten ist, wird der Rückstand abermals in 4 ml Dichlormethan gelöst und mit 1 ml Trifluoressigsäure versetzt. Nach 3.5 h bei RT wird mit 50 ml Dichlormethan verdünnt und mit gesättigter wässriger Natriumhydrogencarbonat-Lösung sowie mit Wasser gewaschen. Dann wird über Natriumsulfat getrocknet und anschließend vom Lösungsmittel befreit. Der Rückstand wird durch Chromatographie an Kieselgel (Dichlormethan/Essigsäureethylester 20:1) gereinigt. Man erhält 83 mg (70% d. Th.) des gewünschten Produktes.
¹H-NMR (400 MHz, DMSO-*d₆*, *δ*/*ppm*): 8.98 (t, 1H), 7.70 (d, 1H), 7.38 (dd, 2H), 7.20 (d, 1H), 6.99-6.90 (m, 2H), 6.26 (dd, 1H), 4.97-4.80 (m, 2H), 4.22 (dd, 1H), 3.95 (dd, 2H); 3.86 (dd, 1H), 3.76-3.70 (m, 2H), 3.60 (dd, 2H), 1.96 (s, 6H).
HPLC (Methode 1): Rₜ = 3.93 min.
MS (DCI, *m*/*z*): 518 (M+H)⁺.

### Beispiel 17

### 5-Chlor-N-[((5S)-3-{4-[3-(difluormethoxy)-2-oxopyridin-1(2H)-yl]-3-methylphenyl}-2-oxo-1,3-oxazolidin-5-yl)methyl]thiophen-2-carboxamid

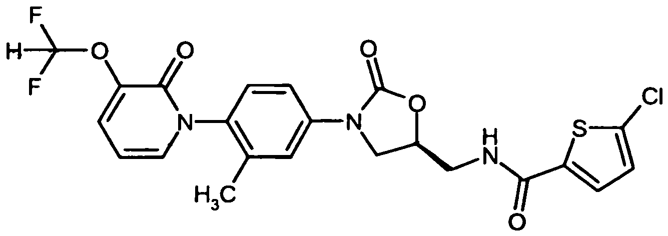

100 mg (0.172 mmol) Beispiel 15A werden in 1.5 ml wasserfreiem N,N-Dimethylformamid gelöst, mit 121 mg (0.87 mmol) Kaliumcarbonat versetzt und 30 min nachgerührt. 68.9 mg (0.435 mmol) Ethyl-chlor(difluor)acetat werden zugegeben und es wird 6 h auf 60°C erhitzt. Dann wird filtriert und mit präparativer HPLC mit einem Wasser/Acetonitril Gradient gereinigt. Die Produktfraktionen werden im Vakuum zur Trockene eingedampft und der Rückstand im Vakuum getrocknet. Man erhält 21 mg (19% d. Th.) der gewünschten Verbindung.
¹H-NMR (400 MHz, DMSO-*d₆*, *δ*/*ppm*): δ = 9.0 (t, 1H), 7.6 (d, 1H), 7.05 (b, 2H), 7.5 (d, 2H), 7.3 (d, 1H), 7.2 (d, 1H), 7.05 (t, 1H), 6.3 (t, 1H), 4.85 (m, 1H), 4.2 (t, 1H), 3.9 (m, 1H), 3.6 (t, 2H), 2.0 (s, 3H).
LC-MS (Methode 4): Rₜ = 2.04 min
MS (ESIpos): m/z = 510 (M+H)⁺

### Beispiel 18

### 5-Chlor-N-({(5S)-3-[4-(3-ethoxy-2-oxopyrazin-1(2H)-yl)-3-methylphenyl]-2-oxo-1,3-oxazolidin-5-yl}methyl)thiophen-2-carboxamid

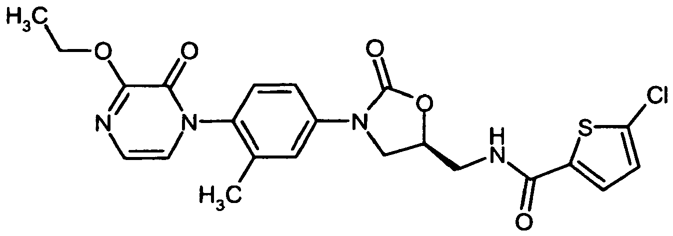

400 mg (1.63 mmol) Beispiel 114A werden in 16 ml wasserfreiem Acetonitril gelöst und bei 0°C mit 443 mg (1.71 mmol) Beispiel 1A versetzt. Es wird mit 546 mg (2.45 mmol) Magnesiumperchlorat versetzt, die Kühlung entfernt und 5 h bei RT gerührt. Dann werden 528 mg (3.26 mmol) 1,1-Carbonyldiimidazol und 4 mg (0.03 mmol) N,N-Dimethylaminopyridin zugefügt und es wird 15 h am Rückfluß erhitzt. Man lässt erkalten und destilliert das Lösemittel im Vakuum ab. Der Rückstand wird in Essigsäureethylester aufgenommen und zweimal mit Wasser, mit gesättigter Natriumhydrogencarbonatlösung sowie mit gesättigter Natriumchloridlösung gewaschen, über Magnesiumsulfat getrocknet. Nach Filtration wird im Vakuum zur Trockene eingedampft. Man löst den Rückstand in Acetonitril an und es wird mit präparativer HPLC mit einem Gradienten aus Wasser und Acetonitril aufgereinigt. Die Produktfraktionen werden vereinigt und im Vakuum zur Trockene eingedampft. Man erhält 273 mg (34% d. Th.) der gewünschten Verbindung.
¹H-NMR (400 MHz, DMSO-*d₆*, *δ*/*ppm*): δ = 8.98 (t, 1H), 7.70 (d, 1H), 7.56-7.50 (m, 2H), 7.30 (d, 1H), 7.20 (d, 1H), 7.07 (d, 1H), 6.88 (d, 1H), 4.90-4.82 (m, 1H), 4.30 (q, 2H), 4.21 (t, 1H), 3.90-3.84 (m, 1H), 3.61 (t, 2H), 2.05 (s, 3H), 1.34 (t, 3H).
LC-MS (Methode 6): Rₜ = 2.22 min
MS (ESIpos): m/z = 489 (M+H)⁺

### Beispiel 19

### 5-Chlor-N-({(5S)-3-[3-(ethoxymethyl)-4-(2-oxopyridin-1(2H)-yl)phenyl]-2-oxo-1,3-oxazolidin-5-yl}methyl)thiophen-2-carboxamid

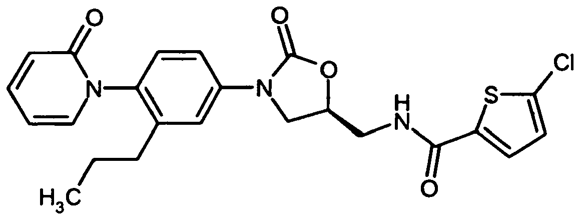

90 mg (0.0.39 mmol) Beispiel 38A werden in 6 ml wasserfreiem Acetonitril gelöst und bei 0°C mit 122 mg (0.47 mmol) Beispiel 1A versetzt. Es werden 131 mg (0.59 mmol) Magnesiumperchlorat zugegeben, die Kühlung entfernt und es wird 15 h bei RT gerührt. Das Lösemittel wird im Vakuum eingedampft und der Rückstand mit 9 ml Butyronitril verdünnt. Dann wird mit 127 mg (0.79 mmol) 1,1-Carbonyldiimidazol und 1 mg (0.01 mmol) N,N-Dimethylaminopyridin versetzt und 15 h am Rückfluß erhitzt. Man lässt erkalten und destilliert das Lösemittel im Vakuum ab, löst den Rückstand in Acetonitril an und reinigt mit präparativer HPLC mit einem Gradienten aus Wasser und Acetonitril auf. Die Produktfraktionen werden vereinigt und im Vakuum zur Trockene eingedampft. Man erhält 33 mg (18% d. Th.) der gewünschten Verbindung.
¹H-NMR (400 MHz, DMSO-*d₆*, *δ*/*ppm*): δ = 8.98 (t, 1H), 7.70 (d, 1H), 7.56-7.46 (m, 4H), 7.24-7.17 (m, 2H), 6.47 (d, 1H), 6.30 (dt, 1H), 4.90-4.82 (m, 1H), 4.26-4.19 (m, 1H), 3.92-3.85 (m, 1H), 3.62 (t, 2H), 2.30 (t, 2H), 1.49-1.36 (m, 2H), 0.71 (t, 3H).
LC-MS (Methode 6): Rₜ = 2.20 min
MS (ESIpos): m/z = 472 (M+H)⁺

### Beispiel 20

### 5-Chlor-N-[((5S)-3-{3-methyl-4-[2-oxo-3-(trifluormethyl)pyridin-1(2H)-yl]phenyl}-2-oxo-1,3-oxazolidin-5-yl) methyl]thiophen-2-carboxamid

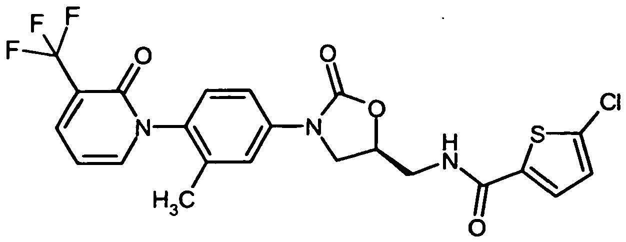

750 mg (2.8 mmol) Beispiel 40A werden in 28 ml wasserfreiem Acetonitril gelöst und bei 0°C mit 730 mg (3.36 mmol) Beispiel 1A versetzt. Es werden 936 mg (4.19 mmol) Magnesiumperchlorat zugegeben, die Kühlung entfernt und es wird 15 h bei RT gerührt. Das Lösemittel wird im Vakuum eingedampft und der Rückstand mit 55 ml Butyronitril verdünnt. Dann wird mit 906 mg (5.59 mmol) 1,1-Carbonyldiimidazol und 7 mg (0.06 mmol) N,N-Dimethylaminopyridin versetzt und 15 h am Rückfluß erhitzt. Man lässt erkalten, destilliert das Lösemittel im Vakuum ab, löst den Rückstand in Acetonitril an und reinigt mit präparativer HPLC mit einem Gradienten aus Wasser und Acetonitril auf. Die Produktfraktionen werden vereinigt und im Vakuum zur Trockene eingedampft. Man erhält 828 mg (58% d. Th.) der gewünschten Verbindung.
¹H-NMR (400 MHz, DMSO-*d*₆ *δ*/*ppm*): δ = 8.98 (t, 1H), 8.07 (d, 1H), 7.89 (d, 1H), 7.70 (d, 1H), 7.58-7.52 (m, 2H), 7.33 (d, 1H), 7.20 (d, 1H), 6.47 (t, 1H), 4.91-4.82 (m, 1H), 4.23 (t, 1H), 3.92-3.85 (m, 1H), 3.62 (t, 2H), 2.04 (s, 3H).
LC-MS (Methode 6): Rₜ = 2.14 mm
MS (ESIpos): m/z = 512 (M+H)⁺

### Beispiel 21

### 5-Chlor-N-[((5S)-3-{5-methyl-4-(3-methyl-2-oxopyridin-1(2H)-yl)-2-[methyl(propyl)amino]-phenyl}-2-oxo-1,3-oxazolidin-5-yl)methyl]thiophen-2-carboxamid

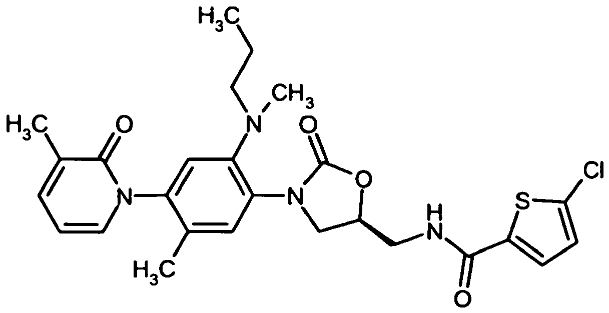

Eine Lösung von 180 mg (0.631 mmol) des Produktes aus Beispiel 105A in 5 ml Acetonitril wird mit 151 mg (0.694 mmol) des Produktes aus Beispiel 1A versetzt. Der Suspension werden 211 mg (0.95 mmol) Magnesiumperchlorat zugefügt, wonach man 17 h bei RT rühren läßt. Dann werden 256 mg (1.58 mmol) 1,1'-Carbonyldiimidazol und 7.7 mg (0.06 mmol) DMAP hinzugesetzt und es wird 17 h bei 60°C gerührt. Das Rohgemisch wird mittels präparativer HPLC mit einem Acetonitril/Wasser-Gemisch gereinigt. Es werden 106 mg (30% d. Th.) des gewünschten Produktes erhalten.
¹H-NMR (400 MHz, DMSO-*d₆*, *δ*/*ppm*): 9.04 (t, 1H), 7.77 (d, 1H), 7.44-7.34 (m, 2 H), 7.22 (d, 1H), 7.14 (d, 1H), 6.88 (s, 1H), 6.22 (dd, 1H), 4.95-4.82 (m, 1H), 4.00 (ddd, 1H), 3.72-3.50 (m, 3H), 2.90-2.69 (m, 2H), 2.58 (s, 3H), 2.04 (s, 3H), 1.86 (d, 3H), 1.46-1.31 (m, 2H), 0.80 (s, 3H).
HPLC (Methode 2): Rₜ = 4.07 min.
MS (DCI, *mlz*): 529 (M+H)⁺.

### Beispiel 22

### 5-Chlor-N-({(5S)-3-[5-methyl-4-(3-methyl-2-oxopyridin-1(2H)-yl)-2-propoxyphenyl]-2-oxo-1,3-oxazolidin-5-yl}methyl)thiophen-2-carboxamid

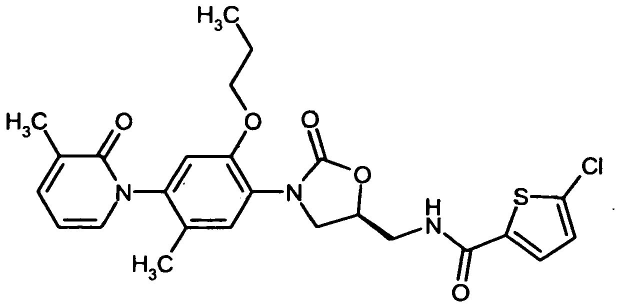

Eine Lösung von 415 mg (1.52 mmol) des Produktes aus Beispiel 109A in 10 ml Acetonitril wird mit 365 mg (1.68 mmol) des Produktes aus Beispiel 1A versetzt. Der Suspension werden 510 mg (2.29 mmol) Magnesiumperchlorat zugefügt, wonach man 17 h bei RT rühren läßt. Dann werden 617 mg (3.81 mmol) 1,1'-Carbonyldiimidazol und 18 mg (0.15 mmol) DMAP hinzugesetzt und es wird auf 60°C erhitzt. Nach 48 h wird vom Lösungsmittel befreit und das eingeengte Filtrat mittels präparativer HPLC mit einem Acetonitril/Wasser-Gemisch gereinigt. Es werden 58 mg (7% d. Th.) des gewünschten Produktes erhalten.
¹H-NMR (400 MHz, DMSO-*d₆*, *δ*/*ppm*): 8.99 (t, 1H), 7.74 (d, 1H), 7.44-7.33 (m, 2H), 7.22 (br.s, 2H), 6.99 (s, 1H), 6.24 (dd, 1H), 4.88-4.80 (m, 1H), 4.07-3.88 (m, 3H), 3.76-3.53 (m, 3H), 2.04 (s, 3H), 1.89 (s, 3H), 1.70-1.61 (m, 2H), 0.92 (t, 3H).
HPLC (Methode 2): Rₜ = 4.43 min.

### Beispiel 23

### 5-Chlor-N-({(5S)-3-[4-(1-ethyl-2-oxo-1,2-dihydropyridin-3-yl)-3-methoxyphenyl]-2-oxo-1,3-oxazolidin-5-yl} methyl)thiophen-2-carboxamid

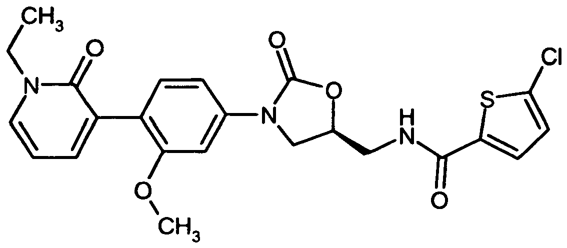

160 mg (0.67 mmol) der Verbindung aus Beispiel 57A werden in 6 ml Acetonitril gelöst. Man kühlt auf 0°C, versetzt mit 157 mg (0.72 mmol) der Verbindung aus Beispiel 1A und 219 mg (0.98 mmol) Magnesiumperchlorat, wonach 20 h bei RT gerührt wird. Dann fügt man 159 mg (0.98 mmol) 1,1'-Carbonyldiimidazol und 8 mg (0.065 mmol) DMAP hinzu und erhitzt 20 h lang auf 60°C. Man reinigt direkt mittels präparativer HPLC. Es werden 190 mg (59% d. Th.) des gewünschten Produktes erhalten.
¹H-NMR (400 MHz, DMSO-*d₆*, *δ*/*ppm*): 8.99 (t, 1H), 7.70 (d, 1H), 7.68 (dd, 1H), 7.36-7.32 (m, 2H), 7.25 (d, 1H), 7.20 (d, 1H), 7.01 (dd, 1H), 6.26 (t, 1H), 4.89-4.81 (m, 1H), 4.22 (t, 1H), 3.94 (q, 2H), 3.88 (dd, 1H), 3.70 (s, 3H), 3.62 (t, 2H), 1.23 (t, 3H).
HPLC (Methode 3): Rₜ = 1.89 min.
MS (ESIpos, *m*/*z*): 488/490 (³⁵Cl/³⁷Cl) (M+H)⁺.

### Beispiel 24

### 5-Chlor-N-({(5S)-3-[3-methoxy-4-(1-methyl-2-oxo-1,2-dihydropyridin-3-yl)phenyl]-2-oxo-1,3-oxazolidin-5-yl}methyl)thiophen-2-carboxamid

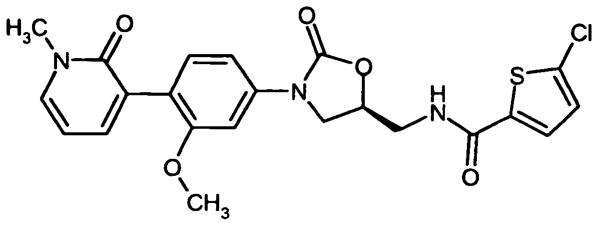

260 mg (1.13 mmol) des Produktes aus Beispiel 59A werden analog Beispiel 23 mit dem Produkt aus Beispiel 1A umgesetzt. Man reinigt mittels präparativer HPLC. Es werden 310 mg (58% d. Th.) des gewünschten Produktes erhalten.
¹H-NMR (400 MHz, DMSO-*d₆*, *δ*/*ppm*): 8.98 (t, 1H), 7.70 (d, 1H), 7.67 (dd, 1H), 7.36-7.33 (m, 2H), 7.23 (d, 1H), 7.20 (d, 1H), 7.01 (dd, 1H), 6.24 (t, 1H), 4.89-4.81 (m, 1H), 4.23 (t, 1H), 3.88 (dd, 1H), 3.70 (s, 3H), 3.62 (t, 2H), 3.46 (s, 3H).
HPLC (Methode 2): Rₜ = 3.99 min.
MS (ESIpos, *m*/*z*): 474/476 (³⁵Cl³⁷Cl) (M+H)⁺.

### Beispiel 25

### 5-Chlor-N-({(5S)-3-[3-methoxy-4-(2-oxo-1-propyl-1,2-dihydropyridin-3-yl)phenyl]-2-oxo-1,3-oxazolidin-5-yl}methyl)thiophen-2-carboxamid

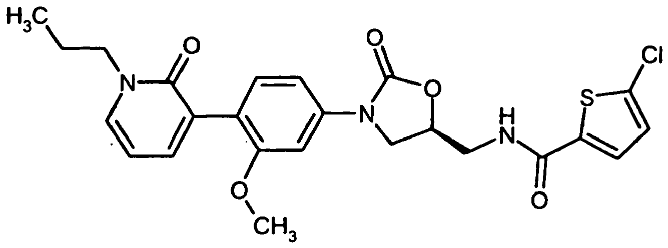

110 mg (0.43 mmol) des Produktes aus Beispiel 53A werden analog Beispiel 23 mit dem Produkt aus Beispiel 1A umgesetzt. Man reinigt mittels präparativer HPLC. Es werden 113 mg (51% d. Th.) des gewünschten Produktes erhalten.
¹H-NMR (400 MHz, DMSO-*d₆*, δ/*ppm*): 9.00 (t, 1H), 7.70 (d, 1H), 7.44 (d, 1H), 7.32-7.28 (m, 2H), 7.26 (d, 1HP, 7.20 (d, 1H), 7.11 (d, 1H), 6.19 (t, 1H), 4.91-4.83 (m, 1H), 4.25 (t, 1H), 3.90 (dd, 1H), 3.73 (s, 3H), 3.63 (t, 2H), 2.52-2.49 (m, 2H), 1.50-1.48 (m, 2H), 0.91 (t, 3H).
HPLC (Methode 4): Rₜ = 2.43 min.
MS (ESIpos, *m*/*z*): 502/504 (³⁵Cl/³⁷Cl) (M+H)⁺.

### Beispiel 26

### 5-Chlor-N-({(5S)-3-[3-ethoxy-4-(2-oxopiperidin-1-yl)phenyl]-2-oxo-1,3-oxazolidin-5-yl}methyl)-thiophen-2-carboxamid

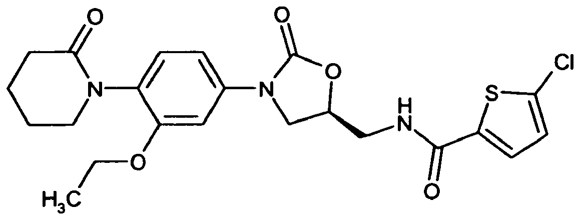

Eine Lösung von 220 mg (0.939 mmol) des Produktes aus Beispiel 91A in 10 ml Acetonitril wird mit 224 mg (1.03 mmol) des Produktes aus Beispiel 1A versetzt. Der Suspension werden 314 mg (1.41 mmol) Magnesiumperchlorat zugefügt, wonach man 5 h bei RT rühren lässt. Dann werden 380 mg (2.35 mmol) 1,1'-Carbonyldiimidazol und 11 mg (0.09 mmol) DMAP hinzugesetzt und es wird bei RT gerührt. Nach 18 h wird mit 100 ml Wasser verdünnt. Die Wasserphase wird dreimal mit Essigsäureethylester extrahiert und die vereinigten organischen Phasen über Natriumsulfat getrocknet. Anschließende Reinigung mittels präparativer HPLC mit einem Gradienten aus Wasser und Acetonitril liefert 137 mg (30% d. Th.) des gewünschten Produktes.
¹H-NMR (400 MHz, DMSO-*d₆*, *δ*/*ppm*): 8.97 (t, 1H); 7.69 (d, 1H), 7.32 (d, 1H), 7.20 (d, 1H), 7.11 (d, 1H), 6.98 (dd, 1H), 4.90-4.78 (m, 1H), 4.19 (dd, 1H), 3.99 (q, 2H), 3.85 (dd, 1H), 3.60 (dd, 2H), 3.44-3.35 (m, 2H), 2.37-2.28 (m, 2H), 1.88-1.77 (m, 4H), 1.30 (t, 3H).
LC-MS (Methode 5): Rₜ = 4.03 min.
MS (ESIpos, *m*/*z*): 478 (M+H)⁺.

### Beispiel 27

### 5-Chlor-N-({(5S)-3-[3-(methoxymethyl)-4-(2-oxopiperidin-1-yl)phenyl]-2-oxo-1,3-oxazolidin-5-yl}methyl)thiophen-2-carboxamid

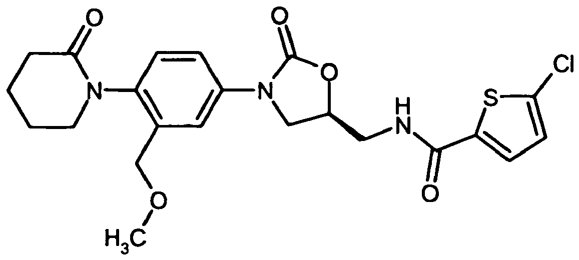

560 mg (2.39 mmol) Beispiel 116A werden in 24 ml wasserfreiem Acetonitril gelöst und bei 0°C mit 624 mg (2.87 mmol) Beispiel 1A versetzt. Es werden 800 mg (3.89 mmol) Magnesiumperchlorat zugegeben, die Kühlung entfernt und 15 h bei RT gerührt. Das Lösemittel wird im Vakuum entfernt, der Rückstand mit 50 ml wasserfreiem Butyronitril gelöst und dann mit 775 mg (4.78 mmol) 1,1-Carbonyldiimidazol und 6 mg (0.05 mmol) N,N-Dimethylaminopyridin versetzt. Nach Erhitzen für 16 h am Rückfluss, wird mit Essigsäureethylester versetzt, die organische Phase abgetrennt, zweimal mit Wasser, einmal mit gesättigter Natriumhydrogencarbonatlösung und einmal mit gesättigter Natriumchloridlösung gewaschen, getrocknet, filtriert und im Vakuum zur Trockene eingedampft. Der Rückstand wird in Dimethylsulfoxid angelöst und mit präparativer HPLC mit einem Gradienten aus Wasser und Acetonitril aufgereinigt. Die Produktfraktionen werden vereinigt und im Vakuum zur Trockene eingedampft. Man erhält 560 mg (49% d. Th.) der gewünschten Verbindung.
¹H-NMR (400 MHz, DMSO-*d₆*, *δ*/*ppm*): δ = 8.97 (t, 1H), 7.70 (d, 1H), 7.59-7.54 (m, 1H), 7.52-7.45 (m, 1H), 7.24-7.17 (m, 2H), 4.87-4.78 (m, 1H), 4.30 (d, 1H), 4.22-4.14 (m, 2H), 3.90-3.83 (m, 1H), 3.63-3.52 (m, 3H), 3.37-3.27 (m, 4H), 2.40-2.32 (m, 2H), 1.90-1.80 (m, 4H).
LC-MS (Methode 5): Rₜ = 1.93 min
MS (ESIpos): m/z = 478 (M+H)⁺

### Beispiel 28

### 5-Chlor-N-({(5S)-3-[3-(ethoxymethyl)-4-(2-oxopiperidin-1-yl)phenyl]-2-oxo-1,3-oxazolidin-5-yl}methyl)thiophen-2-arboxamid

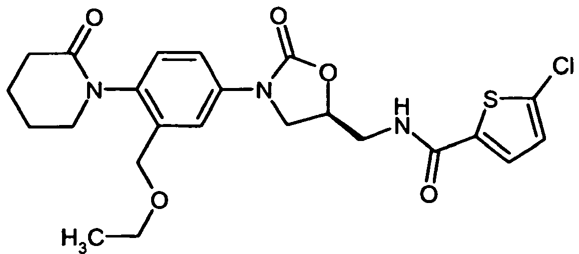

147 mg (0.58 mmol) Beispiel 75A werden in 6 ml wasserfreiem Acetonitril gelöst und bei 0°C mit 184 mg (0.71 mmol) Beispiel 1A versetzt. Es werden 198 mg (0.89 mmol) Magnesiumperchlorat zugegeben, die Kühlung entfernt und 15 h bei RT gerührt. Das Lösemittel wird im Vakuum entfernt, der Rückstand mit 10 ml wasserfreiem Butyronitril verdünnt und mit 192 mg (1.18 mmol) 1,1-Carbonyldiimidazol und 2 mg (0.01 mmol) N,N-Dimethylaminopyridin versetzt. Nach 16 h am Rückfluss wird mit Essigsäureethylester versetzt, die organische Phase abgetrennt, zweimal mit Wasser, einmal mit gesättigter Natriumhydrogencarbonat-Lösung und einmal mit gesättigter Natriumchloridlösung gewaschen, getrocknet, filtriert und im Vakuum zur Trockene eingedampft. Der Rückstand wird in Dimethylsulfoxid angelöst und es wird mit präparativer HPLC mit einem Gradienten aus Wasser und Acetonitril auf gereinigt, die Produktfraktionen werden vereinigt und im Vakuum zur Trockene eingedampft. Man erhält 190 mg (65% d. Th.) der gewünschten Verbindung.
¹H-NMR (400 MHz, DMSO-*d₆*, *δ*/*ppm*): δ = 8.97 (t, 1H), 7.70 (d, 1H), 7.57 (d, 1H), 7.52-7.44 (m, 1H), 7.24-7.17 (m, 2H), 4.87-4.78 (m, 1H), 4.33 (d, 1H), 4.23-4.15 (m, 2H), 3.89-3.82 (m, 1H), 3.64-3.51 (m, 3H), 3.45 (q, 2H), 3.39-3.33 (m, 1H), 2.39-2.30 (m, 2H), 1.88-1.78 (m, 4H) 1.20-1.11 (m, 3H).
LC-MS (Methode 5): Rₜ = 2.06 min
MS (ESIpos): m/z = 492 (M+H)⁺

### Vergleichsbeispiel 29

### 5-Chlor-N-({(5S)-3-[3-methyl-4-(5-oxo-1,4-oxazepan-4-yl)phenyl]-2-oxo-1,3-oxazolidin-5-yl}methyl)thiophen-2-carboxamid

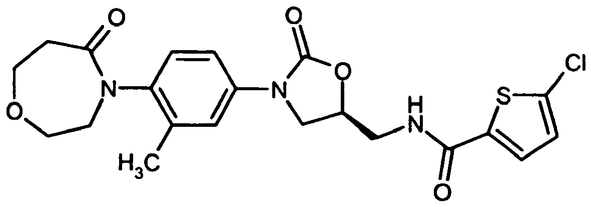

373 mg (1.69 mmol) Beispiel 77A werden in 20 ml wasserfreiem Acetonitril gelöst und bei 0°C mit 526 mg (2.03 mmol) Beispiel 1A versetzt. 567 mg (2.54 mmol) Magnesiumperchlorat werden zugegeben, die Kühlung entfernt und es wird 15 h bei RT gerührt. Dann wird im Vakuum zur Trockene eingedampft und in 30 ml Butyronitril aufgenommen. Es wird mit 549 mg (3.39 mmol) 1,1-Carbonyldiimidazol und 4 mg (0.03 mmol) N,N-Dimethylaminopyridin versetzt und 5 h am Rückfluß erhitzt. Man lässt erkalten und destilliert das Lösemittel im Vakuum ab. Der Rückstand wird in Acetonitril gelöst und mit präparativer HPLC mit einem Gradienten aus Wasser und Acetonitril aufgereinigt. Die Produktfraktionen werden vereinigt und im Vakuum zur Trockene eingedampft. Man erhält 462 mg (59% d. Th.) der gewünschten Verbindung.
¹H-NMR (400 MHz, DMSO-*d*₆, *δ*/*ppm*): δ = 8.97 (t, 1H), 7.70 (d, 1H), 7.42-7.35 (m, 2H), 7.20 (d, 1H), 7.13 (d, 1H), 4.87-4.79 (m, 1H), 4.17 (t, 1H), 3.85-3.78 (m, 6H), 3.65-3.56 (m, 3H), 2.92-2.84 (m, 1H), 2.75-2.67 (m, 1H), 2.15 (s, 3H).
LC-MS (Methode 6): Rₜ = 1.88 min
MS (ESIpos): m/z = 464 (M+H)⁺

### Vergleichsbeispiel 30

### 5-Chlor-N-({(5S)-3-[3-cyclopropyl-4-(5-oxo-1,4-oxazepan-4-yl)phenyl]-2-oxo-1,3-oxazolidin-5-yl}methyl)thiophen-2-carboxamid

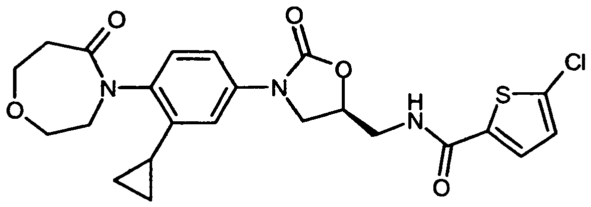

Eine Lösung von 236 mg (0.95 mmol) des Produktes aus Beispiel 80A in 12 ml Acetonitril wird mit 229 mg (1.05 mmol) des Produktes aus Beispiel 1A versetzt. Der Suspension werden 320 mg (1.44 mmol) Magnesiumperchlorat zugefügt, wonach man 5 h bei RT rühren lässt. Dann werden 388 mg (2.40 mmol) 1,1'-Carbonyldiimidazol und 12 mg (0.09 mmol) DMAP hinzugesetzt und es wird 12 h bei RT gerührt. Anschließend wird mit Wasser verdünnt, die Wasserphase dreimal mit Essigsäureethylester extrahiert und die vereinigten organischen Phasen über Natriumsulfat getrocknet. Nach Filtration wird vom Lösungsmittel befreit und der Rückstand mittels präparativer HPLC mit einem Wasser/Acetonitril Gemisch gereinigt. Man erhält 138 mg (29% d. Th.) des gewünschten Produktes.
¹H-NMR (400 MHz, DMSO-*d*₆, *δ*/*ppm*): 8.94 (t, 1H), 7.67 (d, 1H), 7.32 (ddd, 1H), 7.18 (d, 1H), 7.12 (d, 1H), 7.06 (dd, 1H), 4.86-4.75 (m, 1H), 4.16 (ddd, 1H), 3.91-3.72 (m, 6H), 3.67-3.52 (m, 3H), 2.92 (ddd, 1H), 2.69 (ddd, 1H), 1.92-1.84 (m, 1H), 1.01-0.82 (m, 2H), 0.77-0.68 (m, 1H), 0.50-0.41 (m, 1H).
HPLC (Methode 1): Rₜ = 3.99 min
MS (DCI, *m*/*z*): 507 (M+NH₄)⁺.

### Beispiel 31

### 5-Chlor-N-({(5S)-3-[3-ethyl-4-(2-oxotetrahydropyrimidin-1(2H)-yl)phenyl]-2-oxo-1,3-oxazolidin-5-yl}methyl)thiophen-2-carboxamid

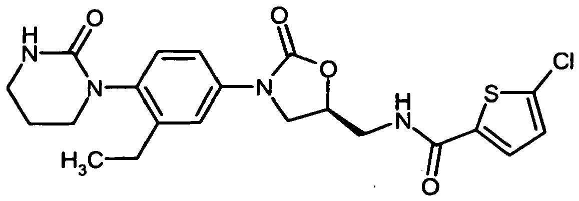

Eine Lösung von 12.8 mg (0.058 mmol) des Produktes aus Beispiel 83A in 1 ml Acetonitril wird mit 14 mg (0.06 mmol) des Produktes aus Beispiel 1A versetzt. Der Suspension werden 19 mg (0.09 mmol) Magnesiumperchlorat zugefügt, wonach man 4.5 h bei RT rühren lässt. Dann werden 23.6 mg (0.146 mmol) 1,1'-Carbonyldiimidazol und 1 mg (0.01 mmol) DMAP hinzugesetzt und es wird 18 h bei RT gerührt. Anschließend wird mit DMSO versetzt und mittels präparativer HPLC mit einem Wasser/Acetonitril Gemisch gereinigt. Man erhält 10 mg (36% d. Th.) des gewünschten Produktes.
¹H-NMR (400 MHz, DMSO-*d*₆, *δ*/*ppm*): 8.95 (t, 1H), 7.68 (d, 1H), 7.42-7.29 (m, 2H), 7.21-7.12 (m, 2H), 6.42 (br. s, 1H), 4.89-4.78 (m, 1H), 4.18 (ddd, 1H), 3.88-3.82 (m, 1H), 3.65-3.48 (m, 4H), 3.40-3.18 (m, 4H), 1.98-1.87 (m, 2H), 1.13 (t, 3H).
HPLC (Methode 1): Rₜ = 3.98 min
MS (ESIpos, *m*/*z*): 463 (M+H)⁺.

### Beispiel 32

### 5-Chlor-N-({(5S)-3-[3-isopropyl-4-(2-oxotetrahydropyrimidin-1(2H)-yl)phenyl]-2-oxo-1,3-oxazolidin-5-yl}methyl)thiophen-2-carboxamid

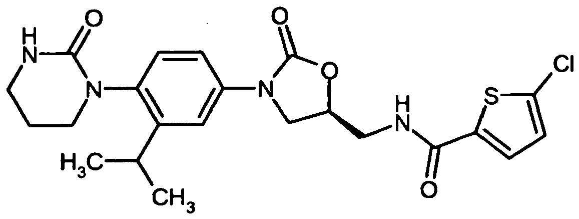

Eine Lösung von 75 mg (0.32 mmol) des Produktes aus Beispiel 85A in 4 ml Acetonitril wird mit 77 mg (0.35 mmol) des Produktes aus Beispiel 1A versetzt. Der Suspension werden 107 mg (0.482 mmol) Magnesiumperchlorat zugefügt, wonach man 5 h bei RT rühren lässt. Dann werden 130 mg (0.804 mmol) 1,1'-Carbonyldiimidazol und 4 mg (0.03 mmol) DMAP hinzugesetzt und es wird 18 h bei RT gerührt. Anschließend wird mit Wasser verdünnt und dreimal mit Essigsäureethylester extrahiert. Die vereinigten organischen Phasen werden über Natriumsulfat getrocknet. Nach Filtration wird im Vakuum vom Lösungsmittel befreit und der Rückstand mittels präparativer HPLC mit einem Wasser/Acetonitril Gemisch gereinigt. Man erhält 51 mg (33% d. Th.) des gewünschten Produktes.
¹H-NMR (400 MHz, *DMSO-d₆, δ*/*ppm*): 8.96 (t, 1H), 7.68 (d, 1H), 7.44 (m, 1H), 7.29 (ddd, 1H), 7.18 (d, 1H), 7.12 (d, 1H), 6.41 (br. s, 1H), 4.88-4.78 (m, 1H), 4.19 (dd, 1H), 3.86 (dd, 1H), 3.60 (dd, 2H), 3.57-3.48 (m, 1H), 3.31-3.17 (m, 3H), 3.03-2.93 (m, 1H), 2.00-1.89 (m, 2H), 1.18-1.08 (m, 6H).
HPLC (Methode 1): Rₜ = 4.07 min
MS (ESIpos, *m*/*z*): 477 (M+H)⁺.

### Beispiel 33

### 5-Chlor-N-({(5S)-2-oxo-3-[4-(2-oxopyridin-1(2H)-yl)-3-propylphenyl]-1,3-oxazolidin-5-yl}methyl)thiophen-2-carboxamid

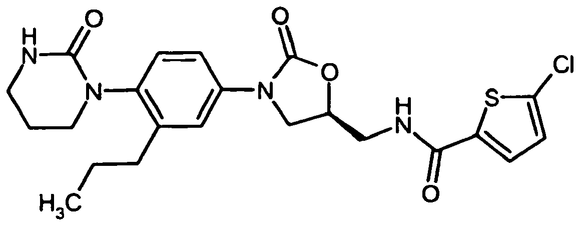

42 mg (0.18 mmol) Beispiel 87A werden in 3 ml wasserfreiem Acetonitril gelöst und bei 0°C mit 56 mg (0.22 mmol) Beispiel 1A versetzt. 60 mg (0.27 mmol) Magnesiumperchlorat werden zugegeben, die Kühlung entfernt und es wird 15 h bei RT gerührt. Das Lösemittel wird im Vakuum eingedampft, der Rückstand mit 5 ml Butyronitril verdünnt und mit 58 mg (0.36 mmol) 1,1-Carbonyldiimidazol und 0.4 mg (0.004 mmol) N,N-Dimethylaminopyridin versetzt. Nach 15 h am Rückfluß lässt man erkalten und destilliert das Lösemittel im Vakuum ab. Man löst den Rückstand in Acetonitril und reinigt mit präparativer HPLC mit einem Gradienten aus Wasser und Acetonitril. Die Produktfraktionen werden vereinigt und im Vakuum zur Trockene eingedampft. Man erhält 44 mg (51% d. Th.) der gewünschten Verbindung.
¹H-NMR (400 MHz, DMSO-*d*₆, *δ*/*ppm*): δ = 8.98 (t, 1H), 7.70 (d, 1H), 7.39-7.29 (m, 2H), 7.20 (d, 1H), 7.14 (d, 1H), 6.45 (br. s, 1H), 4.85-4.79 (m, 1H), 4.17 (t, 1H), 3.86-3.81 (m, 1H), 3.55-3.35 (m, 5H), 2.52-2.48 (m, 2H), 2.49-2.38 (m, 1H), 1.97-1.90 (m, 2H), 1.59-1.45 (m, 2H), 0.90 (t, 3H).
LC-MS (Methode 6): Rₜ = 2.10 min
MS (ESIpos): m/z = 477 (M+H)⁺

### Beispiel 34

### 5-Chlor-N-({(5S)-3-[3-methyl-4-(3-methyl-2-oxotetrahydropyrimidin-1(2H)-yl)phenyl]-2-oxo-1,3-oxazolidin-5-yl}methyl)thiophen-2-carboxamid

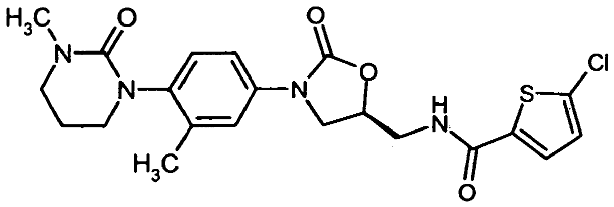

Eine Lösung von 45 mg (0.205 mmol) des Produktes aus Beispiel 61A in 2 ml Acetonitril wird mit 49.1 mg (0.226 mmol) des Produktes aus Beispiel 1A versetzt. Der Suspension werden 68.7 mg (0.308 mmol) Magnesiumperchlorat zugefügt, wonach man 3 h bei RT rühren lässt. Dann werden 83.2 mg (0.513 mmol) 1,1'-Carbonyldiimidazol und 2.5 mg (0.02 mmol) DMAP hinzugesetzt, und es wird bei RT gerührt. Nach 24 h wird mit 50 ml Wasser verdünnt und die wässrige Phase dreimal mit Essigsäureethylester extrahiert. Die vereinigten organischen Phasen werden über Natriumsulfat getrocknet. Nach Filtration wird vom Lösungsmittel befreit und der Rückstand mittels präparativer HPLC mit einem Acetonitril/Wasser-Gemisch gereinigt. Es werden 68 mg (71% d. Th.) des gewünschten Produktes erhalten.
¹H-NMR (400 MHz, DMSO-*d*₆, *δ*/*ppm*): 8.96 (dd, 1H), 7.68 (dd, 1H), 7.38-7.31 (m, 2H), 7.19 (d, 1H), 7.13 (d, 1H), 4.85-4.76 (m, 1H), 4.16 (dd, 1H), 3.83 (dd, 1H), 3.62-3.52 (m, 3H), 3.43-3.25 (m, 3H), 2.84 (s, 3H), 2.13 (s, 3H), 2.07-1.99 (m, 2H).
HPLC (Methode 2): Rₜ = 4.09 min.
MS (DCIpos, *m*/*z*): 463 (M+H)⁺.

### Beispiel 35

### 5-Chlor-N-({(5S)-3-[3-methoxy-4-(3-methyl-2-oxotetrahydropyrimidin-1(2H)-yl)phenyl]-2-oxo-1,3-oxazolidin-5-yl}methyl)thiophen-2-carboxamid

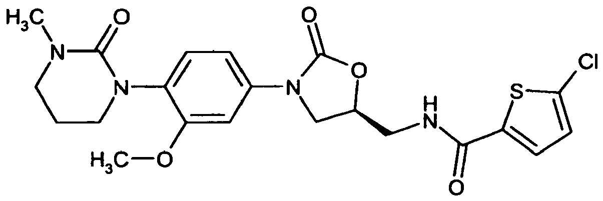

Eine Lösung von 350 mg (1.49 mmol) des Produktes aus Beispiel 63A in 10 ml Acetonitril wird mit 356 mg (1.64 mmol) des Produktes aus Beispiel 1A versetzt. Der Suspension werden 498 mg (2.23 mmol) Magnesiumperchlorat zugefügt, wonach man 2 h bei RT rühren lässt. Dann werden 603 mg (3.72 mmol) 1,1'-Carbonyldiimidazol und 18 mg (0.15 mmol) DMAP hinzugesetzt, und es wird bei RT gerührt. Nach 48 h wird in 200 ml Wasser gegeben und die wässrige Phase dreimal mit Essigsäureethylester extrahiert. Der gebildete Niederschlag wird abfiltriert und im Vakuum getrocknet. Es werden 232 mg (32% d. Th.) des gewünschten Produktes erhalten.
¹H-NMR (400 MHz, DMSO-*d*₆, *δ*/*ppm*): 8.97 (t, 1H), 7.69 (d, 1H), 7.29 (d, 1H), 7.19 (d, 1H), 7.10 (d, 1H), 6.94 (dd, 1H), 4.87-4.77 (m, 1H), 4.19 (dd, 1H), 3.85 (dd, 1H), 3.74 (s, 3H), 3.60 (t, 2H), 3.48-3.21 (m, 4H), 2.81 (s, 3H), 1.99 (tt, 2H).
HPLC (Methode 2): Rₜ = 4.04 min.
MS (ESIpos, *m*/*z*): 479 (M+H)⁺.

### Beispiel 36

### 5-Chlor-N-{[(5S)-3-{4-[3-(2-hydroxyethyl)-2-oxotetrahydropyrimidin-1(2H)-yl]-3-methylphenyl}-2-oxo-1,3-oxazolidin-5-yl]methyl}thiophen-2-carboxamid

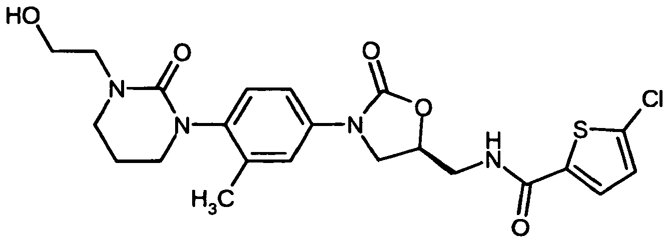

Eine Lösung von 232 mg (0.32 mmol) des Produktes aus Beispiel 67A in 6 ml THF wird mit 195 mg (0.74 mmol) einer 1 molaren Lösung von Tetrabutylammoniumfluorid in THF versetzt. Es wird 2 h bei RT gerührt. Dann wird mit 60 ml Wasser, 30 ml Essigsäureethylester und 6 ml Natriumchlorid-Lösung versetzt und die Phasen werden getrennt. Die organische Phase wird dreimal mit Essigsäureethylester extrahiert und die vereinigten organischen Phasen über Natriumsulfat getrocknet. Nach Filtration wird vom Lösungsmittel befreit und der Rückstand durch Chromatographie an Kieselgel (Dichlormethan/Methanol 20:1) vorgereinigt. Anschließende Aufreinigung mittels präparativer HPLC mit einem Acetonitril/Wasser-Gemisch liefert 29 mg (18% d. Th.) des gewünschten Produktes.
¹H-NMR (400 MHz, DMSO-*d*₆, *δ*/*ppm*): 8.96 (t, 1H), 7.69 (d, 1H), 7.40-7.31 (m, 2H), 7.19 (d, 1H), 7.13 (d, 1H), 4.86-4.77 (m, 1H), 4.64 (t, 1H), 4.16 (dd, 1H), 3.83 (dd, 1H), 3.59 (t, 2H), 3.58-3.40 (m, 5H), 3.38-3.22 (m, 3H), 2.12 (s, 3H), 2.06-1.95 (m, 2H).
HPLC (Methode 1): Rₜ = 3.91 min.
MS (ESIpos, *m*/*z*): 493 (M+H)⁺.

### Beispiel 37

### 5-Chlor-N-{[(5S)-3-{3-chlor-4-[3-(2-hydroxyethyl)-2-oxotetrahydropyrimidin-1(2H)-yl]phenyl}-2-oxo-1,3-oxazolidin-5-yl]methyl}thiophen-2-carboxamid

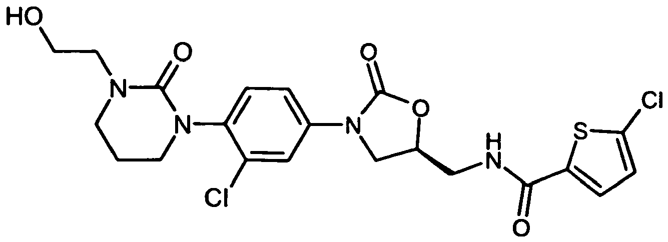

Eine Lösung von 355 mg (0.47 mmol) des Produktes aus Beispiel 70A in 9 ml THF wird mit 290 mg (1.10 mmol) einer 1 molaren Lösung von Tetrabutylammoniumfluorid in THF versetzt. Es wird 2 h bei RT gerührt. Dann wird mit 80 ml Wasser, 50 ml Essigsäureethylester und 6 ml Natriumchlorid-Lösung versetzt und die Phasen werden getrennt. Die organische Phase wird zweimal mit Essigsäureethylester extrahiert und die vereinigten organischen Phasen werden über Natriumsulfat getrocknet. Nach Filtration wird vom Lösungsmittel befreit und der Rückstand durch Chromatographie an Kieselgel (Dichlormethan/Methanol 20:1) vorgereinigt. Anschließende Aufreinigung mittels präparativer HPLC mit einem Actonitril/Wasser-Gemisch liefert 39 mg (16% d. Th.) des gewünschten Produktes.
¹H-NMR (400 MHz, DMSO-*d*₆, *δ*/*ppm*): 8.96 (t, 1H), 7.73-7.67 (m, 2H), 7.46-7.36 (m, 1H), 7.34 (d, 1H), 7.19 (d, 1H), 4.84 (dddd, 1H), 4.64 (t, 1H), 4.19 (dd, 1H), 3.85 (dd, 1H), 3.60 (t, 2H), 3.52-3.33 (m, 6H), 3.33-3.24 (m, 1H), 2.06-1.97 (m, 2H).
HPLC (Methode 1): Rₜ = 3.95 min.
MS (ESIpos, *m*/*z*): 513 (M+H)⁺.

### Beispiel 38

### 5-Chlor-N-{[(5S)-3-{4-[3-(2-hydroxyethyl)-2-oxotetrahydropyrimidin-1(2H)-yl]-3-(trifluormethyl)-phenyl}-2-oxo-1,3-oxazolidin-5-yl]methyl}thiophen-2-carboxamid

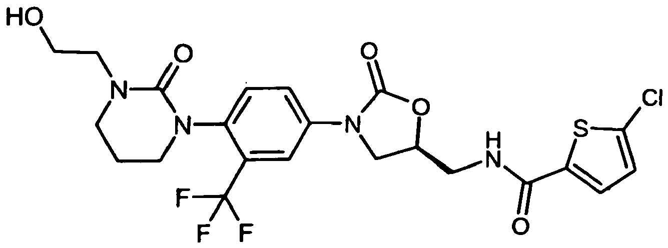

Eine Lösung von 8.88 g (11.3 mmol) des Produktes aus Beispiel 73A in 225 ml THF wird mit 6.95 g (26.6 mmol) einer 1 molaren Lösung von Tetrabutylammoniumfluorid in THF versetzt. Es wird 45 min bei RT gerührt. Dann wird vom Lösungsmittel befreit und der Rückstand durch Chromatographie an Kieselgel (Dichlormethan/Methanol 40:1 -> 10:1) gereinigt. Man erhält 5.24 g (80% d. Th.) des gewünschten Produktes.
¹H-NMR (400 MHz, DMSO-*d*₆, *δ*/*ppm*): 8.96 (dd, 1H), 7.95 (dd, 1H), 7.75-7.66 (m, 2H), 7.46 (d, 1H), 7.19 (d, 1H), 4.91-4.82 (m, 1H), 4.63 (t, 1H), 4.24 (dd, 1H), 3.91 (dd, 1H), 3.63-3.22 (m, 10H), 2.06-1.97 (m, 2H).
HPLC (Methode 2): Rₜ = 4.03 min.
MS (ESIpos, *m*/*z*): 547 (M+H)⁺.

### Beispiel 39

### N-({(5S)-3-[4-(3-(2-Hydroxyethyl)-2-oxopiperidin-1-yl)-3-chlorphenyl]-2-oxo-1,3-oxazolidin-5-yl}methyl)-5-chlorthiophen-2-carbonsäureamid

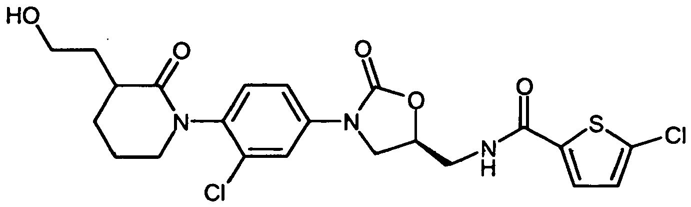

Eine Lösung von 100 mg (0.197 mmol) der Verbindung aus Beispiel 101A in einem Gemisch aus jeweils 1 ml Tetrahydrofuran und Wasser wird mit 49 µl (0.004 mmol) einer 2.5-prozentigen Lösung von Osmiumtetroxid in *tert*.-Butanol und 126 mg (0.59 mmol) Natriumperjodat versetzt. Das Reaktionsgemisch wird 16 Stunden bei RT gerührt. Anschliessend wird mit ca. 5 ml Wasser verdünnt und mit Dichlormethan extrahiert. Der organische Extrakt wird über wasserfreiem Magnesiumsulfat getrocknet, filtriert und das Filtrat am Rotationsverdampfer vom Lösemittel befreit. Der erhaltene Rückstand wird wieder in einem Gemisch aus jeweils 1 ml Tetrahydrofuran und Wasser gelöst und mit 8 mg (0.2 mmol) Natriumborhydrid versetzt. Nach einer Stunde Rühren bei RT wird wiederum mit ca. 5 ml Wasser verdünnt und mit Dichlormethan extrahiert. Der organische Extrakt wird über wasserfreiem Magnesiumsulfat getrocknet, filtriert und das Filtrat am Rotationsverdampfer vom Lösemittel befreit. Der erhaltene Rückstand wird mittels präparativer HPLC mit einem Acetonitril/Wasser-Gemisch gereinigt. Es werden 52 mg (50% d. Th.) der Titelverbindung erhalten.
¹H-NMR (400 MHz, CDCl₃, *δ*/*ppm*): 7.73 und 7.68 (2 dd, zusammen 1H), 7.43 und 7.41 (2 d, zusammen 1H), 7.31 (d, 1H), 7.21 und 7.20 (2 d, zusammen 1H), 6.89 (d, 1H), 6.80 (t, 1H), 4.89-4.82 (m, 1H), 4.07-4.02 (m, 2H), 3.83-3.69 (m, 5H), 3.62-3.47 (m, 2H), 2.71-2.62 (m, 1H), 2.16-1.94 (m, 4H), 1.83-1.74 (m, 2H).
HPLC (Methode 5): Rₜ = 1.96 min.
MS (ESIpos, *m*/*z*): 512/514/516 (Cl₂, ³⁵Cl/³⁷Cl) (M+H)⁺.

### Beispiel 40

### N-({(5S)-3-[4-(3-(2-Hydroxyethyl)-2-oxopiperidin-1-yl)-3-chlorphenyl]-2-oxo-1,3-oxazolidin-5-yl}methyl)-5-chlorthiophen-2-carbonsäureamid (Diastereomer 1)

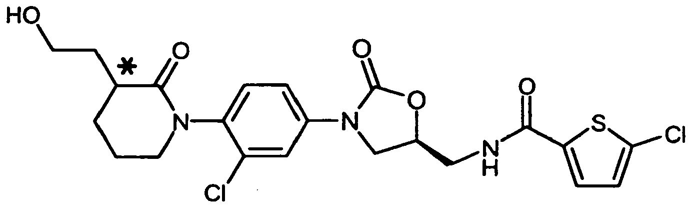

Das Diastereomerengemisch aus Beispiel 39 kann in präparativem Maßstab chromatographisch in die reinen Diastereomere aufgetrennt werden. Dazu werden 50 mg der Verbindung aus Beispiel 39 in 3.5 ml des Laufmittels gelöst und in einer Portion chromatographiert. Es werden 13 mg (26% d. Th.) der Titelverbindung (Diastereomer 1) und 16 mg (32% d. Th.) des Diastereomers 2 erhalten.
Methode: Säule: Daicel Chiralpak AD-H, 5 µm, 250 mm x 20 mm; Laufmittel: Ethanol + 1% Wasser + 0.2% Trifluoressigsäure; Fluß: 15 ml/min; Temperatur: 40°C; UV-Detektion: 220 nm.
Retentionszeit: 15.9 min (Diastereomer 1), 19.3 min (Diastereomer 2)
¹H-NMR (400 MHz, CDCl₃, *δ*/*ppm*): 7.71 (d, 1H), 7.46 und 7.43 (2 dd, zusammen 1H), 7.30 (d, 1H), 7.22 und 7.21 (2 d, zusammen 1H), 6.91 (d, 1H), 6.61 und 6.59 (2 t, zusammen 1H), 4.89-4.82 (m, 1H), 4.09-4.03 (m, 1H), 3.88-3.71 (m, 5H), 3.63-3.45 (m, 2H), 2.72-2.62 (m, 1H), 2.14-1.93 (m, 4H), 1.83-1.74 (m, 1H), 1.71 (breit, 1H).
HPLC (Methode 4): Rₜ = 2.07 min.
MS (ESIpos, *m*/*z*): 512/514/516 (Cl₂, ³⁵Cl/³⁷Cl) (M+H)⁺.

### Beispiel 41

### N-({(5S)-3-[4-(3-(2-Hydroxyethyl)-2-oxopiperidin-1-yl)-3-chlorphenyl]-2-oxo-1,3-oxazolidin-5-yl}methyl)-5-chlorthiophen-2-carbonsäureamid (Diastereomer 2)

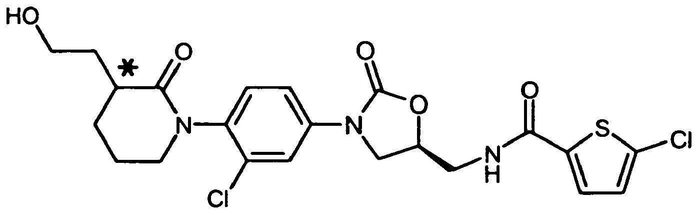

Das Diastereomerengemisch aus Beispiel 39 kann in präparativem Maßstab chromatographisch in die reinen Diastereomere aufgetrennt werden. Dazu werden 50 mg der Verbindung aus Beispiel 39 in 3.5 ml des Laufmittels gelöst und in einer Portion chromatographiert. Es werden 16 mg (32% d. Th.) der Titelverbindung (Diastereomer 2) und 13 mg (26% d. Th.) des Diastereomers 1 erhalten.
Methode: Säule: Daicel Chiralpak AD-H, 5 µm, 250 mm x 20 mm; Laufmittel: Ethanol + 1% Wasser + 0.2% Trifluoressigsäure; Fluß: 15 ml/min; Temperatur: 40°C; UV-Detektion: 220 nm.
Retentionszeit: 15.9 min (Diastereomer 1), 19.3 min (Diastereomer 2)
¹H-NMR (400 MHz, CDCl₃, *δ*/*ppm*): 7.73-7.68 (m, 1H), 7.48-4.41 (m, 1H), 7.30 (d, 1H), 7.25-7.20 (m, 1H, teilweise überdeckt vom CHCl₃-Signal), 6.90 (d, 1H), 6.67-6.61 (m, 1H), 4.89-4.82 (m, 1H), 4.10-4.01 (m, 1H), 3.88-3.69 (m, 5H), 3.63-3.44 (m, 2H), 2.72-2.62 (m, 1H), 2.28 (breit, 1H), 2.16-1.92 (m, 4H), 1.85-1.73 (m, 1H).
HPLC (Methode 4): Rₜ = 2.07 min.
MS (ESIpos, *m*/*z*): 512/514/516 (Cl₂, ³⁵Cl/³⁷Cl) (M+H)⁺.

### Beispiel 42

### 5-Chlor-N-[((5S)-3-{3-chlor-4-[3-(hydroxymethyl)-2-oxopiperidin-1-yl]phenyl}-2-oxo-1,3-oxazolidin-5-yl)methyl]thiophen-2-carbonsäureamid

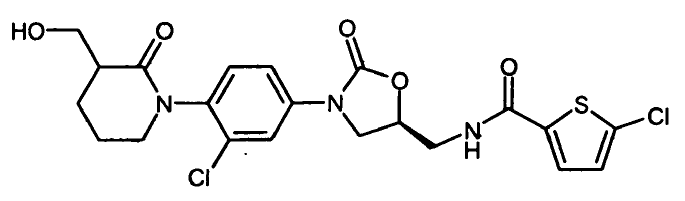

Eine Lösung von 600 mg (0.814 mmol) der Verbindung aus Beispiel 95A in 10 ml Tetrahydrofuran wird bei 0°C mit 855 µl (0.855 mmol) einer 1-molaren Lösung von Tetrabutylammoniumfluorid in Tetrahydrofuran versetzt. Nach fünf Stunden bei RT wird das Reaktionsgemisch am Rotationsverdampfer zur Trockene eingedampft. Der erhaltene Rückstand wird mittels Flash-Chromatographie über Kieselgel mit Cyclohexan/Essigsäureethylester 2:1 → 1:2 als Laufmittel gereinigt. Es werden 342 mg (84% d. Th.) der Titelverbindung erhalten.
¹H-NMR (400 MHz, DMSO-d₆, *δ*/*ppm*): 7.72 und 7.71 (2 t, zusammen 1H), 7.48-7.41 (m, 1H), 7.31 (d, 1H), 7.23 (d, 1H), 6.91 (d, 1H), 6.64 und 6.60 (m, 1H), 4.90-4.83 (m, 1H), 4.07 und 4.05 (2 t, zusammen 1H), 3.87-3.73 (m, 5H), 3.60-3.45 (m, 2H), 2.72-2.61 (m, 1H), 2.10-1.96 (m, 3H), 1.76-1.63 (m, 1H).
HPLC (Methode 2): Rₜ = 4.09 min.
MS (ESIpos, *m*/*z*): 498/500/502 (Cl₂, ³⁵Cl/³⁷Cl) (M+H)⁺.

### Beispiel 43

### 5-Chlor-N-[((5S)-3-{3-chlor-4-[3-(hydroxymethyl)-2-oxopiperidin-1-yl]phenyl}-2-oxo-1,3-oxazolidin-5-yl)methyl]thiophen-2-carbonsäureamid (Diastereomer 1)

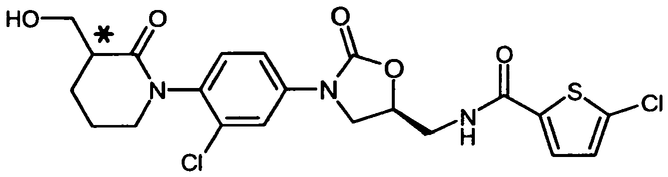

Das Diastereomerengemisch aus Beispiel 42 kann in präparativem Maßstab chromatographisch in die reinen Diastereomere aufgetrennt werden. Dazu werden 320 mg der Verbindung aus Beispiel 46 in 12 ml des Laufmittels gelöst und in drei Portionen chromatographiert. Es werden 167 mg (52% d. Th.) der Titelverbindung (Diastereomer 1) und 128 mg (40% d. Th.) des Diastereomers 2 erhalten.
Methode: Säule: Daicel Chiralpak AD-H, 5 µm, 250 mm x 20 mm; Laufmittel: Ethanol + 1 % Wasser + 0.2% Trifluoressigsäure; Fluß: 15 ml/min; Temperatur: 40°C; UV-Detektion: 220 nm.
Retentionszeit: 12.4 min (Diastereomer 1), 22.3 min (Diastereomer 2)
¹H-NMR (400 MHz, CDCl₃, *δ*/*ppm*): 7.71 (d, 1H), 7.47 und.7.43 (2 d, zusammen 1H), 7.30 und 7.28 (2 d, zusammen 1H), 7.23 und 7.22 (2 d, zusammen 1H, teilweise überdeckt vom CHCl₃-Signal), 6.91 und 6.90 (2 d, zusammen 1H), 6.61 und 6.57 (2 t, zusammen 1H), 4.89-4.83 (m, 1H), 4.10 und 4.03 (m, 1H), 3.87-3.72 (m, 5H), 3.64-3.44 (m, 2H), 2.72-2.61 (m, 1H), 2.13-1.83 (m, 4H), 1.76-1.64 (m, 1H).
HPLC (Methode 4): Rₜ = 2.11 min.
MS (ESIpos, m/z): 498/500/502 (Cl₂, ³⁵Cl/³⁷Cl) (M+H)⁺.

### Beispiel 44

### N-({(5S)-3-[4-(3-(2-Hydroxyethyl)-2-oxopiperidin-1-yl)-3-methylphenyl]-2-oxo-1,3-oxazolidin-5-yl}methyl)-5-chlorthiophen-2-carbonsäureamid

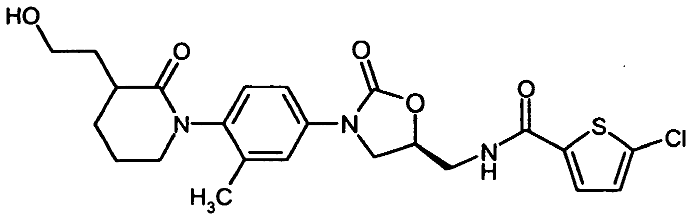

In Analogie zu dem in Beispiel 39 beschriebenen Verfahren werden aus 100 mg (0.205 mmol) der Verbindung aus Beispiel 102A nach präparativer HPLC 60 mg (60% d. Th.) der Titelverbindung erhalten.
¹H-NMR (400 MHz, CDCl₃, *δ*/*ppm*): 7.42-7.36 (m, 2H), 7.30 (d, 1H), 7.09 (dd, 1H), 6.90 (d, 1H), 6.67-6.62 (m, 1H), 4.87-4.80 (m, 1H), 4.09-4.03 (m, 1H), 3.88-3.67 (m, 5H), 3.63-3.53 (m, 1H), 3.48-3.35 (m, 1H), 2.70-2.61 (m, 1H), 2.18 (s, 3H), 2.15-1.73 (m, 7H).
HPLC (Methode 2): Rₜ = 3.94 min.
MS (ESIpos, *mlz*): 492/494 (³⁵Cl/³⁷Cl) (M+H)⁺.

### Beispiel 45

### N-({(5S)-3-[4-(3-(2-Hydroxyethyl)-2-oxopiperidin-1-yl)-3-methylphenyl]-2-oxo-1,3-oxazolidin-5-yl}methyl)-5-chlorthiophen-2-carbonsäureamid (Diastereomer 1)

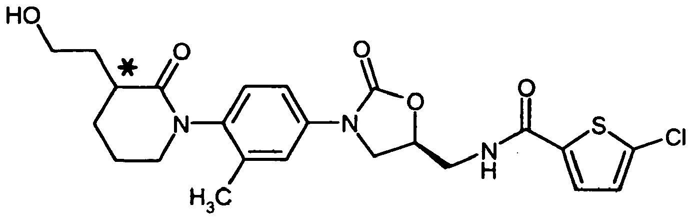

Das Diastereomerengemisch aus Beispiel 44 kann in präparativem Maßstab chromatographisch in die reinen Diastereomere aufgetrennt werden. Dazu werden 53 mg der Verbindung aus Beispiel 44 in 10 ml Ethanol gelöst und in 20 Portionen chromatographiert. Es werden 20 mg (38% d. Th.) der Titelverbindung (Diastereomer 1) und 24 mg (45% d. Th.) des Diastereomers 2 erhalten.
Methode: Säule: Daicel Chiralpak AD-H, 5 µm, 250 mm x 20 mm; Laufmittel: Ethanol/Isohexan 70:30; Fluß:15 ml/min; Temperatur: 30°C; UV-Detektion: 220 nm.
Retentionszeit (Methode 11): 18.52 min (Diastereomer 1); 22.57 min (Diastereomer 2).
¹H-NMR (400 MHz, CDCl₃, *δ*/*ppm*): 7.43-7.37 (m, 2H), 7.29 (d, 1H), 7.10 (dd, 1H), 6.90 (d, 1H), 6.53-6.50 (m, 1H), 4.86-4.81 (m, 1H), 4.33-4.25 (m, 1H), 4.10-4.04 (m, 1H), 3.90-3.65 (m, 5H), 3.61-3.53 (m, 1H), 3.48-3.35 (m, 1H), 2.70-2.61 (m, 1H), 2.19 (s, 3H), 2.15-1.93 (m, 4H), 1.84-1.73 (m, 2H).
HPLC (Methode 1): Rₜ = 3.93 min.

### Beispiel 46

### N-({(5S)-3-[4-(3-(2-Hydroxyethyl)-2-oxopiperidin-1-yl)-3-methylphenyl]-2-oxo-1,3-oxazolidin-5-yl}methyl)-5-chlorthiophen-2-carbonsäureamid (Diastereomer 2)

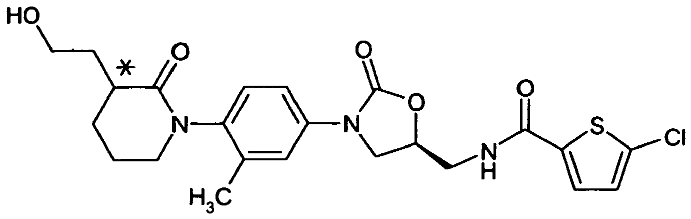

Das Diastereomerengemisch aus Beispiel 44 kann in präparativem Maßstab chromatographisch in die reinen Diastereomere aufgetrennt werden. Dazu werden 53 mg der Verbindung aus Beispiel 44 in 10 ml Ethanol gelöst und in 20 Portionen chromatographiert. Es werden 24 mg (45% d. Th.) der Titelverbindung (Diastereomer 2) und 20 mg (38% d. Th.) des Diastereomers 1 erhalten.
Methode: Säule: Daicel Chiralpak AD-H, 5 µm, 250 mm x 20 mm; Laufmittel: Ethanol/Isohexan 70:30; Fluß:15 ml/min; Temperatur: 30°C; UV-Detektion: 220 nm.
Retentionszeit (Methode 11): 18.52 min (Diastereomer 1); 22.57 min (Diastereomer 2).
¹H-NMR (400 MHz, CDCl₃, *δ*/*ppm*): 7.43-7.37 (m, 2H), 7.30 (d, 1H), 7.10 (dd, 1H), 6.90 (d, 1H), 6.58-6.53 (m, 1H), 4.87-4.81 (m, 1H), 4.35-4.26 (m, 1H), 4.10-4.04 (m, 1H), 3.88-3.68 (m, 5H), 3.63-3.53 (m, 1H), 3.48-3.35 (m, 1H), 2.70-2.60 (m, 1H), 2.19 (s, 3H), 2.14-1.91 (m, 4H), 1.84-1.73 (m, 2H).
HPLC (Methode 1): Rₜ = 3.93 min.

### Beispiel 47

### 5-Chlor-N-[((5S)-3-{3-chlor-4-[3-(3-hydroxypropyl)-2-oxopiperidin-1-yl]phenyl}-2-oxo-1,3-oxazolidin-5-yl)methyl]thiophen-2-carbonsäureamid (Diastereomer 2)

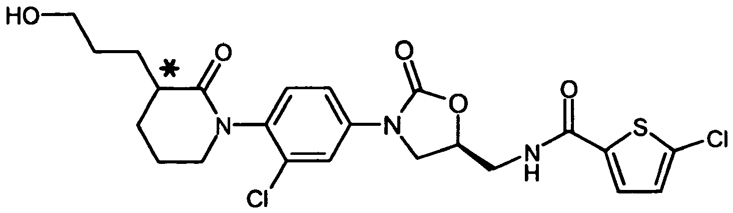

Eine Lösung von 200 mg (0.393 mmol) des Produktes aus Diastereomer 2 des Beispiels 101A in 4 ml wasserfreiem Tetrahydrofuran wird bei 0°C mit 1.2 ml (0.590 mmol) einer 0.5-molaren Lösung von 9-Borabicyclo[3.3.1]nonan in Tetrahydrofuran versetzt. Nach beendetem Zutropfen wird das Reaktionsgemisch zwei Stunden zum Rückfluß erhitzt. Anschließend wird wiederum auf 0°C abgekühlt und bei dieser Temperatur mit 160 µl (0.393 mmol) 10%-iger Natronlauge und 121 µl (1.18 mmol) 30%-iger Wasserstoffperoxid-Lösung versetzt. Das Kühlbad wird entfernt, und wenn das Reaktionsgemisch RT erreicht hat, läßt man noch etwa 30 Minuten rühren. Dann wird mit 20 ml Wasser versetzt und mit Essigsäureethylester extrahiert. Der organische Extrakt wird über wasserfreiem Magnesiumsulfat getrocknet. Nach Filtration wird das Lösemittel am Rotationsverdampfer entfernt und das Rohprodukt mittels präparativer HPLC eines Acetonitril/Wasser-Gemisches isoliert. Es werden 97 mg des Eduktes Beispiel 102A und 60 mg (56% d. Th., bezogen auf Umsatz) der Titelverbindung erhalten.
¹H-NMR (400 MHz, CDCl₃, *δ*/*ppm*): 7.76 und 7.62 (2 d, zusammen 1H), 7.45 und 7.33 (2 dd, zusammen 1H), 7.31 (d, 1H), 7.20 und 7.17 (2 d, zusammen 1H), 6.96 (s, breit, 1H), 6.88 (d, 1H), 4.86-4.78 (m, 1H), 4.01 (quart, 1H), 3.80 (dd, 1H), 3.73 (dd, 1H), 3.70-3.57 (m, 3H), 3.54-3.43 (m, 1H), 2.58-2.51 (m, 1H), 2.13-1.91 (m, 4H), 1.83-1.50 (m, 6H).
HPLC (Methode 4): Rₜ = 2.22 min.
MS (ESIpos, *m*/*z*): 526/528/530 (Cl₂, ³⁵Cl/³⁷Cl) (M+H)⁺.

### Beispiel 48

### 5-Chlor-N-[((5S)-3-{4-[3-(3-hydroxypropyl)-2-oxopiperidin-1-yl]-3-methylphenyl}-2-oxo-1,3-oxazolidin-5-yl)methyl]thiophen-2-carbonsäureamid

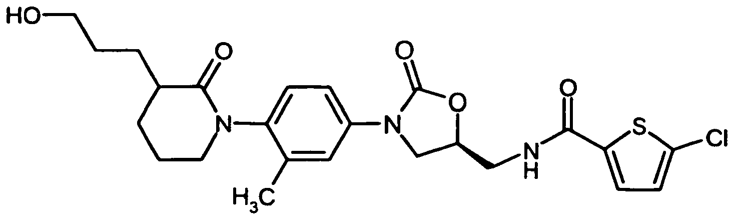

Analog zu dem unter Beispiel 47 beschriebenen Verfahren werden 200 mg (0.410 mmol) der Verbindung aus Beispiel 102A mit 9-Borabicyclo[3.3.1]nonan umgesetzt. Es werden 77 mg des Eduktes und 84 mg (66% d. Th., bezogen auf Umsatz) der Titelverbindung erhalten.
¹H-NMR (400 MHz, CDCl₃, *δ*/*ppm*): 7.43-7.31 (m, 2H), 7.31 (d, 1H), 7.10-7.03 (m, 1H), 6.89 (d, 1H), 6.81-6.72 (m, 1H), 5.86-5.72 (m, 1H), 4.83-4.75 (m, 1H), 4.05-3.99 (m, 1H), 3.87-3.72 (m, 3H), 3.69-3.61 (m, 2H), 3.58-3.51 (m, 1H), 3.47-3.33 (m, 1H), 2.57-2.50 (m, 1H), 2.18 und 2.17 (2 s, zusammen 3H), 2.10-1.87 (m, 4H), 1.79-1.51 (m, 4H).
HPLC (Methode 2): Rₜ = 4.01 min.
MS (ESIpos, *m*/*z*): 506/508 (³⁵Cl/³⁷Cl) (M+H)⁺.

### Beispiel 49

### 5-Chlor-N-[((5S)-3-{4-[3-(3-hydroxypropyl)-2-oxopiperidin-1-yl]-3-methylphenyl}-2-oxo-1,3-oxazolidin-5-yl)methyl]thiophen-2-carbonsäureamid (Diastereomer 1)

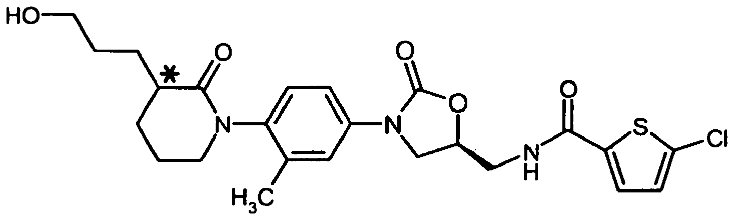

Das Diastereomerengemisch aus Beispiel 48 kann in präparativem Maßstab chromatographisch in die reinen Diastereomere aufgetrennt werden. Dazu werden 32 mg der Verbindung aus Beispiel 48 in 2.5 ml Ethanol gelöst und in einer Portion chromatographiert. Es werden 14 mg (44% d. Th.) der Titelverbindung (Diastereomer 1) und 14 mg (44% d. Th.) des Diastereomers 2 erhalten.
Methode: Säule: Daicel Chiralpak AD-H, 5 µm, 250 mm x 20 mm; Laufmittel: Ethanol/Isohexan 70:30; Fluß: 15 ml/min; Temperatur: 40°C; UV-Detektion: 220 nm.
Retentionszeit (Methode 11): 11.62 min (Diastereomer 1); 17.08 min (Diastereomer 2).
¹H-NMR (400 MHz, CDCl₃, *δ*/*ppm*): 7.40-7.35 (m, 2H), 7.31 (d, 1H), 7.10-7.05 (m, 1H), 6.89 (d, 1H), 6.77-6.73 (m, 1H), 4.82-4.74 (m, 1H), 4.05-4.00 (m, 1H), 3.83-3.76 (m, 2H), 3.69-3.52 (m, 4H), 3.46-3.33 (m, 1H), 2.57-2.50 (m, 1H), 2.36 (s, breit, 1H), 2.18 (s, 3H), 2.13-1.89 (m, 4H), 1.80-1.63 (m, 4H).
HPLC (Methode 1): Rₜ = 4.03 min.

### Beispiel 50

### 5-Chlor-N-[((5S)-3-{4-[3-(3-hydroxypropyl)-2-oxopiperidin-1-yl]-3-methylphenyl}-2-oxo-1,3-oxazolidin-5-yl)methyl]thiophen-2-carbonsäureamid (Diastereomer 2)

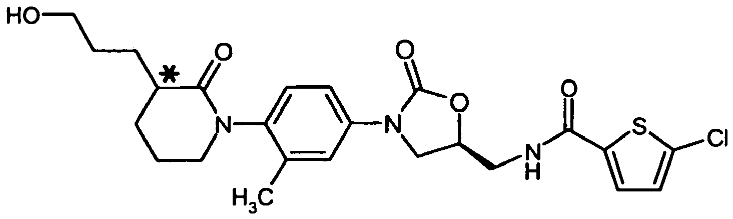

Das Diastereomerengemisch aus Beispiel 48 kann in präparativem Maßstab chromatographisch in die reinen Diastereomere aufgetrennt werden. Dazu werden 32 mg der Verbindung aus Beispiel 48 in 2.5 ml Ethanol gelöst und in einer Portion chromatographiert. Es werden 14 mg (44% d. Th.) der Titelverbindung (Diastereomer 2) und 14 mg (44% d. Th.) des Diastereomers 1 erhalten.
Methode: Säule: Daicel Chiralpak AD-H, 5 µm, 250 mm x 20 mm; Laufmittel: Ethanol/Isohexan 70:30; Fluß: 15 ml/min; Temperatur: 40°C; UV-Detektion: 220 nm.
Retentionszeit (Methode 11): 11.62 min (Diastereomer 1); 17.08min (Diastereomer 2).
¹H-NMR (400 MHz, CDCl₃, *δ*/*ppm*): 7.41-7.32 (m, 2H), 7.30 (d, 1H), 7.10-7.05 (m, 1H), 6.90 (d, 1H), 6.69-6.63 (m, 1H), 4.83-4.78 (m, 1H), 4.07-4.01 (m, 1H), 3.87-3.77 (m, 2H), 3.71-3.60 (m, 3H), 3.59-3.51 (m, 1H), 3.46-3.34 (m, 1H), 2.57-2.50 (m, 1H), 2.36 (s, breit, 1H), 2.18 und 2.17 (2 s, zusammen 3H), 2.13-1.90 (m, 4H), 1.79-1.63 (m, 4H).
HPLC (Methode 1): Rₜ = 4.02 min.

### Beispiel 51

### 5-Chlor-N-({(5S)-3-[3-(methoxymethyl)-4-(3-methyl-2-oxopyridin-1(2H)-yl)phenyl]-2-oxo-1,3-oxazolidin-5-yl}methyl)thiophen-2-carboxamid

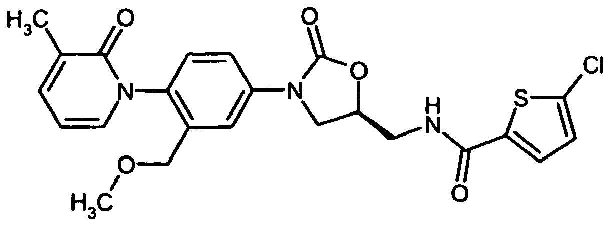

500 mg (2.05 mmol) Beispiel 34A werden in 20 ml wasserfreiem Acetonitril gelöst und bei 0°C mit 686 mg (3.07 mmol) Beispiel 1A versetzt. 685 mg (3.07 mmol) Magnesiumperchlorat werden zugegeben, die Kühlung entfernt und 16 h bei RT gerührt. Das Lösemittel wird im Vakuum entfernt, der Rückstand in Butyronitril aufgenommen, mit 664 mg (4.01 mmol) 1,1-Carbonyldiimidazol und 5 mg (0.03 mmol) N,N Dimethylaminopyridin versetzt und am Rückfluß erhitzt. Nach 16 h wird erneut dieselbe Menge N,N-Dimethylaminopyridin zugegeben und nochmals 16 h am Rückfluß erhitzt. Man lässt erkalten, destilliert das Lösemittel im Vakuum ab und verdünnt mit 300 ml Dichlormethan. Die Dichlormethan-Phase wird mit 1N Salzsäure und gesättigter Natriumchloridlösung gewaschen, filtriert, über Natriumsulfat getrocknet und im Vakuum zur Trockene eingedampft. Man löst den Rückstand in DMSO und reinigt mit präparativer HPLC mit einem Gradienten aus Wasser und Acetonitril. Die Produktfraktionen werden vereinigt und im Vakuum zur Trockene eingedampft. Man erhält 576 mg (58% d. Th.) der gewünschten Verbindung.
¹H-NMR (400 MHz, DMSO-*d*₆, *δ*/*ppm*): δ = 8.98 (t, 1H), 7.73-7.65 (m, 2H), 7.62-7.55 (m, 1H), 7.42 (d, 1H), 7.35 (d, 1H), 7.27 (d, 1H), 7.18 (d, 1H), 6.23 (t, 1H), 4.91-4.82 (m, 1H), 4.28-4.08 (m, 3H), 3.95-3.87 (m, 1H), 3.68-3.58 (m, 2H), 3.35 (s, 3H), 2.04 (s, 3H).
LC-MS (Methode 3): Rₜ= 1.88 min
MS (ESIpos): m/z = 488 (M+H)⁺

### Beispiel 52

### 5-Chlor-N-({(5S)-3-[3-(ethoxymethyl)-4-(3-methyl-2-oxopyridin-1(2H)-yl)phenyl]-2-oxo-1,3-oxazolidin-5-yl}methyl)thiophen-2-carboxamid

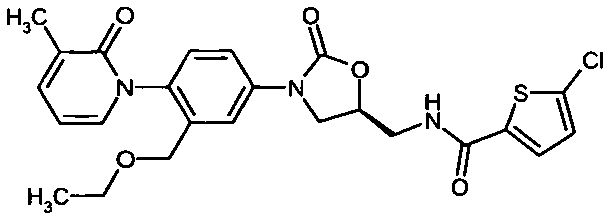

388 mg (1.5 mmol) Beispiel 34A werden in 15 ml wasserfreiem Acetonitril gelöst und bei 0°C mit 467 mg (1.80 mmol) Beispiel 1A versetzt. 503 mg (2.25 mmol) Magnesiumperchlorat werden zugegeben, die Kühlung entfernt und 16 h bei RT gerührt. Das Lösemittel wird im Vakuum entfernt, der Rückstand in Butyronitril aufgenommen, mit 487 mg (3.00 mmol) 1,1-Carbonyldiimidazol und 3.6 mg (0.03 mmol) N,N-Dimethylaminopyridin versetzt und 15 h am Rückfluß erhitzt. Man lässt erkalten, destilliert das Lösemittel im Vakuum ab, löst den Rückstand in Dimethylsulfoxid und reinigt mit präparativer HPLC mit einem Gradienten aus Wasser und Acetonitril. Die Produktfraktionen werden vereinigt und im Vakuum zur Trockene eingedampft. Man erhält 395 mg (52% d. Th.) der gewünschten Verbindung.
¹H-NMR (400 MHz, DMSO-*d*₆, *δ*/*ppm*): δ = 8.99 (t, 1H), 7.72-7.67 (m, 2H), 7.62-7.53 (m, 1H), 7.41 (d, 1H), 7.34 (d, 1H), 7.26 (d, 1H), 7.20 (d, 1H), 6.22 (t, 1H), 4.91-4.82 (m, 1H), 4.28-4.14 (m, 3H), 3.93-3.87 (m, 1H), 3.68-3.59 (m, 2H), 3.38-3.25 (m, 2H), 2.04 (s, 3H), 1.03 (t, 3H).
LC-MS (Methode 5): Rₜ = 2.15 min
MS (ESIpos): m/z = 502 (M+H)⁺

### B. Bewertung der pharmakologischen Wirksamkeit

Die Eignung der erfindungsgemäßen Verbindungen zur Behandlung von thromboembolischen Erkrankungen kann in folgenden Assaysystemen gezeigt werden:

### a) Testbeschreibungen (in vitro)

### a.1) Messung der Faktor Xa-Hemmung in Puffer

Zur Bestimmung der Faktor Xa-Hemmung der oben aufgeführten Substanzen wird ein biochemisches Testsystem aufgebaut, in dem die Umsetzung eines Faktor Xa-Substrates zur Ermittlung der enzymatischen Aktivität von humanem Faktor Xa benutzt wird. Dabei spaltet Faktor Xa aus dem peptischen Substrat Aminomethylcoumarin ab, das fluoreszent gemessen wird. Die Bestimmungen werden in Mikrotiterplatten durchgeführt.

Zu testende Substanzen werden in unterschiedlichen Konzentrationen in Dimethylsulfoxid gelöst und 30 min mit humanem Faktor Xa (1.3 nmol/l gelöst in 50 mmol/l Tris-Puffer [C,C,C-Tris(hydroxymethyl)-aminomethan], 100 mmol/l Natriumchlorid, 5 mmol/l Calciumchlorid, 0.1% BSA [bovines Serumalbumin], ph 7.4) bei 22°C inkubiert. Anschließend wird das Substrat (5 µmol/l Boc-Ile-Glu-Gly-Arg-AMC von der Firma Bachem) hinzugefügt. Nach einer Inkubation von 30 min wird die Probe bei einer Wellenlänge von 360 nm angeregt und die Emission bei 460 nm gemessen. Die gemessenen Emissionen der Testansätze mit Prüfsubstanz werden mit den Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

### a.2) Messung der Thrombin-Hemmung in Puffer

Zur Bestimmung der Thrombin-Hemmung der oben aufgeführten Substanzen wird ein biochemisches Testsystem aufgebaut, in dem die Umsetzung eines Thrombin-Substrates zur Ermittlung der enzymatischen Aktivität von humanem Thrombin benutzt wird. Dabei spaltet Thrombin aus dem peptischen Substrat Aminomethylcoumarin ab, das fluoreszent gemessen wird. Die Bestimmungen werden in Mikrötiterplatten durchgeführt.

Zu testende Substanzen werden in unterschiedlichen Konzentrationen in Dimethylsulfoxid gelöst und 15 min mit humanem Thrombin (0.06 nmol/l gelöst in 50 mmol/l Tris-Puffer [C,C,C-Tris(hydroxymethyl)-aminomethan], 100 mmol/l Natriumchlorid, 0.1% BSA [bovines Serumalbumin], ph 7.4) bei 22°C inkubiert. Anschließend wird das Substrat (5 µmol/l Boc-Asp(OBzl)-Pro-Arg-AMC von der Firma Bachem) hinzugefügt. Nach einer Inkubation von 30 min wird die Probe bei einer Wellenlänge von 360 nm angeregt und die Emission bei 460 nm gemessen. Die gemessenen Emissionen der Testansätze mit Prüfsubstanz werden mit den Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

### a.3) Bestimmung der Selektivität

Zum Nachweis der Selektivität der Substanzen bezüglich Thrombin- und Faktor Xa-Hemmung werden die Prüfsubstanzen auf ihre Hemmung anderer humaner Serinproteasen wie Faktor XIIa, Faktor XIa, Trypsin und Plasmin hin untersucht. Zur Bestimmung der enzymatischen Aktivität von Faktor XIIa (10 nmol/l von Kordia), Faktor XIa (0.4 nmol/l von Kordia), Trypsin (83 mU/ml von Sigma) und Plasmin (0.1 µg/ml von Kordia) werden diese Enzyme gelöst (50 mmol/l Tris-Puffer [C,C,C-Tris(hydroxymethyl)-aminomethan], 100 mmol/l Natriumchlorid, 0.1% BSA [bovines Serumalbumin], 5 mmol/l Calciumchlorid, pH 7.4) und für 15 min mit Prüfsubstanz in verschiedenen Konzentrationen in Dimethylsulfoxid sowie mit Dimethylsulfoxid ohne Prüfsubstanz inkubiert. Anschließend wird die enzymatische Reaktion durch Zugabe der entsprechenden Substrate gestartet (5 µmol/l H-Pro-Phe-Arg-AMC von Bachem für Faktor XIIa, 5 µmol/l Boc-Ile-Glu-Gly-Arg-AMC von Bachem für Trypsin, 5 µmol/l Boc-Glu(OBzl)-Ala-Arg-AMC von Bachem für Faktor XIa, 50 µmol/l MeOSuc-Ala-Phe-Lys-AMC von Bachem für Plasmin). Nach einer Inkubationszeit von 30 min bei 22°C wird die Fluoreszenz gemessen (Anregung: 360 nm, Emission: 460 nm). Die gemessenen Emissionen der Testansätze mit Prüfsubstanz werden mit den Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

### a.4) Bestimmung der Faktor Xa-inhibitorischen Wirkung der potentiellen Inhibitoren in Plasmaproben

Zur Bestimmung der Hemmung von Faktor Xa in Plasmaproben wird der im Plasma vorhandene Faktor X durch eine Protease aus dem Gift der Klapperschlange aktiviert. Anschließend wird die Faktor Xa-Aktivität bzw. deren Hemmung durch potentielle Inhibitoren mittels Zugabe eines chromogenen Substrats gemessen.

Die zu testenden Substanzen werden in unterschiedlichen Konzentrationen in Dimethylsulfoxid gelöst und mit einer wässrigen Refludan-Lösung (10 µg/ml) verdünnt. In klaren 96-Loch-Flachbodenplatten werden 30 µl Citratplasma (Octapharma) mit 10 µl der Substanzverdünnung gemischt. Dann werden entweder 20 µl einer Lösung eines Klapperschlangengifts (Russel viper venom (RVV); RVV Reagenz: Pentapharm 121-06, Endkonzentration 0.6 mU) in einem wässrigen Calciumchlorid-Lösung-Puffer (Endkonzentration Calciumchlorid 0.05 *M*) oder 20 µl der wässrigen Calciumchlorid-Lösung (Endkonzentration Calciumchlorid 0.05 M) ohne RVV Reagenz (als Referenz für eine unstimmulierte Probe) hinzugegeben. Nach Zugabe von 20 µl ChromozymXSubstrat (Endkonzentration 1.6 mmol/l, Bachem L-1565, verdünnt in Wasser) wird im SpectraFluor Reader mit einem Messfilter von 405 nm über 20 Minuten jede Minute gemessen. Die Ermittlung des IC₅₀-Wertes erfolgt, wenn ca. 70% des Maximalsignals erreicht ist (ca. 12 min).

Repräsentative Wirkdaten aus diesem Test sind in der folgenden Tabelle 1 aufgerührt:

**Tabelle 1**

| **Beispiel Nr.** | **IC₅₀ [nM]** |
|---|---|
| 4 | 201 |
| 7 | 206 |
| 9 | 149 |
| 16 | 213 |
| 18 | 319 |
| 19 | 361 |
| 27 | 39 |
| 30* | 25 |
| 32 | 70 |
| 35 | 552 |
| 38 | 252 |

| | |
|---|---|
| * Vergleichsbeispiel | |

### a.5) Bestimmung der Thrombin-inbibitorischen Wirkung der potentiellen Inhibitoren in Plasmaproben

Die zu testenden Substanzen werden in unterschiedlichen Konzentrationen in Dimethylsulfoxid gelöst und mit Wasser verdünnt. In weissen 96-Loch-Flachbodenplatten werden 20 µl Substanzverdünnung mit 20 µl Ecarin-Lösung (Ecarin Reagenz, Firma Sigma E-0504, Endkonzentration 20 mU pro Ansatz) in Ca-Puffer (200 mM Herpes + 560 mM Natriumchlorid + 10 mM Calciumchlorid + 0.4% PEG) bzw. mit 20 µl Ca-Puffer (als unstimulierte Kontrolle) gemischt. Desweiteren werden 20 µl fluorogenes Thrombinsubstrat (Firma Bachem I-1120, Endkonzentration 50 µmol/l) und 20 µl Citratplasma (Firma Octapharma) zugegeben und gut homogenisiert. Die Platte wird im SpectraFluorplus Reader mit einem Excitationsfilter 360 nm und Emissionsfilter 465 nm über 20 Minuten jede Minute gemessen. Die Ermittlung des IC₅₀-Wertes erfolgt, wenn ca. 70% des Maximalsignals erreicht ist (ca. 12 min).

Repräsentative Wirkdaten aus diesem Test sind in der folgenden Tabelle 2 aufgeführt:

**Tabelle 2**

| **Beispiel Nr.** | **Ic₅₀ [nM]** |
|---|---|
| 4 | 286 |
| 7 | 277 |
| 9 | 247 |
| 16 | 14 |
| 18 | 502 |
| 19 | 8 |
| 27 | 54 |
| 30* | 34 |
| 32 | 151 |
| 35 | 390 |
| 38 | 632 |

| | |
|---|---|
| * Vergleichsbeispiel | |

### a.6) Thrombin Generation Assay (Thrombogramm)

Die Wirkung der Prüfsubstanzen auf das Thrombogramm (Thrombin Generation Assay nach Hemker) wird in vitro in Humanplasma (Octaplas® von der Firma Octapharma) bestimmt. Im Thrombin Generation Assay nach Hemker wird die Aktivität von Thrombin in gerinnendem Plasma durch die Messung der fluoreszenten Spaltprodukte des Substrats I-1140 (Z-Gly-Gly-Arg-AMC, Bachem) bestimmt. Um die Gerinnungsreaktion zu starten werden Reagenzien der Firma Thrombinoscope verwendet (PPP Reagenz: 30 pM recombinant tissue factor, 24 µM phospholipids in HEPES). Die Reaktion wird in Gegenwart variierender Konzentrationen an Prüfsubstanz oder dem entsprechenden Lösungsmittel durchgeführt. Außerdem wird ein Thrombin-Kalibrator der Firma Thrombinoscope verwendet, dessen amidolytische Aktivität zur Berechnung der Thrombinaktivität in einer Plasmaprobe benötigt wird.

Die Testdurchführung erfolgt nach Herstellerangaben (Thrombionsocpe BV): 4 µl der Prüfsubstanz oder des Lösungsmittels, 76 µl Plasma und 20 µl PPP-Reagenz oder Thrombin Calibrator werden 5 min bei 37°C inkubiert. Nach Zugabe von 20 µl 2.5 mM Thrombinsubstrat in 20 mM Hepes, 60 mg/ml BSA, 102 mM Calciumchlorid wird die Thrombin-Generierung über 120 min alle 20 s gemessen. Die Messung wird mit einem Fluorometer (Fluoroskan Ascent) der Firma Thermo Electron durchgeführt, der mit einem 390/460 nM Filterpaar und einem Dispenser ausgestattet ist. Durch die Verwendung der "thrombinoscope software" wird das Thrombogramm berechnet und graphisch dargestellt. Die folgenden Parameter werden berechnet: lag time, time to Peak, Peak, ETP (endogenous thrombin potential) und start tail.

### a.7) Bestimmung der antikoagulatorischen Wirkung

Die antikoagulatorische Wirkung der Prüfsubstanzen wird in vitro in Human-, Kaninchen- und Rattenplasma bestimmt. Dazu wird Blut unter Verwendung einer 0.11 molaren Natriumcitrat-Lösung als Vorlage in einem Mischungsverhältnis Natriumcitrat/Blut 1/9 abgenommen. Das Blut wird unmittelbar nach der Abnahme gut gemischt und 15 Minuten bei ca. 4000 g zentrifugiert. Der Überstand wird abpipettiert.

Die Prothrombinzeit (PT, Synonyme: Thromboplastinzeit, Quick-Test) wird in Gegenwart variierender Konzentrationen an Prüfsubstanz oder dem entsprechenden Lösungsmittel mit einem handelsüblichen Testkit (Neoplastin® von der Firma Boehringer Mannheim oder Hemoliance® RecombiPlastin von der Firma Instrumentation Laboratory) bestimmt. Die Testverbindungen werden 3 Minuten bei 37°C mit dem Plasma inkubiert. Anschließend wird durch Zugabe von Thromboplastin die Gerinnung ausgelöst und der Zeitpunkt des Gerinnungseintritts bestimmt. Es wird die Konzentration an Prüfsubstanz ermittelt, die eine Verdoppelung der Prothrombinzeit bewirkt.

Die Thrombinzeit (TT) wird in Gegenwart variierender Konzentrationen an Prüfsubstanz oder dem entsprechenden Lösungsmittel mit einem handelsüblichen Testkit (Thrombin Reagent von der Firma Roche) bestimmt. Die Testverbindungen werden 3 Minuten bei 37°C mit dem Plasma inkubiert. Anschließend wird durch Zugabe des Thrombin Reagenz die Gerinnung ausgelöst und der Zeitpunkt des Gerinnungseintritts bestimmt. Es wird die Konzentration an Prüfsubstanz ermittelt, die eine Verdoppelung der Thrombinzeit bewirkt.

Die aktivierte partielle Thromboplastinzeit (APTT) wird in Gegenwart variierender Konzentrationen an Prüfsubstanz oder dem entsprechenden Lösungsmittel mit einem handelsüblichen Testkit (PTT Reagent von der Firma Roche) bestimmt. Die Testverbindungen werden 3 Minuten bei 37°C mit dem Plasma und dem PTT Reagenz (Cephalin, Kaolin) inkubiert. Anschließend wird durch Zugabe von 25 mM Calciumchlorid die Gerinnung ausgelöst und der Zeitpunkt des Gerinnungseintritts bestimmt. Es wird die Konzentration an Prüfsubstanz ermittelt, die eine Verdoppelung der APTT bewirkt.

### a.8) Thromboetastographie (Thromboelastogramm)

Die Thromboelastographie wird mit Hilfe des Thromboelastographen ROTEM der Firma Pentapharm und dem dazu gehörenden Zubehör Cup and pin durchgeführt. Die Messung erfolgt in Vollblut, welches zuvor in Natrium-Citrat Monovetten der Firma Sarstedt entnommen wird. Das Blut wird in den Monovetten mit Hilfe eines Schüttlers in Bewegung gehalten und bei 37°C für 30 min. vorinkubiert. Es wird eine 2 molare Stammlösung von Calciumchlorid in Wasser erstellt. Diese wird 1:10 mit einer wässrigen 0.9%igen Natriumchlorid-Lösung verdünnt. Zur Messung werden 20 µl dieser 200 mM Calciumchlorid-Lösung in die Cups vorgelegt (Endkonzentration Calciumchlorid 12.5 mM). Es werden 3.2 µl Substanz oder Lösemittel zugegeben. Die Messung wird durch Zugabe von 300 µl Vollblut gestartet. Nach der Zugabe wird kurz mit der Pipettenspitze auf und ab pipettiert ohne Luftblasen zu erzeugen. Die Messung erfolgt über 2.5 Stunden oder wird bei Beginn der Fibrinolyse gestoppt. Zur Auswertung werden folgende Parameter bestimmt: CT(clotting time/ [sec.]), CFT (clotting formation time/ [sec.]), MCF (maximum clot firmness / [mm]) und der alpha-Winkel [°]. Die Messpunkte werden alle 3 Sekunden erhoben und graphisch über die y-Achse als MCF [mm] und auf der x-Achse als Zeit [sec.] dargestellt.

### a.9) Inhibierung der an Thrombus gebundenen Gerinnungsfaktoren Thrombin und Faktor Xa

Blutgerinnsel, die sich entweder vor Therapiebeginn mit Antikoagulantien, während Therapiepausen oder trotz Therapie bilden, enthalten große Mengen Gerinnungsfaktoren, die eine fortschreitende Thrombusbildung begünstigen können. Diese Gerinnungsfaktoren sind fest an den Thromus gebunden und können nicht ausgewaschen werden. In bestimmten klinischen Situationen kann hieraus ein Risiko für den Patienten entstehen. In den unten aufgeführten Versuchen lassen sich in humanen Thromben sowohl Thrombin als auch FXa mit biologischer (prokoagulatorischer) Aktivität nachweisen.

### In vitro gebildete Thromben

Thromben werden *in vitro* aus humanem Plasma gebildet und auf Aktivität der gebundenen Gerinnungsfaktoren Thrombin und FXa untersucht. Hierzu werden 300µl Plasma mit 30 µl Lipidvesikeln und 30 µl einer wässrigen Calciumchlorid-Lösung in einer 48 MTP Platte gemischt, und 30 min inkubiert. Dieser und die folgenden Schritte werden bei 37°C und unter konstanter Bewegung durchgeführt (300 U/min). Die gebildeten Thromben werden in eine neue 48 MTP Platte transferiert und in 0.9%iger Natriumchlorid-Lösung zweimal 10 min gewaschen, wobei der Thrombus zwischen den Waschgängen auf Filterpapier abgetupft wird. Der Thrombus wird in Puffer B transferiert (Owens Veronal Puffer, 1% BSA) und 15 min inkubiert, auf Filterpapier abgetupft und 30 min in Testsubstanz in verschiedenen Konzentrationen in Puffer B inkubiert. Anschließend werden die Clots wie oben beschrieben zweimalig gewaschen. Die Thromben werden abgetupft und in Puffer D transferiert: (240 µl Owren's veronal Puffer, 1% BSA und 15.6 mM Calciumchlorid) und mit oder ohne 0.6 µM Prothrombin 45 min inkubiert. Die Reaktion wird durch 75 µl 1% EDTA-Lösung gestoppt. Thrombinaktivität wird separat im Thrombus in Puffer A (7.5 mM Na₂EDTAx2H₂O, 175 mM Natriumchlorid, 1 % BSA, pH 8.4) oder im Überstand aus dem letzten Schritt gemessen. Hierzu wird das Substrat I-1120 in der Endkonzentration 50 µM eingesetzt, und die resultierende Fluoreszenz im Fluoreszenz-Plattenausleser ausgemessen (360/465nm).

Die Aktivität dieses thrombusgebundenen Thrombins kann durch einen selektiven FXa-Inhibitor in therapeutisch relevanten Konzentrationen nicht unterdrückt werden. Hingegen kann diese mit dualen FIIa/FXa Inhibitoren oder einem FIIa-Referenz-Inhibitor gehemmt werden.

Nach Zugabe von Prothrombin wird bei Vorhandensein von thrombusgebundenem FXa (Prothrombinase-Komplex) neues Thrombin gebildet, das durch das fluoreszierende Substrat nachgewiesen wird. Diese Neubildung von Thrombin kann durch einen reinen Thrombininhibitor nicht verhindert werden, jedoch durch duale FIIa/FXa Inhibitoren oder den selektiven FXa-Inhibitor Referenzinhibitor.

Die biologische Wirksamkeit der Thrombus-gebundenen Thrombinaktivität wird durch Zugabe von fluoreszierend markiertem Fibrinogen überprüft, das durch aktives Thrombin in Fibrin umgewandelt und an den Thrombus gebunden wird. Hierzu wird der Thrombus wie oben beschrieben gebildet und in 250 µl einer Alexa488-markiertem Fibrinogen-Lösung (100 µg/ml) und 30 µl einer wässrigen 100 mM Calciumchlorid-Lösung inkubiert (mit oder ohne Testsubstanzen in verschiedenen Konzentrationen). Die Fluoreszenz des Überstandes wird in einem Fluoreszenz-Plattenausleser bei geeigneter Wellenlänge gemessen. Außerdem werden die Thromben viermal jeweils 15 min gewaschen, und fluoreszenz-mikroskopisch evaluiert. Der Abfall der Fluoreszenz aus dem Überstand und die Zunahme der Fluoreszenz der Thromben kann durch duale FIIa/FXa Inhibitoren, nicht jedoch durch den FXa-Referenzinhibitor gehemmt werden.

### In vivo entstandene, intrakardiale Thromben (Patientenmaterial)

Die Untersuchungen werden wiederholt an Thromben, die Patienten im Rahmen von Herzoperationen aus dem linken Ventrikel entnommen worden waren. Hierzu werden die Thromben aufgetaut und in Stücke unterteilt (10 - 100mg Feuchtgewicht). Die Thromben werden, je nach Protokoll, mehrmalig gewaschen oder ungewaschen eingesetzt und die Thrombinaktivität mit dem Substrat I-1120 (Endkonzentration 100 µM) analog dem oben beschrieben Verfahren gemessen.

### a.10) Spezielle Diagnostik der Gerinnungsstörung und Organsfunktion bei endotoxämischen Mäusen und Ratten

### Thrombin-Antithrombin-Komplexe

Thrombin-Antithrombin-Komplexe (nachfolgend als "TAT" bezeichnet) sind ein Maß für das durch Gerinnungsaktivierung endogen gebildete Thrombin. TAT werden mittels eines ELISA-Assays bestimmt (Enzygnost TAT micro, Dade-Behring). Aus Zitratblut wird durch Zentrifugation Plasma gewonnen. Zu 50 µl Plasma wird 50 µl TAT-Probenpuffer gegeben, kurz geschüttelt und 15 min bei Raumtemperatur inkubiert. Die Proben werden abgesaugt, und das Well 3-malig mit Waschpuffer gewaschen (300 µl/Well). Die Platte wird zwischen den Waschgängen abgeklopft. Es wird Konjugatlösung (100 µl) hinzugegeben und 15 min bei Raumtemperatur inkubiert. Die Proben werden abgesaugt, und das Well 3-malig mit Waschpuffer gewaschen (300 µl/Well). Anschließend wird chromogenes Susbtrat hinzugegeben (100 µl/Well), 30 min im Dunkeln bei Raumtemperatur inkubiert, Stopplösung hinzugegeben (100 µl/ Well), und die Farbbildung bei 492 nm gemessen (Saphire Plate reader).

### Parameter für Organfunktion

Es werden verschiedene Parameter bestimmt, aufgrund derer Rückschlüsse auf die Funktionseinschränkung verschiedener innerer Organe durch die LPS-Gabe gezogen werden können, und der therapeutische Effekt von Prüfsubstanzen abgeschätzt werden kann. Zitratblut oder ggf. Lithium-Heparin-Blut wird zentrifugiert, und die Parameter aus dem Plasma bestimmt. Folgende Parameter werden typischerweise erhoben: Kreatinin, Harnstoff, Aspartat-Aminotransferase (AST), Alanin-Aminotransferase (ALT), Gesamt-Bilirubin, Laktatdehydrogenase (LDH), Gesamt-Protein, Gesamt-Albumin und Fibrinogen. Die Werte geben Aufschluss auf die Funktion der Niere, der Leber, des Kreislaufes und der Gefäße.

### Parameter für Entzündung

Das Ausmaß der durch Endotoxin ausgelösten Entzündungsreaktion lässt sich aus dem Anstieg von Entzündungsmediatoren im Plasma nachweisen, z.B. Interleukine (1, 6, 8 und 10), Tumornekrosefaktor alpha oder Monocyte Chemoattractant Protein-1. Hierzu können ELISAs oder das Luminex-System verwendet werden.

### b) Bestimmung der antithrombotischen Wirkung (in vivo)

### b.1) Arteriovenöses Shunt- und Blutungs-Modell (Kombi-Modell Ratte)

Nüchterne männliche Ratten (Stamm: HSD CPB:WU) mit einem Gewicht von 300-350 g werden mit Inactin (150 - 180 mg/kg) narkotisiert. Die Thrombusbildung wird in einem arteriovenösen Shunt in Anlehnung an die von Christopher N. Berry et al., Br. J. Pharmacol. (1994), 113, 1209-1214 beschriebene Methode ausgelöst. Dazu werden die linke Vena jugularis und die rechte Arteria carotis freipräpariert. Ein extracorporaler Shunt wird mittels eines 10 cm langen Polyethylenschlauchs (PE 60) zwischen den beiden Gefäßen gelegt. Dieser Polyethylenschlauch ist in der Mitte in einen weiteren 3 cm langen Polyethylenschlauch (PE 160), der zur Erzeugung einer thrombogenen Oberfläche einen aufgerauhten und zu einer Schlinge gelegten Nylonfaden enthält, eingebunden. Der extrakorporale Kreislauf wird 15 Minuten lang aufrechterhalten. Dann wird der Shunt entfernt und der Nylonfaden mit dem Thrombus sofort gewogen. Das Leergewicht des Nylonfadens ist vor Versuchsbeginn ermittelt worden.

Zur Bestimmung der Blutungszeit wird unmittelbar nach Öffnung des Shunt-Kreislaufs die Schwanzspitze der Ratten mit einer Rasierklinge um 3 mm kupiert. Der Schwanz wird in 37°C temperierte physiologische Kochsalzlösung gelegt und die Blutung aus der Schnittwunde über 15 Minuten beobachtet. Es werden die Zeit bis zum Sistieren der Blutung für mind. 30 Sekunden (initiale Blutungszeit), die Gesamtblutungszeit innerhalb von 15 Minuten (kumulative Blutungszeit) sowie die Menge des Blutverlusts über die photometrische Bestimmung des aufgefangenen Hämoglobins ermittelt.

Die Prüfsubstanzen werden vor Anlegung des extrakorporalen Kreislaufs und des Schwanzspitzenschnitts entweder intravenös über die kontralaterale Jugularvene als Einzelbolus bzw. als Bolus mit anschließender Dauerinfusion oder oral mittels Schlundsonde wachen Tieren verabreicht.

### c) Bestimmung der Pharmakokinetik (in vivo)

Zur Bestimmung der *in vivo* Pharmakokinetik werden die Testsubstanzen in verschiedenen Formulierungsmitteln (z.B. Plasma, Ethanol, DMSO, PEG400 etc.) oder Gemischen dieser Lösungsvennittler gelöst Mäusen, Ratten, Hunden oder Affen intravenös oder peroral appliziert. Die intravenöse Applikation erfolgt wahlweise als Bolus oder als Infusion. Die applizierten Dosen liegen im Bereich von 0.1 bis 5 mg/kg. Blutproben werden mittels eines Katheters oder als Tötungsplasma zu verschiedenen Zeitenpunkten über ein Intervall von bis zu 26 h entnommen. Des weiteren werden teilweise auch Organ-, Gewebs- und Urinproben gewonnen. Die quantitative Bestimmung der Substanzen in den Versuchsproben erfolgt mittels Eichproben die in der jeweiligen Matrix eingestellt werden. In den Proben enthaltene Proteine werden durch Fällung mit Acetonitril oder Methanol entfernt. Anschließend werden die Proben mittels HPLC auf einer 2300 HTLC Anlage (Cohesive Technologies, Franklin, MA, USA) unter Verwendung von Reversed Phase Säulen aufgetrennt. Das HPLC System ist über ein Turbo Ion Spray Interface an ein Triple Quadropole Massenspektrometer API 3000 (Applied Biosystems, Darmstadt, Deutschland) gekoppelt. Die Auswertung des Plasmakonzentrations-Zeitverlaufs erfolgt unter Verwendung eines validierten Kinetikauswerteprogramms.

Die Affinität einer Substanz für ein Transportprotein wird mittels *in vitro* Testung in einem Flux Assay unter Verwendung von Caco-2 Zellen oder Zellen die ein spezifischen Transporter überexpremieren untersucht (Troutman MD, Thakker DR, Pharm. Res. 20 (8) 1210-1224 (2003); Schwab D, Fischer H, Tabatabaei A, Poli S, Huwyler J, J. Med. Chem. 46, 1716-1725 (2003); Merino G, Jonker JW, Wagenaar E, Pulido MM, Molina AJ, Alvarez AI, Schinkel AH, Drug Metab. Dispos. 33 (5) 614- 618 (2005)). Hierzu werden die Zellen auf 24- oder 96-Loch-Filter-Platten für 4 bis 15 Tage kultiviert. Zur Bestimmung der Permeation werden die Substanzen in einem HEPES-Puffer entweder apikal (A) oder basal (B) auf die Zellen gegeben und für 2 h inkubiert. Nach 0 h und 2 h werden Proben aus den Cis- und Transkompartimenten gezogen und mittels LC-MS/MS analysiert. Der Papp-Wert wird mittels der von Schwab et al. publizierten Formel berechnet. Eine Substanz wird als aktiv transportiert klassifiziert, wenn das Ratio von Papp (B-A)/Papp (A-B) > 2 oder < 0.5 ist.

### d) Bestimmung der Wirkung bei Endotoxinämie (in vivo)

Die Untersuchung wird an Ratten oder Mäusen durchgeführt. Im Modell an Mäusen (NMRI, männlich) wird LPS *(Escherichia coli* Serotyp 055:B5, Sigma-Aldrich) 50 mg/kg intraperitoneal injiziert. Die Prüfsubstanzen werden bis zu einer Stunde vor LPS-Injektion entweder intravenös über die Schwanzvene, subkutan, intraperitoneal oder oral mittels Schlundsonde verabreicht. Vier Stunden nach LPS-Applikation wird das Tier narkotisiert (Ketavet/Rompun) und das Abdomen operativ eröffnet. Natrium-Zitratlösung (3.2% w/v) (Formel: Körpergewicht in g / 13 mal 100 µl) wird in die untere Hohlvene injiziert, und nach 30 Sek. Blut entnommen (ca. 1 ml). Aus dem Blut werden verschiedene Parameter bestimmt, z.B. die zellulären Blutbestandteile (insbesondere Erythrozyten, Leukozyten und Thrombozyten), der Laktatspiegel, die Gerinnungsaktivierung (TAT) oder Parameter der Organdysfunktion oder des Organversagens und Sterblichkeit.

### e) Methodikbeschreibung zu DIC-Versuchen an der Ratte

Bei männlichen Wistar-Ratten wird LPS (E. coli O55 B5, Hersteller Sigma, gelöst in PBS) in einer Dosierung von 250 µg/ kg intravenös in die Schwanzvene injiziert (Applikationsvolumen 2 ml/kg). Die Prüfsubstanz wird in PEG 400/H₂O 60%/40% gelöst und oral (Applikationsvolumen 5 ml/kg) 30 Minuten vor LPS-Injektion appliziert. 1, 5 oder 4 Stunden nach LPS-Injektion werden die Tiere in Terminalnarkose (Trapanal^{®} 100 mg/kg i.p.) durch Herzpunktion entblutet und Citratplasma für die Bestimmung von Fibrinogen, PT, TAT und Plättchenzahl gewonnen. Optional wird Serum zur Bestimmung von Leberenzymen, Nierenfunktionsparamertern und Cytokinen gewonnen. TNFα und IL-6 werden mit kommerziell erhältlichen ELISAs (R&D Systems) bestimmt.

Es können auch direkte Parameter der Organfunktion gemessen werden, z.B. links- und rechtsventrikuläre Drucke, arterielle Drucke, Urinausscheidung, Nierendurchblutung und Blutgase und Säure-Basenstatus.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Natriumchloridlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel 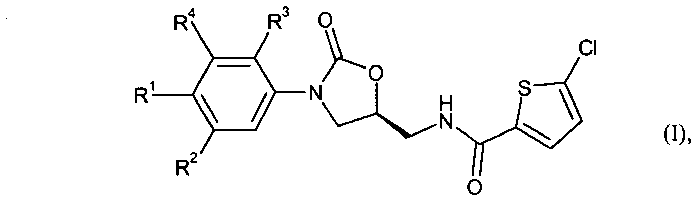 in welcher
R¹ für eine Gruppe der Formel 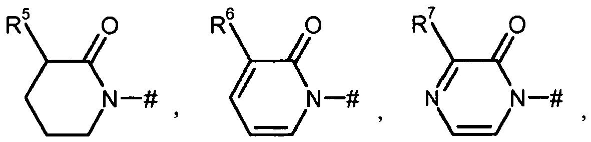 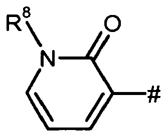 oder 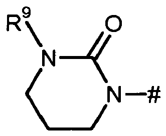 steht,
wobei
# die Anknüpfstelle an den Phenylring ist,
R⁵ für Wasserstoff oder C₁-C₃-Alkyl steht, worin Alkyl substituiert sein kann mit einem Substituenten Hydroxy,
R⁶ für Wasserstoff, Trifluormethyl, Trifluormethoxy, Difluormethoxy, C₁-C₃-Alkyl oder C₁-C₄-Alkoxy steht,
worin Alkoxy substituiert sein kann mit einem Substituenten; wobei der Substituent ausgewählt wird aus der Gruppe bestehend aus Halogen, Hydroxy, Trifluormethyl, Aminocarbonyl, C₁-C₃-Alkoxy, C₁-C₃-Alkylcarbonyl, C₁-C₄-Alkylaminocarbonyl und Morpholinocarbonyl,
R⁷ für Wasserstoff, C₁-C₃-Alkyl oder C₁-C₄-Alkoxy steht,
worin C₂-C₃-Akl und C₂-C₄-Alkoxy substituiert sein können mit einem Substituenten Hydroxy,
R⁸ für Wasserstoff oder C₁-C₃-Alkyl steht,
worin C₂-C₃-Alkyl substituiert sein kann mit einem Substituenten Hydroxy,
R⁹ für Wasserstoff oder C₁-C₃-Alkyl steht,
worin C₂-C₃-Alkyl substituiert sein kann mit einem Substituenten Hydroxy,
R² für Chlor, Trifluormethyl, Trifluormethoxy, Difluormethoxy, Methyl, Ethyl, n-Propyl, iso-Propyl, Methoxy, Ethoxy, Methoxymethyl, Ethoxymethyl oder Cyclopropyl steht,
R³ für Wasserstoff, C₁-C₃-Alkoxy oder C₁-C₃-Alkylamino steht,
R⁴ für Wasserstoff oder Methyl steht,
oder eines ihrer Salze, ihrer Solvate oder der Solvate ihrer Salze.

2. Verbindung nach Anspruch 1, **dadurch gekennzeichnet, dass**
R¹ für eine Gruppe der Formel 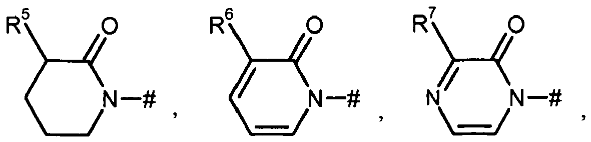 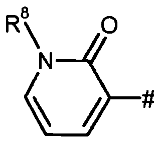 oder 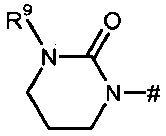 steht, wobei
# die Anknüpfstelle an den Phenylring ist,
R⁵ für Wasserstoff oder C₁-C₃-Alkyl steht,
worin Alkyl substituiert sein kann mit einem Substituenten Hydroxy,
R⁶ für Wasserstoff, Trifluormethyl, Trifluormethoxy, C₁-C₃-Alkyl oder C₁-C₄-Alkoxy steht,
worin Alkoxy substituiert sein kann mit einem Substituenten, wobei der Substituent ausgewählt wird aus der Gruppe bestehend aus Halogen, Hydroxy, Trifluormethyl, Aminocarbonyl, C₁-C₃-Alkoxy, C₁-C₃-Alkylcarbonyl, C₁-C₄-Alkylaminocarbonyl und Morpholinocarbonyl,
R⁷ für C₁-C₄-Alkoxy steht,
R⁸ für C₁-C₃-Alkyl steht,
R⁹ für Wasserstoff oder C₁-C₃-Alkyl steht, worin C₂-C₃-Alkyl substituiert sein kann mit einem Substituenten Hydroxy,
R² für Chlor, Trifluormethyl, Methyl, Ethyl, n-Propyl, iso-Propyl, Methoxy, Ethoxy, Methoxymethyl, Ethoxymethyl oder Cyclopropyl steht,
R³ für Wasserstoff, C₁-C₃-Alkoxy oder C₁-C₃-Alkylamino steht,
R⁴ für Wasserstoff oder Methyl steht,
oder eines ihrer Salze, ihrer Solvate oder der Solvate ihrer Salze.

3. Verbindung nach einem der Ansprüche 1 oder 2, **dadurch gekennzeichnet, dass**
R¹ für eine Gruppe der Formel 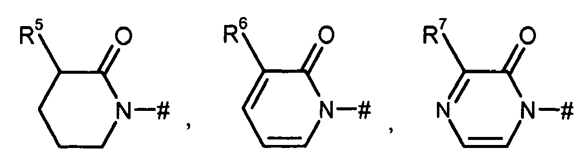 oder 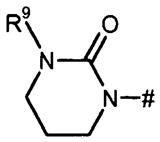 steht,
wobei
# die Anknüpfstelle an den Phenylring ist,
R⁵ für Wasserstoff oder C₁-C₃-Alkyl steht,
worin Alkyl substituiert sein kann mit einem Substituenten Hydroxy,
R⁶ für Wasserstoff, Trifluormethyl, Trifluormethoxy, C₁-C₃-Alkyl oder C₁-C₄-Alkoxy steht,
worin Alkoxy substituiert sein kann mit einem Substituenten, wobei der Substituent ausgewählt wird aus der Gruppe bestehend aus Halogen, Hydroxy, Trifluormethyl, Aminocarbonyl, C₁-C₃-Alkoxy, C₁-C₃-Alkylcarbonyl, C₁-C₄-Alkylaminocarbonyl und Morpholinocarbonyl,
R⁷ für C₁-C₄-Alkoxy steht,
R⁹ für Wasserstoff oder C₁-C₃-Alkyl steht,
worin C₂-C₃-Alkyl substituiert sein kann mit einem Substituenten Hydroxy,
R² für Chlor, Trifluormethyl, Methyl, Ethyl, n-Propyl, iso-Propyl, Methoxy, Ethoxy, Methoxymethyl, Ethoxymethyl oder Cyclopropyl steht,
R³ für Wasserstoff, C₁-C₃-Alkoxy oder C₁-C₃-Alkylamino steht,
R⁴ für Wasserstoff oder Methyl steht,
oder eines ihrer Salze, ihrer Solvate oder der Solvate ihrer Salze.

4. Verbindung nach einem der Ansprüche 1 bis 3, **dadurch gekennzeichnet, dass**
R¹ für eine Gruppe der Formel 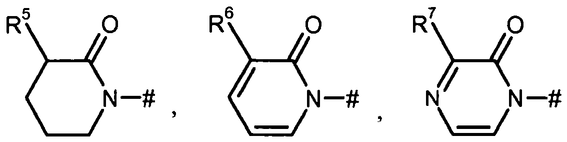 oder 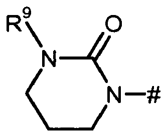 steht,
wobei
# die Anknüpfstelle an den Phenylring ist,
R⁵ für Wasserstoff steht,
R⁶ für Wasserstoff oder C₁-C₄-Alkoxy steht,
worin Alkoxy substituiert sein kann mit einem Substituenten Hydroxy,
R⁷ für Ethoxy steht,
R⁹ für Wasserstoff, Methyl oder 2-Hydroxyeth-1-yl steht,
R² für Methyl, iso-Propyl, Methoxy, Ethoxy, Methoxymethyl oder Cyclopropyl steht,
R³ für Wasserstoff steht,
R⁴ für Wasserstoff oder Methyl steht,
oder eines ihrer Salze, ihrer Solvate oder der Solvate ihrer Salze.

5. Verfahren zur Herstellung einer Verbindung der Formel (I) oder eines ihrer Salze, ihrer Solvate oder der Solvate ihrer Salze nach Anspruch 1, **dadurch gekennzeichnet, dass**
[A] die Verbindung der Formel 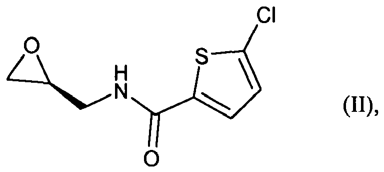 in der ersten Stufe mit einer Verbindung der Formel 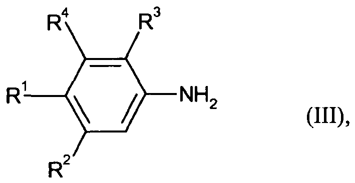
in welcher R¹, R², R³ und R⁴ die in Anspruch 1 angegebene Bedeutung haben,
zu einer Verbindung der Formel 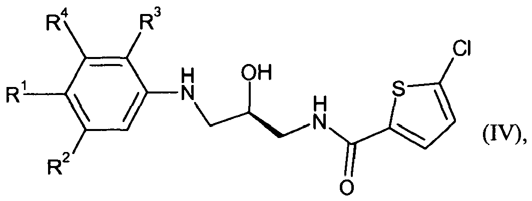 in welcher R¹, R², R³ und R⁴ die in Anspruch 1 angegebene Bedeutung haben,
umgesetzt wird,
und in der zweiten Stufe in Anwesenheit von Phosgen oder Phosgenäquivalenten zu einer Verbindung der Formel (I) cyclisiert wird
oder
[B] eine Verbindung der Formel 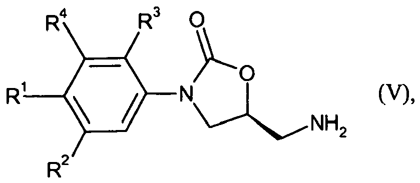 in welcher R¹, R², R³ und R⁴ die in Anspruch 1 angegebene Bedeutung haben,
mit eienr Verbindung der Formel 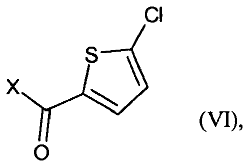 in welcher
X für Halogen, bevorzugt Brom oder Chlor, oder Hydroxy steht,
umgesetzt wird.

6. Verbindung nach einem der Ansprüche 1 bis 4 zur Verwendung in der Behandlung und/oder Prophylaxe von Krankheiten.

7. Verwendung einer Verbindung nach einem der Ansprüche 1 bis 4 zur Herstellung eines Arzneimittels zur Behandlung und/oder Prophylaxe von Krankheiten.

8. Verwendung einer Verbindung nach einem der Ansprüche 1 bis 4 zur Herstellung eines Arzneimittels zur Behandlung und/oder Prophylaxe von thromboembolischen Erkrankungen.

9. Arzneimittel enthaltend eine Verbindung nach einem der Ansprüche 1 bis 4 in Kombination mit einem inerten, nichttoxischen, pharmazeutisch geeigneten Hilfsstoff.

10. Arzneimittel enthaltend eine Verbindung nach einem der Ansprüche 1 bis 4 in Kombination mit einem weiteren Wirkstoff.

11. Arzneimittel nach Anspruch 10 oder 11 zur Verwendung in der Behandlung und/oder Prophylaxe von thromboembolischen Erkrankungen.

12. Verfahren zur Verhinderung der Blutkoagulation in vitro, **dadurch gekennzeichnet, dass** eine antikoagulatorisch wirksame Menge einer Verbindung nach einem der Ansprüche 1 bis 4 zugegeben wird.

13. Verwendung einer Verbindung nach einem der Ansprüche 1 bis 4 zur Herstellung eines Arzneimittels zur Behandlung und/oder Prophylaxe von pulmonaler Hypertonie.

14. Verwendung einer Verbindung nach einem der Ansprüche 1 bis 4 zur Herstellung eines Arzneimittels zur Behandlung und/oder Prophylaxe von Sepsis, Systemic Inflammatory Syndrome (SIRS), septische Organdysfunktion, septisches Organversagen und Muliorganversagen, Acute Respiratory Distress Syndrome (ARDS), Acute Lung Injury (ALI), Septischer Schock, DIC ("Disseminated Intravascular coagulation") und/oder des septischen Organversagens.

15. Verbindung, wie in einem der Ansprüche 1 bis 4 definiert, zur Verwendung in einem Verfahren zur Behandlung und/oder Prophylaxe von thromboembolischen Erkrankungen.

## Claims

1. Compound of the formula 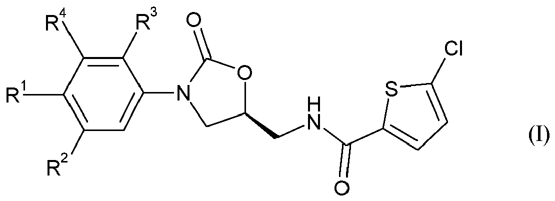 in which
R¹ is a group of the formula 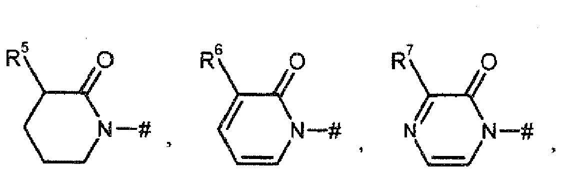 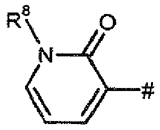 or 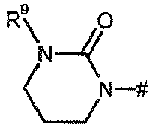 where
# is the attachment site to the phenyl ring,
R⁵ is hydrogen or C₁-C₃-alkyl,
in which alkyl may be substituted by a hydroxyl substituent,
R⁶ is hydrogen, trifluoromethyl, trifluoromethoxy, difluoromethoxy, C₁-C₃-alkyl or C₁-C₄-alkoxy,
in which alkoxy may be substituted by a substituent, which substituent is selected from the group consisting of halogen, hydroxy, trifluoromethyl, aminocarbonyl, C₁-C₃-alkoxy, C₁-C₃-alkylcarbonyl, C₁-C₄-alkylaminocarbonyl and morpholinocarbonyl,
R⁷ is hydrogen, C₁-C₃-alkyl or C₁-C₄-alkoxy,
in which C₂-C₃-alkyl and C₂-C₄-alkoxy may be substituted by a hydroxyl substituent,
R⁸ is hydrogen or C₁-C₃-alkyl,
in which C₂-C₃-alkyl may be substituted by a hydroxyl substituent,
R⁹ is hydrogen or C₁-C₃-alkyl,
in which C₂-C₃-alkyl may be substituted by a hydroxyl substituent,
R² is chlorine, trifluoromethyl, trifluoromethoxy, difluoromethoxy, methyl, ethyl, n-propyl, isopropyl, methoxy, ethoxy, methoxymethyl, ethoxymethyl or cyclopropyl,
R³ is hydrogen, C₁-C₃-alkoxy or C₁-C₃-alkylamino,
R⁴ is hydrogen or methyl,
or one of the salts thereof, solvates thereof or the solvates of salts thereof.

2. Compound according to Claim 1, **characterized in that**
R¹ is a group of the formula 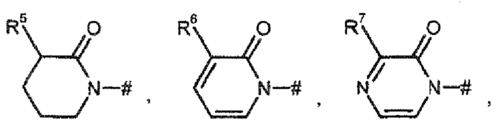 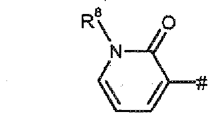 or 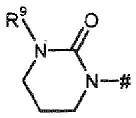 where
# is the attachment site to the phenyl ring,
R⁵ is hydrogen or C₁-C₃-alkyl,
in which alkyl may be substituted by a hydroxyl substituent,
R⁶ is hydrogen, trifluoromethyl, trifluoromethoxy, C₁-C₃-alkyl or C₁-C₄-alkoxy,
in which alkoxy may be substituted by a substituent, which substituent is selected from the group consisting of halogen, hydroxy, trifluoromethyl, aminocarbonyl, C₁-C₃-alkoxy, C₁-C₃-alkylcarbonyl, C₁-C₄-alkylaminocarbonyl and morpholinocarbonyl,
R⁷ is C₁-C₄-alkoxy,
R⁸ is C₁-C₃-alkyl,
R⁹ is hydrogen or C₁-C₃-alkyl,
in which C₂-C₃-alkyl may be substituted by a hydroxyl substituent,
R² is chlorine, trifluoromethyl, methyl, ethyl, n-propyl, isopropyl, methoxy, ethoxy, methoxymethyl, ethoxymethyl or cyclopropyl,
R³ is hydrogen, C₁-C₃-alkoxy or C₁-C₃-alkylamino,
R⁴ is hydrogen or methyl,
or one of the salts thereof, solvates thereof or the solvates of salts thereof.

3. Compound according to either of Claims 1 and 2, **characterized in that**
R¹ is a group of the formula 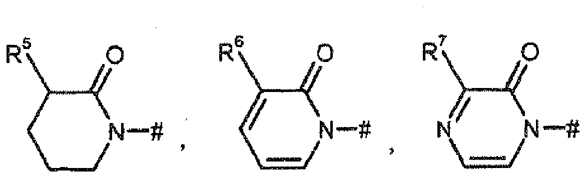 or 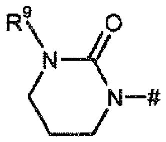 where
# is the attachment site to the phenyl ring,
R⁵ is hydrogen or C₁-C₃-alkyl,
in which alkyl may be substituted by a hydroxyl substituent,
R⁶ is hydrogen, trifluoromethyl, trifluoromethoxy, C₁-C₃-alkyl or C₁-C₄-alkoxy,
in which alkoxy may be substituted by a substituent, which substituent is selected from the group consisting of halogen, hydroxy, trifluoromethyl, aminocarbonyl, C₁-C₃-alkoxy, C₁-C₃-alkylcarbonyl, C₁-C₄-alkylaminocarbonyl and morpholinocarbonyl,
R⁷ is C₁-C₄-alkoxy,
R⁹ is hydrogen or C₁-C₃-alkyl,
in which C₂-C₃-alkyl may be substituted by a hydroxyl substituent,
R² is chlorine, trifluoromethyl, methyl, ethyl, n-propyl, isopropyl, methoxy, ethoxy, methoxymethyl, ethoxymethyl or cyclopropyl,
R³ is hydrogen, C₁-C₃-alkoxy or C₁-C₃-alkylamino,
R⁴ is hydrogen or methyl,
or one of the salts thereof, solvates thereof or the solvates of salts thereof.

4. Compound according to any of Claims 1 to 3, **characterized in that**
R¹ is a group of the formula 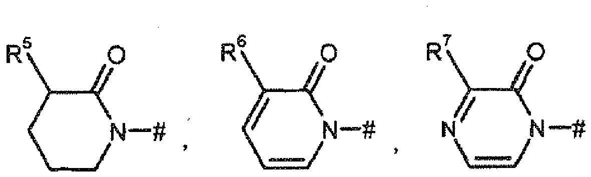 or 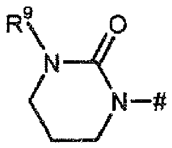 where
# is the attachment site to the phenyl ring,
R⁵ is hydrogen,
R⁶ is hydrogen or C₁-Cₐ-alkoxy,
in which alkoxy may be substituted by a hydroxyl substituent,
R⁷ is ethoxy,
R⁹ is hydrogen, methyl or 2-hydroxyeth-1-yl,
R² is methyl, isopropyl, methoxy, ethoxy, methoxymethyl or cyclopropyl,
R³ is hydrogen,
R⁴ is hydrogen or methyl,
or one of the salts thereof, solvates thereof or the solvates of salts thereof.

5. Process for preparing a compound of the formula (I) or one of the salts thereof, solvates thereof or the solvates of salts thereof according to Claim 1, **characterized in that**
[A] the compound of the formula 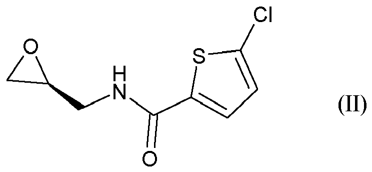 is reacted in the first stage with a compound of the formula 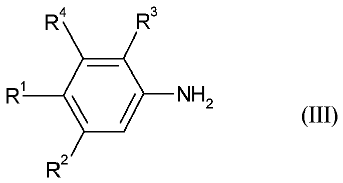 in which R¹, R², R³ and R⁴ are each as defined in Claim 1
to give a compound of the formula 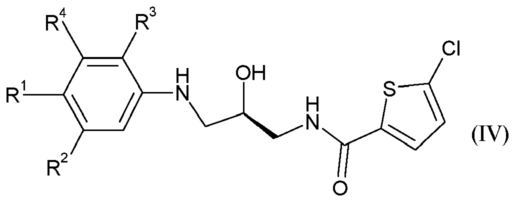 in which R¹, R², R³ and R⁴ are each as defined in Claim 1,
and, in the second stage, in the presence of phosgene or phosgene equivalents, the latter is cyclized to a compound of the formula (I),
or
[B] a compound of the formula 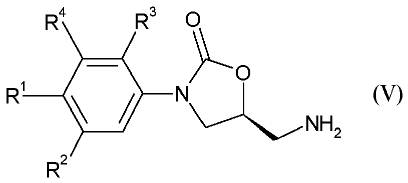 in which R¹, R², R³ and R⁴ are each as defined in Claim 1
is reacted with a compound of the formula 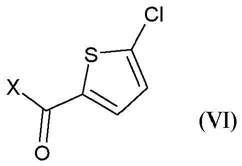 in which
X is halogen, preferably bromine or chlorine, or hydroxy.

6. Compound according to any of Claims 1 to 4 for use in the treatment and/or prophylaxis of diseases.

7. Use of a compound according to any of Claims 1 to 4 for producing a medicament for treatment and/or prophylaxis of diseases.

8. Use of a compound according to any of Claims 1 to 4 for producing a medicament for treatment and/or prophylaxis of thromboembolic disorders.

9. Medicament comprising a compound according to any of Claims 1 to 4 in combination with an inert nontoxic pharmaceutically suitable excipient.

10. Medicament comprising a compound according to any of Claims 1 to 4 in combination with a further active ingredient.

11. Medicament according to Claim 10 or 11 for use in the treatment and/or prophylaxis of thromboembolic disorders.

12. Method for preventing blood coagulation in vitro, **characterized in that** an anticoagulatory amount of a compound according to any of Claims 1 to 4 is added.

13. Use of a compound according to any of Claims 1 to 4 for producing a medicament for treatment and/or prophylaxis of pulmonary hypertension.

14. Use of a compound according to any of Claims 1 to 4 for producing a medicament for treatment and/or prophylaxis of sepsis, systemic inflammatory syndrome (SIRS), septic organ dysfunction, septic organ failure and multiorgan failure, acute respiratory distress syndrome (ARDS), acute lung injury (ALI), septic shock, DIC ("disseminated intravascular coagulation") and/or septic organ failure.

15. Compound as defined in any of Claims 1 to 4 for use in a process for treatment and/or prophylaxis of thromboembolic disorders.

## Revendications

1. Composé de formule 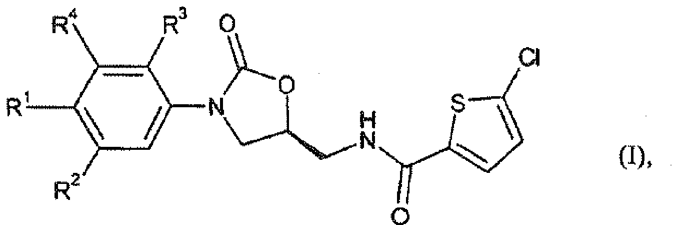 dans laquelle
R¹ est un groupe de formule 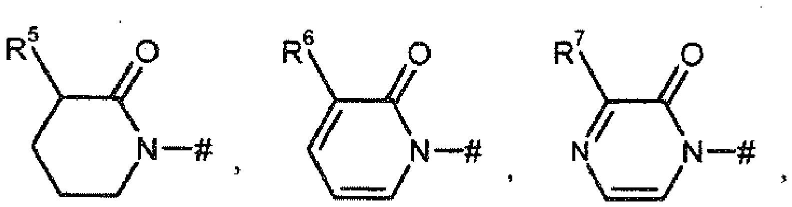 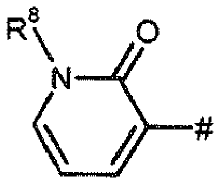 ou 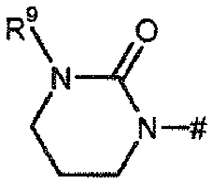 dans laquelle
# est le point de rattachement au noyau phényle,
R⁵ est un atome d'hydrogène ou un groupe alkyle en C₁-C₃, le groupe alkyle pouvant être substitué par un substituant hydroxy,
R⁶ est un atome d'hydrogène, un groupe trifluorométhyle, trifluorométhoxy, difluorométhoxy, alkyle en C₁-C₃ ou alcoxy en C₁-C₄, le groupe alcoxy pouvant être substitué par un substituant, le substituant étant choisi dans le groupe consistant en les groupes halogéno, hydroxy, trifluorométhyle, aminocarbonyle, alcoxy en C₁-C₃, (alkyle en C₁-C₃) carbonyle, (alkyle en C₁-C₄)aminocarbonyle et morpholinocarbonyle,
R⁷ est un atome d'hydrogène, un groupe alkyle en C₁-C₃ ou alcoxy en C₁-C₄, le groupe alkyle en C₂-C₃ et alcoxy en C₂-C₄ pouvant être substitué par un substituant hydroxy,
R⁸ est un atome d'hydrogène ou un groupe alkyle en C₁-C₃, le groupe alkyle en C₂-C₃ pouvant être substitué par un substituant hydroxy,
R⁹ est un atome d'hydrogène ou un groupe alkyle en C₁-C₃, le groupe alkyle en C₂-C₃ pouvant être substitué par un substituant hydroxy,
R² est un groupe chloro, trifluorométhyle, trifluorométhoxy, difluorométhoxy, méthyle, éthyle, n-propyle, isopropyle, méthoxy, éthoxy, méthoxyméthyle, éthoxyméthyle ou cyclopropyle,
R³ est un atome d'hydrogène, un groupe alcoxy en C₁-C₃ ou alkylamino en C₁-C₃,
R⁴ est un atome d'hydrogène ou le groupe méthyle,
ou l'un de ses sels, l'un de ses produits de solvatation ou l'un des produits de solvatation de ses sels.

2. Composé selon la revendication 1, **caractérisé en ce que**
R¹ est un groupe de formule 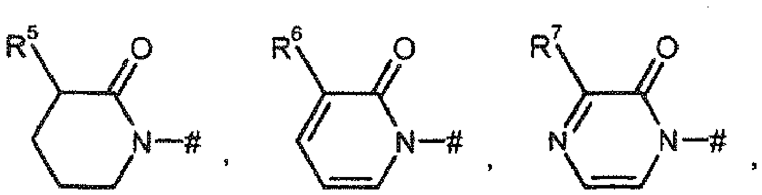 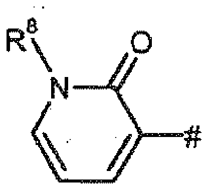 ou 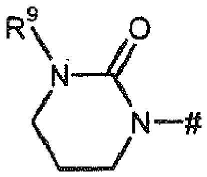 dans laquelle
# est le point de rattachement au noyau phényle,
R⁵ est un atome d'hydrogène ou un groupe alkyle en C₁-C₃, le groupe alkyle pouvant être substitué par un substituant hydroxy,
R⁶ est un atome d'hydrogène, un groupe trifluorométhyle, trifluorométhoxy, alkyle en C₁-C₃ ou alcoxy en C₁-C₄, le groupe alcoxy pouvant être substitué par un substituant, le substituant étant choisi dans le groupe consistant en les groupes halogéno, hydroxy, trifluorométhyle, aminocarbonyle, alcoxy en C₁-C₃, (alkyle en C₁-C₃) carbonyle, (alkyle en C₁-C₄)aminocarbonyle et morpholinocarbonyle,
R⁷ est un groupe alcoxy en C₁-C₄,
R⁸ est un groupe alkyle en C₁-C₃,
R⁹ est un atome d'hydrogène ou un groupe alkyle en C₁-C₃, le groupe alkyle en C₂-C₃ pouvant être substitué par un substituant hydroxy,
R² est un groupe chloro, trifluorométhyle, méthyle, éthyle, n-propyle, isopropyle, méthoxy, éthoxy, méthoxyméthyle, éthoxyméthyle ou cyclopropyle,
R³ est un atome d'hydrogène, un groupe alcoxy en C₁-C₃ ou alkylamino en C₁-C₃,
R⁴ est un atome d'hydrogène ou le groupe méthyle,
ou l'un de ses sels, l'un de ses produits de solvatation ou l'un des produits de solvatation de ses sels.

3. Composé selon l'une des revendications 1 ou 2, **caractérisé en ce que**
R¹ est un groupe de formule 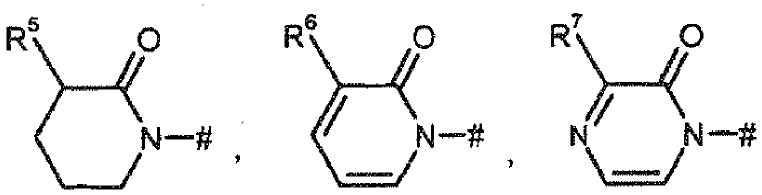 ou 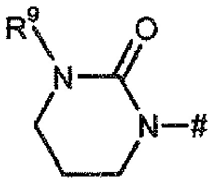 dans laquelle
# est le point de rattachement au noyau phényle,
R⁵ est un atome d'hydrogène ou un groupe alkyle en C₁-C₃, le groupe alkyle pouvant être substitué par un substituant hydroxy,
R⁶ est un atome d'hydrogène, un groupe trifluorométhyle, trifluorométhoxy, alkyle en C₁-C₃ ou alcoxy en C₁-C₄, le groupe alcoxy pouvant être substitué par un substituant, le substituant étant choisi dans le groupe consistant en les groupes halogéno, hydroxy, trifluorométhyle, aminocarbonyle, alcoxy en C₁-C₃, (alkyle en C₁-C₃) carbonyle, (alkyle en C₁-C₄)aminocarbonyle et morpholinocarbonyle,
R⁷ est un groupe alcoxy en C₁-C₄,
R⁹ est un atome d'hydrogène ou un groupe alkyle en C₁-C₃, le groupe alkyle en C₂-C₃ pouvant être substitué par un substituant hydroxy,
R² est un groupe chloro, trifluorométhyle, méthyle, éthyle, n-propyle, isopropyle, méthoxy, éthoxy, méthoxyméthyle, éthoxyméthyle ou cyclopropyle,
R³ est un atome d'hydrogène, un groupe alcoxy en C₁-C₃ ou alkylamino en C₁-C₃,
R⁴ est un atome d'hydrogène ou le groupe méthyle,
ou l'un de ses sels, l'un de ses produits de solvatation ou l'un des produits de solvatation de ses sels.

4. Composé selon l'une des revendications 1 à 3, **caractérisé en ce que**
R¹ est un groupe de formule 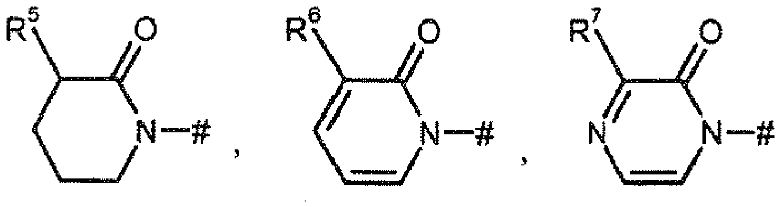 ou 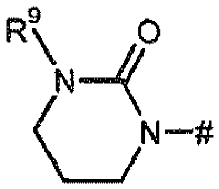 dans laquelle
# est le point de rattachement au noyau phényle,
R⁵ est un atome d'hydrogène,
R⁶ est un atome d'hydrogène ou un groupe alcoxy en C₁-C₄, le groupe alcoxy pouvant être substitué par un substituant hydroxy,
R⁷ est le groupe éthoxy,
R⁹ est un atome d'hydrogène, le groupe méthyle ou 2-hydroxyéth-1-yle,
R² est le groupe méthyle, isopropyle, méthoxy, éthoxy, méthoxyméthyle ou cyclopropyle,
R³ est un atome d'hydrogène,
R⁴ est un atome d'hydrogène ou le groupe méthyle,
ou l'un de ses sels, l'un de ses produits de solvatation ou l'un des produits de solvatation de ses sels.

5. Procédé de préparation d'un composé de formule (I) ou d'un de ses sels, d'un de ses produits de solvatation ou d'un des produits de solvatation de ses sels selon la revendication 1, **caractérisé en ce que**
[A] dans la première étape, on fait réagir le composé de formule 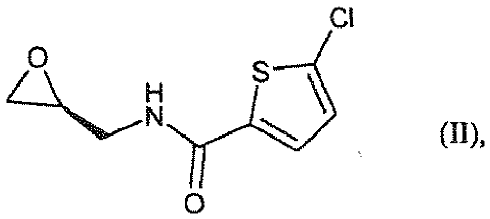 avec un composé de formule 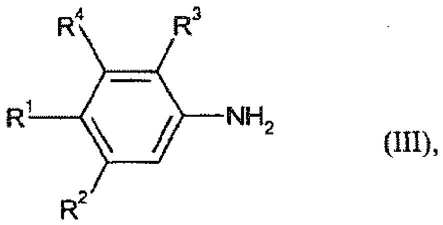 dans laquelle R¹, R², R³ et R⁴ ont les significations données dans la revendication 1, pour donner un composé de formule 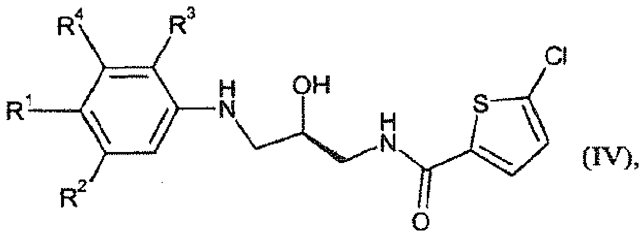 dans laquelle R¹, R², R³ et R⁴ ont les significations données dans la revendication 1,
et, dans la deuxième étape, en présence de phosgène ou d'un équivalent du phosgène, on le cyclise pour obtenir un composé de formule (I)
ou
[B] on fait réagir un composé de formule 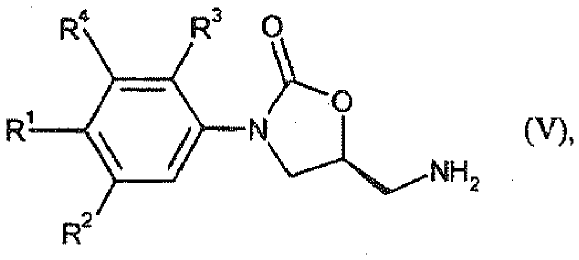 dans laquelle R¹, R², R³ et R⁴ ont les significations données dans la revendication 1, avec un composé de formule 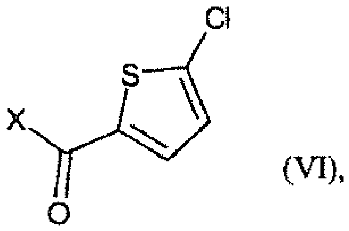 dans laquelle X est un halogène, de préférence le brome ou le chlore, ou le groupe hydroxy.

6. Composé selon l'une des revendications 1 à 4, pour utilisation dans le traitement et/ou la prophylaxie de maladies.

7. Utilisation d'un composé selon l'une des revendications 1 à 4 pour préparer un médicament destiné au traitement et/ou à la prophylaxie de maladies.

8. Utilisation d'un composé selon l'une des revendications 1 à 4 pour la préparation d'un médicament destiné au traitement et/ou à la prophylaxie de maladies thrombo-emboliques.

9. Médicament contenant un composé selon l'une des revendications 1 à 4, en combinaison avec un adjuvant inerte, non toxique, pharmaceutiquement approprié.

10. Médicament contenant un composé selon l'une des revendications 1 à 4 en combinaison avec un autre principe actif.

11. Médicament selon la revendication 10 ou 11, pour utilisation dans le traitement et/ou la prophylaxie de maladies thrombo-emboliques.

12. Procédé pour empêcher la coagulation du sang in vitro, **caractérisé en ce qu'**on administre une quantité à effet anticoagulant d'un composé selon l'une des revendications 1 à 4.

13. Utilisation d'un composé selon l'une des revendications 1 à 4 pour la préparation d'un médicament destiné au traitement et/ou à la prophylaxie de l'hypertension pulmonaire.

14. Utilisation d'un composé selon l'une des revendications 1 à 4 pour préparer un médicament destiné au traitement et/ou à la prophylaxie de la sepsie, du syndrome inflammatoire systémique (SIRS), du dysfonctionnement d'organe d'origine septique, de la défaillance d'organe d'origine septique et de la défaillance d'organes multiples, du syndrome de détresse respiratoire aigu (SDRA), de la lésion pulmonaire aiguë (ALI), du choc septique, de la DIC (coagulation intravasculaire disséminée) et/ou de la défaillance d'organe d'origine septique.

15. Composé tel que défini dans l'une des revendications 1 à 4, pour utilisation dans un procédé destiné au traitement et/ou à la prophylaxie de maladies thrombo-emboliques.
